# EUROPEAN PATENT APPLICATION

(11) **EP 4 335 846 A1**
(43) Date of publication of application: **13.03.2024**
(21) Application number: 23182378.2
(22) Date of filing: 29.06.2023
(51) Int. Cl.: C07D 403/10, H10K 85/00

(54) **ORGANIC ELECTROLUMINESCENT MATERIAL AND DEVICE THEREOF**

(30) Priority: 30.06.2022 CN 202210754672; 28.04.2023 CN 202310481744
(71) Applicant: Beijing Summer Sprout Technology Co., Ltd., Beijing 102308 (CN)
(72) Inventor: Li, Feng, Beijing, 102308 (CN); Wang, Yang, Beijing, 102308 (CN); Yao, Jianfei, Beijing, 102308 (CN); Zhang, Ziyan, Beijing, 102308 (CN); Wang, Qiang, Beijing, 102308 (CN); Wang, Le, Beijing, 102308 (CN); Kwong, Chi Yuen Raymond, Beijing, 102308 (CN); Xia, Chuanjun, Beijing, 102308 (CN)
(74) Representative: Grünecker Patent- und Rechtsanwälte PartG mbB

(57) **Abstract**

Provided are an organic electroluminescent material and a device thereof. The organic electroluminescent material is a compound having a structure of Formula 1. The compound may be used as a host material, an electron transporting material, or a hole blocking material in an organic electroluminescent device and can provide better device performance such as a lower voltage and higher efficiency and can especially improve a device lifetime greatly. Further provided is a compound composition comprising the structure of Formula 1.

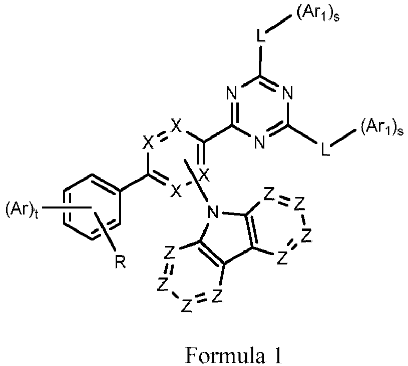

## Description

### TECHNICAL FIELD

The present disclosure relates to compounds for organic electronic devices such as organic light-emitting devices. In particular, the present disclosure relates to a compound having a structure of Formula 1 and an organic electroluminescent device comprising the compound.

### BACKGROUND

Organic electronic devices include, but are not limited to, the following types: organic light-emitting diodes (OLEDs), organic field-effect transistors (O-FETs), organic light-emitting transistors (OLETs), organic photovoltaic devices (OPVs), dye-sensitized solar cells (DSSCs), organic optical detectors, organic photoreceptors, organic field-quench devices (OFQDs), light-emitting electrochemical cells (LECs), organic laser diodes and organic plasmon emitting devices.

In 1987, Tang and Van Slyke of Eastman Kodak reported a bilayer organic electroluminescent device, which comprises an arylamine hole transporting layer and a tris-8-hydroxyquinolato-aluminum layer as the electron and emitting layer (Applied Physics Letters, 1987, 51 (12): 913-915). Once a bias is applied to the device, green light was emitted from the device. This device laid the foundation for the development of modern organic light-emitting diodes (OLEDs). State-of-the-art OLEDs may comprise multiple layers such as charge injection and transporting layers, charge and exciton blocking layers, and one or multiple emissive layers between the cathode and anode. Since the OLED is a self-emitting solid state device, it offers tremendous potential for display and lighting applications. In addition, the inherent properties of organic materials, such as their flexibility, may make them well suited for particular applications such as fabrication on flexible substrates.

The OLED can be categorized as three different types according to its emitting mechanism. The OLED invented by Tang and van Slyke is a fluorescent OLED. It only utilizes singlet emission. The triplets generated in the device are wasted through nonradiative decay channels. Therefore, the internal quantum efficiency (IQE) of the fluorescent OLED is only 25%. This limitation hindered the commercialization of OLED. In 1997, Forrest and Thompson reported phosphorescent OLED, which uses triplet emission from heavy metal containing complexes as the emitter. As a result, both singlet and triplets can be harvested, achieving 100% IQE. The discovery and development of phosphorescent OLED contributed directly to the commercialization of active-matrix OLED (AMOLED) due to its high efficiency. Recently, Adachi achieved high efficiency through thermally activated delayed fluorescence (TADF) of organic compounds. These emitters have small singlet-triplet gap that makes the transition from triplet back to singlet possible. In the TADF device, the triplet excitons can go through reverse intersystem crossing to generate singlet excitons, resulting in high IQE.

OLEDs can also be classified as small molecule and polymer OLEDs according to the forms of the materials used. A small molecule refers to any organic or organometallic material that is not a polymer. The molecular weight of the small molecule can be large as long as it has well defined structure. Dendrimers with well-defined structures are considered as small molecules. Polymer OLEDs include conjugated polymers and non-conjugated polymers with pendant emitting groups. Small molecule OLED can become the polymer OLED if post polymerization occurred during the fabrication process.

There are various methods for OLED fabrication. Small molecule OLEDs are generally fabricated by vacuum thermal evaporation. Polymer OLEDs are fabricated by solution process such as spin-coating, inkjet printing, and slit printing. If the material can be dissolved or dispersed in a solvent, the small molecule OLED can also be produced by solution process.

The emitting color of the OLED can be achieved by emitter structural design. An OLED may comprise one emitting layer or a plurality of emitting layers to achieve desired spectrum. In the case of green, yellow, and red OLEDs, phosphorescent emitters have successfully reached commercialization. Blue phosphorescent device still suffers from non-saturated blue color, short device lifetime, and high operating voltage. Commercial full-color OLED displays normally adopt a hybrid strategy, using fluorescent blue and phosphorescent yellow, or red and green. At present, efficiency roll-off of phosphorescent OLEDs at high brightness remains a problem. In addition, it is desirable to have more saturated emitting color, higher efficiency, and longer device lifetime.

WO2020262861A1 discloses an organic compound having the following formula and an organic light-emitting device comprising the compound: 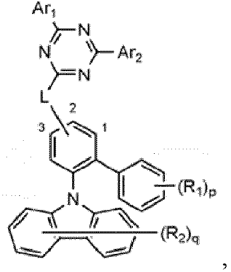 wherein R₂ is each independently selected from substituted or unsubstituted C₆₋₆₀ aryl, benzoxazolyl, benzothiazolyl, dibenzofuryl, or phenyl-substituted benzothiazolyl, and q is an integer from 1 to 8. This application has disclosed the following compounds among specific structures: 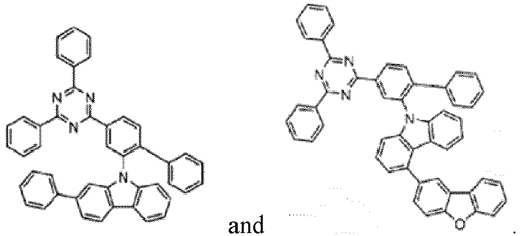 The compound disclosed in this application must have at least one preceding aryl or heteroaryl substituent on carbazole, and this application has neither disclosed nor taught a compound with no aryl or heteroaryl substituent on carbazole and a use of the compound in an organic electroluminescent device.

WO2021040467A1 discloses an organic compound having the following structure and an organic light-emitting device comprising the compound: 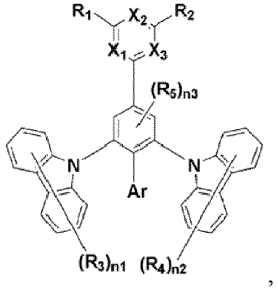 wherein Ar is substituted or unsubstituted C₆₋₆₀ aryl. This application has disclosed the following compounds among specific structures: 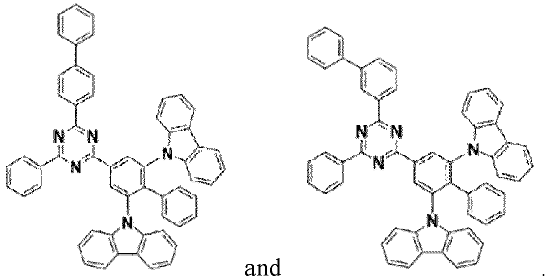 The compound disclosed in this application must have at least two carbazolyl groups on phenylene joined to triazine, and this application has neither disclosed nor taught a compound with only one carbazolyl group on phenylene joined to triazine and a use of the compound in an organic electroluminescent device.

WO2017025164A1 discloses an organic compound having the following structure and an organic light-emitting device comprising the compound: 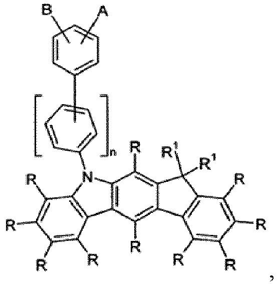 wherein A may be 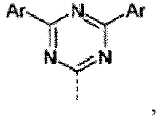 B is an aromatic ring having 6 to 30 aromatic ring atoms or a heteroaromatic ring having 13 to 30 aromatic ring atoms and rich in electrons, and n is 0 or 1. This application has disclosed the following compounds among specific structures: 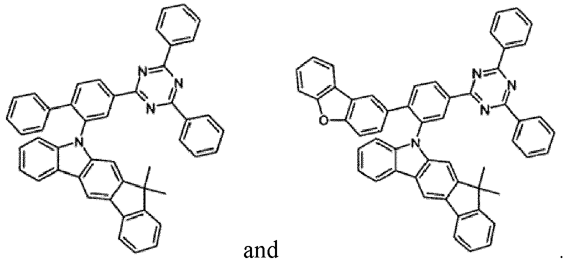 This application has disclosed only the compound having a fluorenoindole structure and has neither disclosed nor taught a compound having a carbazole structure and a use of the compound in an organic electroluminescent device.

KR101926771B1 discloses an organic compound having the following structure and an organic light-emitting device comprising the compound: 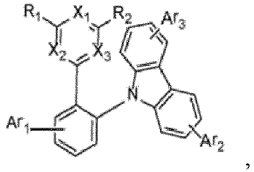 wherein Ar₁ to Ar₃ may be unsubstituted or each independently selected from substituted or unsubstituted C6 to C60 aryl or substituted or unsubstituted C2 to C40 heteroaryl. Further, Ar₁ to Ar₃ may be unsubstituted or each independently selected from any one of 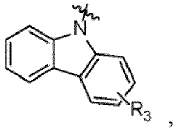 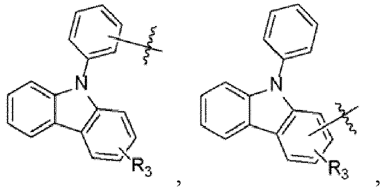 and other structures. This application has disclosed the following compounds among specific structures: 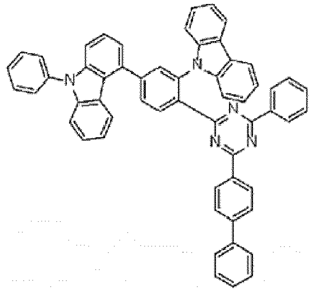 and 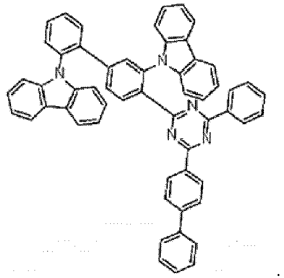 Each Ar₁ in the compound disclosed in this application is unsubstituted or heteroaryl or heteroaryl-substituted aryl, and this application has neither disclosed nor taught a compound where Ar₁ is another substituent and a use of the compound in an organic electroluminescent device.

US20220029109A1 discloses a composition of host materials, wherein one host material has a structure of Formula 1: 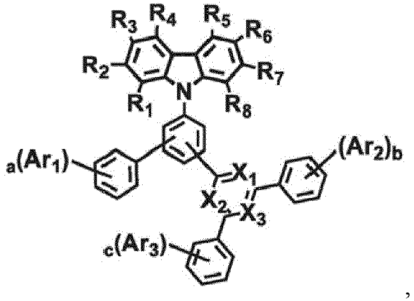 wherein R₁ to R₈ are each independently hydrogen, deuterium, halogen, cyano, substituted or unsubstituted C6 to C30 aryl, or a substituent having a structure represented by Formula 1-1 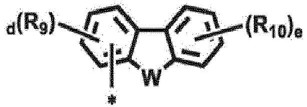 (wherein W is O or S), or two adjacent substituents of R₁ to R₈ can be fused to form a ring having a structure represented by Formula 1-2 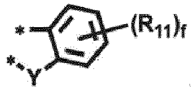 (wherein Y is O or S), the compound of Formula 1 comprises at least one structure represented by Formula 1-1 or Formula 1-2, and when R₂ or R₇ is Formula 1-1, carbazolyl is not joined at position 1 or 2 of carbon in Formula 1-1. This application has disclosed the following compounds among specific structures: 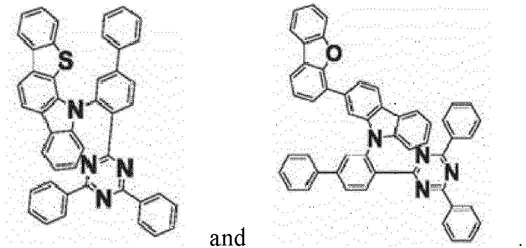 Carbazole in the compound disclosed in this application each has a heteroaryl substituent or is further fused with the structure of Formula 1-2, and this application has neither disclosed nor taught a compound with no heteroaryl substituent on carbazole or with non-fused carbazole and a use of the compound in an organic electroluminescent device.

WO2020149656A1 discloses an organic compound having the following structure and an organic light-emitting device comprising the compound: 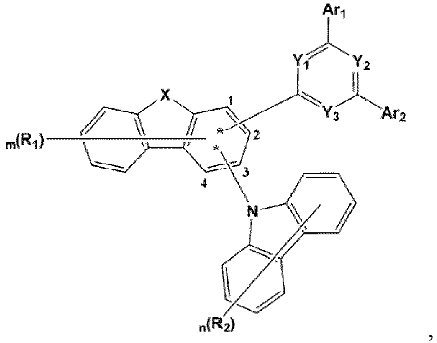 wherein X is O or S, Y₁ to Y₃ are each independently CH or N, two or more of Y₁ to Y₃ are N, Ar₁ and Ar₂ are each independently substituted or unsubstituted C₆₋₆₀ aryl or substituted or unsubstituted C₅₋₆₀ heteroaryl comprising one or more heteroatoms selected from N, O, and S, R₂ is substituted or unsubstituted C₆₋₆₀ aryl, and n is an integer from 1 to 8. This application has disclosed the following compounds among specific structures: 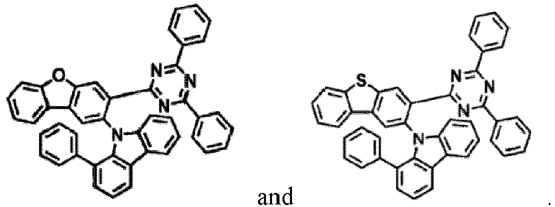 In the compound disclosed in this application, triazine is joined to either dibenzofuryl or dibenzothienyl, and carbazole must have an aryl substituent. This application has neither disclosed nor taught a compound with another structure and a use of the compound in an organic electroluminescent device.

However, currently reported triazine-based organic semiconductor materials have certain limitations in terms of carrier transporting ability and lifetime in optoelectronic devices. Therefore, the application potential of such materials is worthy of further research and development.

### SUMMARY

The present disclosure aims to provide a series of compounds each having a structure of Formula 1 to solve at least part of the preceding problems. The compounds may be applied to organic electroluminescent devices and can provide better device performance such as a lower voltage and higher efficiency and can especially improve a device lifetime greatly.

According to an embodiment of the present disclosure, disclosed is a compound having a structure of Formula 1: 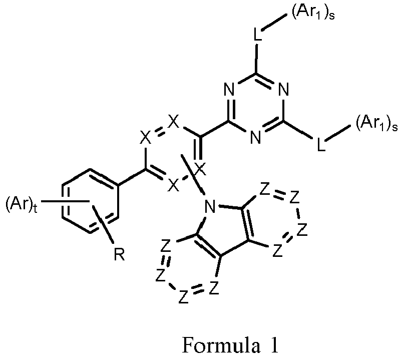 wherein
X is, at each occurrence identically or differently, selected from C, CRₓ, or N; Z is, at each occurrence identically or differently, selected from CR_{z} or N;
Ar is, at each occurrence identically or differently, selected from Formula 2, Formula 3, Formula 4, or a combination thereof, wherein Formula 2, Formula 3, and Formula 4 have the following structures, respectively: 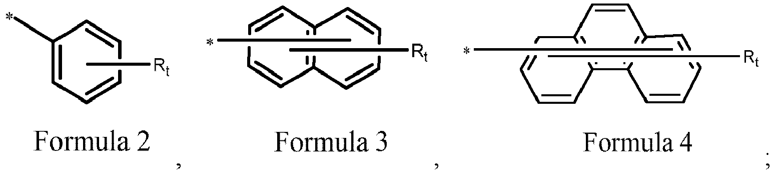 wherein
Rₜ in Formula 2, Formula 3, and Formula 4 represents, at each occurrence identically or differently, mono-substitution, multiple substitutions, or non-substitution;
"*" in the structures of Formula 2, Formula 3, and Formula 4 represents a position where the benzene ring with the substituent R in Formula 1, Formula 2, Formula 3, or Formula 4 is joined;
L is, at each occurrence identically or differently, selected from a single bond, Formula 5, Formula 6, Formula 7, or a combination thereof, wherein Formula 5, Formula 6, and Formula 7 have the following structures, respectively: 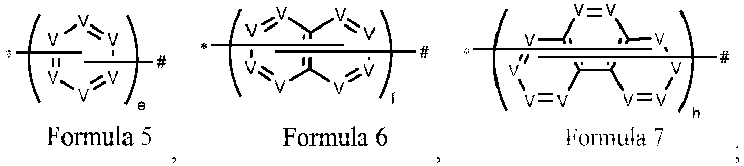
wherein V in Formula 5, Formula 6, and Formula 7 is, at each occurrence identically or differently, selected from C, CRᵥ, or N;
e, f, and h are, at each occurrence identically or differently, selected from 1, 2, or 3;
"*" in Formula 5, Formula 6, and Formula 7 represents a position where triazine shown in Formula 1, Formula 5, Formula 6, or Formula 7 is joined; and "#" represents a position where Ar₁ in Formula 1, Formula 5, Formula 6, or Formula 7 is joined;
Ar₁ is, at each occurrence identically or differently, selected from Formula 8, Formula 9, Formula 10, or a combination thereof, wherein Formula 8, Formula 9, and Formula 10 have the following structures, respectively: 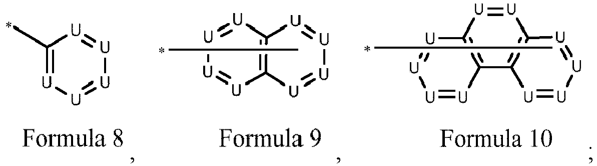
wherein U in Formula 8, Formula 9, and Formula 10 is, at each occurrence identically or differently, selected from C, CRᵤ, or N;
"*" in Formula 8, Formula 9, and Formula 10 represents a position where L in Formula 1, Formula 8, Formula 9, or Formula 10 is joined;
Rₓ, Rᵤ, Rᵥ, and Rₜ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R_{z} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R represents, at each occurrence identically or differently, mono-substitution, multiple substitutions, or non-substitution;
R is, at each occurrence identically or differently, selected from the group consisting of: hydrogen, deuterium, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted amino having 0 to 20 carbon atoms, an acyl group, a carbonyl group, a carboxylic acid group, an ester group, a hydroxyl group, a sulfanyl group, a sulfinyl group, a sulfonyl group, a phosphino group, and combinations thereof;
t is selected from 0, 1, 2, 3, 4, or 5; s is, at each occurrence identically or differently, selected from 1 to a maximum number of available substitutions for L; and
in Formula 1, only adjacent substituents R, adjacent substituents Rₓ, and adjacent substituents R_{z} can be optionally joined to form a 6-membered ring.

According to another embodiment of the present disclosure, further disclosed is an electroluminescent device comprising an anode, a cathode, and an organic layer disposed between the anode and the cathode, wherein the organic layer comprises the compound of Formula 1 in the preceding embodiment.

According to another embodiment of the present disclosure, further disclosed is a compound composition comprising the compound of Formula 1 in the preceding embodiment.

The present disclosure discloses a series of compounds each having a structure of Formula 1. The compounds may be used as host materials, electron transporting materials, or hole blocking materials in organic electroluminescent devices and can provide better device performance such as a lower voltage and higher efficiency and can especially improve a device lifetime greatly.

### BRIEF DESCRIPTION OF DRAWINGS

FIG. 1 is a schematic diagram of an organic light-emitting device that may contain a compound and a compound combination disclosed herein.
FIG. 2 is a schematic diagram of another organic light-emitting device that may contain a compound and a compound combination disclosed herein.

### DETAILED DESCRIPTION

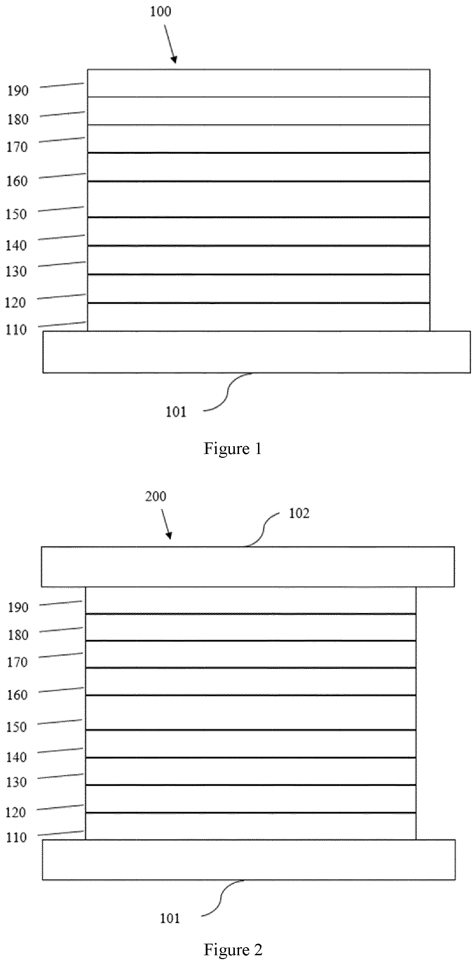

OLEDs can be fabricated on various types of substrates such as glass, plastic, and metal foil. FIG. 1 schematically shows an organic light-emitting device 100 without limitation. The figures are not necessarily drawn to scale. Some of the layers in the figures can also be omitted as needed. Device 100 may include a substrate 101, an anode 110, a hole injection layer 120, a hole transport layer 130, an electron blocking layer 140, an emissive layer 150, a hole blocking layer 160, an electron transport layer 170, an electron injection layer 180 and a cathode 190. Device 100 may be fabricated by depositing the layers described in order. The properties and functions of these various layers, as well as example materials, are described in more detail in U.S. Pat. No. 7,279,704 at cols. 6-10, the contents of which are incorporated by reference herein in its entirety.

More examples for each of these layers are available. For example, a flexible and transparent substrate-anode combination is disclosed in U.S. Pat. No. 5,844,363, which is incorporated by reference herein in its entirety. An example of a p-doped hole transport layer is m-MTDATA doped with F4-TCNQ at a molar ratio of 50:1, as disclosed in U.S. Patent Application Publication No. 2003/0230980, which is incorporated by reference herein in its entirety. Examples of host materials are disclosed in U.S. Pat. No. 6,303,238 to Thompson et al., which is incorporated by reference herein in its entirety. An example of an n-doped electron transport layer is BPhen doped with Li at a molar ratio of 1:1, as disclosed in U.S. Patent Application Publication No. 2003/0230980, which is incorporated by reference herein in its entirety. U.S. Pat. Nos. 5,703,436 and 5,707,745, which are incorporated by reference herein in their entireties, disclose examples of cathodes including composite cathodes having a thin layer of metal such as Mg:Ag with an overlying transparent, electrically-conductive, sputter-deposited ITO layer. The theory and use of blocking layers are described in more detail in U.S. Pat. No. 6,097,147 and U.S. Patent Application Publication No. 2003/0230980, which are incorporated by reference herein in their entireties. Examples of injection layers are provided in U.S. Patent Application Publication No. 2004/0174116, which is incorporated by reference herein in its entirety. A description of protective layers may be found in U.S. Patent Application Publication No. 2004/0174116, which is incorporated by reference herein in its entirety.

The layered structure described above is provided by way of non-limiting examples. Functional OLEDs may be achieved by combining the various layers described in different ways, or layers may be omitted entirely. It may also include other layers not specifically described. Within each layer, a single material or a mixture of multiple materials can be used to achieve optimum performance. Any functional layer may include several sublayers. For example, the emissive layer may have two layers of different emitting materials to achieve desired emission spectrum.

In one embodiment, an OLED may be described as having an "organic layer" disposed between a cathode and an anode. This organic layer may include a single layer or multiple layers.

An OLED can be encapsulated by a barrier layer. FIG. 2 schematically shows an organic light emitting device 200 without limitation. FIG. 2 differs from FIG. 1 in that the organic light emitting device include a barrier layer 102, which is above the cathode 190, to protect it from harmful species from the environment such as moisture and oxygen. Any material that can provide the barrier function can be used as the barrier layer such as glass or organic-inorganic hybrid layers. The barrier layer should be placed directly or indirectly outside of the OLED device. Multilayer thin film encapsulation was described in U.S. Pat. No. 7,968,146, which is incorporated by reference herein in its entirety.

Devices fabricated in accordance with embodiments of the present disclosure can be incorporated into a wide variety of consumer products that have one or more of the electronic component modules (or units) incorporated therein. Some examples of such consumer products include flat panel displays, monitors, medical monitors, televisions, billboards, lights for interior or exterior illumination and/or signaling, heads-up displays, fully or partially transparent displays, flexible displays, smart phones, tablets, phablets, wearable devices, smart watches, laptop computers, digital cameras, camcorders, viewfinders, micro-displays, 3-D displays, vehicles displays, and vehicle tail lights.

The materials and structures described herein may be used in other organic electronic devices listed above.

As used herein, "top" means furthest away from the substrate, while "bottom" means closest to the substrate. Where a first layer is described as "disposed over" a second layer, the first layer is disposed further away from the substrate. There may be other layers between the first and second layers, unless it is specified that the first layer is "in contact with" the second layer. For example, a cathode may be described as "disposed over" an anode, even though there are various organic layers in between.

As used herein, "solution processible" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

It is believed that the internal quantum efficiency (IQE) of fluorescent OLEDs can exceed the 25% spin statistics limit through delayed fluorescence. As used herein, there are two types of delayed fluorescence, i.e. P-type delayed fluorescence and E-type delayed fluorescence. P-type delayed fluorescence is generated from triplet-triplet annihilation (TTA).

On the other hand, E-type delayed fluorescence does not rely on the collision of two triplets, but rather on the transition between the triplet states and the singlet excited states. Compounds that are capable of generating E-type delayed fluorescence are required to have very small singlet-triplet gaps to convert between energy states. Thermal energy can activate the transition from the triplet state back to the singlet state. This type of delayed fluorescence is also known as thermally activated delayed fluorescence (TADF). A distinctive feature of TADF is that the delayed component increases as temperature rises. If the reverse intersystem crossing (RISC) rate is fast enough to minimize the non-radiative decay from the triplet state, the fraction of back populated singlet excited states can potentially reach 75%. The total singlet fraction can be 100%, far exceeding 25% of the spin statistics limit for electrically generated excitons.

E-type delayed fluorescence characteristics can be found in an exciplex system or in a single compound. Without being bound by theory, it is believed that E-type delayed fluorescence requires the luminescent material to have a small singlet-triplet energy gap (ΔE_{S-T}). Organic, non-metal containing, donor-acceptor luminescent materials may be able to achieve this. The emission in these materials is generally characterized as a donor-acceptor charge-transfer (CT) type emission. The spatial separation of the HOMO and LUMO in these donor-acceptor type compounds generally results in small ΔE_{S-T}. These states may involve CT states. Generally, donor-acceptor luminescent materials are constructed by connecting an electron donor moiety such as amino- or carbazole-derivatives and an electron acceptor moiety such as N-containing six-membered aromatic rings.

### Definition of terms of substituents

Halogen or halide - as used herein includes fluorine, chlorine, bromine, and iodine.

Alkyl - as used herein includes both straight and branched chain alkyl groups. Alkyl may be alkyl having 1 to 20 carbon atoms, preferably alkyl having 1 to 12 carbon atoms, and more preferably alkyl having 1 to 6 carbon atoms. Examples of alkyl groups include a methyl group, an ethyl group, a propyl group, an isopropyl group, a n-butyl group, an s-butyl group, an isobutyl group, a t-butyl group, an n-pentyl group, an n-hexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a neopentyl group, a 1-methylpentyl group, a 2-methylpentyl group, a 1-pentylhexyl group, a 1-butylpentyl group, a 1-heptyloctyl group, and a 3-methylpentyl group. Of the above, preferred are a methyl group, an ethyl group, a propyl group, an isopropyl group, a n-butyl group, an s-butyl group, an isobutyl group, a t-butyl group, an n-pentyl group, a neopentyl group, and an n-hexyl group. Additionally, the alkyl group may be optionally substituted.

Cycloalkyl - as used herein includes cyclic alkyl groups. The cycloalkyl groups may be those having 3 to 20 ring carbon atoms, preferably those having 4 to 10 carbon atoms. Examples of cycloalkyl include cyclobutyl, cyclopentyl, cyclohexyl, 4-methylcyclohexyl, 4,4-dimethylcylcohexyl, 1-adamantyl, 2-adamantyl, 1-norbornyl, 2-norbornyl, and the like. Of the above, preferred are cyclopentyl, cyclohexyl, 4-methylcyclohexyl, and 4,4-dimethylcylcohexyl. Additionally, the cycloalkyl group may be optionally substituted.

Heteroalkyl - as used herein, includes a group formed by replacing one or more carbons in an alkyl chain with a hetero-atom(s) selected from the group consisting of a nitrogen atom, an oxygen atom, a sulfur atom, a selenium atom, a phosphorus atom, a silicon atom, a germanium atom, and a boron atom. Heteroalkyl may be those having 1 to 20 carbon atoms, preferably those having 1 to 10 carbon atoms, and more preferably those having 1 to 6 carbon atoms. Examples of heteroalkyl include methoxymethyl, ethoxymethyl, ethoxyethyl, methylthiomethyl, ethylthiomethyl, ethylthioethyl, methoxymethoxymethyl, ethoxymethoxymethyl, ethoxyethoxyethyl, hydroxymethyl, hydroxyethyl, hydroxypropyl, mercaptomethyl, mercaptoethyl, mercaptopropyl, aminomethyl, aminoethyl, aminopropyl, dimethylaminomethyl, trimethylgermanylmethyl, trimethylgermanylethyl, trimethylgermanylisopropyl, dimethylethylgermanylmethyl, dimethylisopropylgermanylmethyl, tert-butyldimethylgermanylmethyl, triethylgermanylmethyl, triethylgermanylethyl, triisopropylgermanylmethyl, triisopropylgermanylethyl, trimethylsilylmethyl, trimethylsilylethyl, trimethylsilylisopropyl, triisopropylsilylmethyl, and triisopropylsilylethyl. Additionally, the heteroalkyl group may be optionally substituted.

Alkenyl - as used herein includes straight chain, branched chain, and cyclic alkene groups. Alkenyl may be those having 2 to 20 carbon atoms, preferably those having 2 to 10 carbon atoms. Examples of alkenyl include vinyl, 1-propenyl group, 1-butenyl, 2-butenyl, 3-butenyl, 1,3-butandienyl, 1-methylvinyl, styryl, 2,2-diphenylvinyl, 1,2-diphenylvinyl, 1-methylallyl, 1,1-dimethylallyl, 2-methylallyl, 1-phenylallyl, 2-phenylallyl, 3-phenylallyl, 3,3-diphenylallyl, 1,2-dimethylallyl, 1-phenyl-1-butenyl, 3-phenyl-1-butenyl, cyclopentenyl, cyclopentadienyl, cyclohexenyl, cycloheptenyl, cycloheptatrienyl, cyclooctenyl, cyclooctatetraenyl, and norbornenyl. Additionally, the alkenyl group may be optionally substituted.

Alkynyl - as used herein includes straight chain alkynyl groups. Alkynyl may be those having 2 to 20 carbon atoms, preferably those having 2 to 10 carbon atoms. Examples of alkynyl groups include ethynyl, propynyl, propargyl, 1-butynyl, 2-butynyl, 3-butynyl, 1-pentynyl, 2-pentynyl, 3,3-dimethyl-1-butynyl, 3-ethyl-3-methyl-1-pentynyl, 3,3-diisopropyl-1-pentynyl, phenylethynyl, phenylpropynyl, etc. Of the above, preferred are ethynyl, propynyl, propargyl, 1-butynyl, 2-butynyl, 3-butynyl, 1-pentynyl, and phenylethynyl. Additionally, the alkynyl group may be optionally substituted.

Aryl or an aromatic group - as used herein includes non-condensed and condensed systems. Aryl may be those having 6 to 30 carbon atoms, preferably those having 6 to 20 carbon atoms, and more preferably those having 6 to 12 carbon atoms. Examples of aryl groups include phenyl, biphenyl, terphenyl, triphenylene, tetraphenylene, naphthalene, anthracene, phenalene, phenanthrene, fluorene, pyrene, chrysene, perylene, and azulene, preferably phenyl, biphenyl, terphenyl, triphenylene, fluorene, and naphthalene. Examples of non-condensed aryl groups include phenyl, biphenyl-2-yl, biphenyl-3-yl, biphenyl-4-yl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-(2-phenylpropyl)phenyl, 4'-methylbiphenylyl, 4"-t-butyl-p-terphenyl-4-yl, o-cumenyl, m-cumenyl, p-cumenyl, 2,3-xylyl, 3,4-xylyl, 2,5-xylyl, mesityl, and m-quarterphenyl. Additionally, the aryl group may be optionally substituted.

Heterocyclic groups or heterocycle - as used herein include non-aromatic cyclic groups. Non-aromatic heterocyclic groups include saturated heterocyclic groups having 3 to 20 ring atoms and unsaturated non-aromatic heterocyclic groups having 3 to 20 ring atoms, where at least one ring atom is selected from the group consisting of a nitrogen atom, an oxygen atom, a sulfur atom, a selenium atom, a silicon atom, a phosphorus atom, a germanium atom, and a boron atom. Preferred non-aromatic heterocyclic groups are those having 3 to 7 ring atoms, each of which includes at least one hetero-atom such as nitrogen, oxygen, silicon, or sulfur. Examples of non-aromatic heterocyclic groups include oxiranyl, oxetanyl, tetrahydrofuranyl, tetrahydropyranyl, dioxolanyl, dioxanyl, aziridinyl, dihydropyrrolyl, tetrahydropyrrolyl, piperidinyl, oxazolidinyl, morpholinyl, piperazinyl, oxepinyl, thiepinyl, azepinyl, and tetrahydrosilolyl. Additionally, the heterocyclic group may be optionally substituted.

Heteroaryl - as used herein, includes non-condensed and condensed hetero-aromatic groups having 1 to 5 hetero-atoms, where at least one hetero-atom is selected from the group consisting of a nitrogen atom, an oxygen atom, a sulfur atom, a selenium atom, a silicon atom, a phosphorus atom, a germanium atom, and a boron atom. A hetero-aromatic group is also referred to as heteroaryl. Heteroaryl may be those having 3 to 30 carbon atoms, preferably those having 3 to 20 carbon atoms, and more preferably those having 3 to 12 carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridoindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group may be optionally substituted.

Alkoxy - as used herein, is represented by -O-alkyl, -O-cycloalkyl, -O-heteroalkyl, or -O-heterocyclic group. Examples and preferred examples of alkyl, cycloalkyl, heteroalkyl, and heterocyclic groups are the same as those described above. Alkoxy groups may be those having 1 to 20 carbon atoms, preferably those having 1 to 6 carbon atoms. Examples of alkoxy groups include methoxy, ethoxy, propoxy, butoxy, pentyloxy, hexyloxy, cyclopropyloxy, cyclobutyloxy, cyclopentyloxy, cyclohexyloxy, tetrahydrofuranyloxy, tetrahydropyranyloxy, methoxypropyloxy, ethoxyethyloxy, methoxymethyloxy, and ethoxymethyloxy. Additionally, the alkoxy group may be optionally substituted.

Aryloxy - as used herein, is represented by -O-aryl or -O-heteroaryl. Examples and preferred examples of aryl and heteroaryl are the same as those described above. Aryloxy groups may be those having 6 to 30 carbon atoms, preferably those having 6 to 20 carbon atoms. Examples of aryloxy groups include phenoxy and biphenyloxy. Additionally, the aryloxy group may be optionally substituted.

Arylalkyl - as used herein, contemplates alkyl substituted with an aryl group. Arylalkyl may be those having 7 to 30 carbon atoms, preferably those having 7 to 20 carbon atoms, and more preferably those having 7 to 13 carbon atoms. Examples of arylalkyl groups include benzyl, 1-phenylethyl, 2-phenylethyl, 1-phenylisopropyl, 2-phenylisopropyl, phenyl-t-butyl, alpha-naphthylmethyl, 1-alpha-naphthylethyl, 2-alpha-naphthylethyl, 1-alpha-naphthylisopropyl, 2-alpha-naphthylisopropyl, beta-naphthylmethyl, 1-beta-naphthylethyl, 2-beta-naphthylethyl, 1-beta-naphthylisopropyl, 2-beta-naphthylisopropyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl, and 1-chloro-2-phenylisopropyl. Of the above, preferred are benzyl, p-cyanobenzyl, m-cyanobenzyl, o-cyanobenzyl, 1-phenylethyl, 2-phenylethyl, 1-phenylisopropyl, and 2-phenylisopropyl. Additionally, the arylalkyl group may be optionally substituted.

Alkylsilyl - as used herein, contemplates a silyl group substituted with an alkyl group. Alkylsilyl groups may be those having 3 to 20 carbon atoms, preferably those having 3 to 10 carbon atoms. Examples of alkylsilyl groups include trimethylsilyl, triethylsilyl, methyldiethylsilyl, ethyldimethylsilyl, tripropylsilyl, tributylsilyl, triisopropylsilyl, methyldiisopropylsilyl, dimethylisopropylsilyl, tri-t-butylsilyl, triisobutylsilyl, dimethyl t-butylsilyl, and methyldi-t-butylsilyl. Additionally, the alkylsilyl group may be optionally substituted.

Arylsilyl - as used herein, contemplates a silyl group substituted with an aryl group. Arylsilyl groups may be those having 6 to 30 carbon atoms, preferably those having 8 to 20 carbon atoms. Examples of arylsilyl groups include triphenylsilyl, phenyldibiphenylylsilyl, diphenylbiphenylsilyl, phenyldiethylsilyl, diphenylethylsilyl, phenyldimethylsilyl, diphenylmethylsilyl, phenyldiisopropylsilyl, diphenylisopropylsilyl, diphenylbutylsilyl, diphenylisobutylsilyl, diphenyl t-butylsilyl. Additionally, the arylsilyl group may be optionally substituted.

Alkylgermanyl - as used herein contemplates a germanyl substituted with an alkyl group. The alkylgermanyl may be those having 3 to 20 carbon atoms, preferably those having 3 to 10 carbon atoms. Examples of alkylgermanyl include trimethylgermanyl, triethylgermanyl, methyldiethylgermanyl, ethyldimethylgermanyl, tripropylgermanyl, tributylgermanyl, triisopropylgermanyl, methyldiisopropylgermanyl, dimethylisopropylgermanyl, tri-t-butylgermanyl, triisobutylgermanyl, dimethyl-t-butylgermanyl, and methyldi-t-butylgermanyl. Additionally, the alkylgermanyl may be optionally substituted.

Arylgermanyl - as used herein contemplates a germanyl substituted with at least one aryl group or heteroaryl group. Arylgermanyl may be those having 6 to 30 carbon atoms, preferably those having 8 to 20 carbon atoms. Examples of arylgermanyl include triphenylgermanyl, phenyldibiphenylylgermanyl, diphenylbiphenylgermanyl, phenyldiethylgermanyl, diphenylethylgermanyl, phenyldimethylgermanyl, diphenylmethylgermanyl, phenyldiisopropylgermanyl, diphenylisopropylgermanyl, diphenylbutylgermanyl, diphenylisobutylgermanyl, and diphenyl-t-butylgermanyl. Additionally, the arylgermanyl may be optionally substituted.

The term "aza" in azadibenzofuran, azadibenzothiophene, etc. means that one or more of C-H groups in the respective aromatic fragment are replaced by a nitrogen atom. For example, azatriphenylene encompasses dibenzo[f,h]quinoxaline, dibenzo[f,h]quinoline and other analogs with two or more nitrogens in the ring system. One of ordinary skill in the art can readily envision other nitrogen analogs of the aza-derivatives described above, and all such analogs are intended to be encompassed by the terms as set forth herein.

In the present disclosure, unless otherwise defined, when any term of the group consisting of substituted alkyl, substituted cycloalkyl, substituted heteroalkyl, substituted heterocyclic group, substituted arylalkyl, substituted alkoxy, substituted aryloxy, substituted alkenyl, substituted alkynyl, substituted aryl, substituted heteroaryl, substituted alkylsilyl, substituted arylsilyl, substituted alkylgermanyl, substituted arylgermanyl, substituted amino, substituted acyl, substituted carbonyl, a substituted carboxylic acid group, a substituted ester group, substituted sulfinyl, substituted sulfonyl, and substituted phosphino is used, it means that any group of alkyl, cycloalkyl, heteroalkyl, heterocyclic group, arylalkyl, alkoxy, aryloxy, alkenyl, alkynyl, aryl, heteroaryl, alkylsilyl, arylsilyl, alkylgermanyl, arylgermanyl, amino, acyl, carbonyl, a carboxylic acid group, an ester group, sulfinyl, sulfonyl, and phosphino may be substituted with one or more groups selected from the group consisting of deuterium, halogen, unsubstituted alkyl having 1 to 20 carbon atoms, unsubstituted cycloalkyl having 3 to 20 ring carbon atoms, unsubstituted heteroalkyl having 1 to 20 carbon atoms, an unsubstituted heterocyclic group having 3 to 20 ring atoms, unsubstituted arylalkyl having 7 to 30 carbon atoms, unsubstituted alkoxy having 1 to 20 carbon atoms, unsubstituted aryloxy having 6 to 30 carbon atoms, unsubstituted alkenyl having 2 to 20 carbon atoms, unsubstituted alkynyl having 2 to 20 carbon atoms, unsubstituted aryl having 6 to 30 carbon atoms, unsubstituted heteroaryl having 3 to 30 carbon atoms, unsubstituted alkylsilyl having 3 to 20 carbon atoms, unsubstituted arylsilyl group having 6 to 20 carbon atoms, unsubstituted alkylgermanyl group having 3 to 20 carbon atoms, unsubstituted arylgermanyl group having 6 to 20 carbon atoms, unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or an attached fragment are considered to be equivalent.

In the compounds mentioned in the present disclosure, hydrogen atoms may be partially or fully replaced by deuterium. Other atoms such as carbon and nitrogen may also be replaced by their other stable isotopes. The replacement by other stable isotopes in the compounds may be preferred due to its enhancements of device efficiency and stability.

In the compounds mentioned in the present disclosure, multiple substitutions refer to a range that includes di-substitutions, up to the maximum available substitutions. When substitution in the compounds mentioned in the present disclosure represents multiple substitutions (including di-, tri-, and tetra-substitutions etc.), that means the substituent may exist at a plurality of available substitution positions on its linking structure, the substituents present at a plurality of available substitution positions may have the same structure or different structures.

In the compounds mentioned in the present disclosure, adjacent substituents in the compounds cannot be joined to form a ring unless otherwise explicitly defined, for example, adjacent substituents can be optionally joined to form a ring. In the compounds mentioned in the present disclosure, the expression that adjacent substituents can be optionally joined to form a ring includes a case where adjacent substituents may be joined to form a ring and a case where adjacent substituents are not joined to form a ring. When adjacent substituents can be optionally joined to form a ring, the ring formed may be monocyclic or polycyclic (including spirocyclic, endocyclic, fusedcyclic, and etc.), as well as alicyclic, heteroalicyclic, aromatic, or heteroaromatic. In such expression, adjacent substituents may refer to substituents bonded to the same atom, substituents bonded to carbon atoms which are directly bonded to each other, or substituents bonded to carbon atoms which are more distant from each other. Preferably, adjacent substituents refer to substituents bonded to the same carbon atom and substituents bonded to carbon atoms which are directly bonded to each other.

The expression that adjacent substituents can be optionally joined to form a ring is also intended to mean that two substituents bonded to the same carbon atom are joined to each other via a chemical bond to form a ring, which can be exemplified by the following formula:

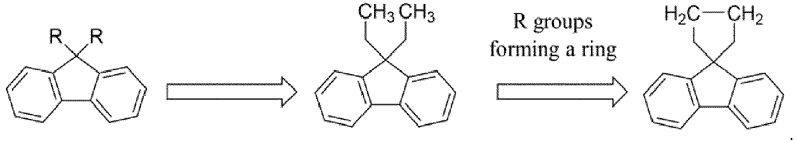

The expression that adjacent substituents can be optionally joined to form a ring is also intended to mean that two substituents bonded to carbon atoms which are directly bonded to each other are joined to each other via a chemical bond to form a ring, which can be exemplified by the following formula:

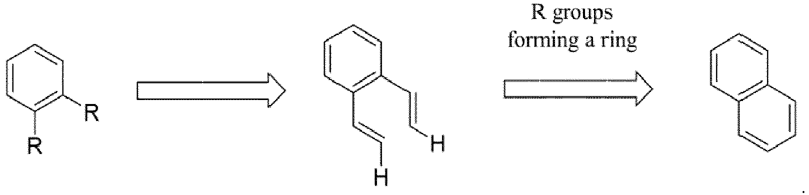

The expression that adjacent substituents can be optionally joined to form a ring is also intended to mean that two substituents bonded to further distant carbon atoms are joined to each other via a chemical bond to form a ring, which can be exemplified by the following formula:

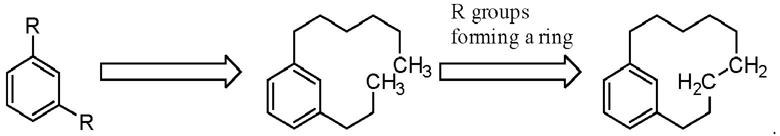

Furthermore, the expression that adjacent substituents can be optionally joined to form a ring is also intended to mean that, in the case where one of the two substituents bonded to carbon atoms which are directly bonded to each other represents hydrogen, the second substituent is bonded at a position at which the hydrogen atom is bonded, thereby forming a ring. This is exemplified by the following formula:

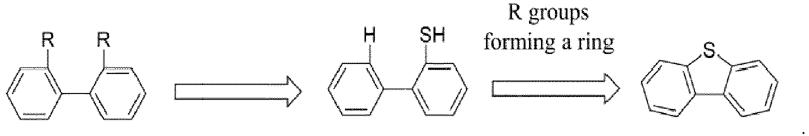

According to an embodiment of the present disclosure, disclosed is a compound having a structure of Formula 1: 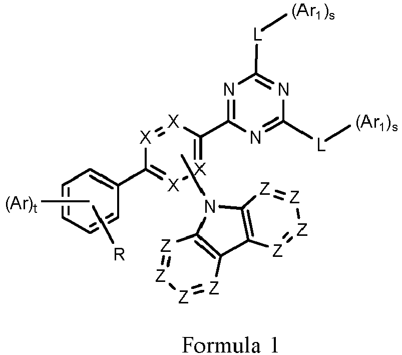 wherein
X is, at each occurrence identically or differently, selected from C, CRₓ, or N, wherein one X in Formula 1 is selected from C and joined to 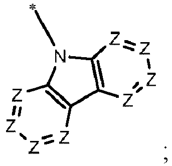 Z is, at each occurrence identically or differently, selected from CR_{z} or N;
Ar is, at each occurrence identically or differently, selected from Formula 2, Formula 3, Formula 4, or a combination thereof, wherein Formula 2, Formula 3, and Formula 4 have the following structures, respectively: 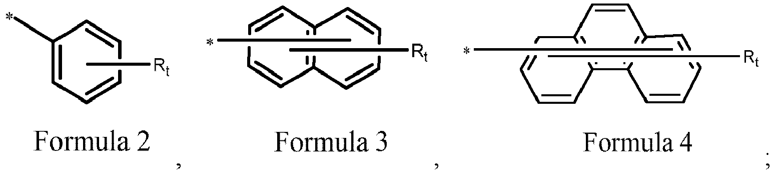 wherein
Rₜ in Formula 2, Formula 3, and Formula 4 represents, at each occurrence identically or differently, mono-substitution, multiple substitutions, or non-substitution;
"*" in the structures of Formula 2, Formula 3, and Formula 4 represents a position where the benzene ring with the substituent R in Formula 1, Formula 2, Formula 3, or Formula 4 is joined;
L is, at each occurrence identically or differently, selected from a single bond, Formula 5, Formula 6, Formula 7, or a combination thereof, wherein Formula 5, Formula 6, and Formula 7 have the following structures, respectively: 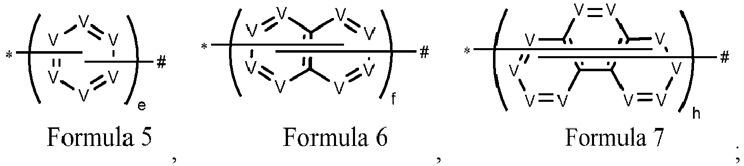
wherein V in Formula 5, Formula 6, and Formula 7 is, at each occurrence identically or differently, selected from C, CRᵥ, or N;
e, f, and h are, at each occurrence identically or differently, selected from 1, 2, or 3;
"*" in Formula 5, Formula 6, and Formula 7 represents a position where triazine shown in Formula 1, Formula 5, Formula 6, or Formula 7 is joined; and "#" represents a position where Ar₁ in Formula 1, Formula 5, Formula 6, or Formula 7 is joined;
Ar₁ is, at each occurrence identically or differently, selected from Formula 8, Formula 9, Formula 10, or a combination thereof, wherein Formula 8, Formula 9, and Formula 10 have the following structures, respectively: 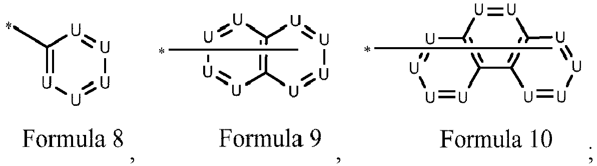
wherein U in Formula 8, Formula 9, and Formula 10 is, at each occurrence identically or differently, selected from C, CRᵤ, or N;
"*" in Formula 8, Formula 9, and Formula 10 represents a position where L in Formula 1, Formula 8, Formula 9, or Formula 10 is joined;
Rₓ, Rᵤ, Rᵥ, and Rₜ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R_{z} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R represents, at each occurrence identically or differently, mono-substitution, multiple substitutions, or non-substitution;
R is, at each occurrence identically or differently, selected from the group consisting of: hydrogen, deuterium, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted amino having 0 to 20 carbon atoms, an acyl group, a carbonyl group, a carboxylic acid group, an ester group, a hydroxyl group, a sulfanyl group, a sulfinyl group, a sulfonyl group, a phosphino group, and combinations thereof;
t is selected from 0, 1, 2, 3, 4, or 5; s is, at each occurrence identically or differently, selected from 1 to a maximum number of available substitutions for L; and
in Formula 1, only adjacent substituents R, adjacent substituents Rₓ, and adjacent substituents R_{z} can be optionally joined to form a 6-membered ring.

Herein, the expression that Ar is, at each occurrence identically or differently, selected from Formula 2, Formula 3, Formula 4, or a combination thereof is intended to mean that Ar may be selected from a group represented by any one alone of the structures of Formula 2, Formula 3, and Formula 4; or Ar may be selected from a group that is a combination of at least any two of Formula 2, Formula 3, and Formula 4. For example, when Ar is selected from a group combined by two structures of Formula 2, Ar is 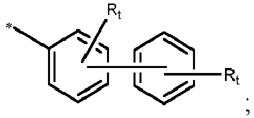 when Ar is selected from a group combined by one structure of Formula 2 and one structure of Formula 3, Ar is 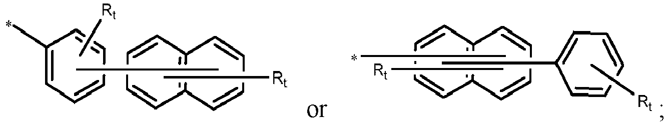 when Ar is selected from a group combined by three structures of Formula 2, Ar is 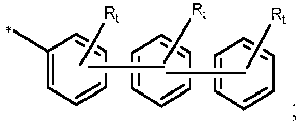 when Ar is selected from a group that is any other combination of the structures of Formula 2, Formula 3, and Formula 4, the case is the same as the above illustration. Therefore, those skilled in the art would acknowledge Ar structures formed by combined groups, which are not listed one by one here.

Herein, the expression that "*" in the structures of Formula 2, Formula 3, and Formula 4 represents a position where the benzene ring with the substituent R in Formula 1, Formula 2, Formula 3, or Formula 4 is joined is intended to mean that when Ar is selected from a group represented by any one alone of the structures of Formula 2, Formula 3, and Formula 4, "*" in the structures of Formula 2, Formula 3, and Formula 4 represents a position where the benzene ring with the substituent R in Formula 1 is joined; when Ar is selected from a group that is a combination of at least any two of Formula 2, Formula 3, and Formula 4, "*" in the structures of Formula 2, Formula 3, and Formula 4 may also represent a position where another structure of Formula 2, Formula 3, or Formula 4 in the combination is joined (the position may be any available substitution position of the ring shown in the structure of Formula 2, Formula 3, or Formula 4). For example, when Ar is selected from a group combined by two structures of Formula 2, Ar is 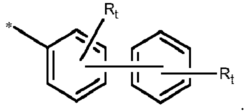 When Ar is selected from a group that is any other combination of Formula 2, Formula 3, and Formula 4, the case is the same as the above illustration. Therefore, those skilled in the art would acknowledge Ar structures formed by combined groups, which are not listed one by one here.

Herein, the expression that "L is, at each occurrence identically or differently, selected from a single bond, Formula 5, Formula 6, Formula 7, or a combination thereof" is intended to mean that L may be selected from a single bond or a group represented by any one alone of Formula 5, Formula 6, and Formula 7; or L may be selected from a group that is a combination of at least any two of Formula 5, Formula 6, and Formula 7. For example, when L is selected from a group combined by two structures of Formula 5, L is 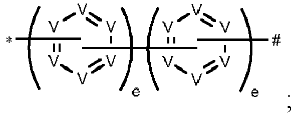 when L is selected from a group combined by one structure of Formula 5 and one structure of Formula 6, L is 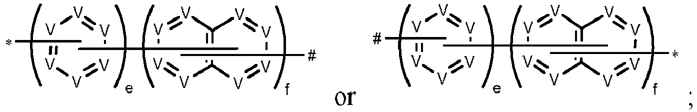 when L is selected from a group that is any other combination of the structures of Formula 5, Formula 6, and Formula 7, the case is the same as the above illustration. Therefore, those skilled in the art would acknowledge L structures formed by combined groups, which are not listed one by one here.

Herein, the expression that "*" in Formula 5, Formula 6, and Formula 7 represents a position where triazine shown in Formula 1, Formula 5, Formula 6, or Formula 7 is joined; and "#" represents a position where Ar₁ in Formula 1, Formula 5, Formula 6, or Formula 7 is joined is intended to mean that when L is selected from a group represented by any one alone of Formula 5, Formula 6, and Formula 7, "*" in Formula 5, Formula 6, and Formula 7 represents a position where triazine shown in Formula 1 is joined and "#" represents a position where Ar₁ in Formula 1 is joined. When L is selected from a group that is a combination of at least any two of Formula 5, Formula 6, and Formula 7, "*" or "#" in the structures of Formula 5, Formula 6, and Formula 7 may also represent a position where another structure of Formula 5, Formula 6, or Formula 7 in the combination is joined (the position may be any available substitution position of a ring shown in the structure of Formula 5, Formula 6, or Formula 7). For example, when L is selected from a group combined by two structures of Formula 5, L is 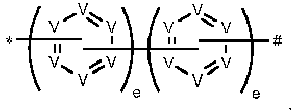 When L is selected from a group that is any other combination of Formula 5, Formula 6, and Formula 7, the case is the same as the above illustration. Therefore, those skilled in the art would acknowledge L structures formed by combined groups, which are not listed one by one here.

Herein, the expression that Ar₁ is, at each occurrence identically or differently, selected from Formula 8, Formula 9, Formula 10, or a combination thereof is intended to mean that Ar₁ may be selected from a group represented by any one alone of Formula 8, Formula 9, and Formula 10; or Ar₁ may be selected from a group that is a combination of at least any two of Formula 8, Formula 9, and Formula 10. For example, when Ar₁ is selected from a group combined by two structures of Formula 8, Ar₁ is 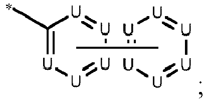 when Ar₁ is selected from a group combined by one structure of Formula 8 and one structure of Formula 9, Ar₁ is 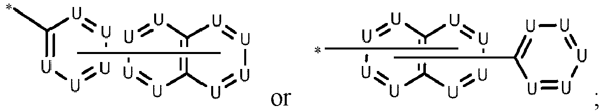 when Ar₁ is selected from a group combined by three structures of Formula 8, Ar₁ is 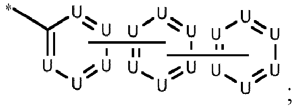 when Ar₁ is selected from a group that is any other combination of the structures of Formula 8, Formula 9, and Formula 10, the case is the same as the above illustration. Therefore, those skilled in the art would acknowledge Ar₁ structures formed by combined groups, which are not listed one by one here.

Herein, the expression that "*" in Formula 8, Formula 9, and Formula 10 represents a position where L in Formula 1, Formula 8, Formula 9, or Formula 10 is joined is intended to mean that when Ar₁ is selected from a group represented by any one alone of Formula 8, Formula 9, and Formula 10, "*" in Formula 8, Formula 9, and Formula 10 represents a position where L in Formula 1 is joined. When Ar₁ is selected from a group that is a combination of at least any two of Formula 8, Formula 9, and Formula 10, "*" in the structures of Formula 8, Formula 9, and Formula 10 may also represent a position where another structure of Formula 8, Formula 9, or Formula 10 in the combination is joined (the position may be any available substitution position of a ring shown in the structure of Formula 8, Formula 9, or Formula 10). For example, when Ar₁ is selected from a group combined by two structures of Formula 8, Ar₁ is 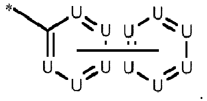 When Ar₁ is selected from a group that is any other combination of Formula 8, Formula 9, and Formula 10, the case is the same as the above illustration. Therefore, those skilled in the art would acknowledge Ar₁ structures formed by groups, which are not listed one by one here.

Herein, "t" represents the number of Ar substituent groups on the benzene ring with the substituent R in Formula 1, and t is selected from 0, 1, 2, 3, 4, or 5. For example, when t is 2, it means that two Ar groups are substituted on the benzene ring with the substituent R in Formula 1, and the two Ar groups may be the same or different.

Herein, "s" represents the number of Ar₁ substituents attached on the structure L in Formula 1, and "s" is an integer. The expression that s is, at each occurrence identically or differently, selected from 1 to a maximum number of available substitutions for L is intended to mean that the structure L has at least one Ar₁ substituent and at most all available substitution positions on the structure L have Ar₁ substituents. For example, when L is 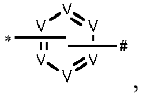 the maximum number of available substitutions on the structure is 5, and s is selected from an integer from 1 to 5; when L is 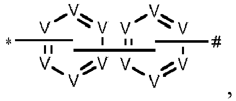 the maximum number of available substitutions on the structure is 9, and s is selected from an integer from 1 to 9; when L is 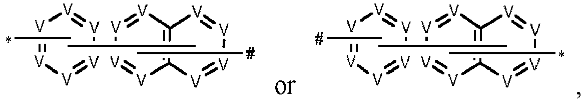 the maximum number of available substitutions on the structure is 11, and s is selected from an integer from 1 to 11; when L is selected from a group that is any other combination of Formula 5, Formula 6, and Formula 7, the case is the same as the above illustration. Therefore, those skilled in the art would acknowledge the maximum number of available substitutions on the group formed by any other combination, that is, would acknowledge the selection range of s, which is not listed one by one here. It is to be noted that when s is selected from an integer greater than or equal to 2, the corresponding multiple Ar₁ groups may be the same or different. Preferably, s is selected from 1, 2, 3, 4, or 5. It is to be noted that in Formula 1, when Ar₁ is joined to V in L, the V is selected from C.

Herein, the expression that only adjacent substituents R, adjacent substituents Rₓ, and adjacent substituents R_{z} can be optionally joined to form a 6-membered ring is intended to mean that in Formula 1, two adjacent substituents R can be optionally joined to form a 6-membered ring, two adjacent substituents Rₓ can be optionally joined to form a 6-membered ring, two adjacent substituents R_{z} can be optionally joined to form a 6-membered ring, and none of other adjacent substituents in Formula 1 are joined to form a ring. Preferably, the 6-membered ring formed by joining adjacent substituents is a 6-membered aromatic or heteroaromatic ring.

According to an embodiment of the present disclosure, the compound having the structure of Formula 1 comprises only one carbazole structure or azacarbazole structure or fused carbazole ring structure or fused azacarbazole ring. The carbazole structure or azacarbazole structure or fused carbazole ring structure or fused azacarbazole ring is shown in the structure of Formula 1, that is, the fused ring structure where Z is located.

According to an embodiment of the present disclosure, Ar is selected from the structure of Formula 2.

According to an embodiment of the present disclosure, Ar is, at each occurrence identically or differently, selected from 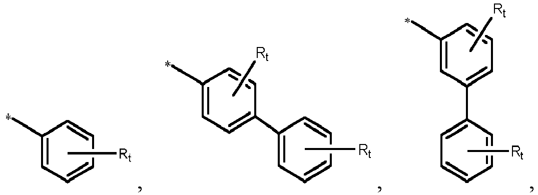 or 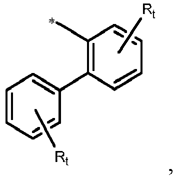 wherein Rₜ represents, at each occurrence identically or differently, mono-substitution, multiple substitutions, or non-substitution.

According to an embodiment of the present disclosure, L is, at each occurrence identically or differently, selected from a single bond.

According to an embodiment of the present disclosure, L is, at each occurrence identically or differently, selected from the structure of Formula 5.

According to an embodiment of the present disclosure, L is, at each occurrence identically or differently, selected from 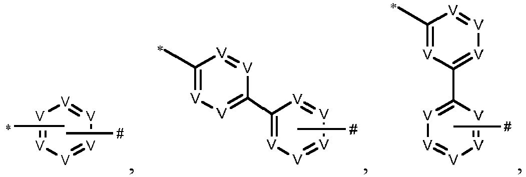 or

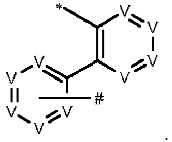

According to an embodiment of the present disclosure, Ar₁ is, at each occurrence identically or differently, selected from 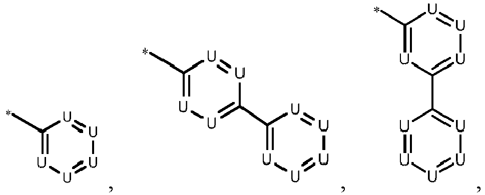 or

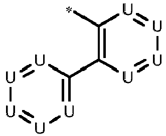

According to an embodiment of the present disclosure, Ar₁ is, at each occurrence identically or differently, selected from the structure of Formula 8.

According to an embodiment of the present disclosure, Ar is, at each occurrence identically or differently, selected from the structure of Formula 2 or Formula 3, L is, at each occurrence identically or differently, selected from the structure of Formula 5 or Formula 6, and Ar₁ is, at each occurrence identically or differently, selected from the structure of Formula 8 or Formula 9.

According to an embodiment of the present disclosure, X is, at each occurrence identically or differently, selected from C or CRₓ.

According to an embodiment of the present disclosure, Z is, at each occurrence identically or differently, selected from CR_{z}.

According to an embodiment of the present disclosure, U is, at each occurrence identically or differently, selected from C or CRᵤ.

According to an embodiment of the present disclosure, V is, at each occurrence identically or differently, selected from C or CRᵥ.

According to an embodiment of the present disclosure, s is 1.

According to an embodiment of the present disclosure, t is selected from 0 or 1.

According to an embodiment of the present disclosure, Rₓ, Rᵤ, Rₜ, Rᵥ, and R_{z} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, and substituted or unsubstituted alkenyl having 2 to 20 carbon atoms.

According to an embodiment of the present disclosure, Rₓ, Rᵤ, Rₜ, Rᵥ, and R_{z} are, at each occurrence identically or differently, selected from hydrogen or deuterium.

According to an embodiment of the present disclosure, R is, at each occurrence identically or differently, selected from the group consisting of: hydrogen, deuterium, halogen, substituted or unsubstituted cycloalkyl having 3 to 20 ring carbon atoms, and substituted or unsubstituted alkenyl having 2 to 20 carbon atoms.

According to an embodiment of the present disclosure, R is, at each occurrence identically or differently, selected from hydrogen or deuterium.

According to an embodiment of the present disclosure, the compound of Formula 1 has a structure represented by Formula 1-1: 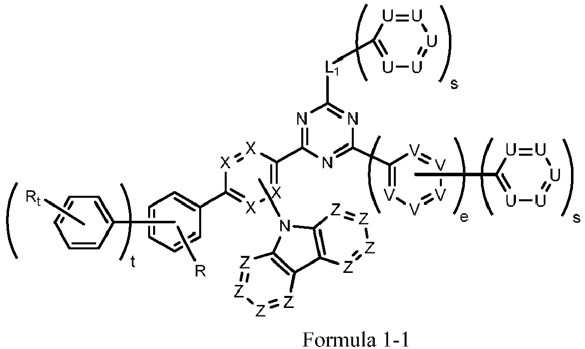 wherein
X is, at each occurrence identically or differently, selected from C, CRₓ, or N, wherein one X in Formula 1-1 is selected from C and joined to 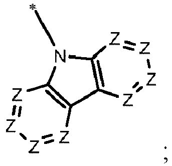 Z is, at each occurrence identically or differently, selected from CR_{z} or N; U is, at each occurrence identically or differently, selected from CRᵤ or N; V is, at each occurrence identically or differently, selected from C, CRᵥ, or N;
L₁ is, at each occurrence identically or differently, selected from a single bond or 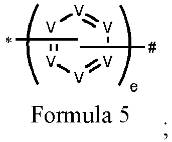
e is, at each occurrence identically or differently, selected from 1, 2, or 3;
Rₓ, Rᵤ, Rᵥ, and Rₜ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R_{z} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R and Rₜ represent, at each occurrence identically or differently, mono-substitution, multiple substitutions, or non-substitution;
R is, at each occurrence identically or differently, selected from the group consisting of: hydrogen, deuterium, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted amino having 0 to 20 carbon atoms, an acyl group, a carbonyl group, a carboxylic acid group, an ester group, a hydroxyl group, a sulfanyl group, a sulfinyl group, a sulfonyl group, a phosphino group, and combinations thereof;
t is selected from 0, 1, 2, 3, 4, or 5; s is, at each occurrence identically or differently, selected from 1 to a maximum number of available substitutions in 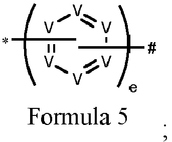 preferably, s is selected from 1, 2, 3, 4, or 5; and
in Formula 1-1, only adjacent substituents R, adjacent substituents Rₓ, and adjacent substituents R_{z} can be optionally joined to form a 6-membered ring.

According to an embodiment of the present disclosure, the compound is selected from the group consisting of Compound A-1 to Compound A-581, wherein the specific structures of Compound A-1 to Compound A-581 are referred to claim 7.

According to an embodiment of the present disclosure, the compound is selected from the group consisting of Compound A-1 to Compound A-584, wherein the specific structures of Compound A-1 to Compound A-581 are referred to claim 7, and the specific structures of Compound A-582 to Compound A-584 are shown as follows:

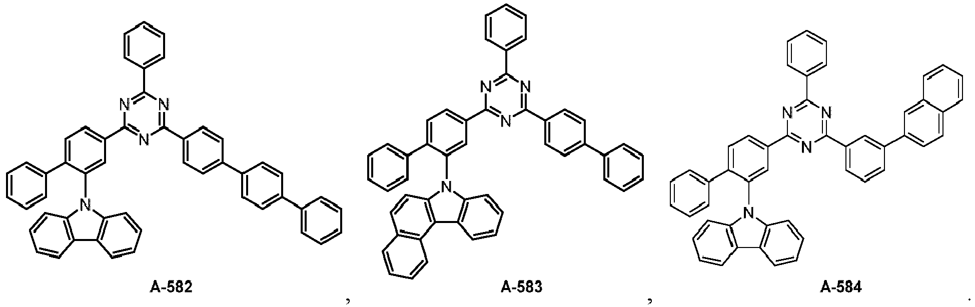

According to an embodiment of the present disclosure, further disclosed is an electroluminescent device comprising:
an anode,
a cathode, and
an organic layer disposed between the anode and the cathode, wherein the organic layer comprises a compound of Formula 1, wherein the compound having the structure of Formula 1 is any one of the preceding embodiments.

According to an embodiment of the present disclosure, the organic layer is a light-emitting layer, and the compound is a host compound.

According to an embodiment of the present disclosure, the compound is a green phosphorescent host material, a red phosphorescent host material, or a yellow phosphorescent host material.

According to an embodiment of the present disclosure, the organic layer is an electron transporting layer, and the compound is an electron transporting compound.

According to an embodiment of the present disclosure, the organic layer is a hole blocking layer, and the compound is a hole blocking compound.

According to an embodiment of the present disclosure, the light-emitting layer further comprises a first metal complex having a general formula of M(Lₐ)ₘ(L_{b})ₙ(L_{c})_{q}; wherein
the metal M is selected from a metal with a relative atomic mass greater than 40;
Lₐ, L_{b}, and L_{c} are a first ligand, a second ligand, and a third ligand coordinated to the metal M, respectively; Lₐ, L_{b}, and L_{c} may be the same or different;
the ligands Lₐ, L_{b}, and L_{c} can be optionally joined to form a multidentate ligand; for example, any two of Lₐ, L_{b}, and L_{c} may be joined to form a tetradentate ligand; in another example, Lₐ, L_{b}, and L_{c} may be joined to each other to form a hexadentate ligand; in another example, none of Lₐ, L_{b}, and L_{c} are joined so that no multidentate ligand is formed;
m is 1, 2, or 3, n is 0, 1, or 2, q is 0, 1, or 2, and m+n+q is equal to an oxidation state of the metal M; when m is greater than or equal to 2, multiple Lₐ may be the same or different; when n is 2, two L_{b} may be the same or different; when q is 2, two L_{c} may be the same or different;
the ligand Lₐ has a structure represented by Formula 11: 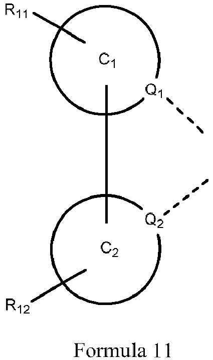
wherein the ring C₁ and the ring C₂ are, at each occurrence identically or differently, selected from an aromatic ring having 5 to 30 ring atoms, a heteroaromatic ring having 5 to 30 ring atoms, or a combination thereof;
Q₁ and Q₂ are, at each occurrence identically or differently, selected from C or N;
R₁₁ and R₁₂ represent, at each occurrence identically or differently, mono-substitution, multiple substitutions, or non-substitution;
R₁₁ and R₁₂ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
adjacent substituents R₁₁, R₁₂ can be optionally joined to form a ring; and
the ligands L_{b} and L_{c} are, at each occurrence identically or differently, selected from a monoanionic bidentate ligand.

In this embodiment, the expression that adjacent substituents R₁₁, R₁₂ can be optionally joined to form a ring is intended to mean that any one or more of groups of adjacent substituents, such as two substituents R₁₁, two substituents R₁₂, and substituents R₁₁ and R₁₂, may be joined to form a ring. Obviously, it is also possible that none of these groups of adjacent substituents are joined to form a ring.

According to an embodiment of the present disclosure, the ligands L_{b} and L_{c} are, at each occurrence identically or differently, selected from the group consisting of the following structures: 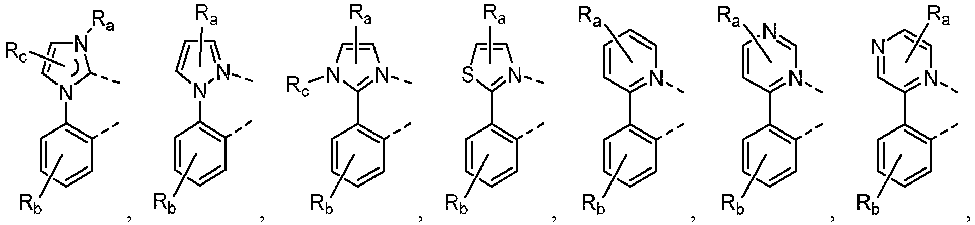 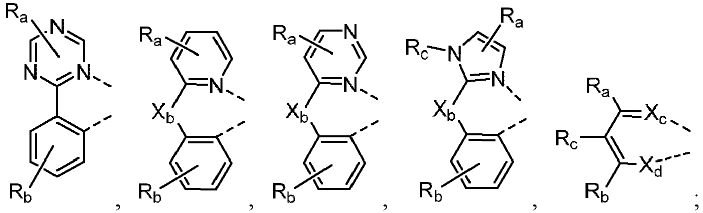 wherein
Rₐ and R_{b} represent, at each occurrence identically or differently, mono-substitution, multiple substitutions, or non-substitution;
X_{b} is, at each occurrence identically or differently, selected from the group consisting of: O, S, Se, NR_{N1}, and CR_{C1}R_{C2};
X_{c} and X_{d} are, at each occurrence identically or differently, selected from the group consisting of: O, S, Se, and NR_{N2};
Rₐ, R_{b}, R_{c}, R_{N1}, R_{N2}, R_{C1}, and R_{C2} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof; and
adjacent substituents Rₐ, R_{b}, R_{c}, R_{N1}, R_{N2}, R_{C1}, and R_{C2} can be optionally joined to form a ring.

In this embodiment, the expression that adjacent substituents Rₐ, R_{b}, R_{c}, R_{N1}, R_{N2}, R_{C1}, and R_{C2} can be optionally joined to form a ring is intended to mean that any one or more of groups of adjacent substituents, such as two substituents Rₐ, two substituents R_{b}, substituents Rₐ and R_{b}, substituents Rₐ and R_{c}, substituents R_{b} and R_{c}, substituents Rₐ and R_{N1}, substituents R_{b} and R_{N1}, substituents Rₐ and R_{C1}, substituents Rₐ and R_{C2}, substituents R_{b} and R_{C1}, substituents R_{b} and R_{C2}, substituents R_{C1} and R_{C2}, substituents Rₐ and R_{N2}, and substituents R_{b} and R_{N2}, may be joined to form a ring. Obviously, it is also possible that none of these substituents are joined to form a ring.

According to an embodiment of the present disclosure, the first metal complex has a general formula of Ir(Lₐ)ₘ(L_{b})₃₋ₘ and has a structure represented by Formula 11-1: 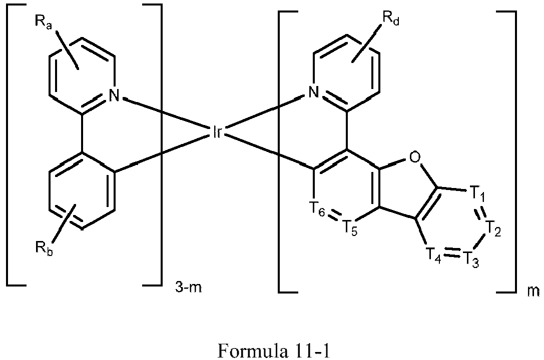 wherein
m is 0, 1, 2, or 3; when m is 2 or 3, multiple Lₐ are the same or different; when m is 0 or 1, multiple L_{b} are the same or different;
T₁ to T₆ are, at each occurrence identically or differently, selected from CR_{T} or N;
Rₐ, R_{b}, and R_{d} represent, at each occurrence identically or differently, mono-substitution, multiple substitutions, or non-substitution;
Rₐ, R_{b}, R_{d}, and R_{T} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a sulfanyl group, a hydroxyl group, a sulfinyl group, a sulfonyl group, a phosphino group, and combinations thereof;
adjacent substituents Rₐ, R_{b} can be optionally joined to form a ring; and
adjacent substituents R_{d}, R_{T} can be optionally joined to form a ring.

In this embodiment, the expression that adjacent substituents Rₐ, R_{b} can be optionally joined to form a ring is intended to mean that any one or more of groups of adjacent substituents, such as two substituents Rₐ, two substituents R_{b}, and substituents Rₐ and R_{b}, may be joined to form a ring. Obviously, it is also possible that none of these groups of adjacent substituents are joined to form a ring.

In this embodiment, the expression that adjacent substituents R_{d}, R_{T} can be optionally joined to form a ring is intended to mean that any one or more of groups of adjacent substituents, such as two substituents R_{T} and two substituents R_{d}, may be joined to form a ring. Obviously, it is also possible that none of these groups of adjacent substituents are joined to form a ring.

According to an embodiment of the present disclosure, at least one of T₁ to T₆ is selected from CR_{T}, and the R_{T} is selected from substituted or unsubstituted alkyl having 1 to 20 carbon atoms, substituted or unsubstituted cycloalkyl having 3 to 20 ring carbon atoms, substituted or unsubstituted aryl having 6 to 30 carbon atoms, or substituted or unsubstituted heteroaryl having 3 to 30 carbon atoms.

According to an embodiment of the present disclosure, at least one of T₁ to T₆ is selected from CR_{T}, and the R_{T} is selected from fluorine or cyano.

According to an embodiment of the present disclosure, at least two of T₁ to T₆ are selected from CR_{T}, wherein one R_{T} is selected from fluorine or cyano, and another R_{T} is selected from substituted or unsubstituted alkyl having 1 to 20 carbon atoms, substituted or unsubstituted cycloalkyl having 3 to 20 ring carbon atoms, substituted or unsubstituted aryl having 6 to 30 carbon atoms, or substituted or unsubstituted heteroaryl having 3 to 30 carbon atoms.

According to an embodiment of the present disclosure, T₁ to T₆ are, at each occurrence identically or differently, selected from CR_{T}.

According to an embodiment of the present disclosure, T₁ to T₆ are, at each occurrence identically or differently, selected from CR_{T} or N, and at least one of T₁ to T₆ is selected from N; for example, one of T₁ to T₆ is selected from N or two of T₁ to T₆ are selected from N.

According to an embodiment of the present disclosure, the first metal complex is selected from the group consisting of Compound GD1 to Compound GD76, wherein the specific structures of Compound GD 1 to Compound GD76 are referred to claim 10.

According to an embodiment of the present disclosure, the first metal complex is selected from the group consisting of Compound GD1 to Compound GD77, wherein the specific structures of Compound GD1 to Compound GD76 are referred to claim 10, and the specific structure of Compound GD77 is

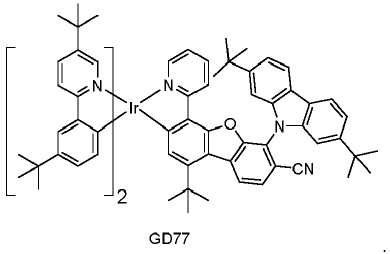

According to an embodiment of the present disclosure, the first metal complex is selected from the group consisting of Compound GD1 to Compound GD78, wherein the specific structures of Compound GD1 to Compound GD76 are referred to claim 10, the specific structure of Compound GD77 is 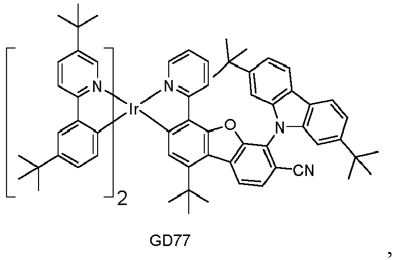 and the specific structure of Compound GD78 is

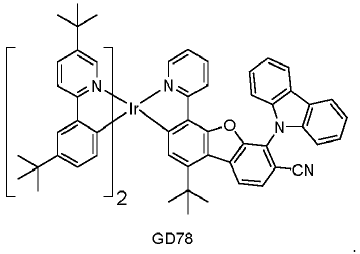

According to an embodiment of the present disclosure, hydrogens in Compound GD1 to Compound GD76 can be partially or fully substituted with deuterium.

According to an embodiment of the present disclosure, the first metal complex has a general formula of M(Lₐ₁)ⱼ(L_{b1})ₖ, wherein M is selected from a metal with a relative atomic mass greater than 40;
Lₐ₁ and L_{b1} are a first ligand and a second ligand coordinated to M, respectively; Lₐ₁ and L_{b1} can be optionally joined to form a multidentate ligand;
j is 1, 2, or 3; k is 0, 1, or 2; j+k is equal to an oxidation state of M; when j is greater than or equal to 2, multiple Lₐ₁ may be the same or different; when k is 2, two L_{b1} may be the same or different;
Lₐ₁ has a structure represented by Formula 11-2: 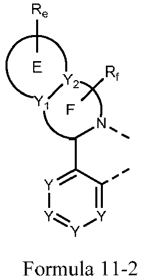 wherein
the ring F is selected from a 5-membered heteroaromatic ring or a 6-membered heteroaromatic ring;
the ring E is selected from a 5-membered unsaturated carbocyclic ring, a benzene ring, a 5-membered heteroaromatic ring, or a 6-membered heteroaromatic ring;
the ring F and the ring E are fused via Y₁ and Y₂;
Y₁ and Y₂ are, at each occurrence identically or differently, selected from C or N;
R_{f} and Rₑ represent, at each occurrence identically or differently, mono-substitution, multiple substitutions, or non-substitution;
Y is, at each occurrence identically or differently, selected from CR_{y} or N;
R_{f}, Rₑ, and R_{y} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
adjacent substituents R_{f}, Rₑ, R_{y} can be optionally joined to form a ring;
the ligand L_{b1} has a structure represented by Formula 11-3: 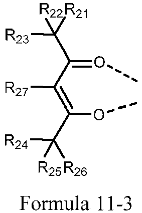
wherein R₂₁ to R₂₇ are each independently selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted amino having 0 to 20 carbon atoms, an acyl group, a carbonyl group, a carboxylic acid group, an ester group, a cyano group, an isocyano group, a sulfanyl group, a sulfinyl group, a sulfonyl group, a phosphino group, and combinations thereof.

Herein, the expression that adjacent substituents R_{f}, Rₑ, R_{y} can be optionally joined to form a ring is intended to mean that in the presence of substituents R_{f}, Rₑ, R_{y}, any one or more of groups of adjacent substituents, such as adjacent substituents R_{f}, adjacent substituents Rₑ, adjacent substituents R_{y}, adjacent substituents R_{f} and Rₑ, adjacent substituents R_{f} and R_{y}, and adjacent substituents Rₑ and R_{y}, may be joined to form a ring. Obviously, in the presence of substituents R_{f}, Rₑ, R_{y}, it is also possible that none of these groups of substituents is joined to form a ring.

According to an embodiment of the present disclosure, the ligand L_{b1} has a structure represented by Formula 11-3: 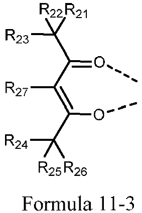 wherein at least one of R₂₁ to R₂₃ is selected from substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, or a combination thereof; and/or at least one of R₂₄ to R₂₆ is selected from substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, or a combination thereof.

According to an embodiment of the present disclosure, the ligand L_{b1} has a structure represented by Formula 11-3: 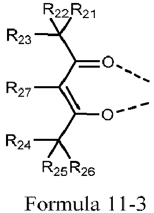 wherein at least two of R₂₁ to R₂₃ are selected from substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, or a combination thereof; and/or at least two of R₂₄ to R₂₆ are selected from substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, or a combination thereof.

According to an embodiment of the present disclosure, the ligand L_{b1} has a structure represented by Formula 11-3: 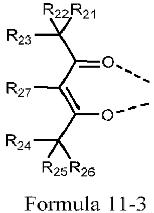 wherein at least two of R₂₁ to R₂₃ are, at each occurrence identically or differently, selected from substituted or unsubstituted alkyl having 2 to 20 carbon atoms, substituted or unsubstituted cycloalkyl having 3 to 20 ring carbon atoms, substituted or unsubstituted heteroalkyl having 2 to 20 carbon atoms, or a combination thereof; and/or at least two of R₂₄ to R₂₆ are, at each occurrence identically or differently, selected from substituted or unsubstituted alkyl having 2 to 20 carbon atoms, substituted or unsubstituted cycloalkyl having 3 to 20 ring carbon atoms, substituted or unsubstituted heteroalkyl having 2 to 20 carbon atoms, or a combination thereof.

According to an embodiment of the present disclosure, the first metal complex has a general formula of Ir(Lₐ₁)₂(L_{b1}).

According to an embodiment of the present disclosure, the first metal complex is an Ir complex and comprises a ligand Lₐ₁, and Lₐ₁ has the structure represented by Formula 11-2 and comprises at least one structural unit selected from the group consisting of an aromatic ring formed by fusing a 6-membered ring to a 6-membered ring, a heteroaromatic ring formed by fusing a 6-membered ring to a 6-membered ring, an aromatic ring formed by fusing a 6-membered ring to a 5-membered ring, and a heteroaromatic ring formed by fusing a 6-membered ring to a 5-membered ring.

According to an embodiment of the present disclosure, the first metal complex is an Ir complex and comprises a ligand Lₐ₁, and Lₐ₁ has the structure represented by Formula 11-2 and comprises at least one structural unit selected from the group consisting of naphthalene, phenanthrene, quinoline, isoquinoline, and azaphenanthrene.

According to an embodiment of the present disclosure, the first metal complex is selected from the group consisting of the following structures:

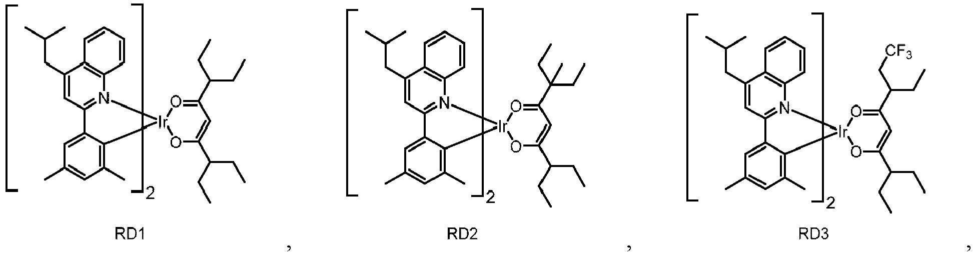

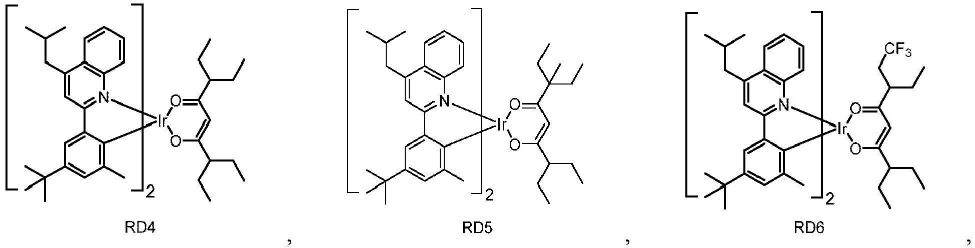

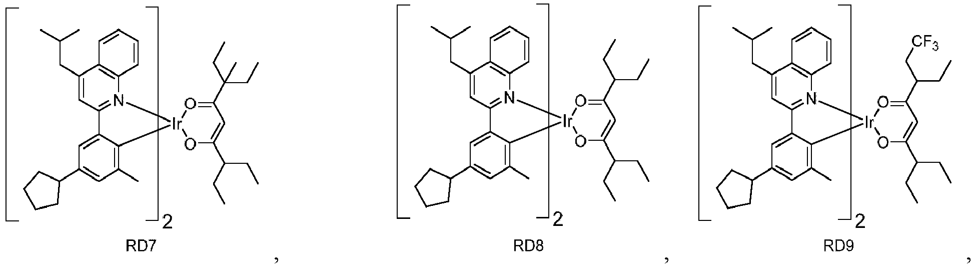

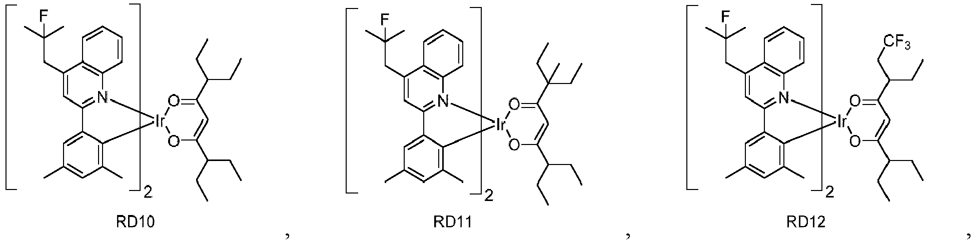

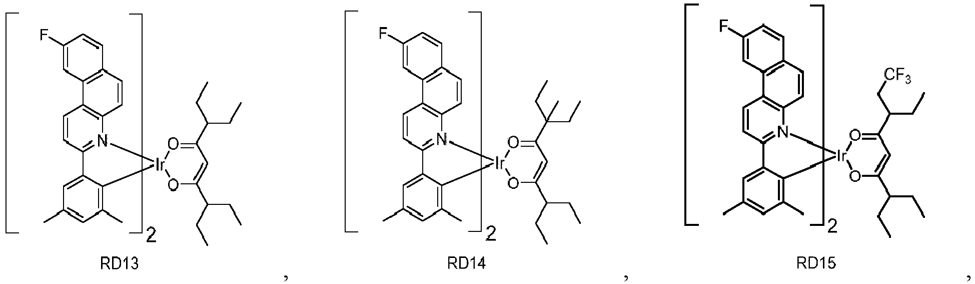

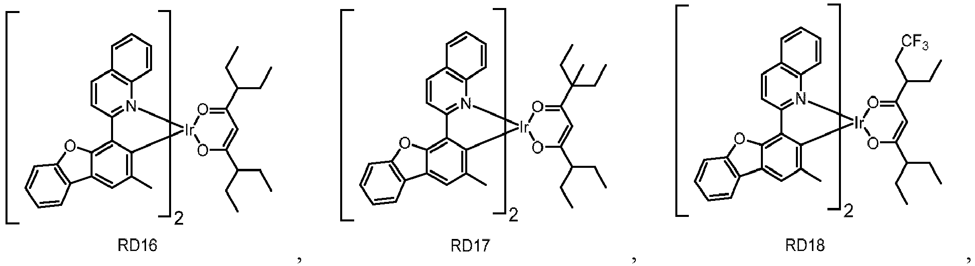

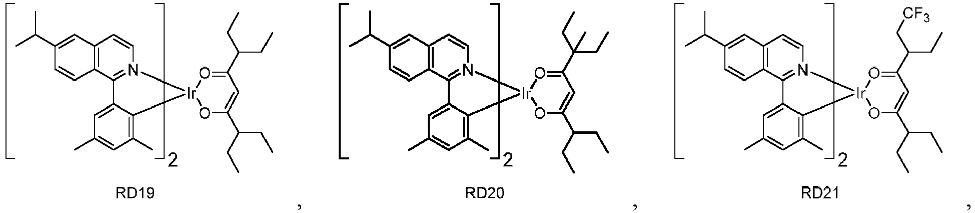

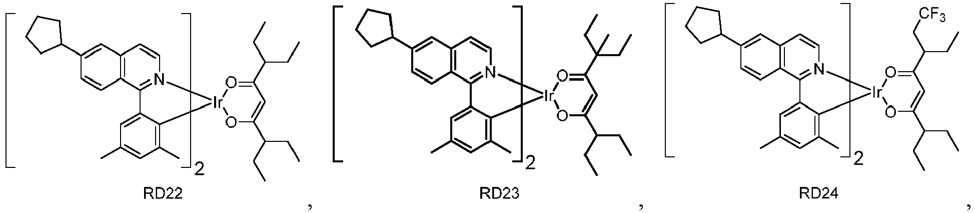

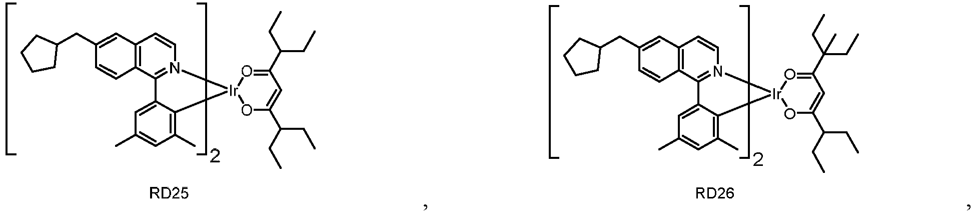

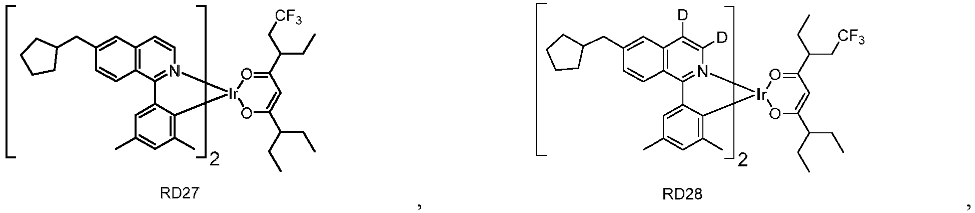

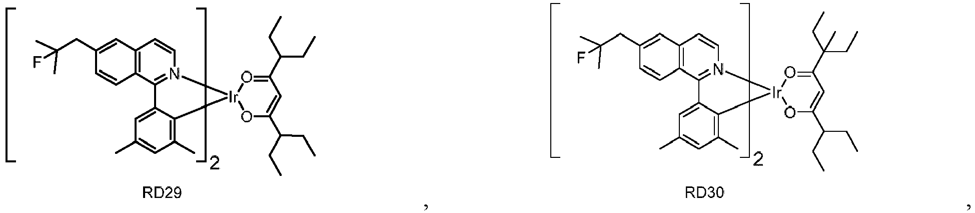

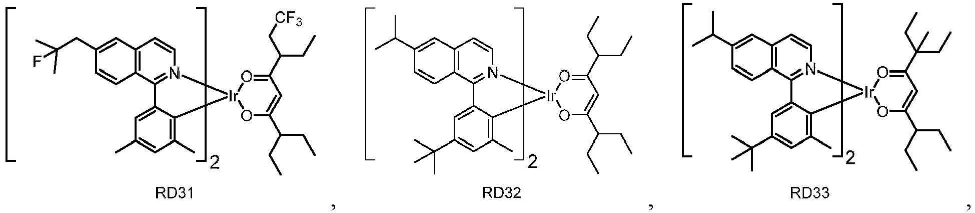

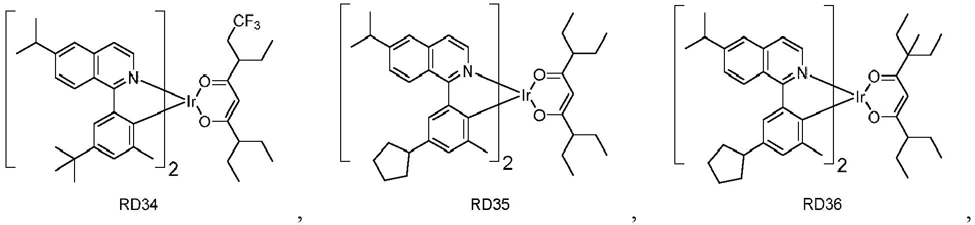

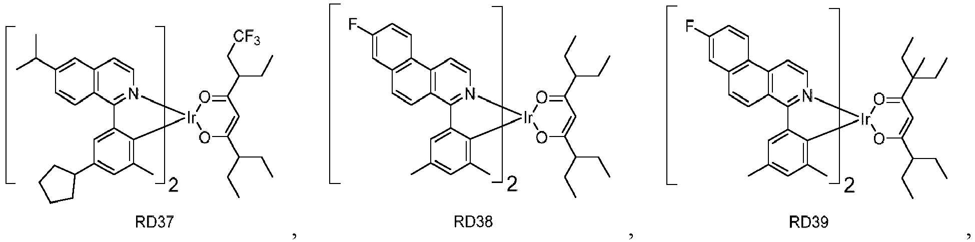

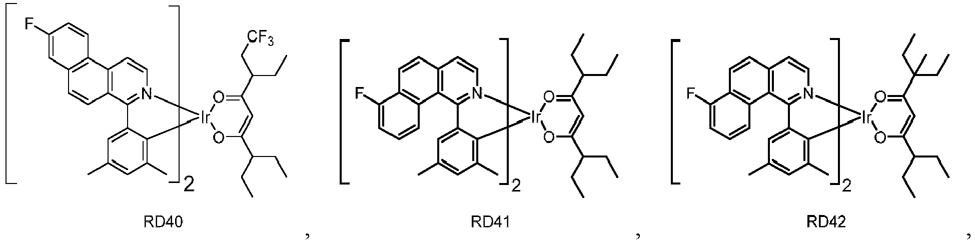

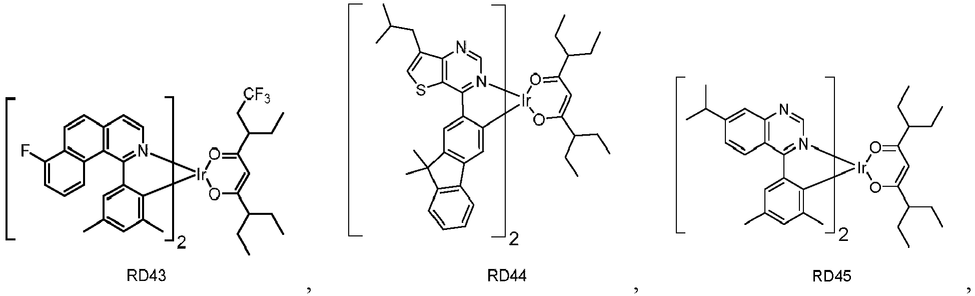

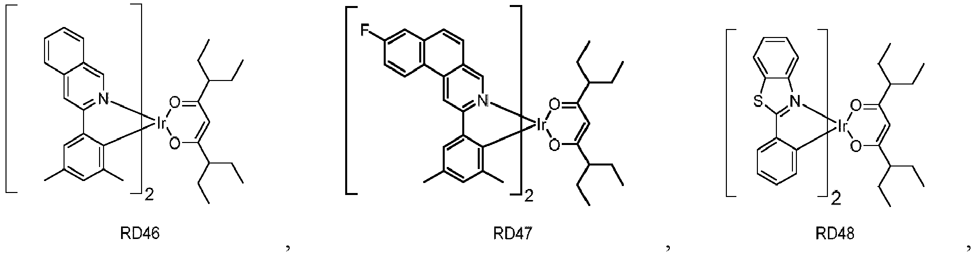

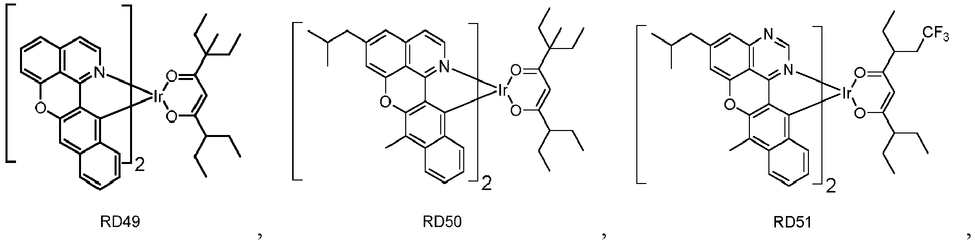

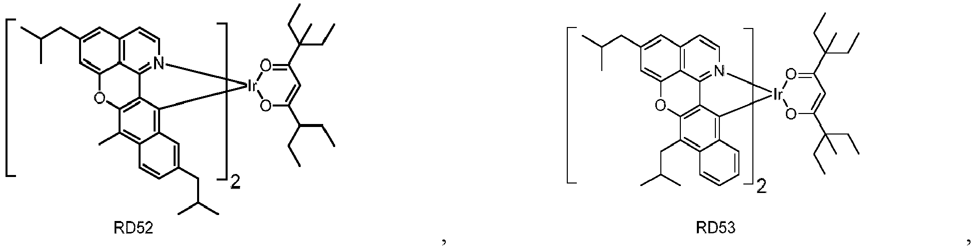

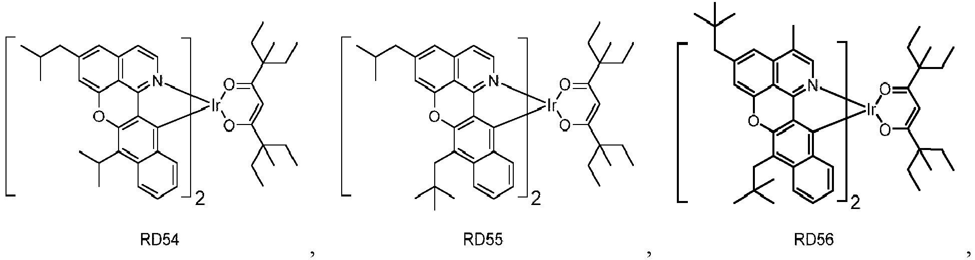

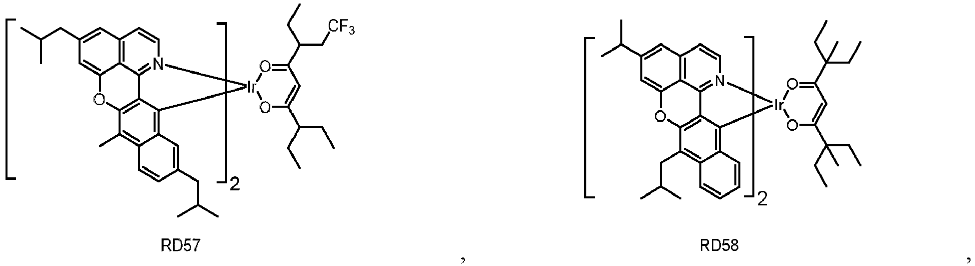

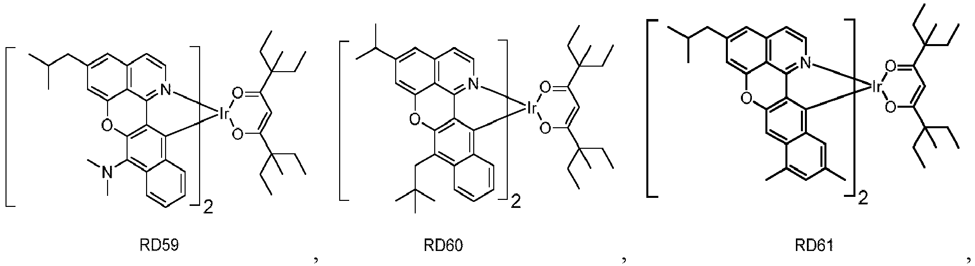

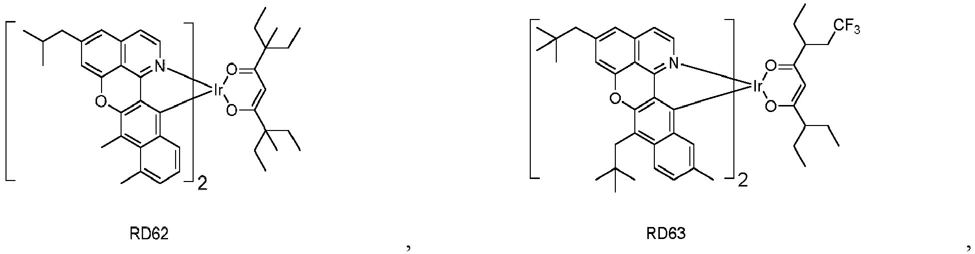

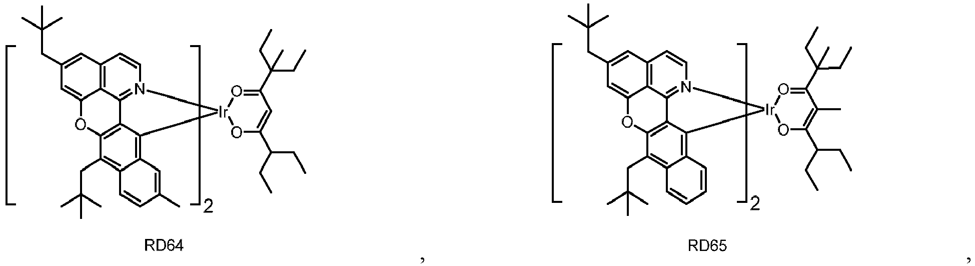

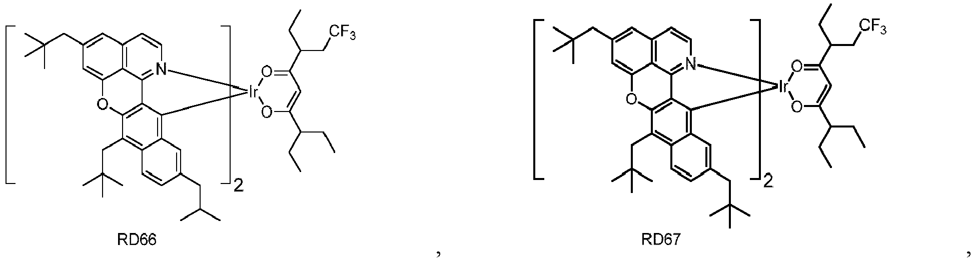

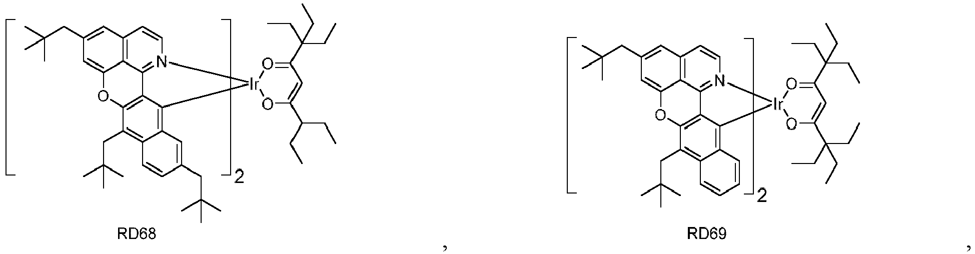

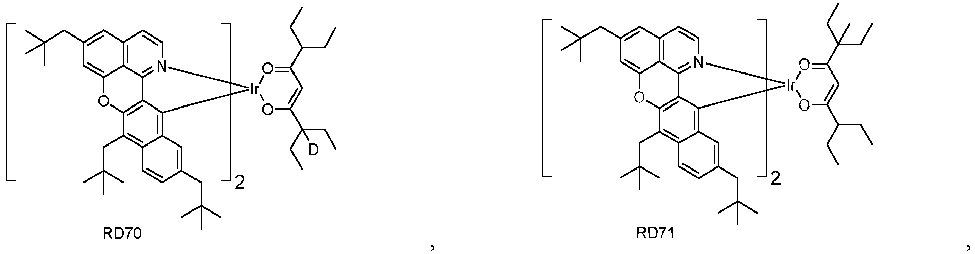

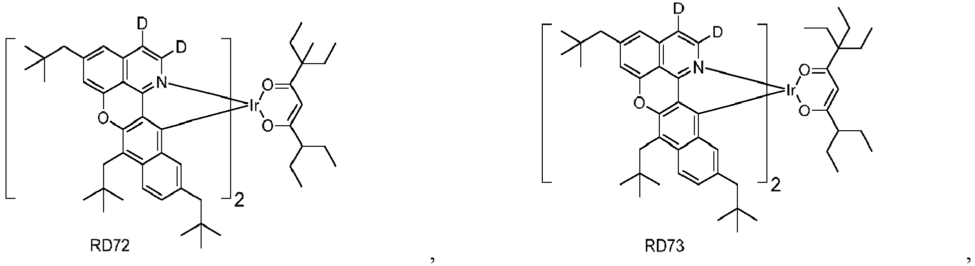

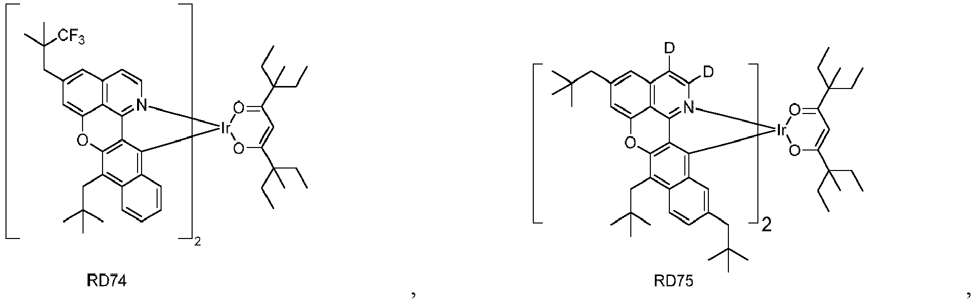

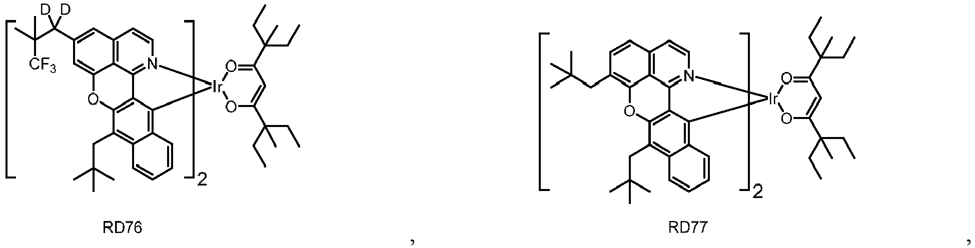

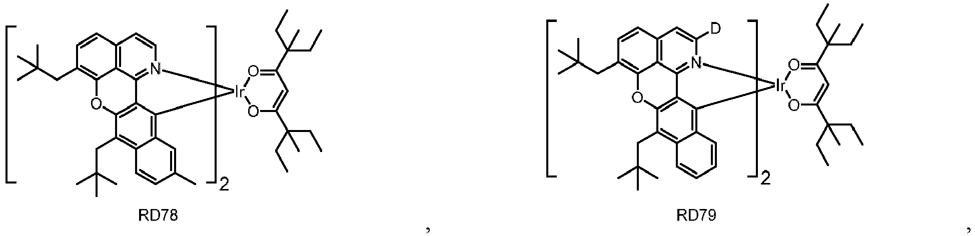

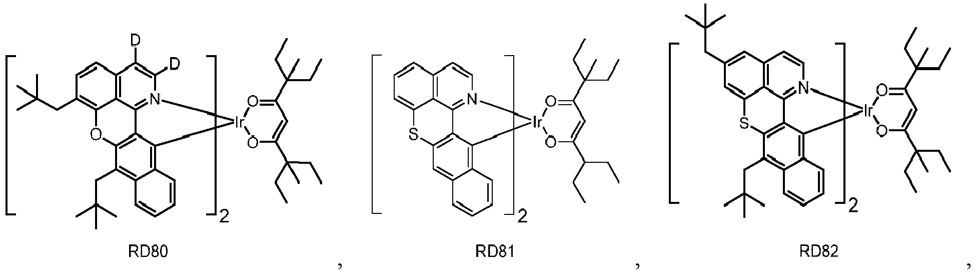

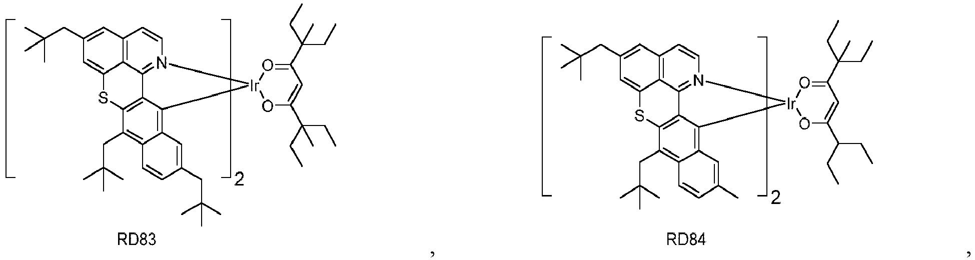

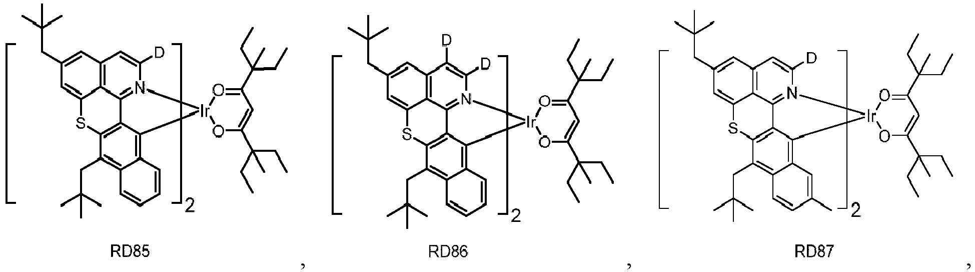

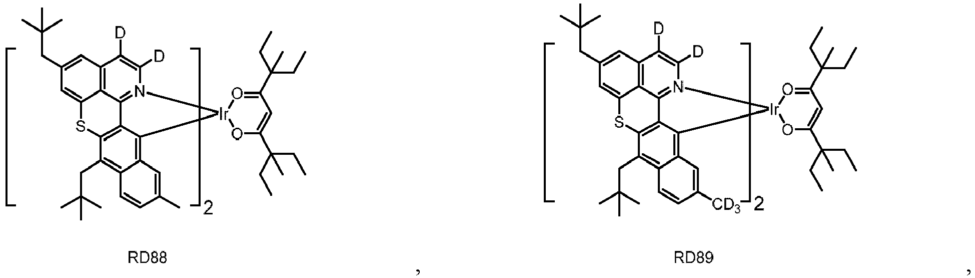

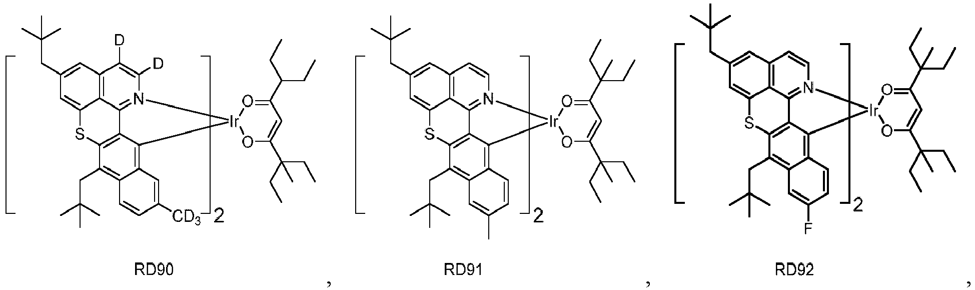

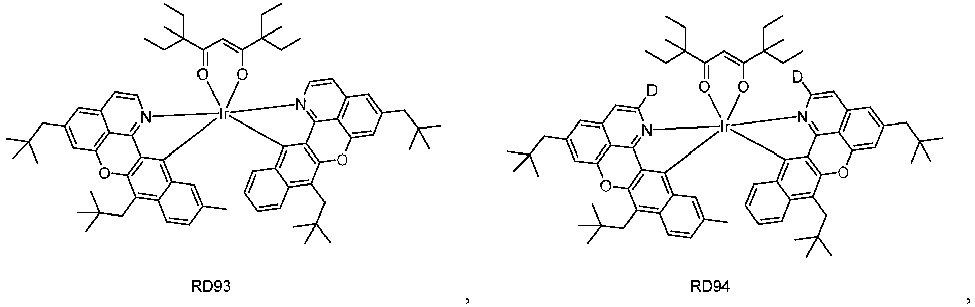

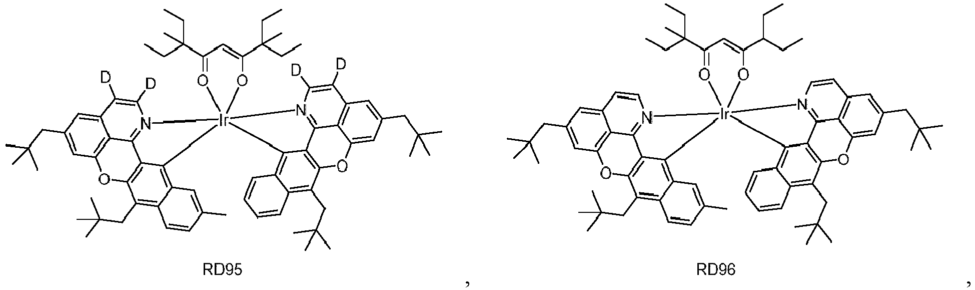

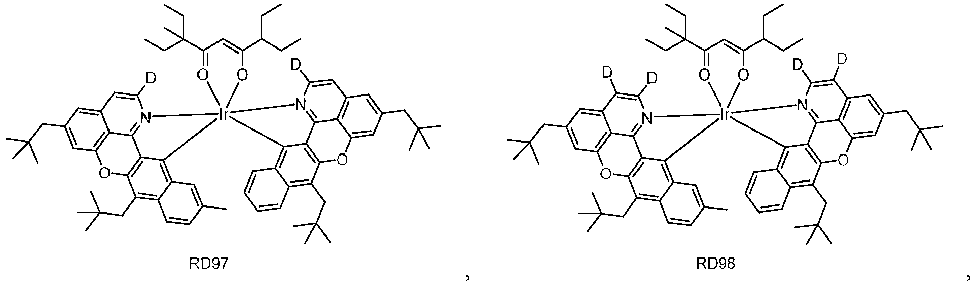

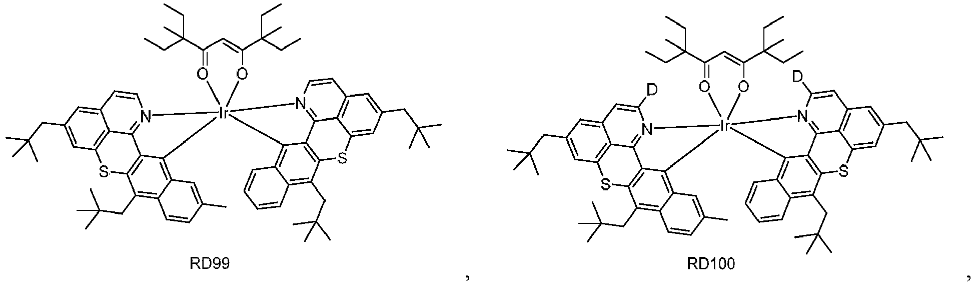

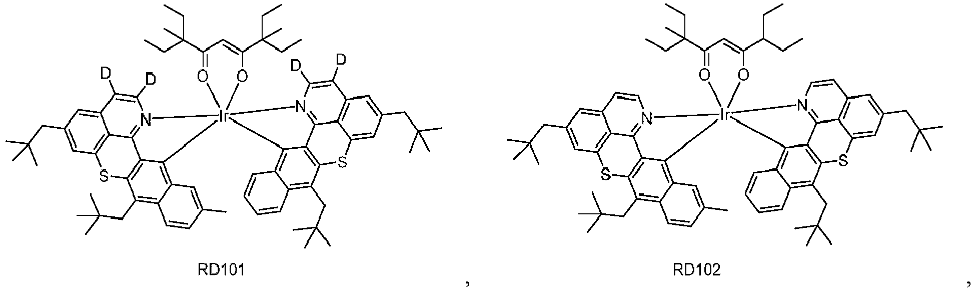

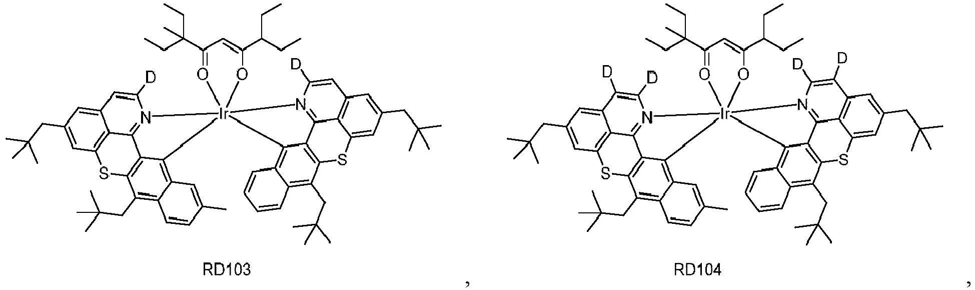

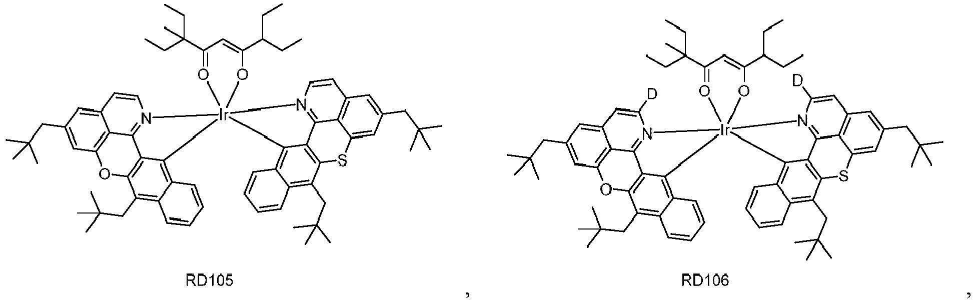

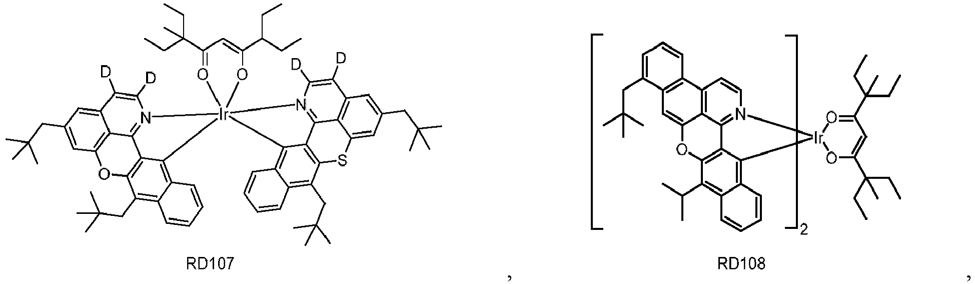

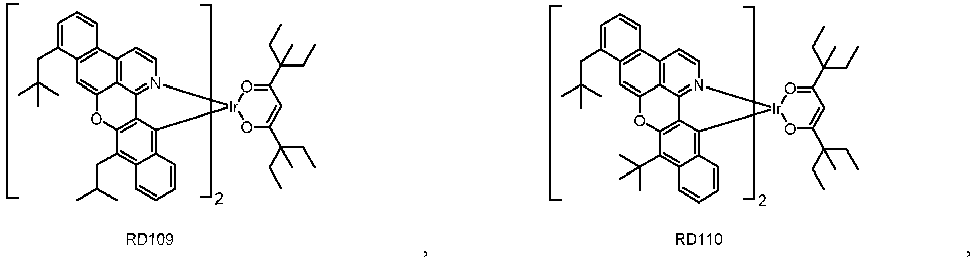

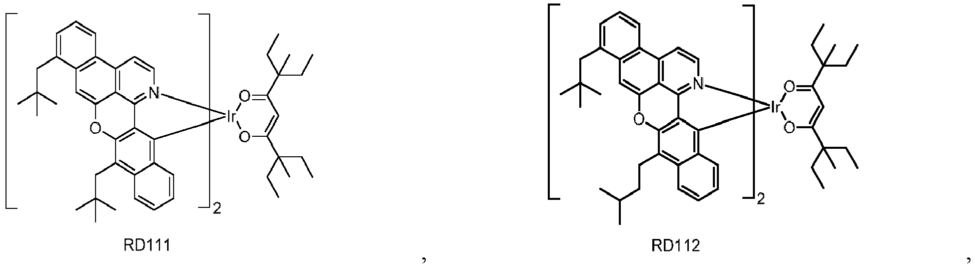

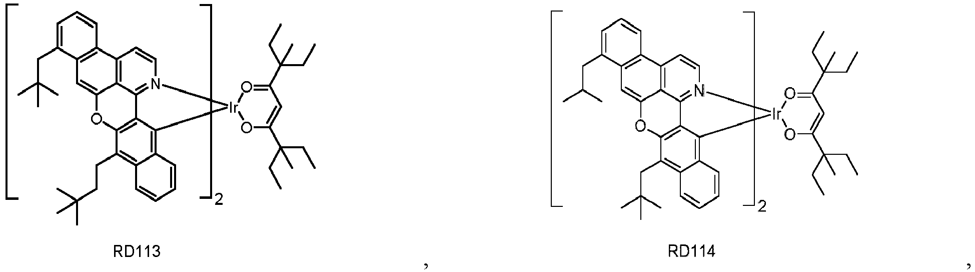

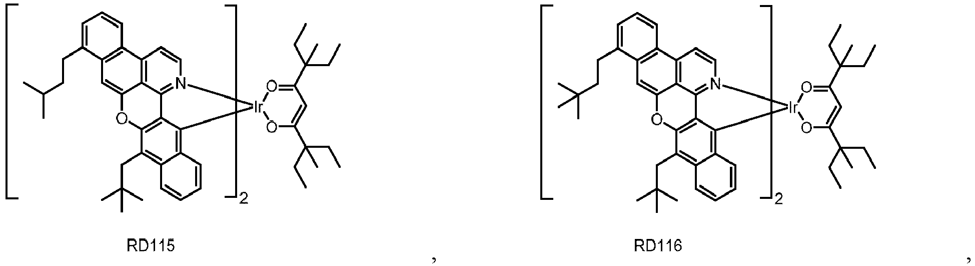

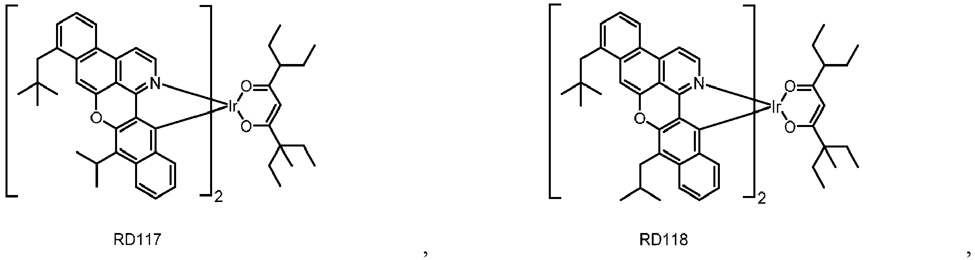

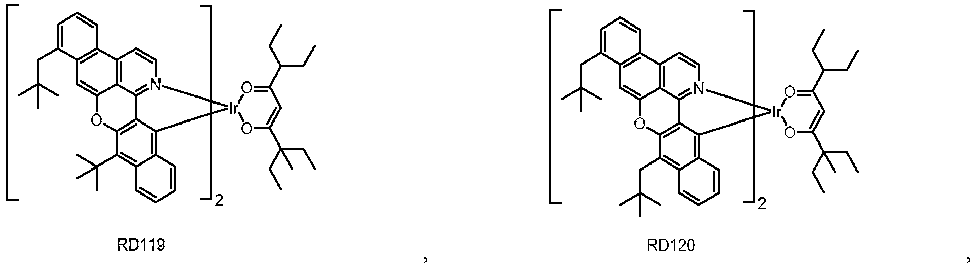

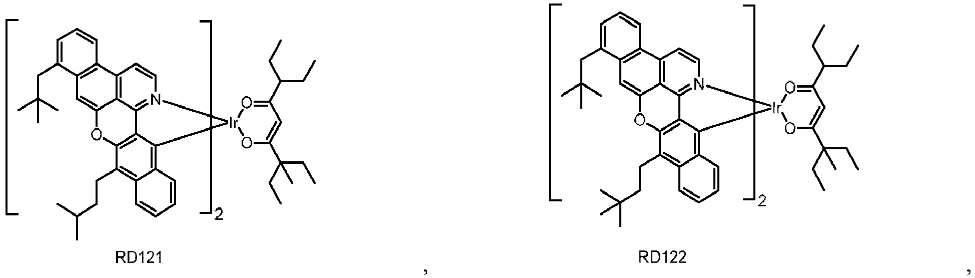

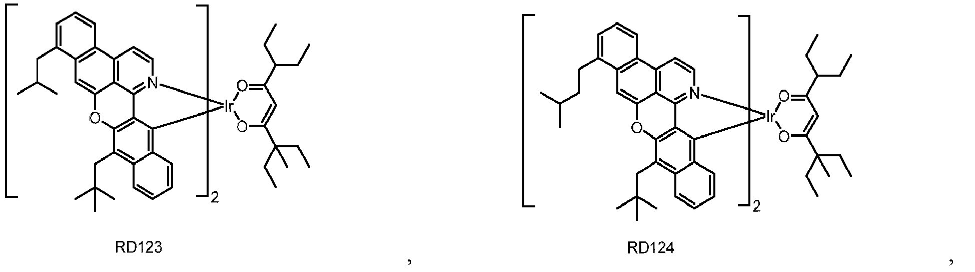

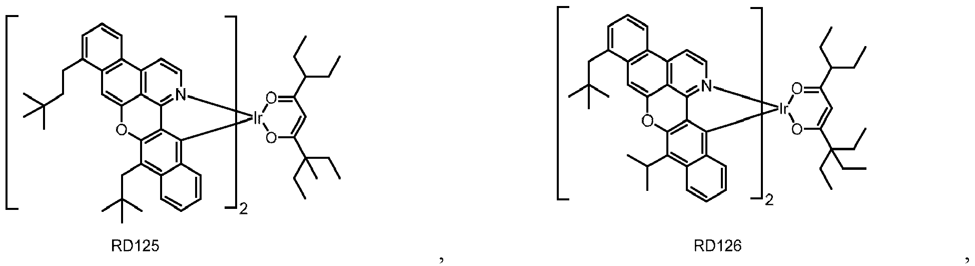

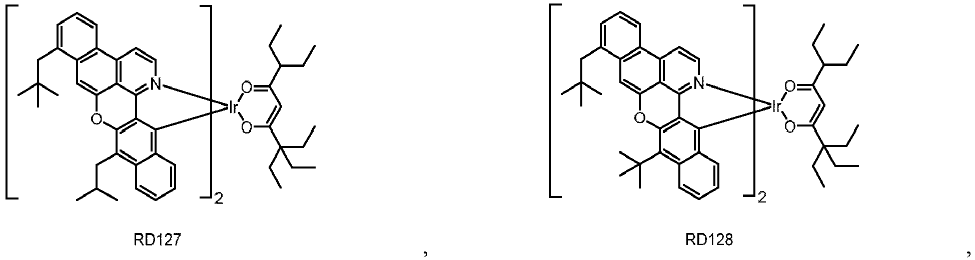

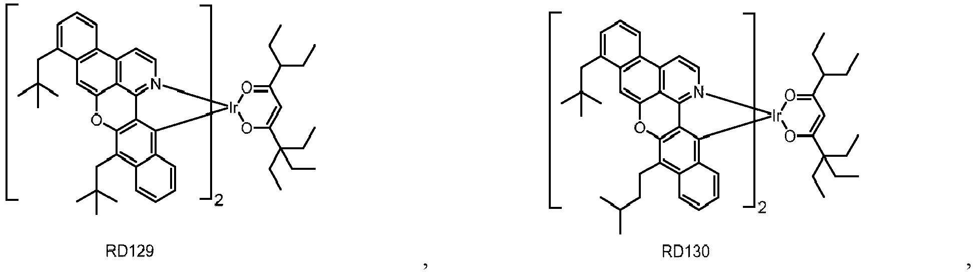

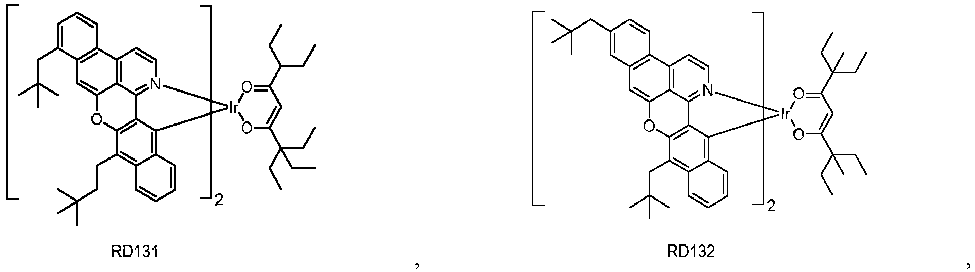

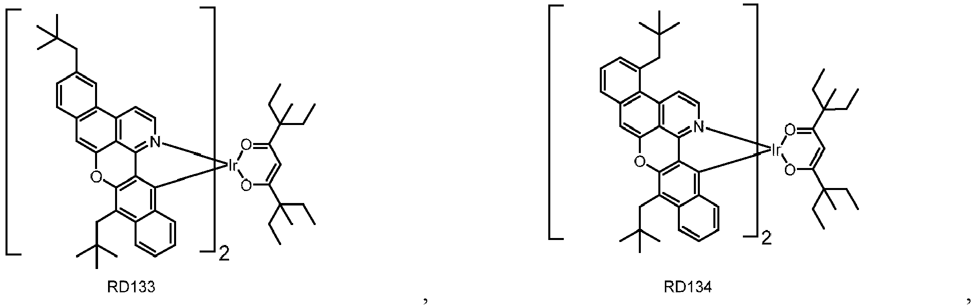

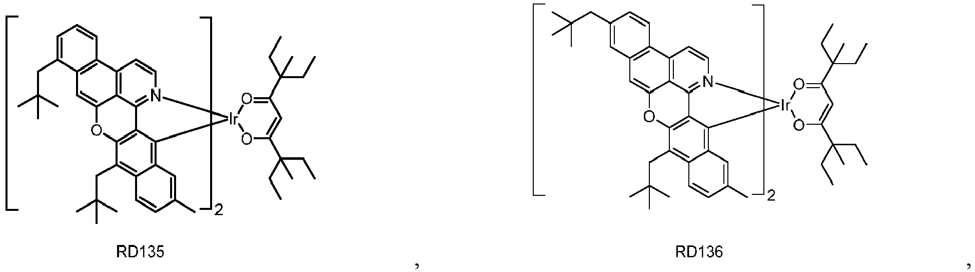

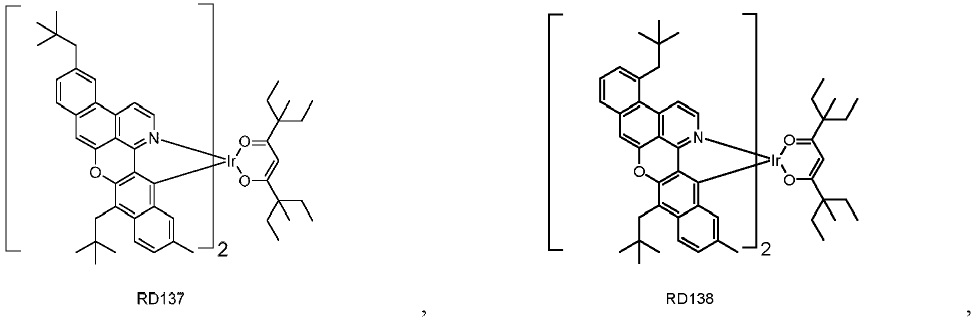

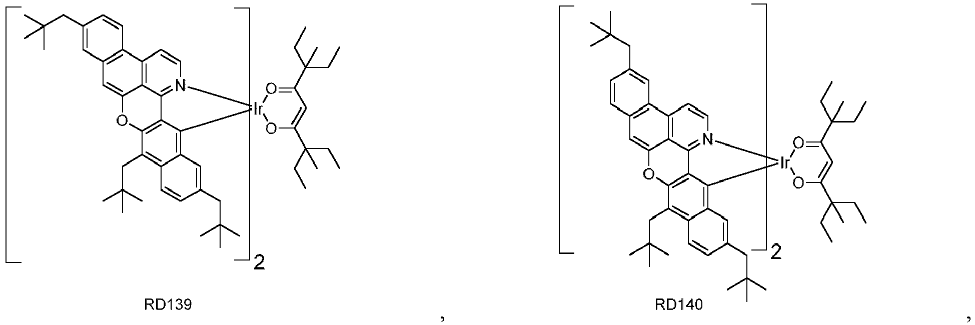

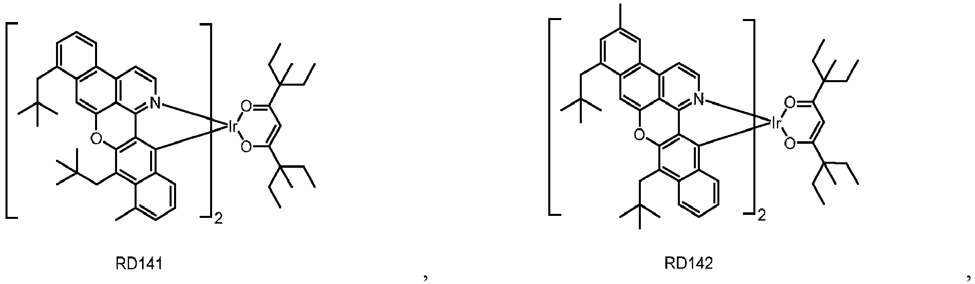

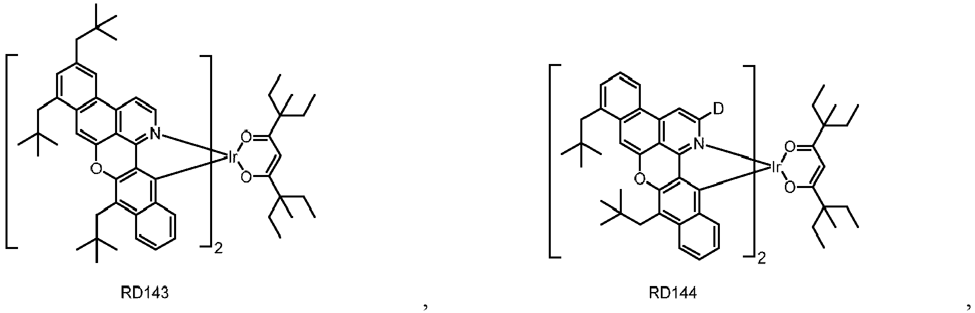

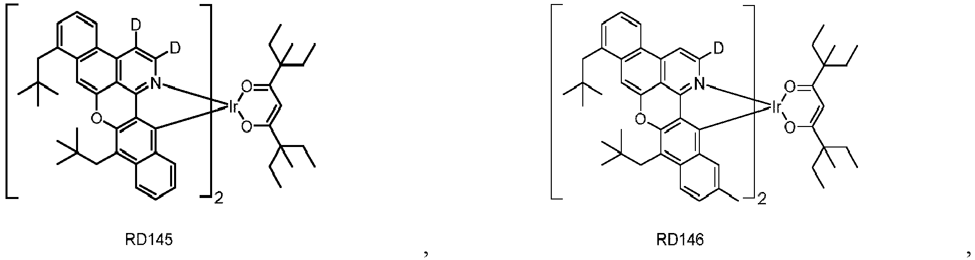

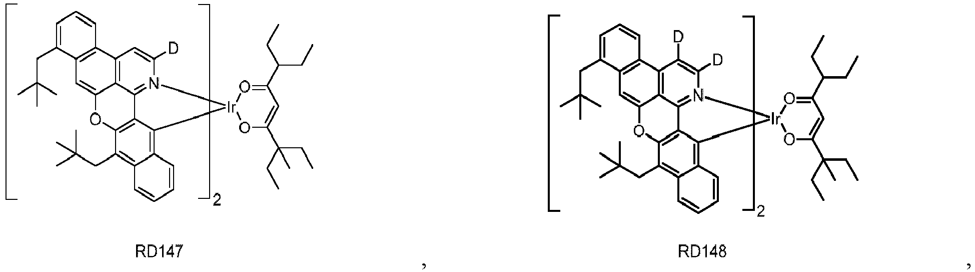

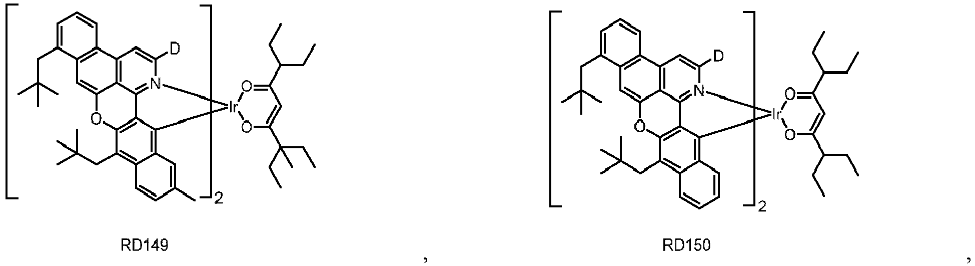

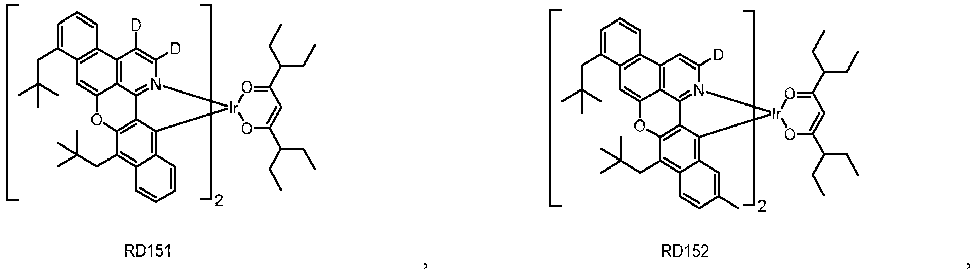

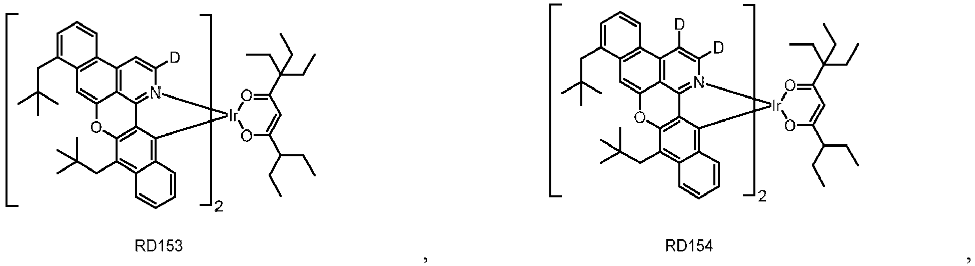

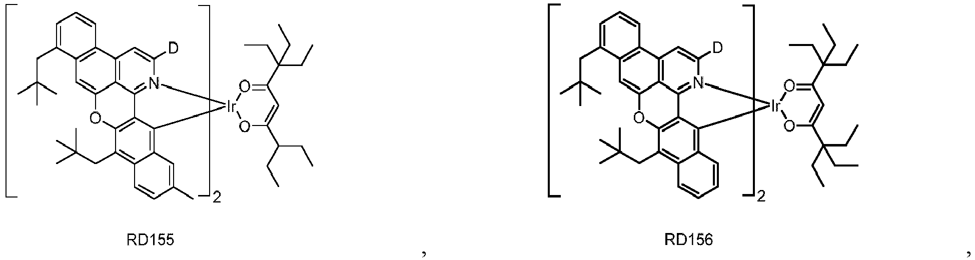

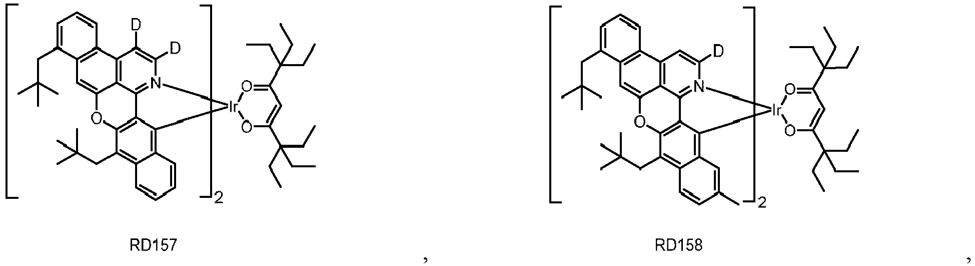

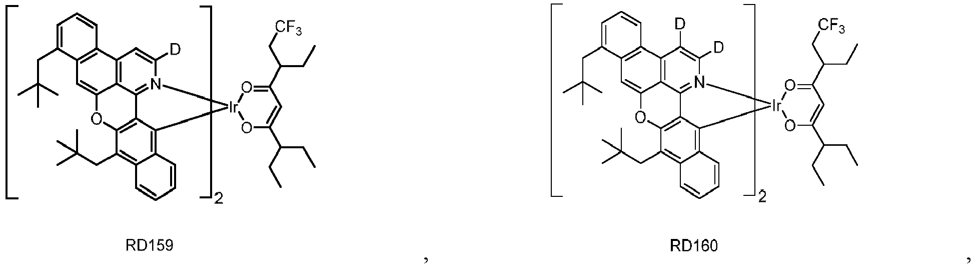

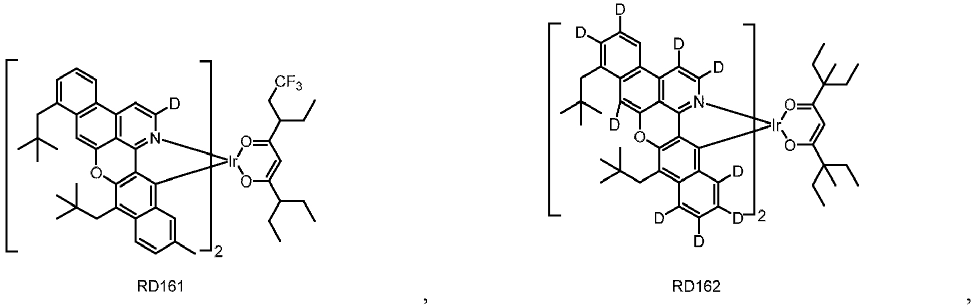

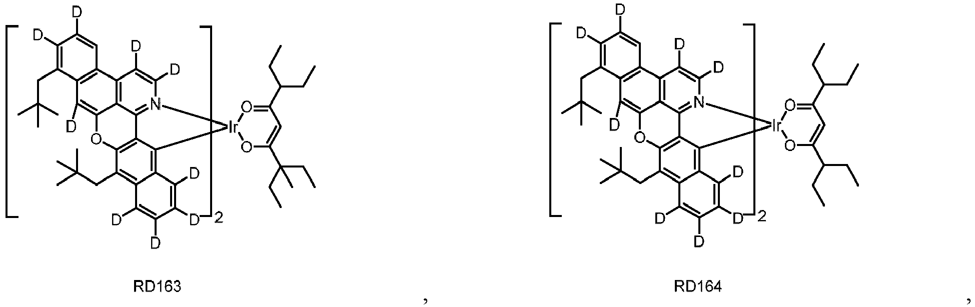

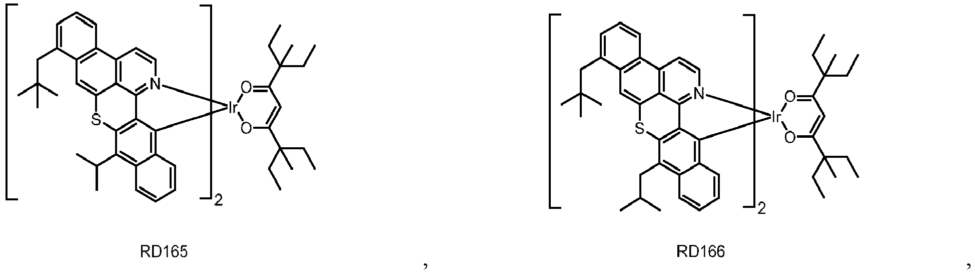

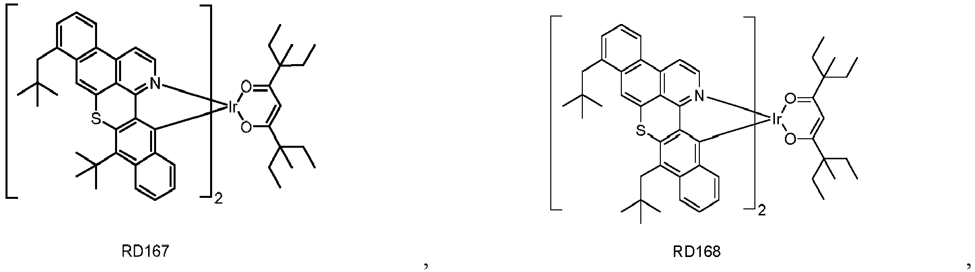

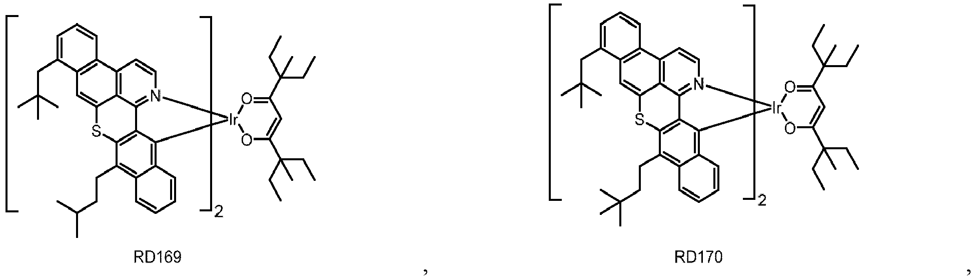

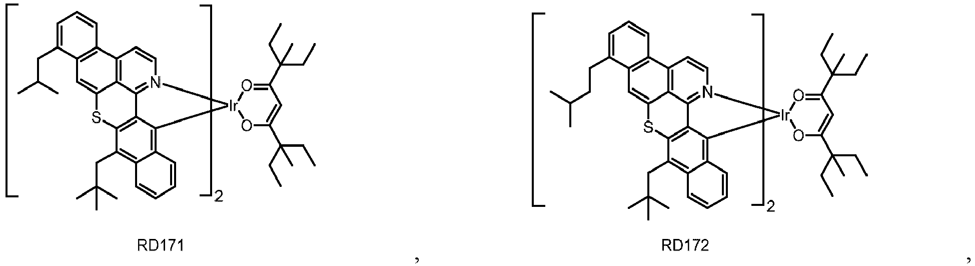

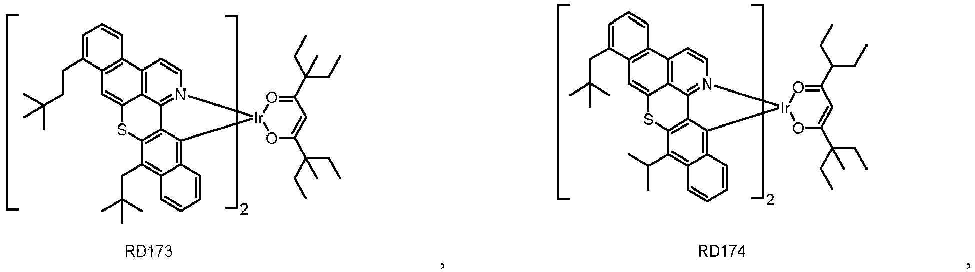

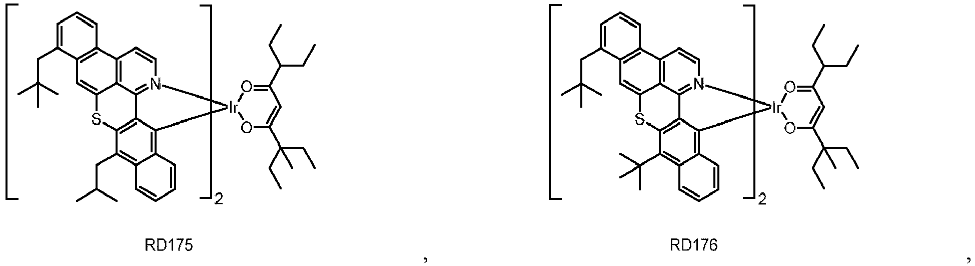

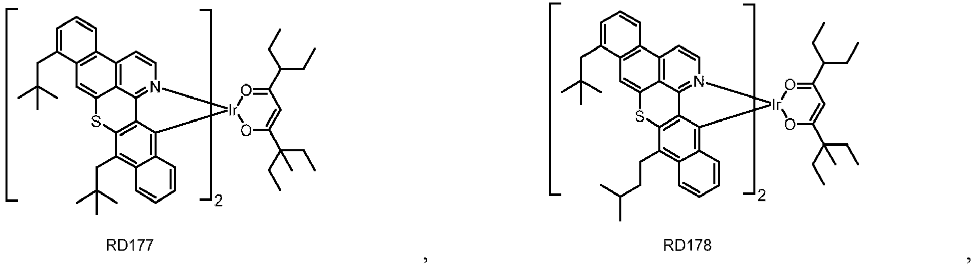

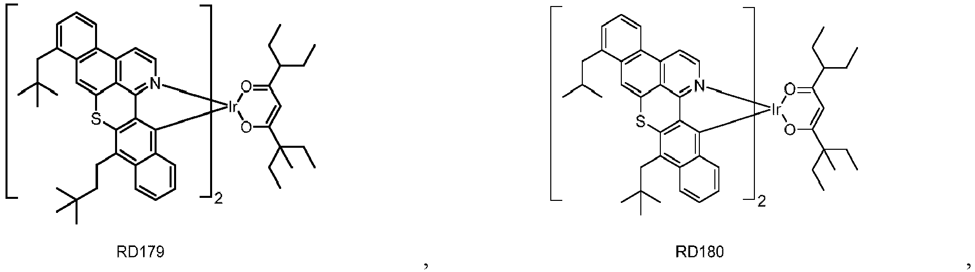

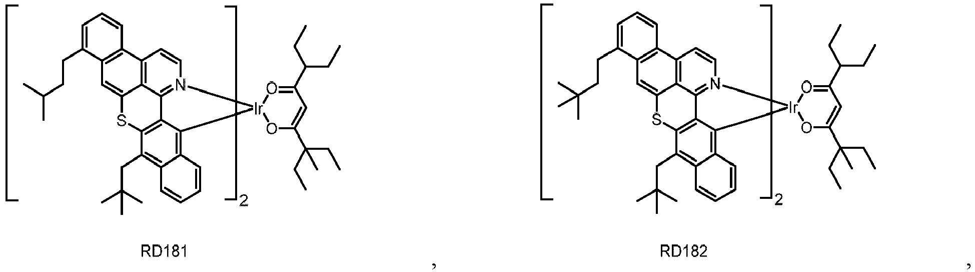

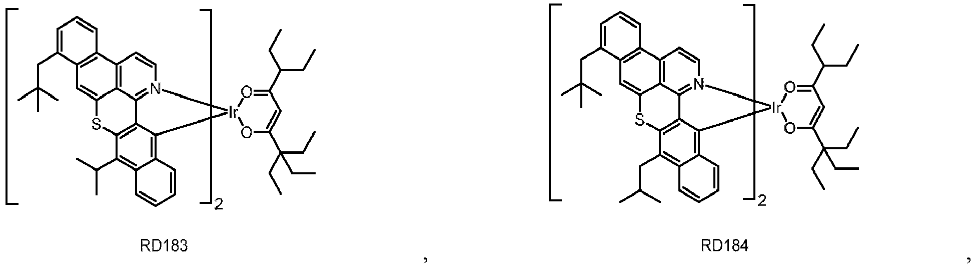

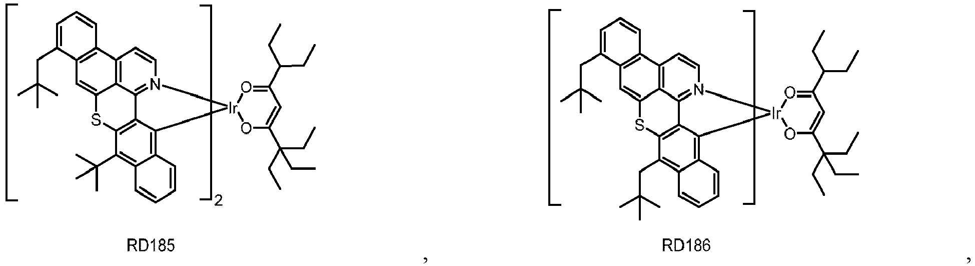

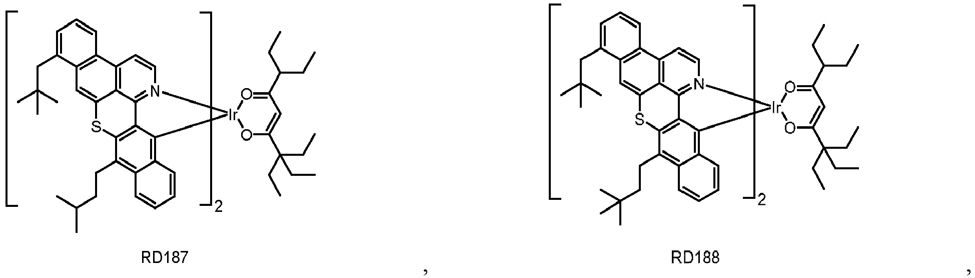

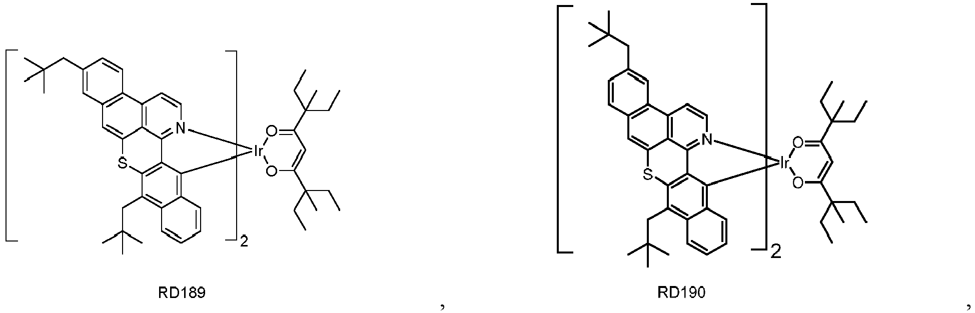

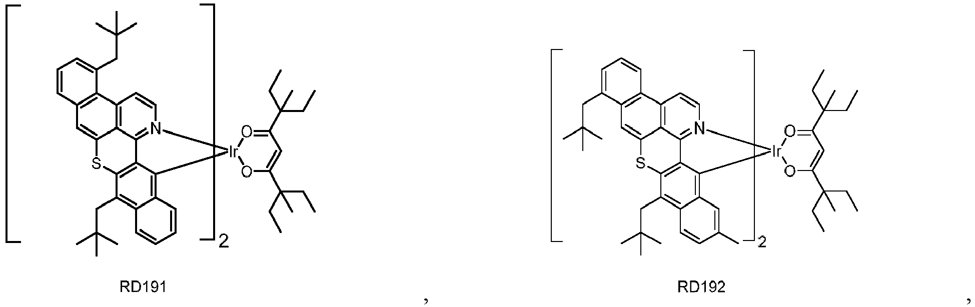

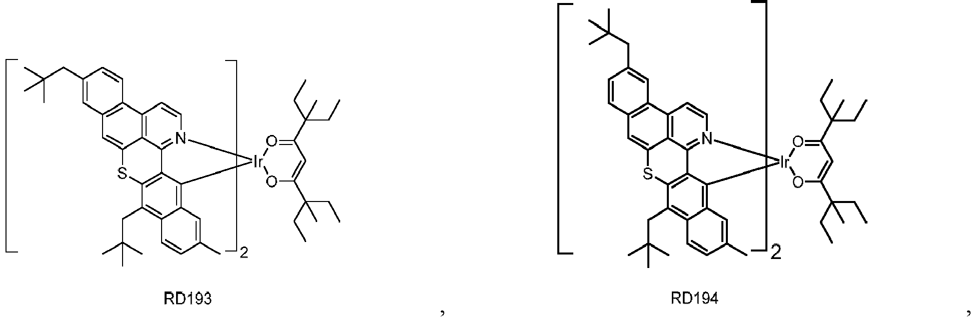

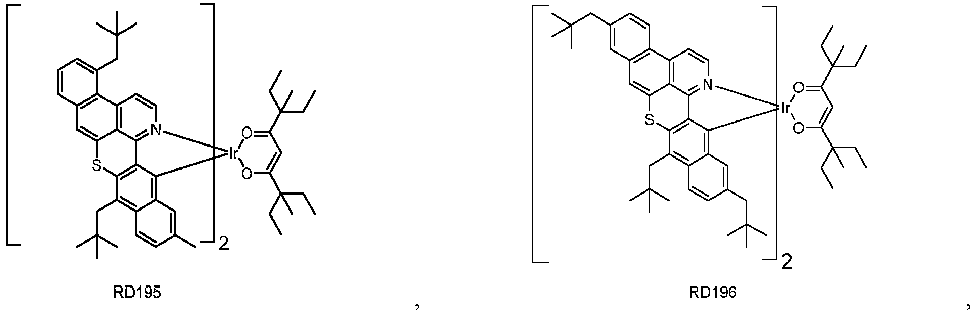

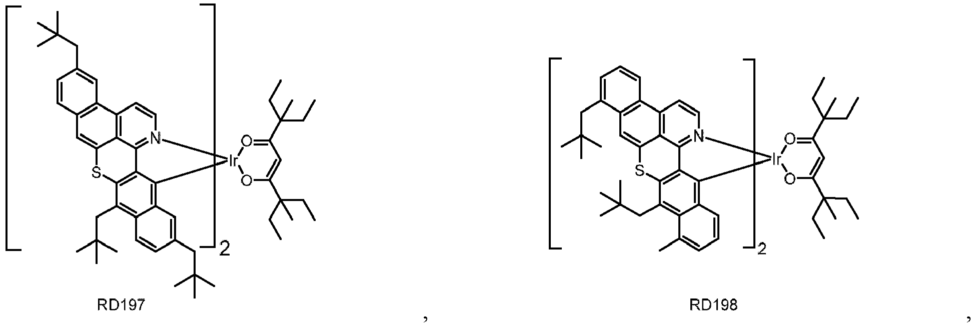

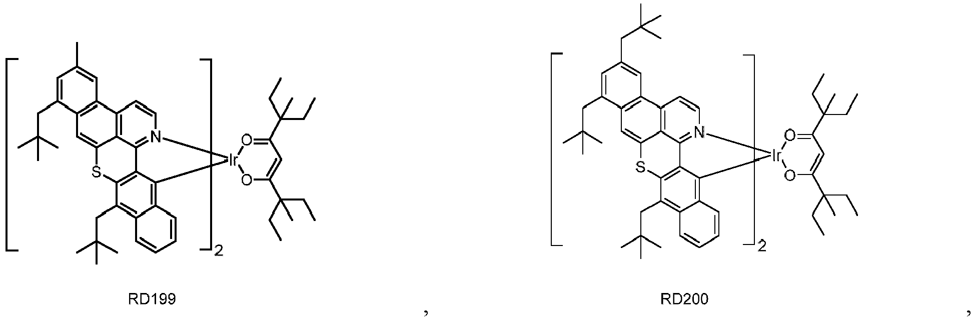

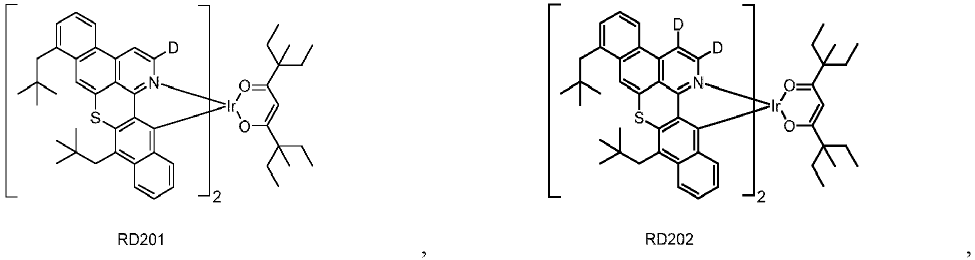

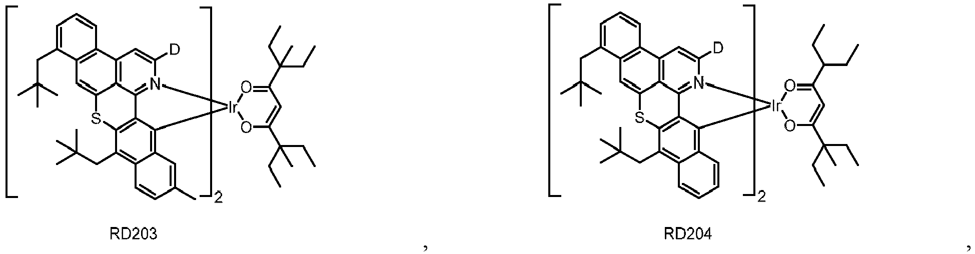

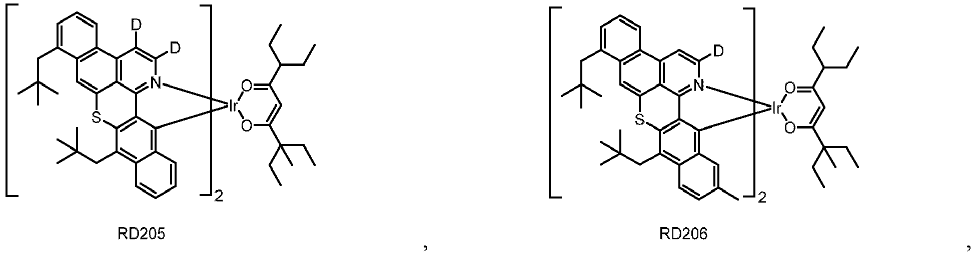

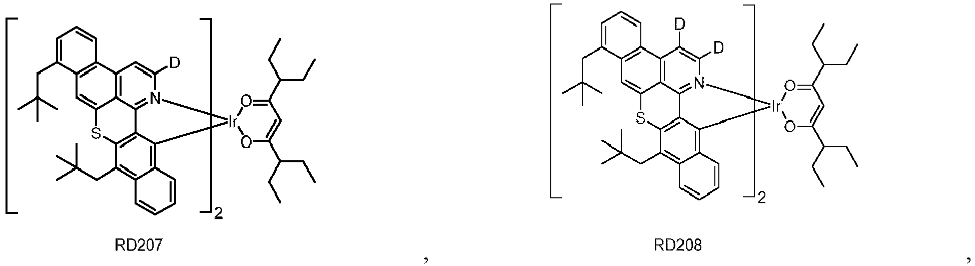

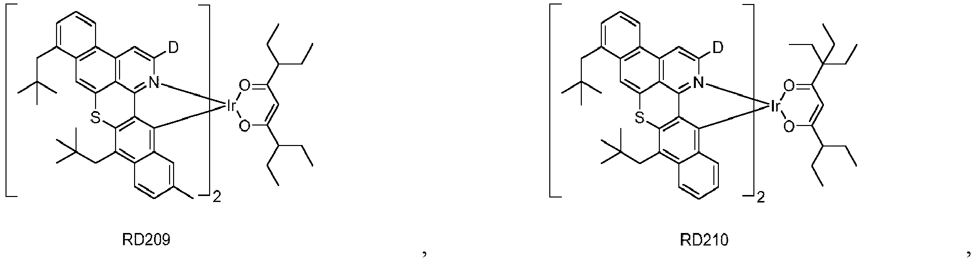

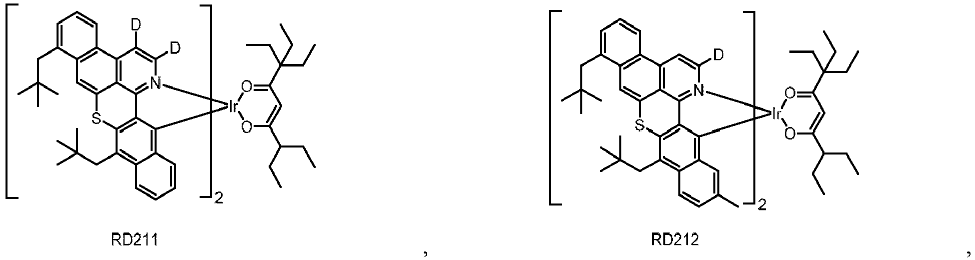

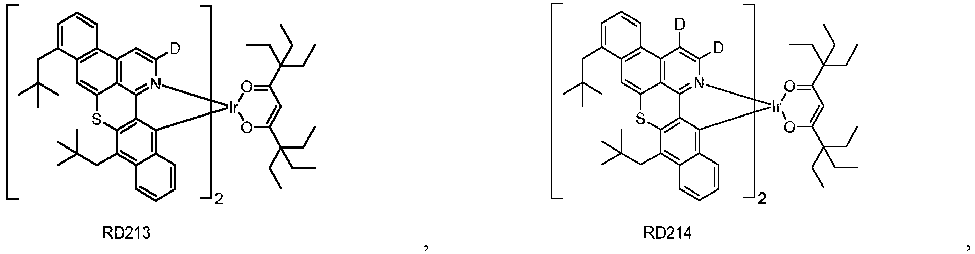

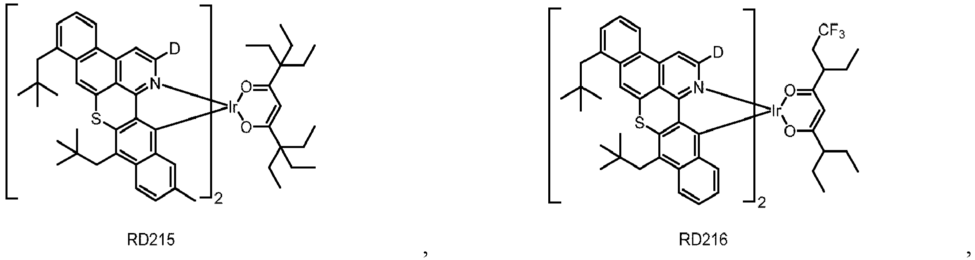

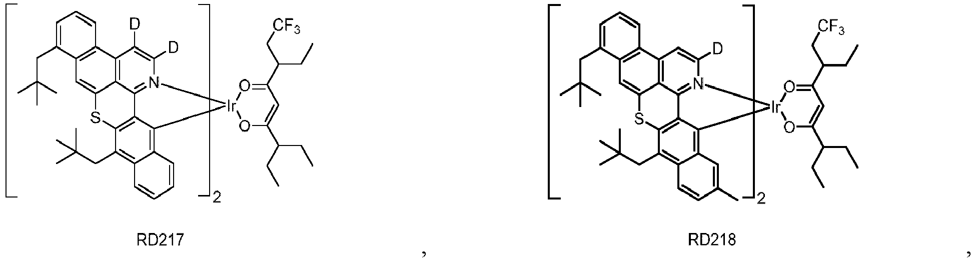

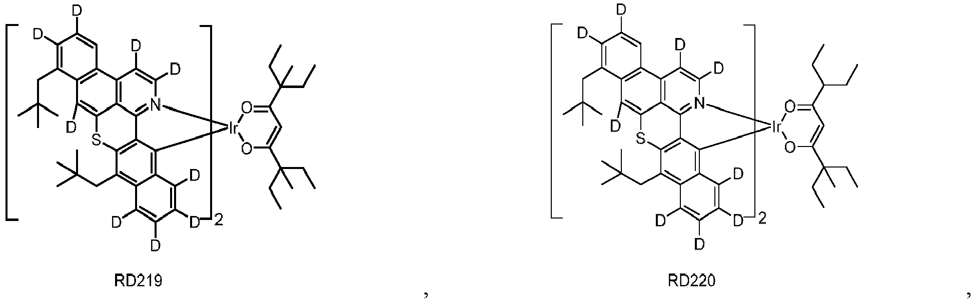

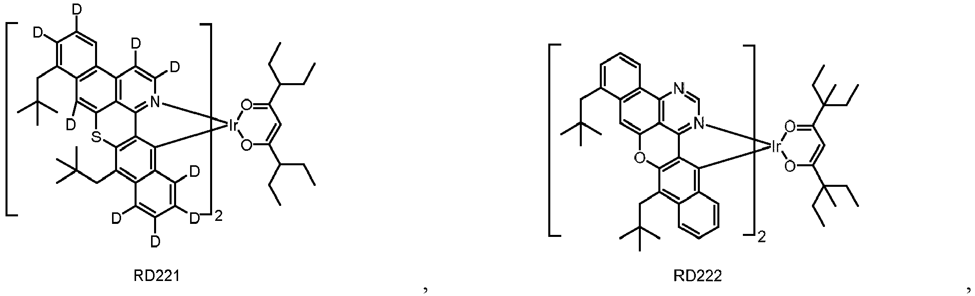

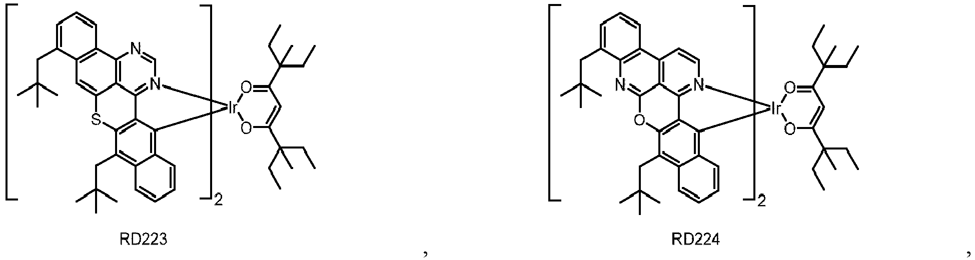

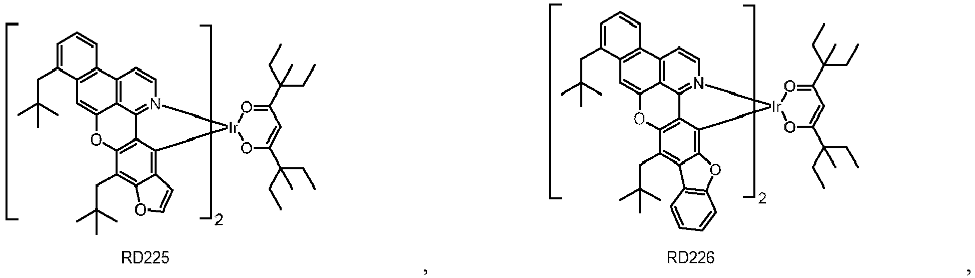

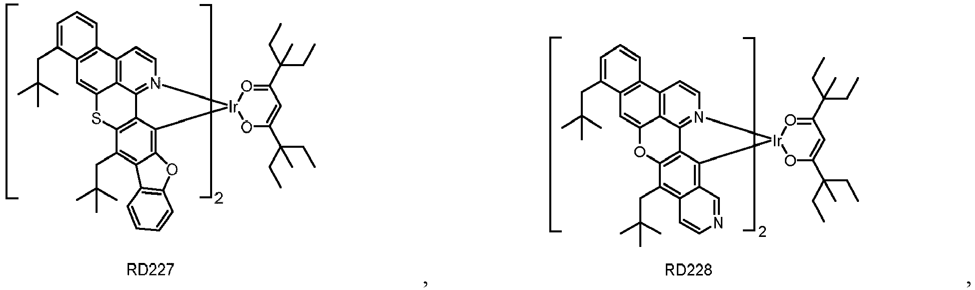

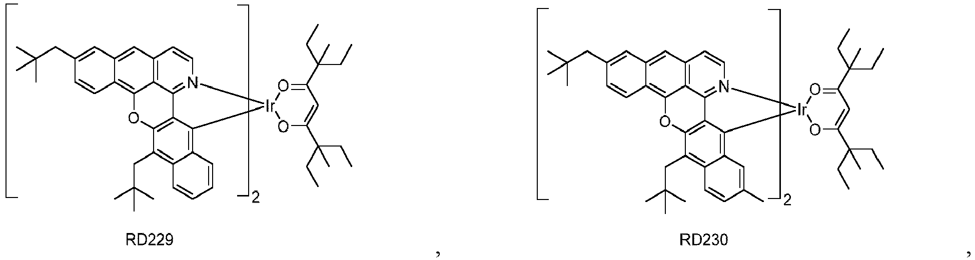

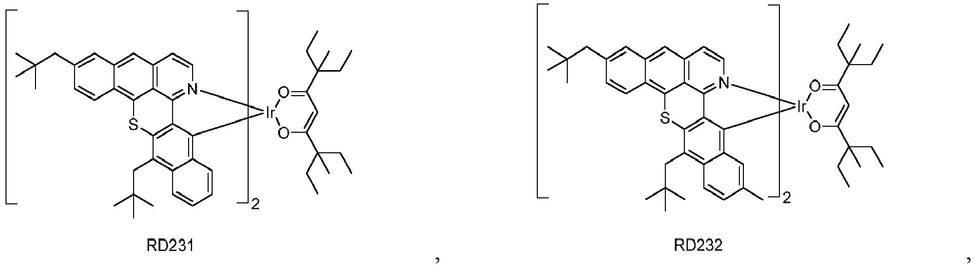

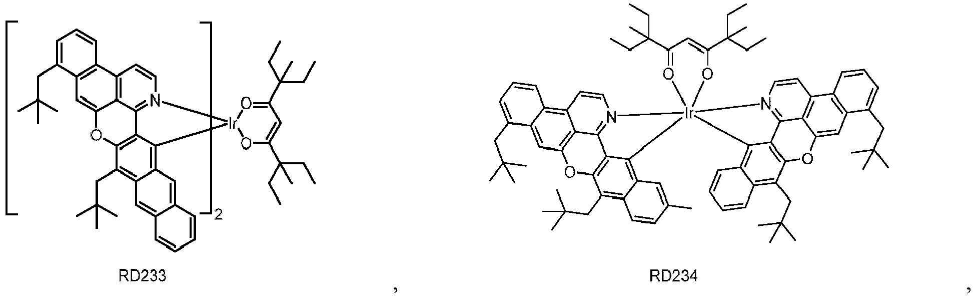

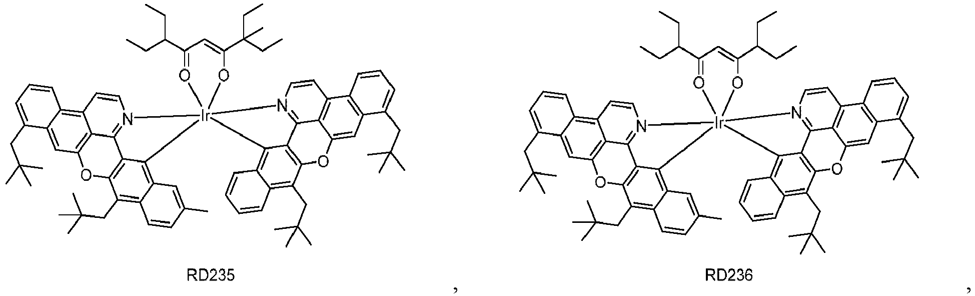

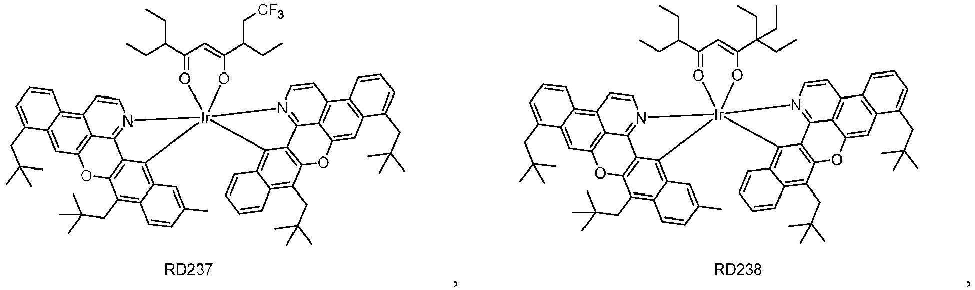

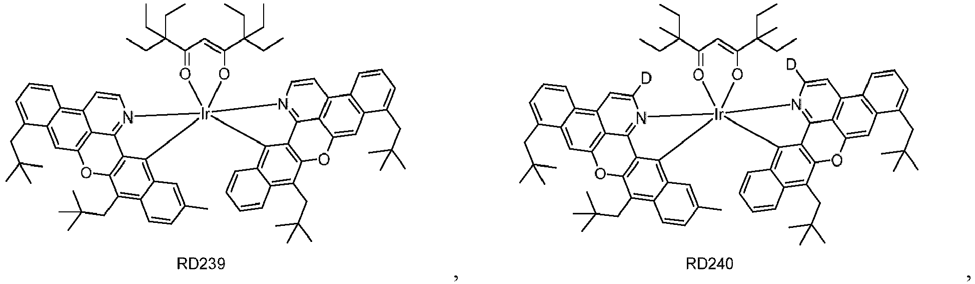

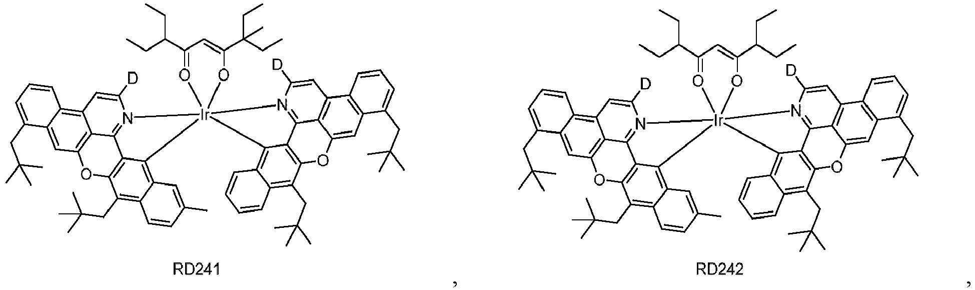

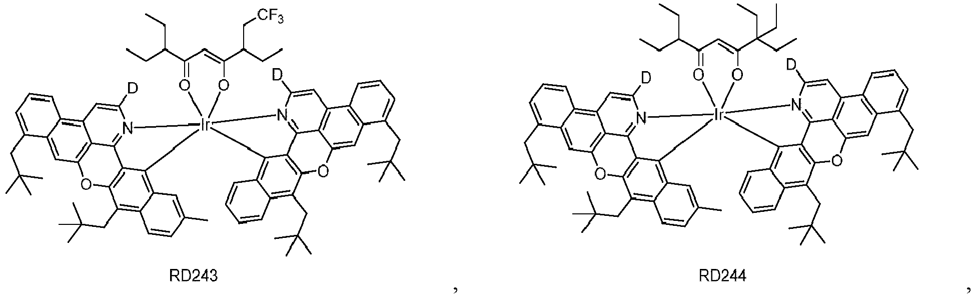

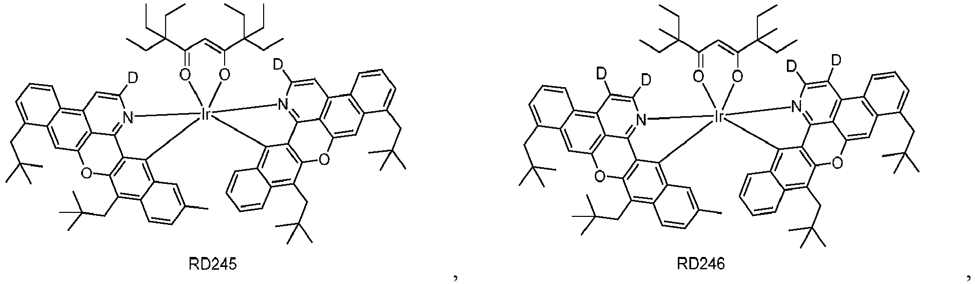

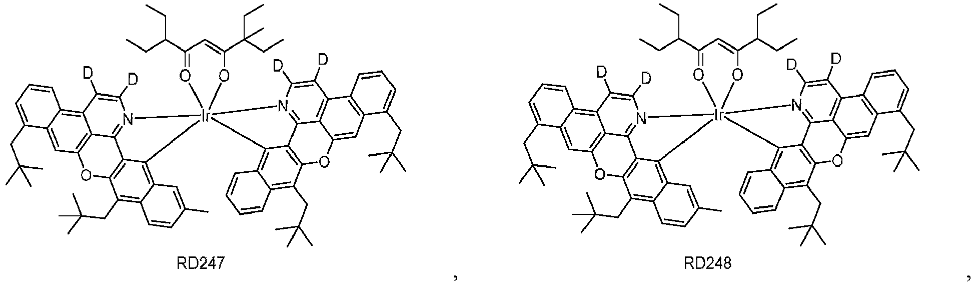

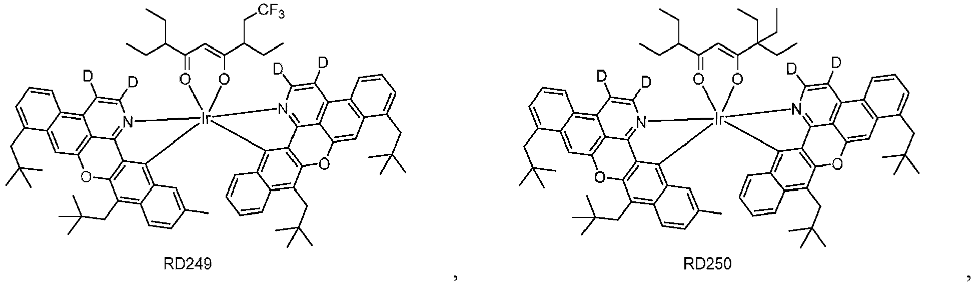

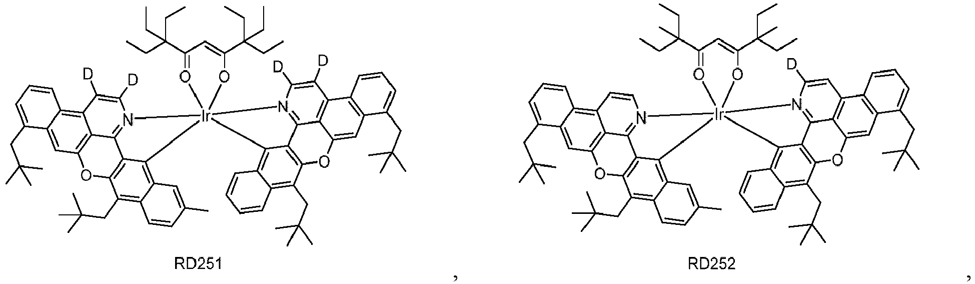

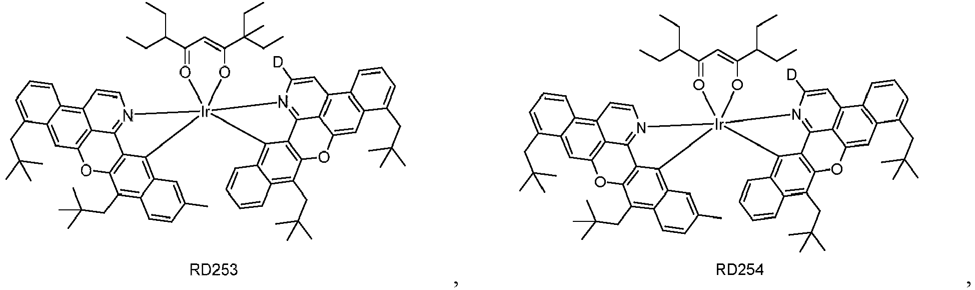

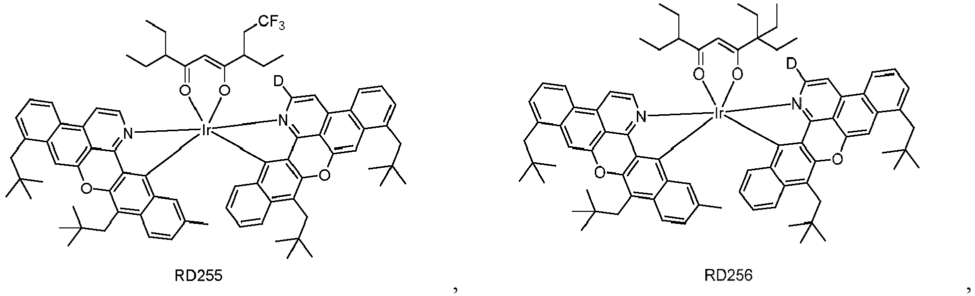

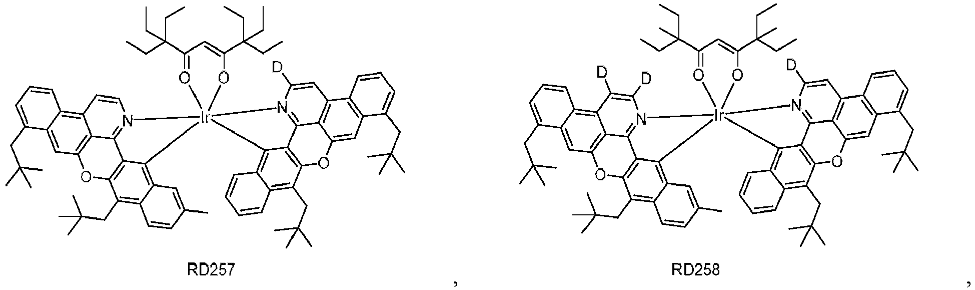

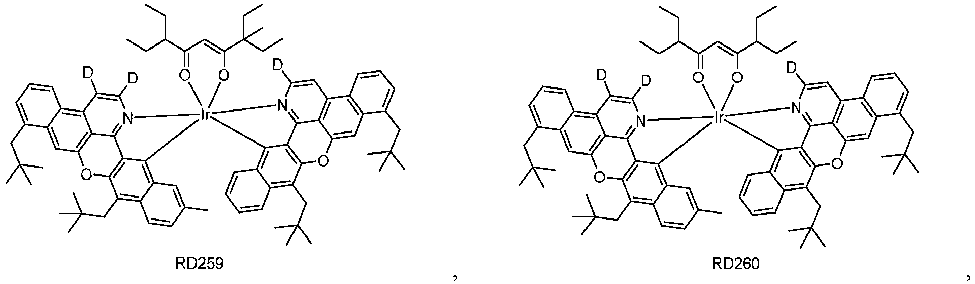

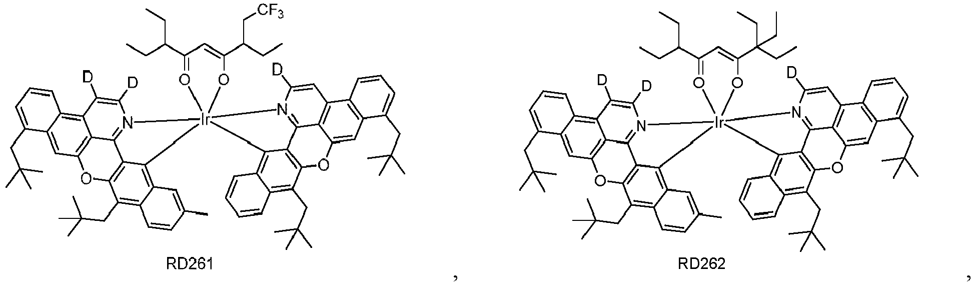

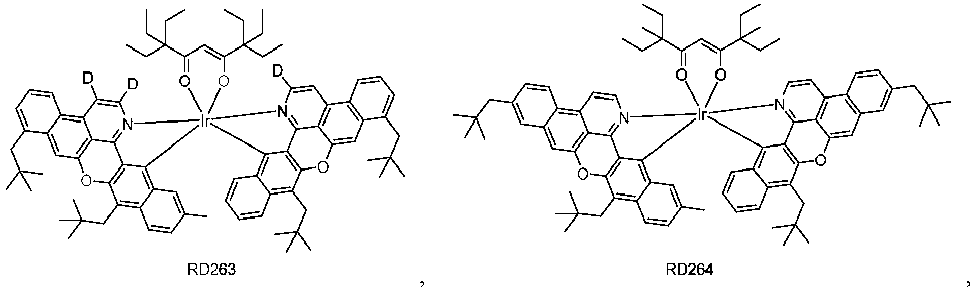

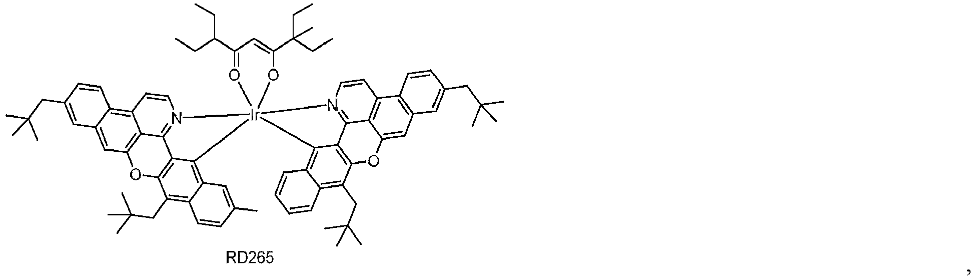

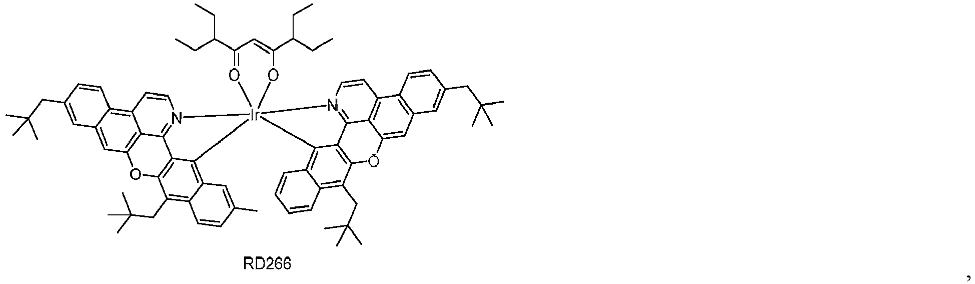

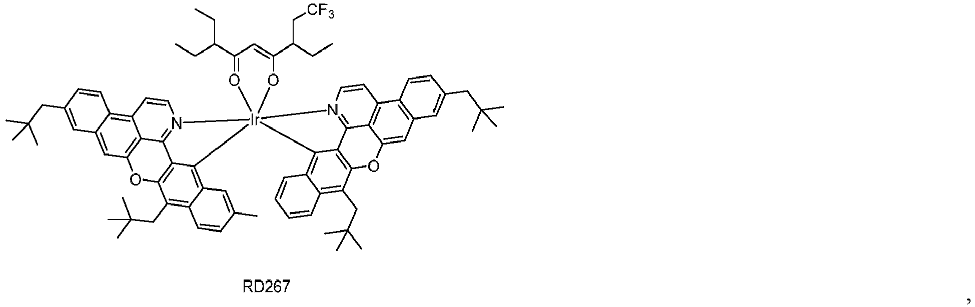

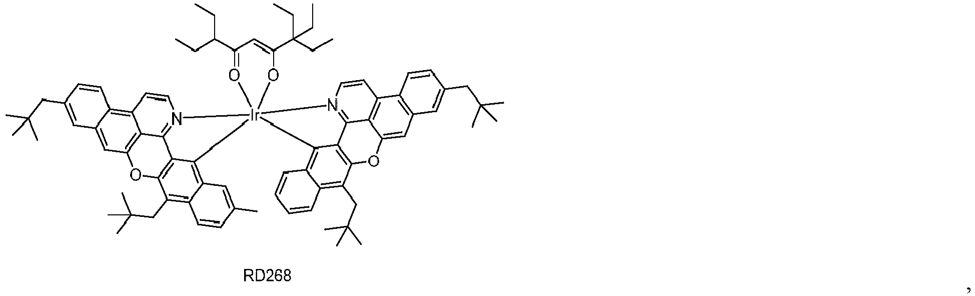

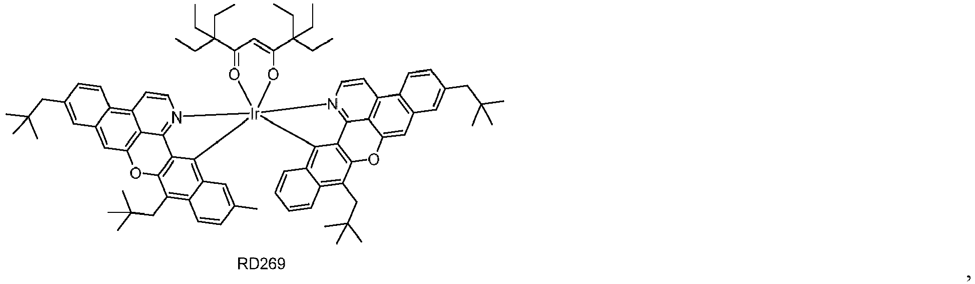

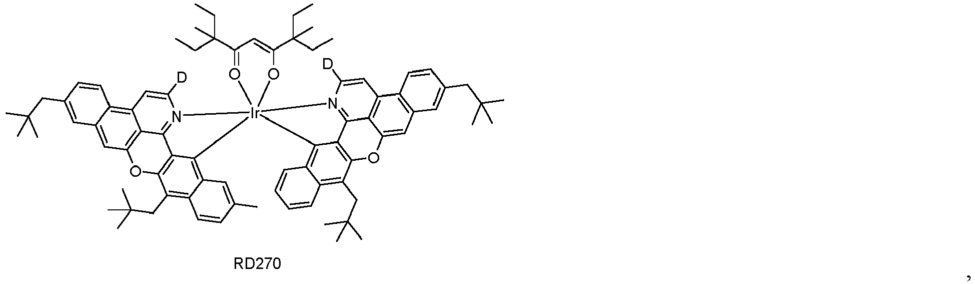

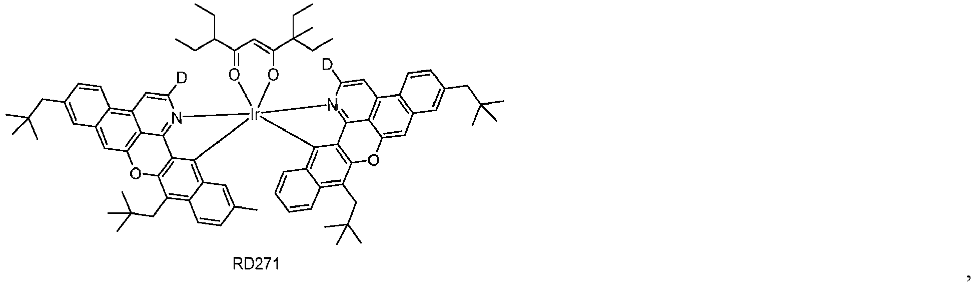

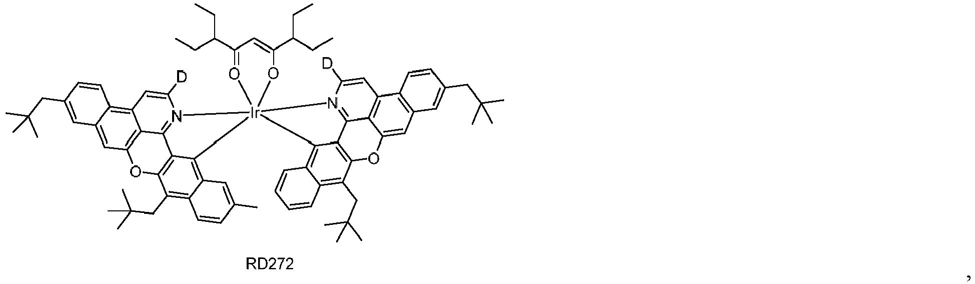

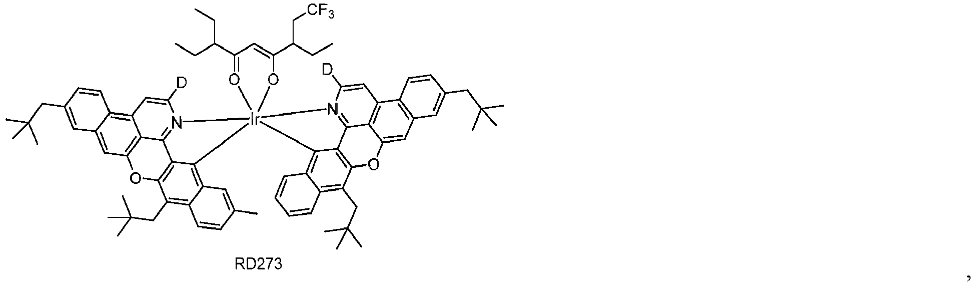

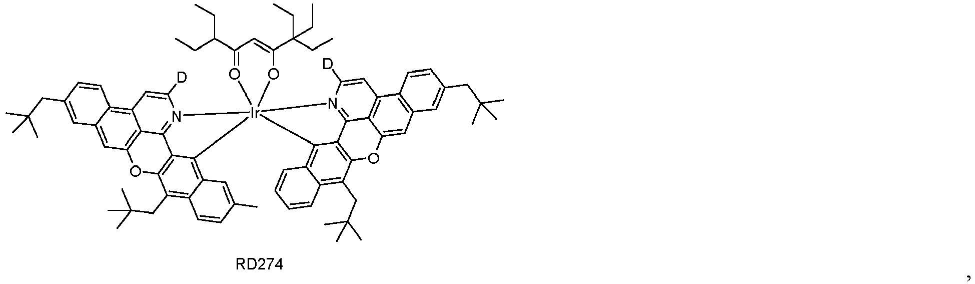

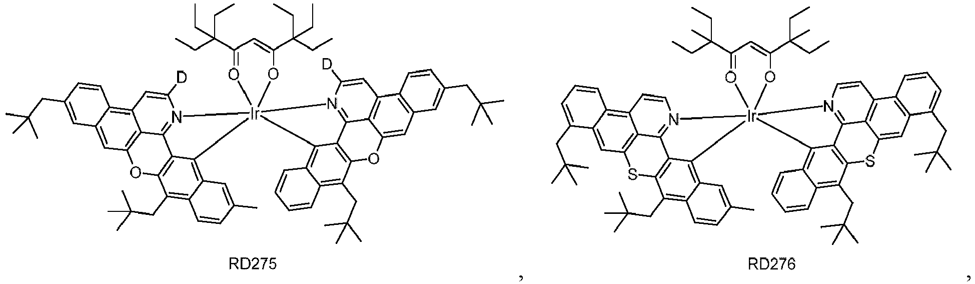

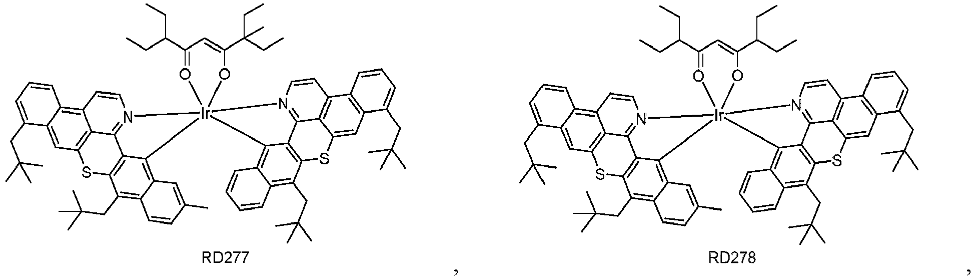

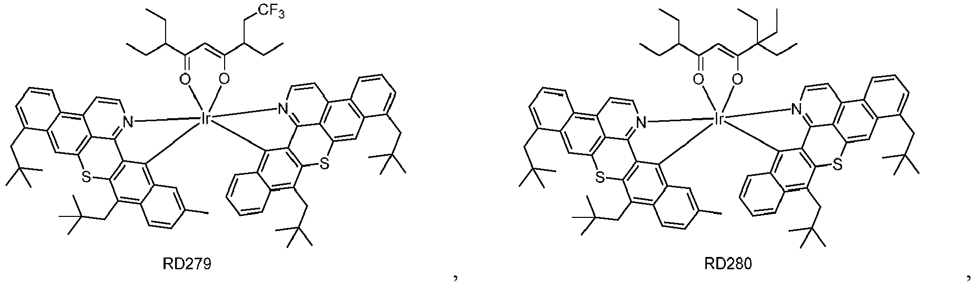

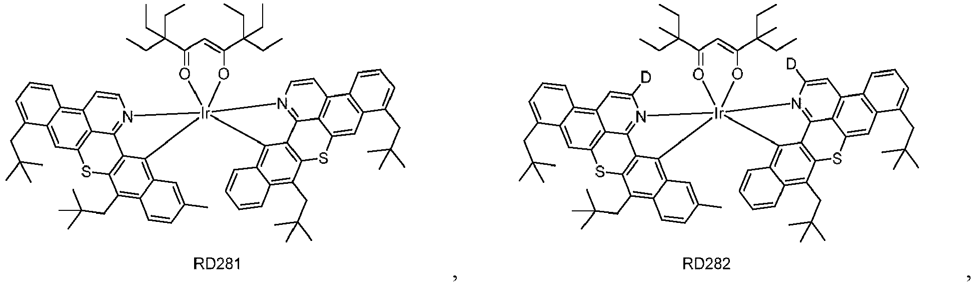

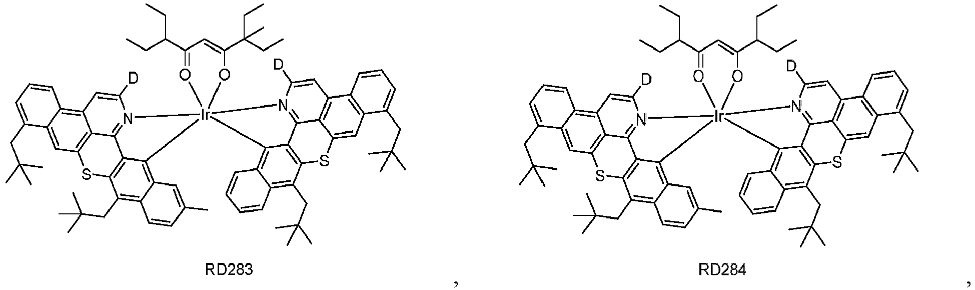

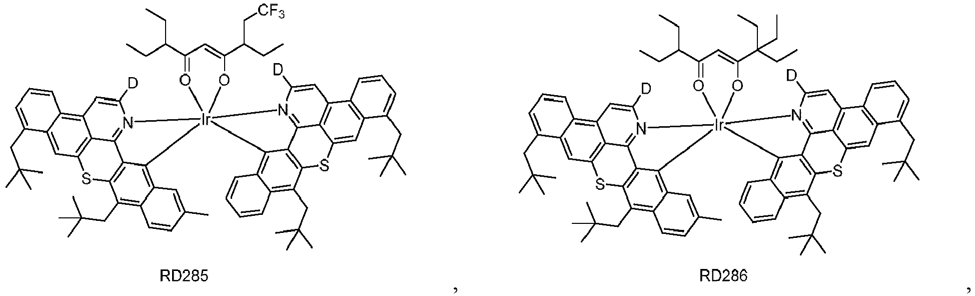

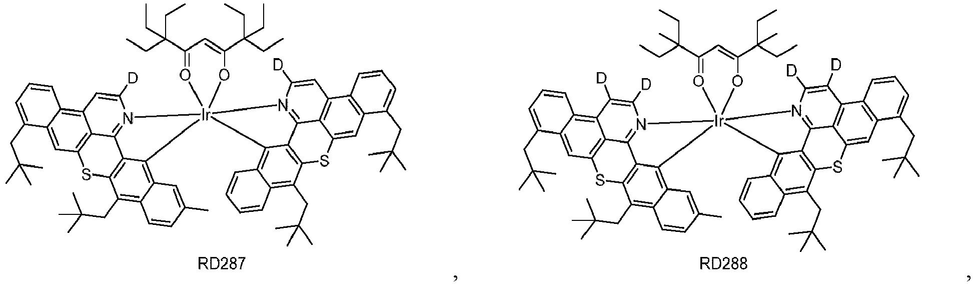

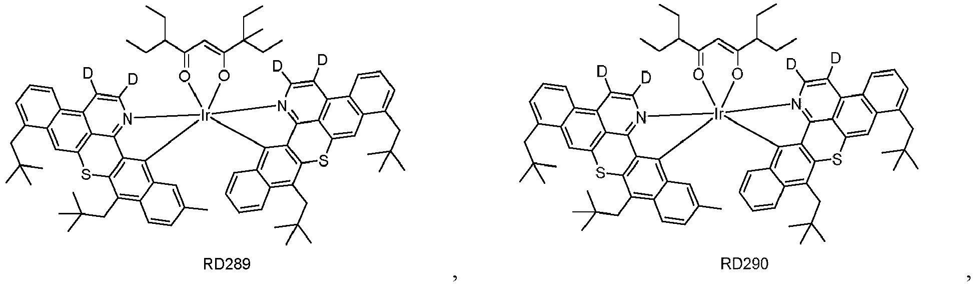

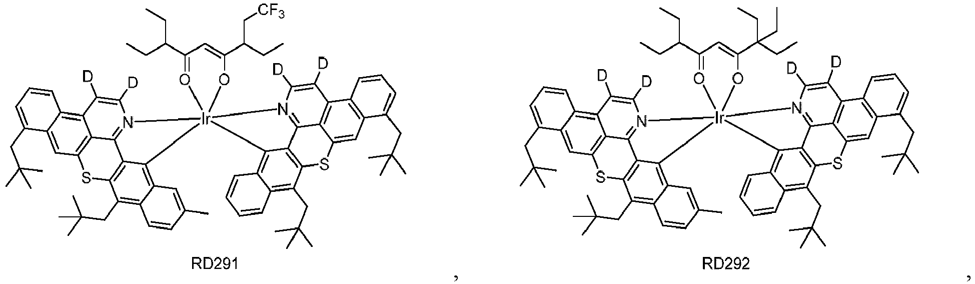

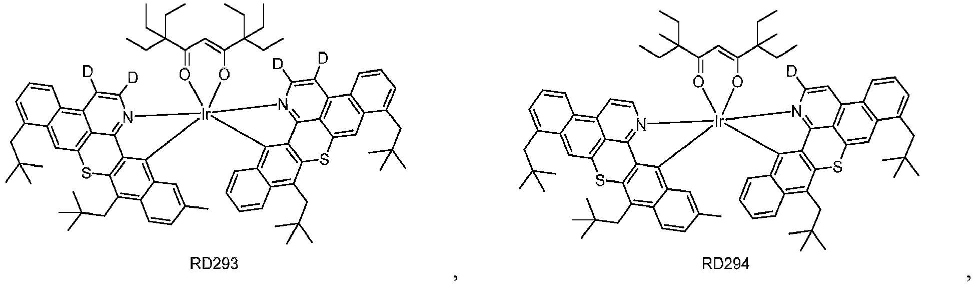

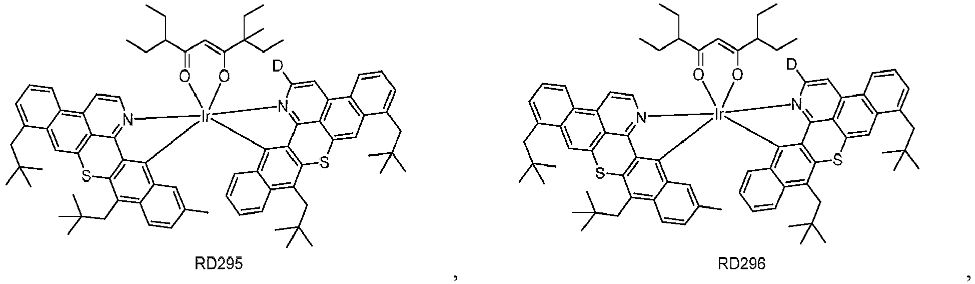

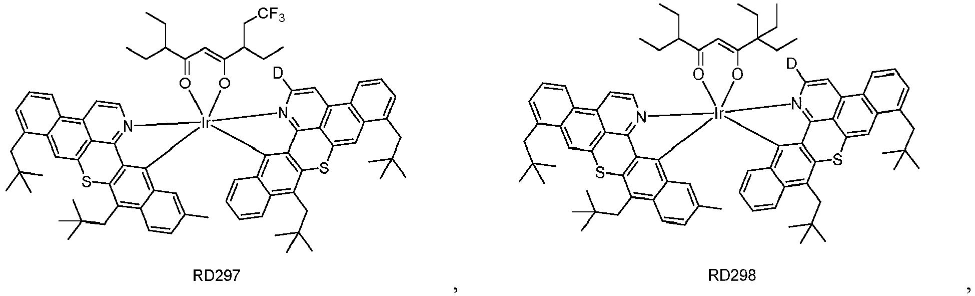

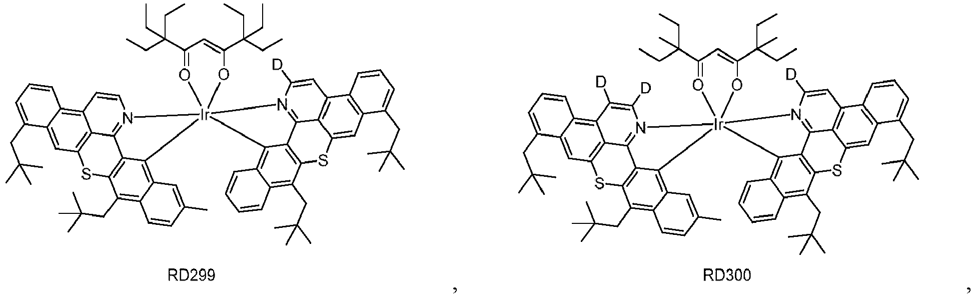

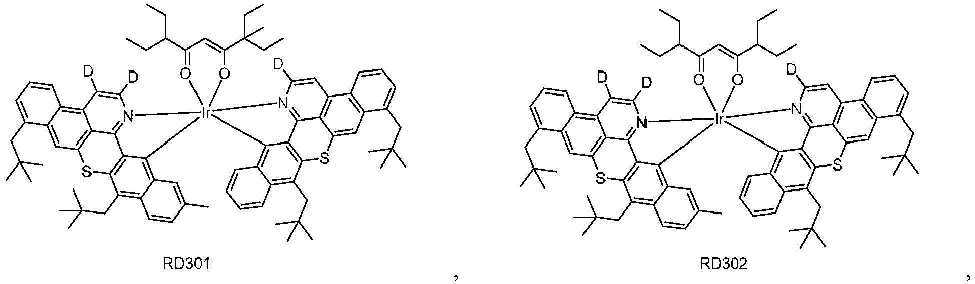

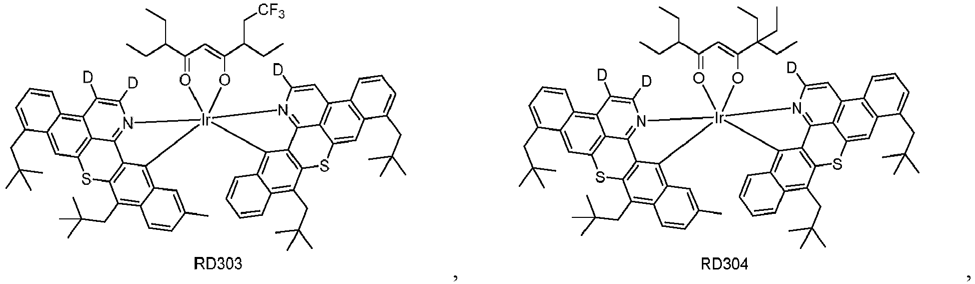

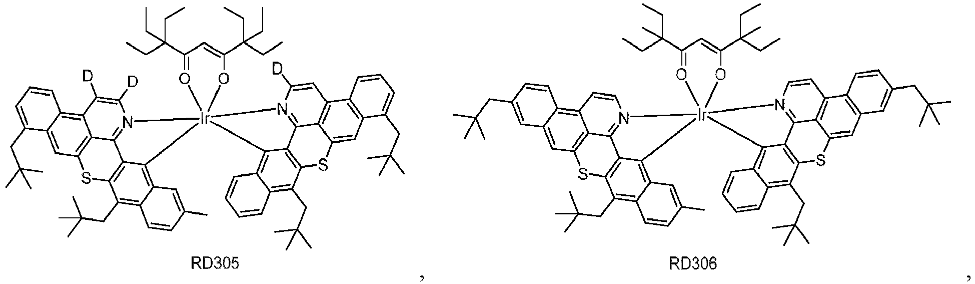

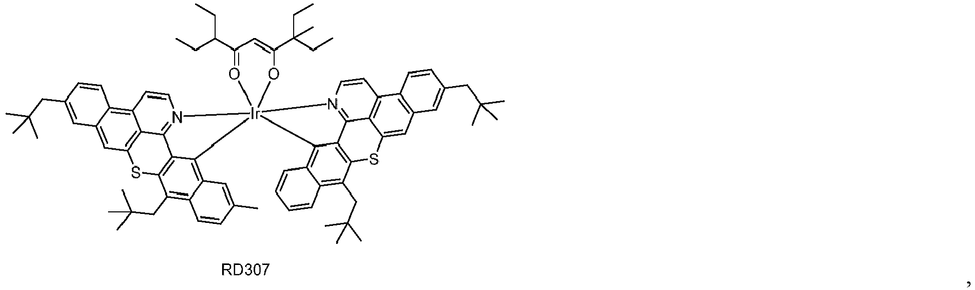

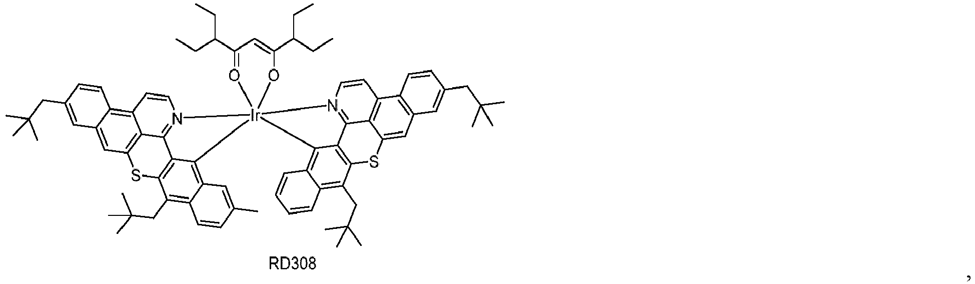

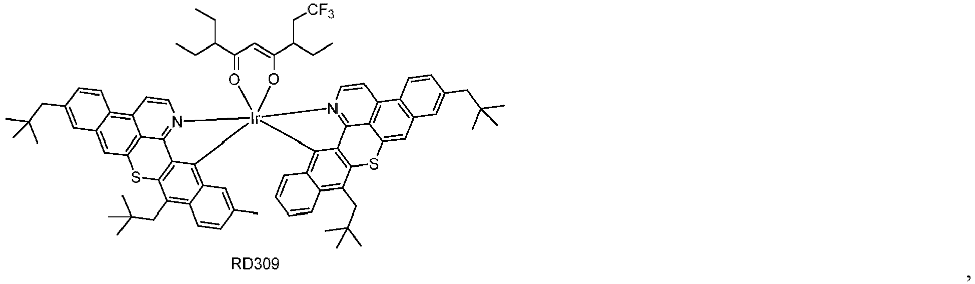

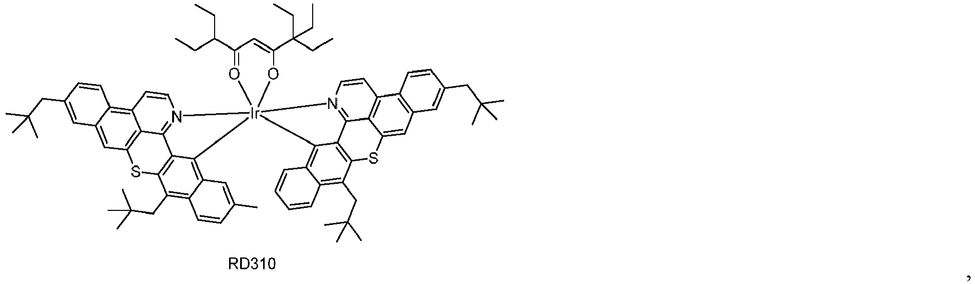

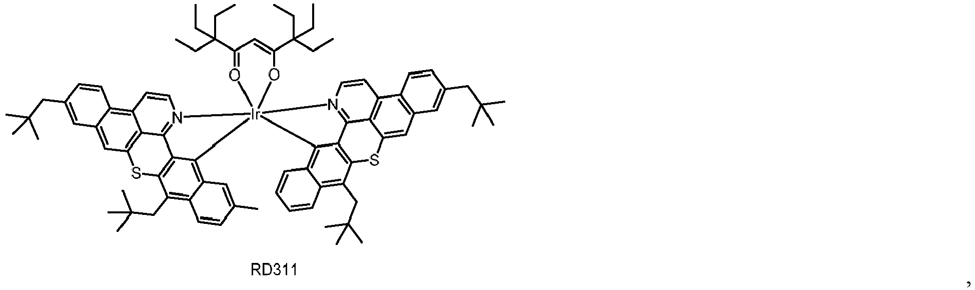

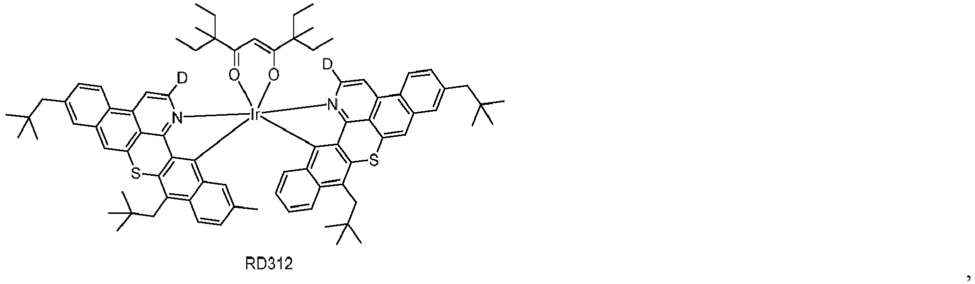

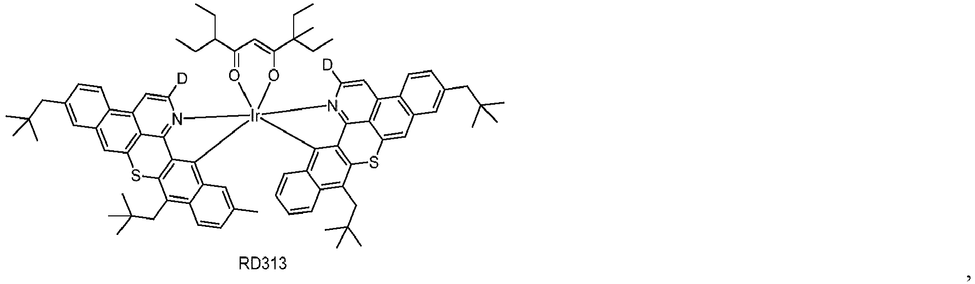

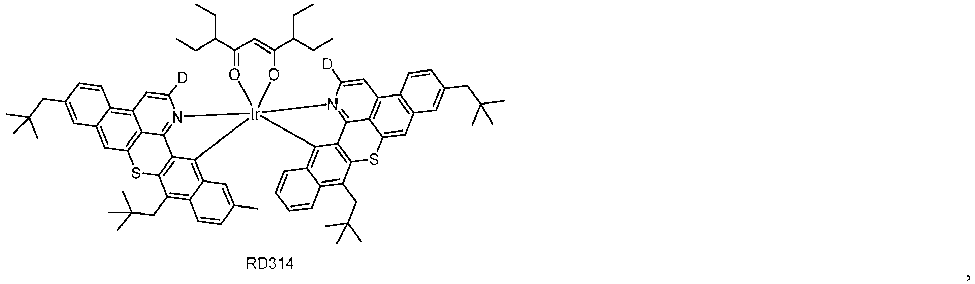

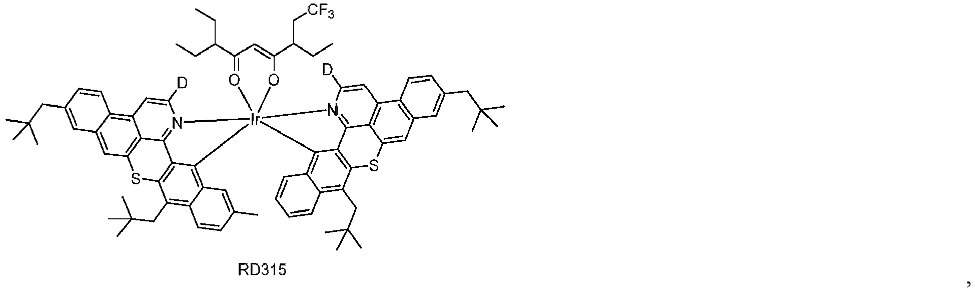

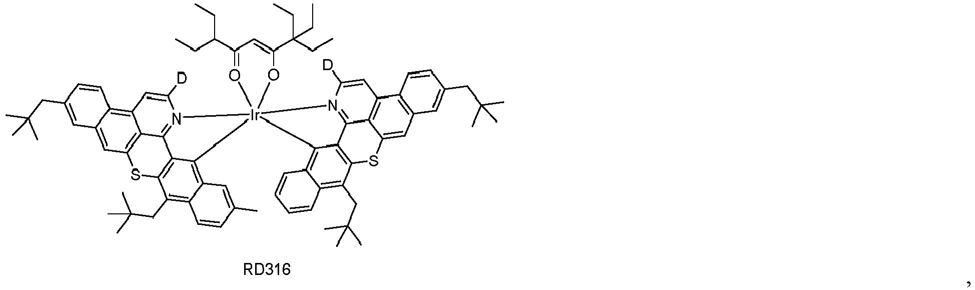

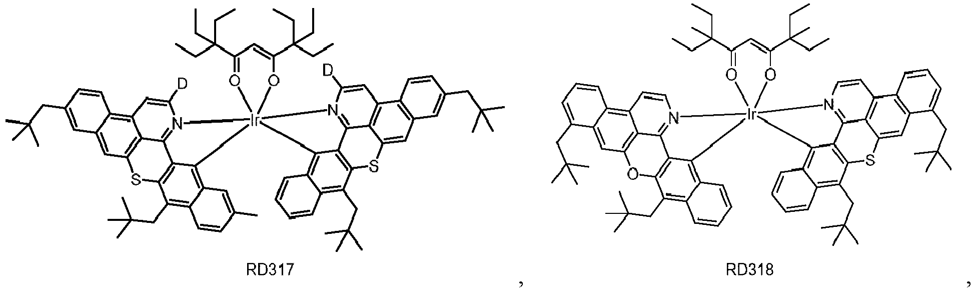

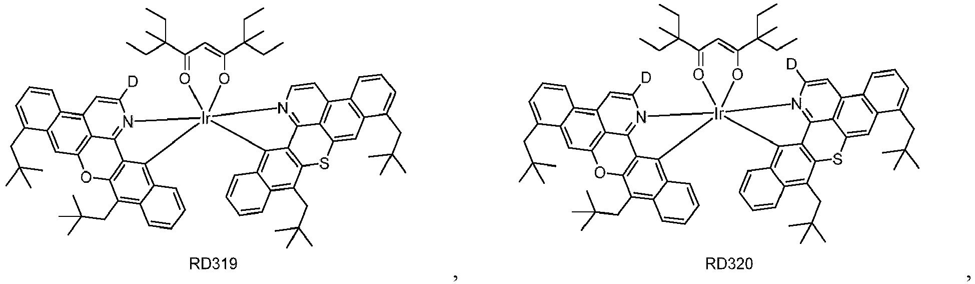

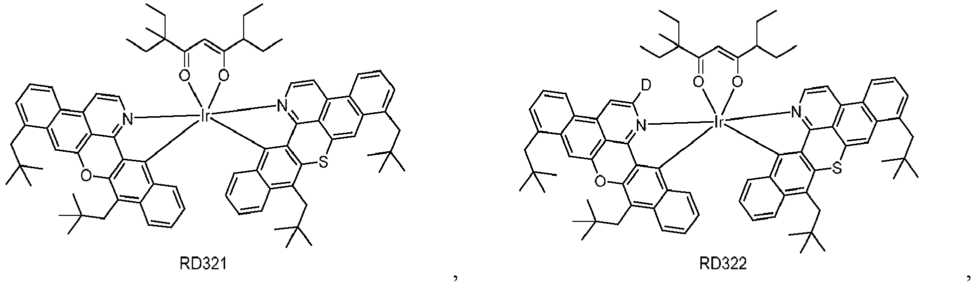

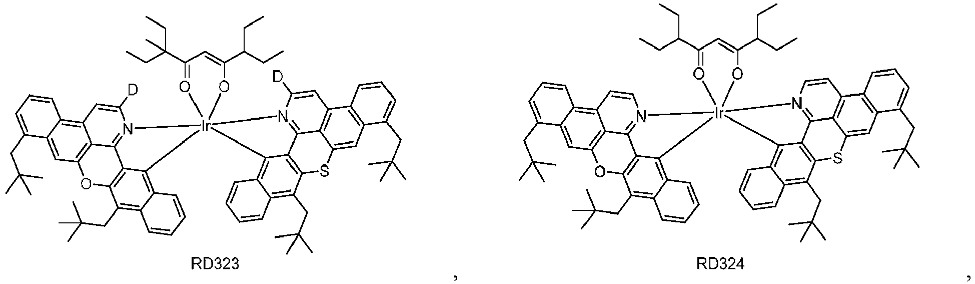

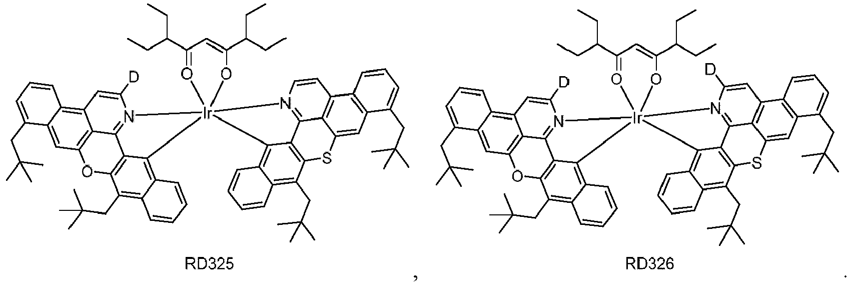

According to an embodiment of the present disclosure, in the organic electroluminescent device, the light-emitting layer further comprises a second host compound, wherein the second host compound comprises at least one chemical group selected from the group consisting of: benzene, pyridine, pyrimidine, triazine, carbazole, azacarbazole, indolocarbazole, dibenzothiophene, aza-dibenzothiophene, dibenzofuran, azadibenzofuran, dibenzoselenophene, triphenylene, azatriphenylene, fluorene, silafluorene, naphthalene, quinoline, isoquinoline, quinazoline, quinoxaline, phenanthrene, azaphenanthrene, and combinations thereof.

According to an embodiment of the present disclosure, in the organic electroluminescent device, the light-emitting layer further comprises a second host compound, wherein the second host compound comprises at least one chemical group selected from the group consisting of: benzene, carbazole, indolocarbazole, dibenzothiophene, dibenzofuran, fluorene, silafluorene, and combinations thereof.

According to an embodiment of the present disclosure, the second host compound has a structure represented by Formula 12 or Formula 13: 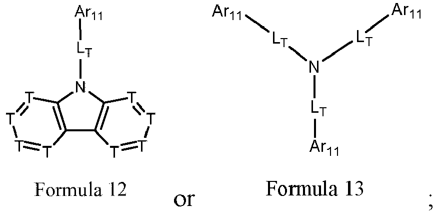 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from C, CR_{w}, or N;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof;
R_{w} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof; and
adjacent substituents R_{w} can be optionally joined to form a ring.

Herein, the expression that adjacent substituents R_{w} can be optionally joined to form a ring is intended to mean that any one or more of groups of adjacent substituents, such as any two substituents R_{w}, may be joined to form a ring. Obviously, it is also possible that none of these substituents are joined to form a ring.

According to an embodiment of the present disclosure, the second host compound has a structure represented by Formula 12-1: 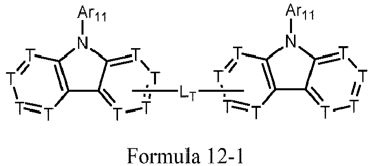 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from C, CR_{w}, or N;
R_{w} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w} can be optionally joined to form a ring.

According to an embodiment of the present disclosure, in the organic electroluminescent device, the second host compound has a structure represented by Formula 12-2: 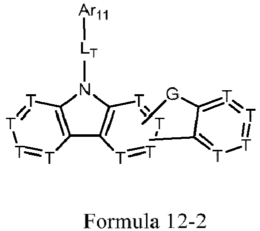 wherein
G is, at each occurrence identically or differently, selected from C(R_{g})₂, NR_{g}, O, or S;
T is, at each occurrence identically or differently, selected from C, CR_{w}, or N;
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
R_{w} and R_{g} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w}, R_{g} can be optionally joined to form a ring.

Herein, the expression that adjacent substituents R_{w}, R_{g} can be optionally joined to form a ring is intended to mean that any one or more of groups of adjacent substituents, such as two substituents R_{w}, two substituents R_{g}, and substituents R_{w} and R_{g}, may be joined to form a ring. Obviously, it is also possible that none of these substituents are joined to form a ring.

According to an embodiment of the present disclosure, the second host compound has a structure represented by Formula 12-3: 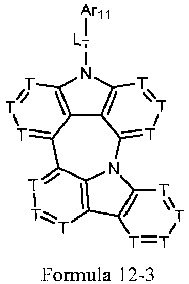 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from C, CR_{w}, or N;
R_{w} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w} can be optionally joined to form a ring.

According to an embodiment of the present disclosure, in Formula 12-1, Formula 12-2, and Formula 12-3, T is, at each occurrence identically or differently, selected from C or CR_{w}.

According to an embodiment of the present disclosure, in Formula 12-1, Formula 12-2, and Formula 12-3, T is, at each occurrence identically or differently, selected from C, CR_{w}, or N, and at least one of T is selected from N, for example, one of T is selected from N or two of T are selected from N.

According to an embodiment of the present disclosure, in the organic electroluminescent device, the second host compound has a structure represented by one of Formula 12-a to Formula 12-j: 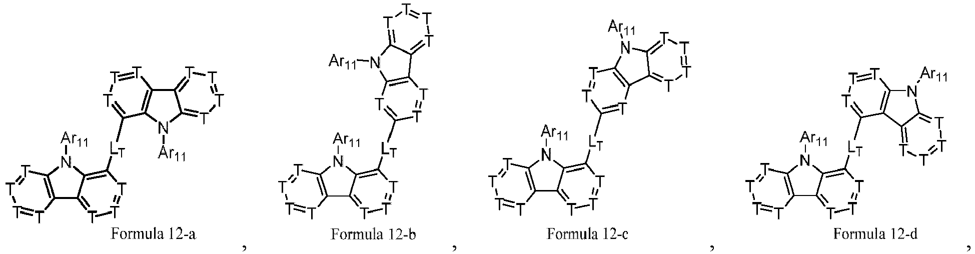 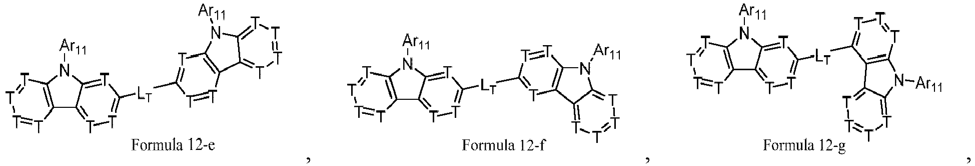 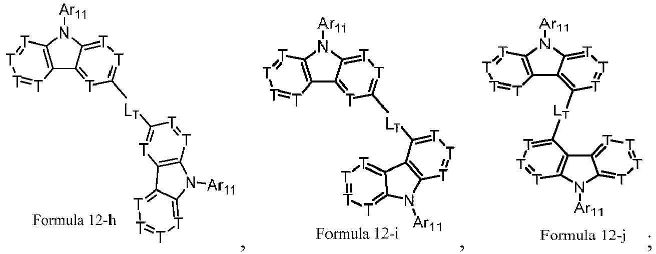 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from CR_{w} or N;
R_{w} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w} can be optionally joined to form a ring.

According to an embodiment of the present disclosure, in Formulas 12-a to 12-j, T is, at each occurrence identically or differently, selected from CR_{w}.

According to an embodiment of the present disclosure, in Formulas 12-a to 12-j, T is, at each occurrence identically or differently, selected from CR_{w} or N, and at least one of T is selected from N, for example, one of T is selected from N or two of T are selected from N.

According to an embodiment of the present disclosure, in the organic electroluminescent device, the second host compound has a structure represented by one of Formula 13-a to Formula 13-f: 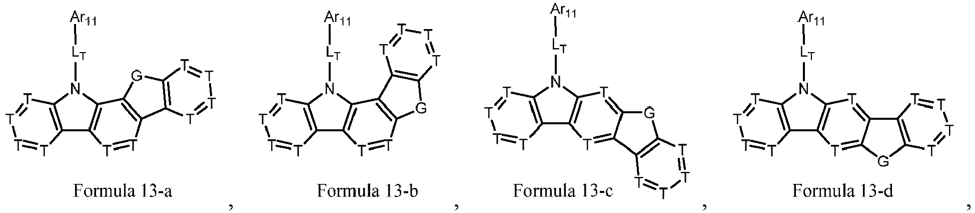 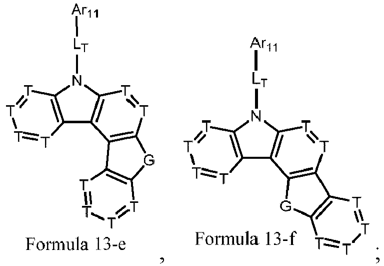 wherein
G is, at each occurrence identically or differently, selected from C(R_{g})₂, NR_{g}, O, or S;
T is, at each occurrence identically or differently, selected from CR_{w} or N;
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
R_{w} and R_{g} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w}, R_{g} can be optionally joined to form a ring.

According to an embodiment of the present disclosure, in Formulas 13-a to 13-f, T is, at each occurrence identically or differently, selected from CR_{w}.

According to an embodiment of the present disclosure, in Formulas 13-a to 13-f, T is, at each occurrence identically or differently, selected from CR_{w} or N, and at least one of T is selected from N, for example, one of T is selected from N or two of T are selected from N.

According to an embodiment of the present disclosure, the second host compound is selected from the group consisting of Compound PH-1 to Compound PH-101, wherein the specific structures of Compound PH-1 to Compound PH-101 are referred to claim 12.

According to an embodiment of the present disclosure, the second host compound is selected from the group consisting of Compound PH-1 to Compound PH-137, wherein the specific structures of Compound PH-1 to Compound PH-101 are referred to claim 12, and the specific structures of Compound PH-102 to Compound PH-137 are shown as follows:

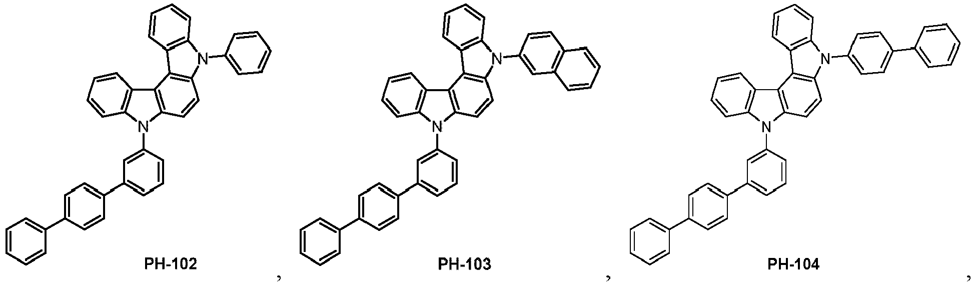

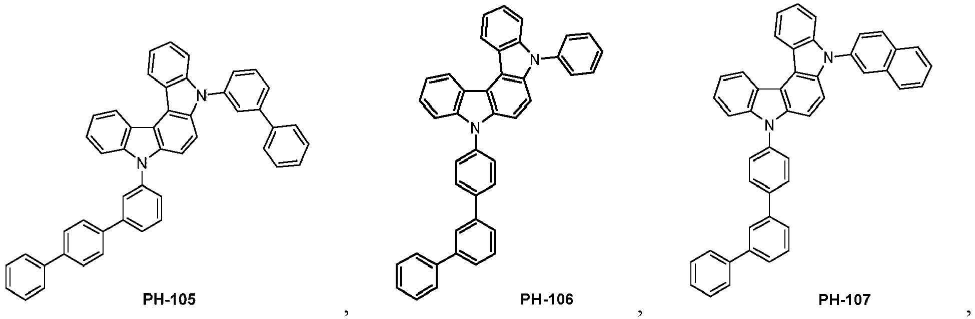

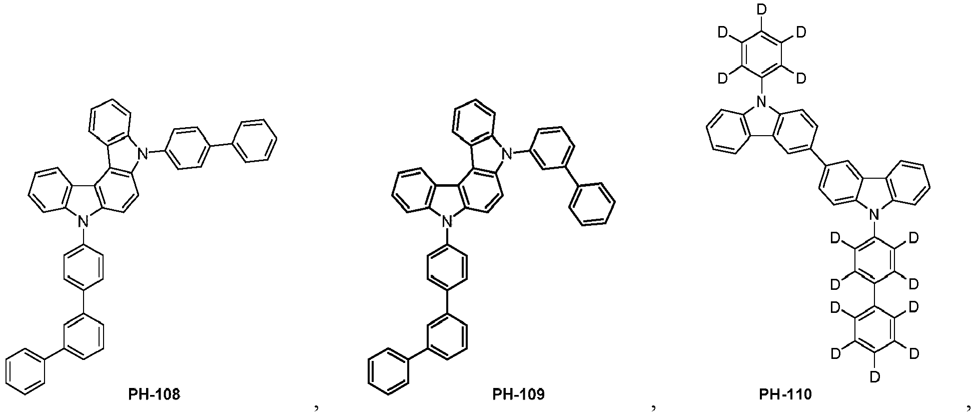

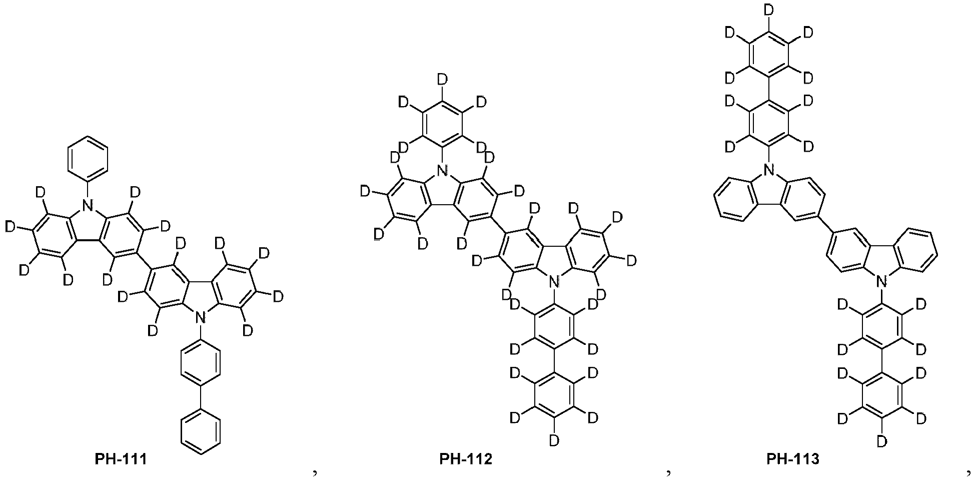

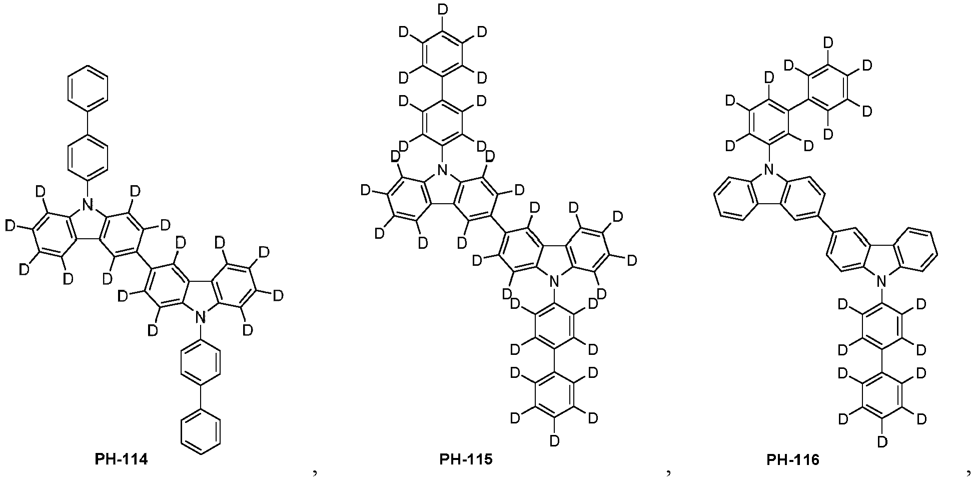

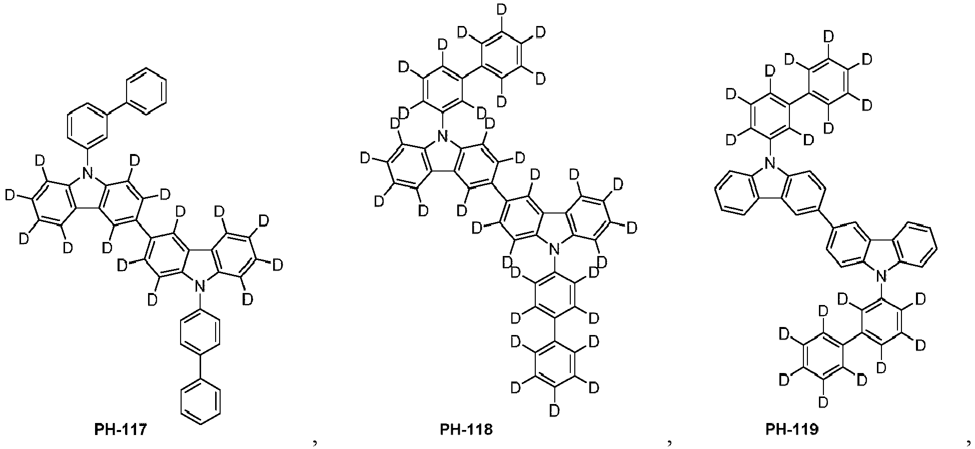

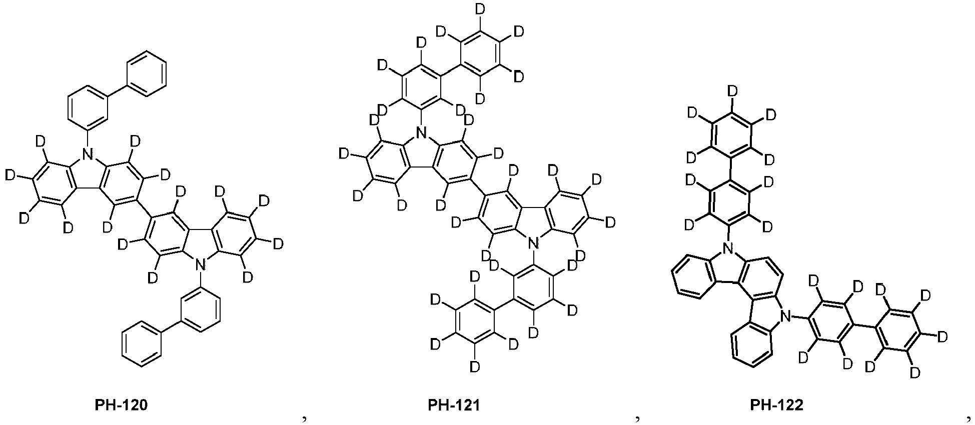

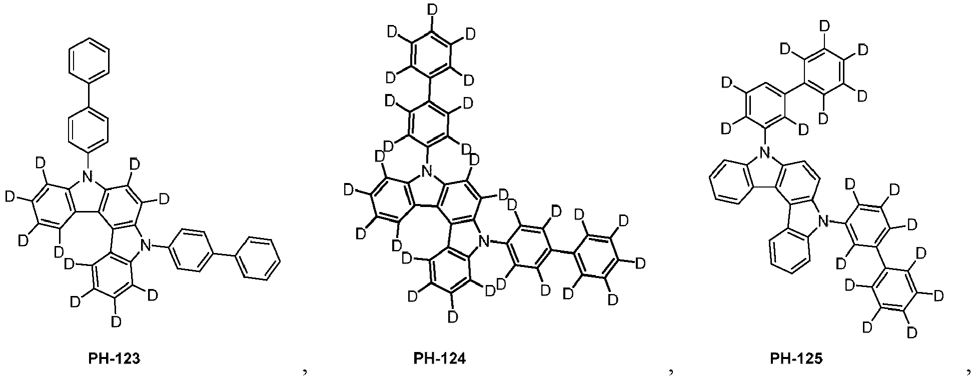

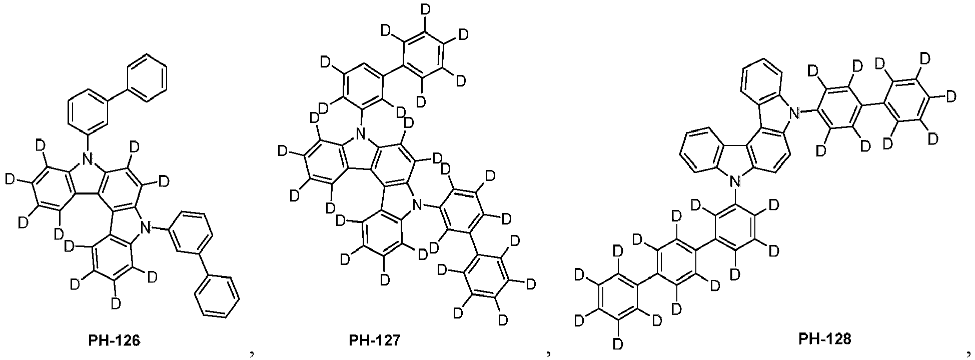

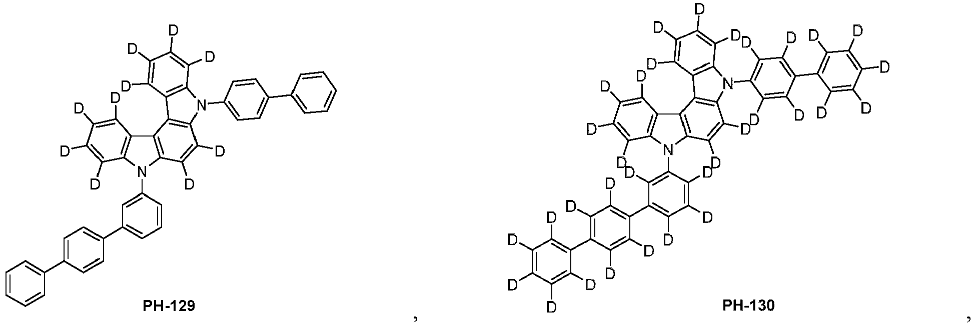

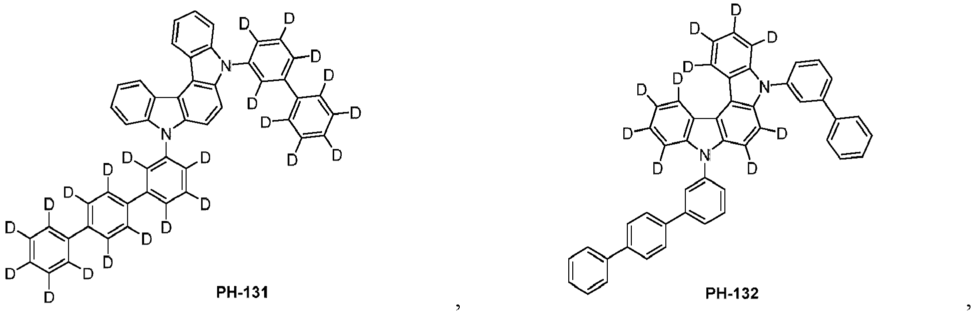

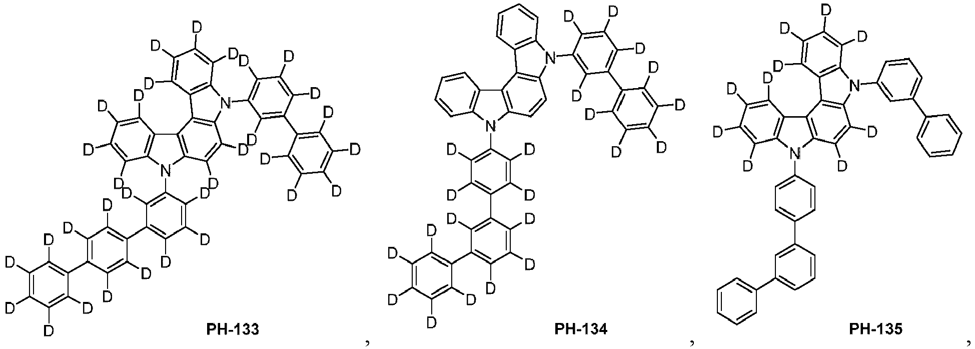

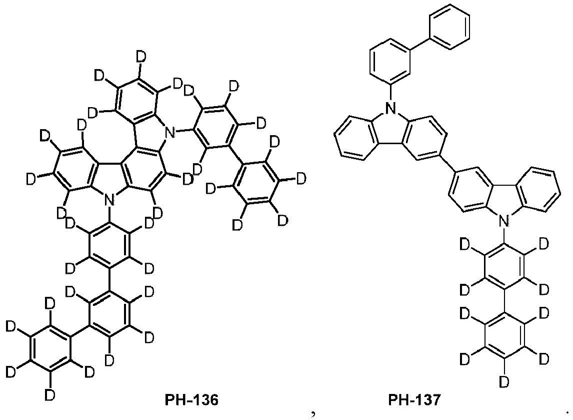

In the preparation of a device, when more than two host materials together with a luminescent material are to be co-deposited to form an emissive layer, this may be implemented in either of the following manners: (1) co-depositing the more than two host materials and the luminescent material from respective evaporation sources, to form the emissive layer; or (2) pre-mixing the more than two host materials to obtain a mixture, and co-depositing the mixture from an evaporation source with the luminescent material from another evaporation source, to form the emissive layer. The latter pre-mixing method further saves evaporation sources.

According to an embodiment of the present disclosure, it may be implemented in either of the following manners: (1) co-depositing the compound of Formula 1, the second host compound, and the first metal complex in the present disclosure from respective evaporation sources, to form the emissive layer; or (2) pre-mixing the compound of Formula 1 and the second host compound to obtain a mixture, and co-depositing the mixture from an evaporation source with the first metal complex from another evaporation source, to form the emissive layer. The compound of Formula 1 and the second host compound in the present disclosure can form a stable pre-mixture to be co-deposited. Therefore, the emissive layer is suitable for being formed through evaporation by the pre-mixing method.

According to an embodiment of the present disclosure, the organic electroluminescent device emits green light.

According to an embodiment of the present disclosure, the organic electroluminescent device emits yellow light.

According to an embodiment of the present disclosure, the organic electroluminescent device emits red light.

According to an embodiment of the present disclosure, the organic electroluminescent device emits white light.

According to an embodiment of the present disclosure, the first metal complex is doped with the compound and the second host compound, and the first metal complex accounts for 1% to 30% of the total weight of the light-emitting layer.

According to an embodiment of the present disclosure, the first metal complex is doped with the compound and the second host compound, and the first metal complex accounts for 3% to 13% of the total weight of the light-emitting layer.

According to an embodiment of the present disclosure, further disclosed is a compound composition comprising a compound represented by Formula 1, wherein the compound is shown in any one of the preceding embodiments.

### Combination with Other Materials

The materials described in the present disclosure for a particular layer in an organic light emitting device can be used in combination with various other materials present in the device. The combinations of these materials are described in more detail in U.S. Pat. App. No. 20160359122 at paragraphs 0132-0161, which is incorporated by reference herein in its entirety. The materials described or referred to the disclosure are non-limiting examples of materials that may be useful in combination with the compounds disclosed herein, and one of skill in the art can readily consult the literature to identify other materials that may be useful in combination.

The materials described herein as useful for a particular layer in an organic light emitting device may be used in combination with a variety of other materials present in the device. For example, materials disclosed herein may be used in combination with a wide variety of dopants, transport layers, blocking layers, injection layers, electrodes and other layers that may be present. The combination of these materials is described in detail in paragraphs 0080-0101 of U.S. Pat. App. No. 20150349273, which is incorporated by reference herein in its entirety. The materials described or referred to the disclosure are non-limiting examples of materials that may be useful in combination with the compounds disclosed herein, and one of skill in the art can readily consult the literature to identify other materials that may be useful in combination.

In the embodiments of material synthesis, all reactions were performed under nitrogen protection unless otherwise stated. All reaction solvents were anhydrous and used as received from commercial sources. Synthetic products were structurally confirmed and tested for properties using one or more conventional equipment in the art (including, but not limited to, nuclear magnetic resonance instrument produced by BRUKER, liquid chromatograph produced by SHIMADZU, liquid chromatograph-mass spectrometry produced by SHIMADZU, gas chromatograph-mass spectrometry produced by SHIMADZU, differential Scanning calorimeters produced by SHIMADZU, fluorescence spectrophotometer produced by SHANGHAI LENGGUANG TECH., electrochemical workstation produced by WUHAN CORRTEST, and sublimation apparatus produced by ANHUI BEQ, etc.) by methods well known to the persons skilled in the art. In the embodiments of the device, the characteristics of the device were also tested using conventional equipment in the art (including, but not limited to, evaporator produced by ANGSTROM ENGINEERING, optical testing system produced by SUZHOU FSTAR, life testing system produced by SUZHOU FSTAR, and ellipsometer produced by BEIJING ELLITOP, etc.) by methods well known to the persons skilled in the art. As the persons skilled in the art are aware of the above-mentioned equipment use, test methods and other related contents, the inherent data of the sample can be obtained with certainty and without influence, so the above related contents are not further described in this present disclosure.

### Synthesis Example 1: Synthesis of Compound A-27

### Step 1: Synthesis of Intermediate C

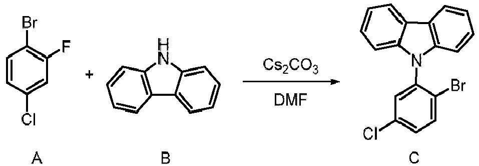

In a three-necked round-bottom flask, Intermediate A (22.2 g, 106.0 mmol), Intermediate B (17.7 g, 106.0 mmol), cesium carbonate (Cs₂CO₃) (69.1 g, 212.0 mmol), and 200 mL of *N*,*N*-dimethylformamide (DMF) were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected and concentrated under reduced pressure to obtain a crude product. The crude product was pulped with absolute ethanol to obtain Intermediate C (26.7 g, 74.9 mmol) as a white solid with a yield of 70.7%.

### Step 2: Synthesis of Intermediate E

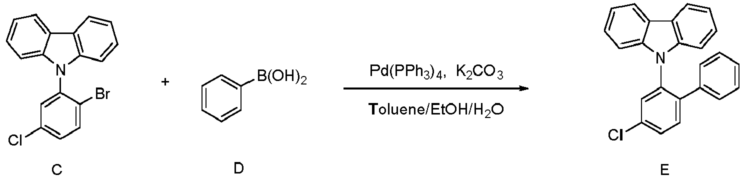

In a three-necked round-bottom flask, Intermediate C (18.9 g, 53.0 mmol), Intermediate D (9.5 g, 77.9 mmol), Pd(PPh₃)₄ (2.4 g, 2.1 mmol), K₂CO₃ (14.6 g, 106.0 mmol), 160 mL of toluene, 40 mL of EtOH, and 40 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 10:1 to 8:1) to obtain Intermediate E (16.8 g, 47.5 mmol) as a white solid with a yield of 89.6%.

### Step 3: Synthesis of Intermediate F

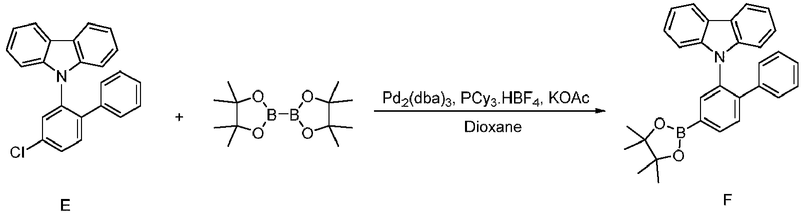

In a three-necked round-bottom flask, Intermediate E (16.8 g, 47.5 mmol), bis(pinacolato)diboron (18.1 g, 71.3 mmol), Pd₂(dba)₃ (0.87 g, 0.95 mmol), tricyclohexylphosphonium tetrafluoroborate (PCy₃·HBF₄) (0.87 g, 0.95 mmol), KOAc (9.3 g, 95.0 mmol), and 150 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, and the filtrate was concentrated under reduced pressure to obtain a crude product. The crude product was purified through silica gel column chromatography (PE:DCM = 5:1 to 2:1) to obtain Intermediate F (18.0 g, 40.4 mmol) as a white solid with a yield of 85.0%.

### Step 4: Synthesis of Compound A-27

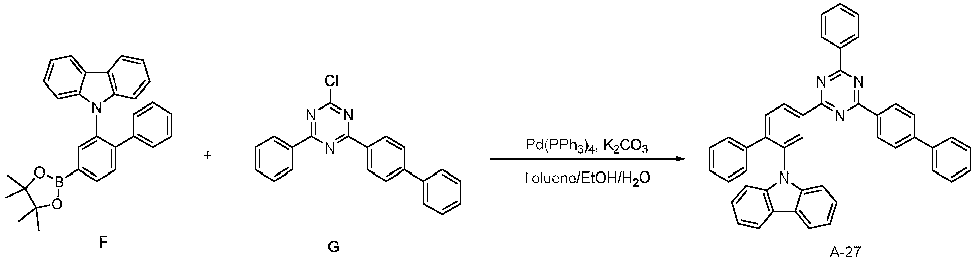

In a three-necked round-bottom flask, Intermediate F (7.2 g, 16.2 mmol), Intermediate G (5.6 g, 16.2 mmol), Pd(PPh₃)₄ (0.37 g, 0.32 mmol), K₂CO₃ (3.2 g, 32.4 mmol), 60 mL of toluene, 15 mL of EtOH, and 15 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain a light yellow solid (8.4 g, 13.4 mmol) with a yield of 82.7%. The product was confirmed as the target product A-27 with a molecular weight of 626.2.

### Synthesis Example 2: Synthesis of Compound A-28

### Step 1: Synthesis of Intermediate I

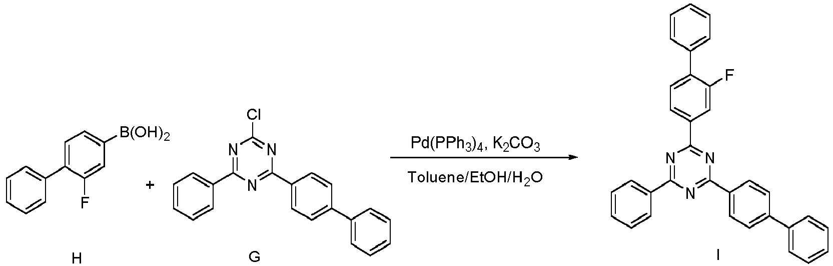

In a three-necked round-bottom flask, Intermediate H (3.1 g, 14.4 mmol), Intermediate G (4.9 g, 14.4 mmol), Pd(PPh₃)₄ (0.33 g, 0.29 mmol), K₂CO₃ (3.9 g, 28.8 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate I (6.0 g, 12.5 mmol) as a white solid with a yield of 86.8%.

### Step 2: Synthesis of Compound A-28

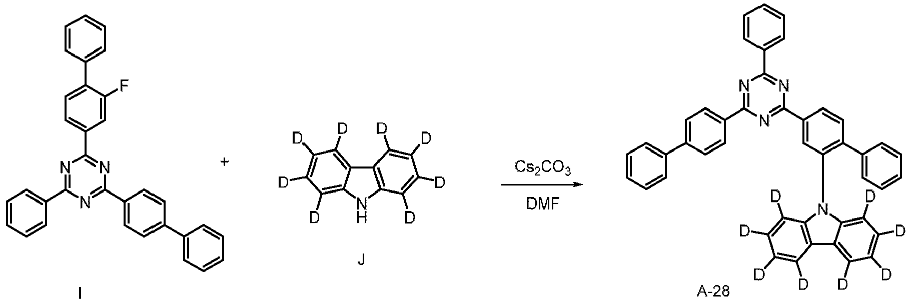

In a three-necked round-bottom flask, Intermediate I (3.6 g, 7.5 mmol), Intermediate J (1.3 g, 7.5 mmol), cesium carbonate (4.9 g, 15.0 mmol), and 60 mL of DMF were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected, dried over anhydrous Na₂SO₄, filtered, and concentrated under reduced pressure to obtain a crude product. The crude product was purified through silica gel column chromatography (PE:DCM = 10:1 to 3:1) to obtain a light yellow solid (2.5 g, 3.9 mmol) with a yield of 52.0%. The product was confirmed as the target product A-28 with a molecular weight of 634.3.

### Synthesis Example 3: Synthesis of Compound A-49

### Step 1: Synthesis of Compound A-49

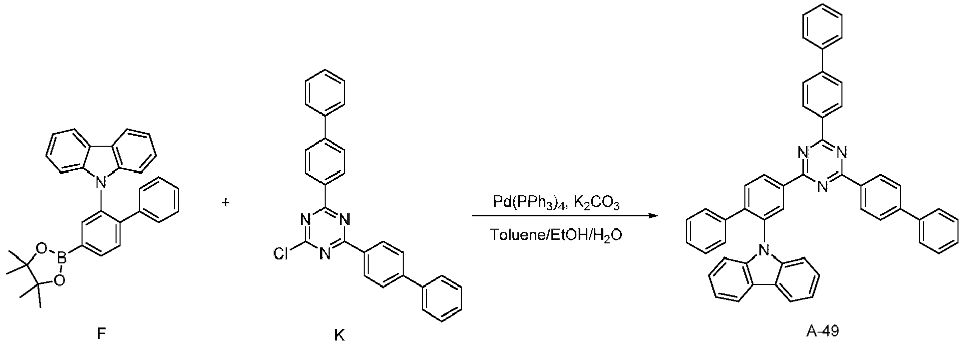

In a three-necked round-bottom flask, Intermediate F (3.6 g, 8.1 mmol), Intermediate K (3.4 g, 8.1 mmol), Pd(PPh₃)₄ (0.19 g, 0.16 mmol), K₂CO₃ (2.2 g, 16.2 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain a light yellow solid (4.2 g, 6.0 mmol) with a yield of 74.1%. The product was confirmed as the target product A-49 with a molecular weight of 702.3.

### Synthesis Example 4: Synthesis of Compound A-50

### Step 1: Synthesis of Intermediate L

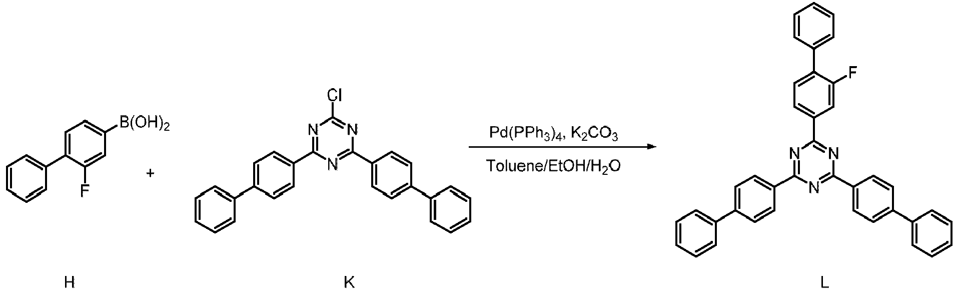

In a three-necked round-bottom flask, Intermediate H (3.1 g, 14.4 mmol), Intermediate K (6.0 g, 14.4 mmol), Pd(PPh₃)₄ (0.33 g, 0.29 mmol), K₂CO₃ (3.9 g, 28.8 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate L (5.4 g, 9.7 mmol) as a white solid with a yield of 67.4%.

### Step 2: Synthesis of Compound A-50

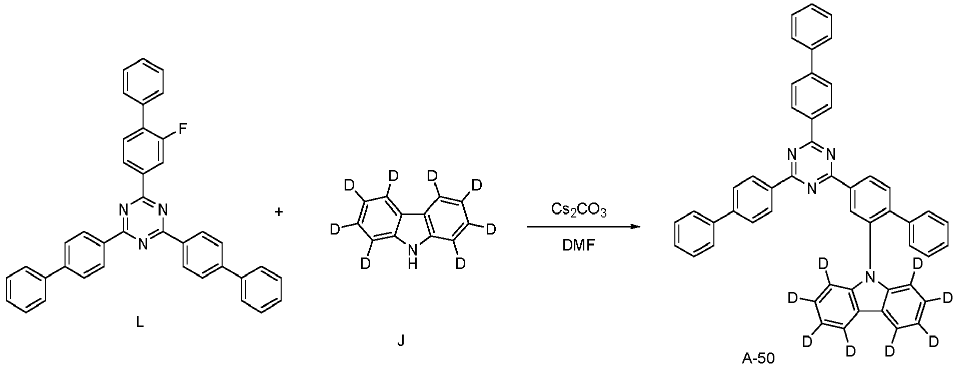

In a three-necked round-bottom flask, Intermediate L (4.2 g, 7.5 mmol), Intermediate J (1.3 g, 7.5 mmol), cesium carbonate (4.9 g, 15.0 mmol), and 60 mL of DMF were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected, dried over anhydrous Na₂SO₄, filtered, and concentrated under reduced pressure to obtain a crude product. The crude product was purified through silica gel column chromatography (PE:DCM = 10:1 to 3:1) to obtain a light yellow solid (2.5 g, 3.5 mmol) with a yield of 46.7%. The product was confirmed as the target product A-50 with a molecular weight of 710.3.

### Synthesis Example 5: Synthesis of Compound A-56

### Step 1: Synthesis of Compound A-56

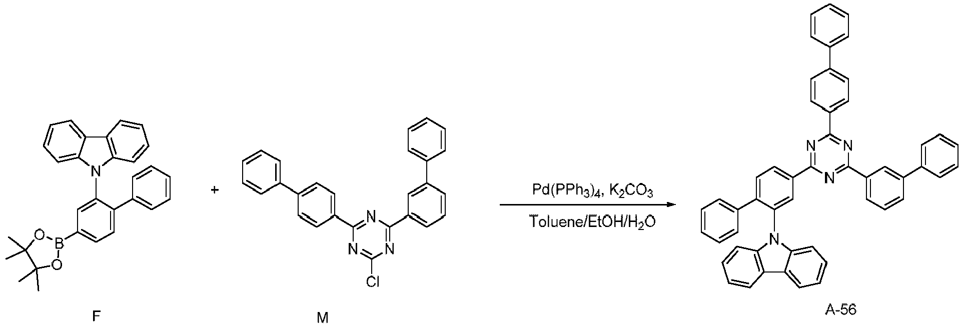

In a three-necked round-bottom flask, Intermediate F (4.0 g, 9.0 mmol), Intermediate M (3.8 g, 9.0 mmol), Pd(PPh₃)₄ (0.20 g, 0.18 mmol), K₂CO₃ (2.5 g, 18.0 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene to obtain a light yellow solid (3.2 g, 4.6 mmol) with a yield of 51.1%. The product was confirmed as the target product A-56 with a molecular weight of 702.3.

### Synthesis Example 6: Synthesis of Compound A-163

### Step 1: Synthesis of Intermediate O

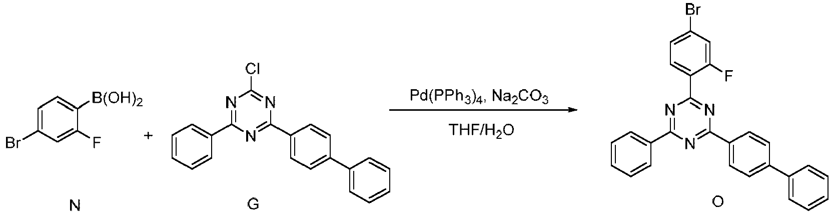

In a three-necked round-bottom flask, Intermediate N (8.8 g, 40.2 mmol), Intermediate G (13.8 g, 40.2 mmol), Pd(PPh₃)₄ (0.93 g, 0.80 mmol), Na₂CO₃ (8.5 g, 80.4 mmol), 120 mL of THF, and 30 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate O (9.5 g, 19.7 mmol) as a white solid with a yield of 49.0%.

### Step 2: Synthesis of Intermediate P

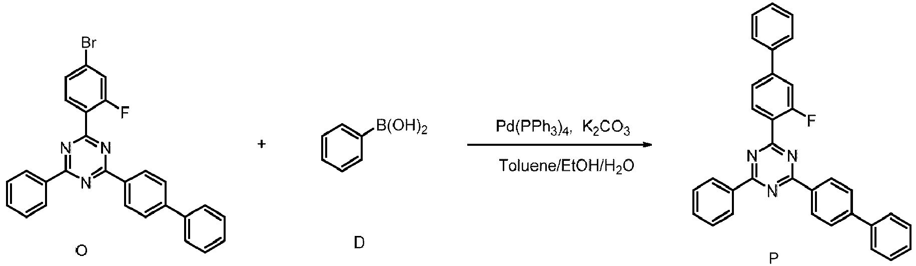

In a three-necked round-bottom flask, Intermediate O (9.5 g, 19.7 mmol), Intermediate D (2.6 g, 21.6 mmol), Pd(PPh₃)₄ (0.50 g, 0.43 mmol), K₂CO₃ (5.4 g, 39.4 mmol), 60 mL of toluene, 15 mL of EtOH, and 15 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate P (4.8 g, 10.0 mmol) as a white solid with a yield of 50.8%.

### Step 3: Synthesis of Compound A-163

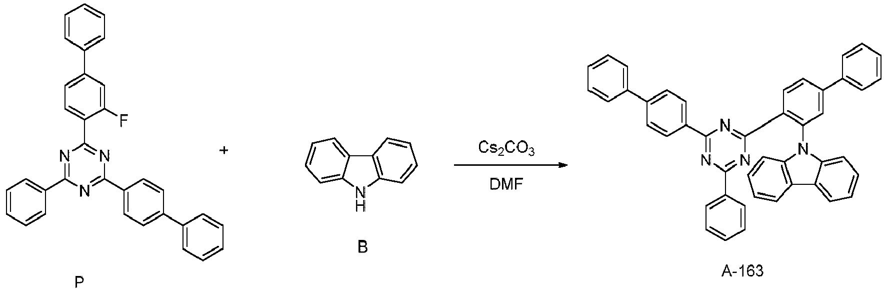

In a three-necked round-bottom flask, Intermediate P (3.6 g, 7.5 mmol), Intermediate B (1.3 g, 7.5 mmol), cesium carbonate (4.9 g, 15.0 mmol), and 60 mL of DMF were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected, dried over anhydrous Na₂SO₄, filtered, and concentrated under reduced pressure to obtain a crude product. The crude product was purified through silica gel column chromatography (PE:DCM = 10:1 to 3:1) to obtain a light yellow solid (2.5 g, 4.0 mmol) with a yield of 53.3%. The product was confirmed as the target product A-163 with a molecular weight of 626.2.

### Synthesis Example 7: Synthesis of Compound A-185

### Step 1: Synthesis of Intermediate R

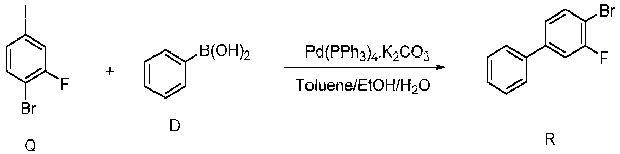

In a three-necked round-bottom flask, Intermediate Q (30.0 g, 99.7 mmol), Intermediate D (13.4 g, 109.7 mmol), Pd(PPh₃)₄ (1.2 g, 1.0 mmol), K₂CO₃ (27.5 g, 199.4 mmol), 320 mL of toluene, 80 mL of EtOH, and 80 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through column chromatography (PE) to obtain Intermediate R (22.6 g, 90.0 mmol) as a white solid with a yield of 90.3%.

### Step 2: Synthesis of Intermediate S

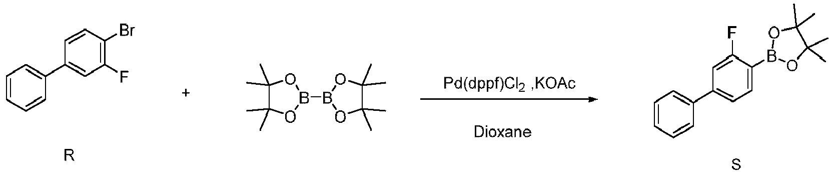

In a three-necked round-bottom flask, Intermediate R (14.5 g, 57.7 mmol), bis(pinacolato)diboron (22.0 g, 86.6 mmol), Pd(dppf)Cl₂ (0.84 g, 1.15 mmol), KOAc (11.3 g, 115.4 mmol), and 300 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, the filtrate was concentrated under reduced pressure to obtain a crude product, and the crude product was purified through silica gel column chromatography (PE:DCM = 10:1 to 2:1) to obtain Intermediate S (15.0 g, 50.3 mmol) as a white solid with a yield of 87.2%.

### Step 3: Synthesis of Intermediate T

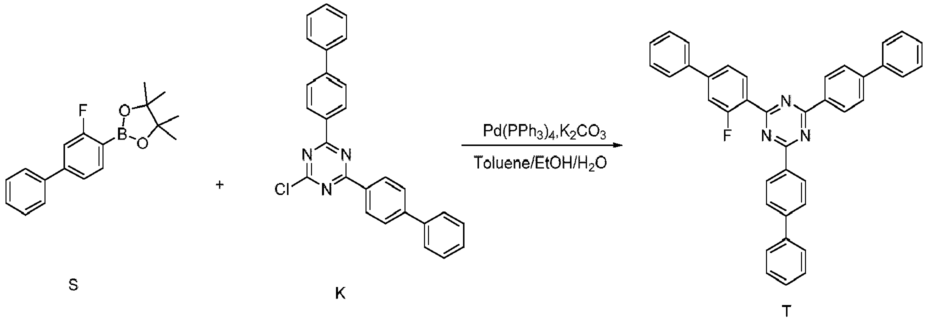

In a three-necked round-bottom flask, Intermediate S (6.0 g, 20.0 mmol), Intermediate K (8.4 g, 20.0 mmol), Pd(PPh₃)₄ (0.46 g, 0.4 mmol), K₂CO₃ (5.5 g, 40.0 mmol), 60 mL of toluene, 15 mL of EtOH, and 15 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate T (10.4 g, 18.7 mmol) as a white solid with a yield of 93.5%.

### Step 4: Synthesis of Compound A-18 5

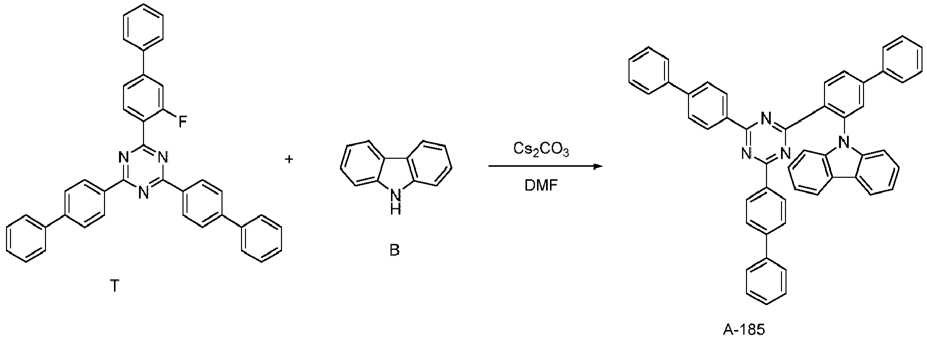

In a three-necked round-bottom flask, Intermediate T (5.7 g, 10.3 mmol), Intermediate B (1.7 g, 10.3 mmol), cesium carbonate (6.7 g, 20.5 mmol), and 60 mL of DMF were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected and concentrated under reduced pressure to obtain a crude product. The crude product was purified through silica gel column chromatography (PE:DCM = 10:1 to 3:1) to obtain a light yellow solid (5.0 g, 7.1 mmol) with a yield of 68.9%. The product was confirmed as the target product A-185 with a molecular weight of 702.3.

### Synthesis Example 8: Synthesis of Compound A-192

### Step 1: Synthesis of Intermediate U

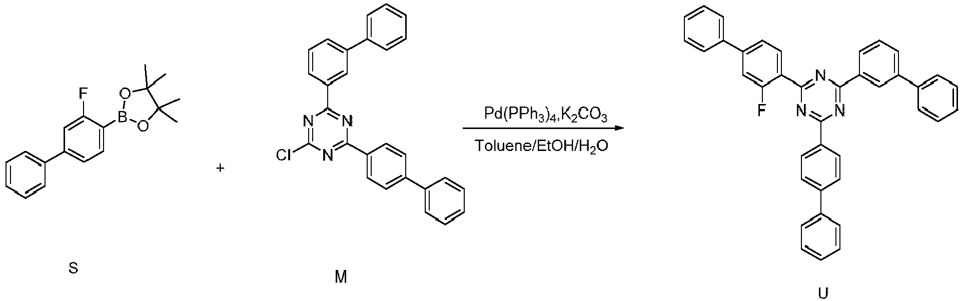

In a three-necked round-bottom flask, Intermediate S (6.0 g, 20.0 mmol), Intermediate M (8.4 g, 20.0 mmol), Pd(PPh₃)₄ (0.46 g, 0.4 mmol), K₂CO₃ (5.5 g, 40.0 mmol), 60 mL of toluene, 15 mL of EtOH, and 15 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was recrystallized from toluene and pulped with ethanol to obtain Intermediate U (10.4 g, 18.7 mmol) as a white solid with a yield of 93.5%.

### Step 2: Synthesis of Compound A-192

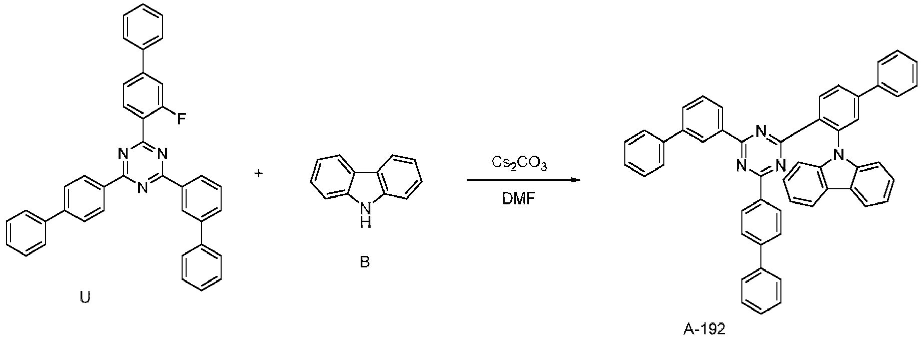

In a three-necked round-bottom flask, Intermediate U (5.7 g, 10.3 mmol), Intermediate B (1.7 g, 10.3 mmol), cesium carbonate (6.7 g, 20.5 mmol), and 60 mL of DMF were added in sequence. Under N₂ protection, the system was heated to reflux overnight. After the reaction was completed as confirmed by TLC, heating was stopped and the system was cooled to room temperature. The reaction solution was poured into a large amount of water and extracted with ethyl acetate. The organic phases were collected and concentrated under reduced pressure to obtain a crude product. The crude product was purified through silica gel column chromatography (PE:DCM = 10:1 to 3:1) to obtain a light yellow solid (5.0 g, 7.1 mmol) with a yield of 68.9%. The product was confirmed as the target product A-192 with a molecular weight of 702.3.

### Synthesis Example 9: Synthesis of Compound A-273

### Step 1: Synthesis of Intermediate W

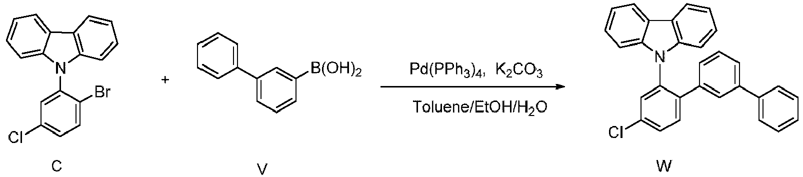

In a three-necked round-bottom flask, Intermediate C (6.5 g, 18.2 mmol), Intermediate V (4.0 g, 20.2 mmol), Pd(PPh₃)₄ (0.42 g, 0.36 mmol), K₂CO₃ (5.0 g, 36.4 mmol), 60 mL of toluene, 15 mL of EtOH, and 15 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 10:1 to 8:1) to obtain Intermediate W (7.5 g, 17.4 mmol) as a white solid with a yield of 95.6%.

### Step 2: Synthesis of Intermediate X

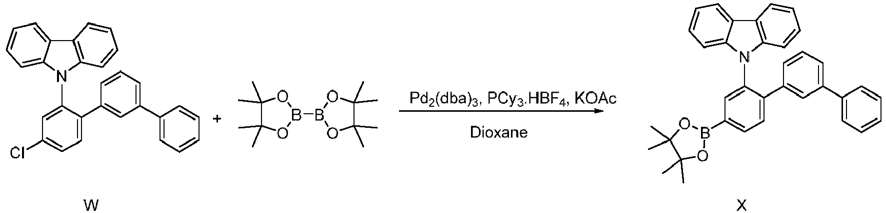

In a three-necked round-bottom flask, Intermediate W (7.5 g, 17.4 mmol), bis(pinacolato)diboron (6.6 g, 26.0 mmol), Pd₂(dba)₃ (0.32 g, 0.35 mmol), tricyclohexylphosphonium tetrafluoroborate (PCy₃·HBF₄) (0.32 g, 0.35 mmol), KOAc (9.3 g, 95.0 mmol), and 80 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, the filtrate was concentrated under reduced pressure to obtain a crude product, and the crude product was purified through silica gel column chromatography (PE:DCM = 5:1 to 2:1) to obtain Intermediate X (7.5 g, 14.4 mmol) as a white solid with a yield of 82.8%.

### Step 3: Synthesis of Compound A-273

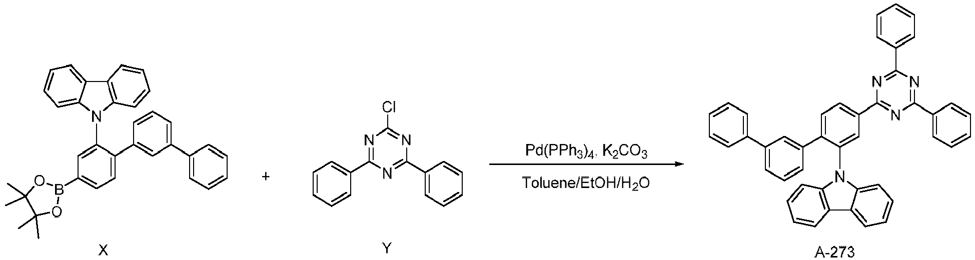

In a three-necked round-bottom flask, Intermediate X (4.3 g, 8.2 mmol), Intermediate Y (2.2 g, 8.2 mmol), Pd(PPh₃)₄ (0.19 g, 0.16 mmol), K₂CO₃ (2.3 g, 16.4 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 10:1 to 3:1) to obtain a light yellow solid (3.5 g, 5.6 mmol) with a yield of 68.3%. The product was confirmed as the target product A-273 with a molecular weight of 626.2.

### Synthesis Example 10: Synthesis of Compound A-448

### Step 1: Synthesis of Intermediate AC

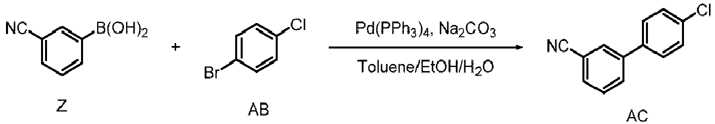

In a three-necked round-bottom flask, Intermediate Z (25 g, 170 mmol), Intermediate AB (39 g, 204 mmol), Pd(PPh₃)₄ (3.93 g, 3.4 mmol), and Na₂CO₃ (36 g, 340 mmol ) were added to a mixed solvent of toluene (80 mL), EtOH (20 mL), and H₂O (20 mL). Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was subjected to silica gel column chromatography (PE:DCM = 5:1 to 3:1) to obtain Intermediate AC (33.3 g, 155.8 mmol) as a white solid with a yield of 91.6%.

### Step 2: Synthesis of Intermediate AD

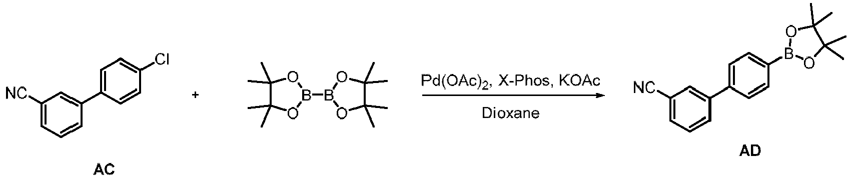

In a three-necked round-bottom flask, Intermediate AC (33.3 g, 155.8 mmol), bis(pinacolato)diboron (59.3 g, 233.7 mmol), Pd(OAc)₂ (0.7 g, 3.1 mmol), 2-dicyclohexylphosphino-2',4',6'-triisopropylbiphenyl (X-Phos) (3.0 g, 6.2 mmol), and KOAc (31 g, 311.6 mmol) were added to 1,4-dioxane (300 mL). Under N₂ protection, the system was heated to reflux overnight. After the reaction was completed as confirmed by TLC, heating was stopped and the system was cooled to room temperature. The reaction system was filtered through Celite, the filtrate was concentrated under reduced pressure to obtain a crude product, and the crude product was purified through silica gel column chromatography (PE:DCM = 5:1 to 2:1) to obtain Intermediate AD (28.2 g, 92.4 mmol) as a white solid with a yield of 59.3%.

### Step 3: Synthesis of Intermediate AF

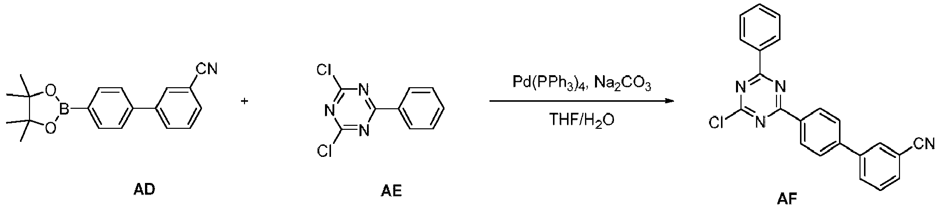

In a three-necked round-bottom flask, Intermediate AD (24.4 g, 80 mmol), Intermediate AE (27.0 g, 120 mmol), Pd(PPh₃)₄ (1.85 g, 1.6 mmol), and Na₂CO₃ (25 g, 240 mmol ) were added to a mixed solvent of THF (400 mL) and H₂O (100 mL). Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 5:1 to 1:1) to obtain Intermediate AF (12.2 g, 33.1 mmol) as a white solid with a yield of 41.3%.

### Step 4: Synthesis of Compound A-448

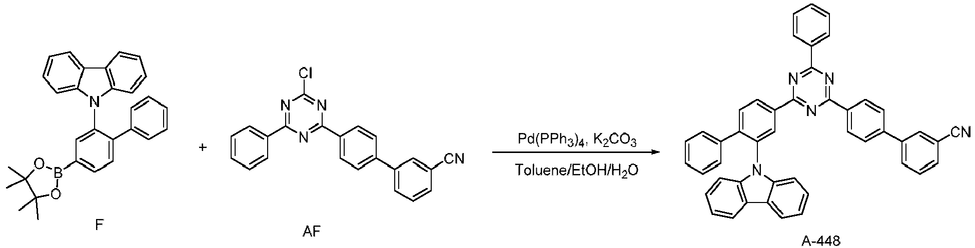

In a three-necked round-bottom flask, Intermediate F (3.86 g, 8.68 mmol), Intermediate AF (3.2 g, 8.68 mmol), Pd(PPh₃)₄ (0.30 g, 0.26 mmol), K₂CO₃ (2.4 g, 17.36 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 4:1 to 1:1) to obtain a light yellow solid (3.7 g, 5.68 mmol) with a yield of 65.4%. The product was confirmed as the target product A-448 with a molecular weight of 651.2.

### Synthesis Example 11: Synthesis of Compound A-525

### Step 1: Synthesis of Intermediate AH

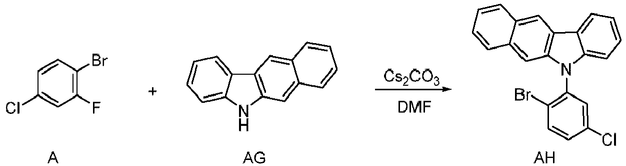

In a three-necked round-bottom flask, Intermediate A (2.8 g, 13.8 mmol), Intermediate AG (2.0 g, 9.2 mmol), cesium carbonate (Cs₂CO₃) (6.0 g, 18.5 mmol), and 30 mL of A A-dimethylformamide (DMF) were added in sequence. Under N₂ protection, the system was heated to 130 °C. After the reaction was completed as confirmed by TLC, heating was stopped and the system was cooled to room temperature. The reaction solution was poured into a large amount of water and extracted with ethyl acetate. The organic phases were collected and concentrated under reduced pressure to obtain a crude product. The crude product was subjected to silica gel column chromatography (PE:DCM = 5:1) to obtain Intermediate AH (3.5 g, 8.6 mmol) as a white solid with a yield of 93.5%.

### Step 2: Synthesis of Intermediate AI

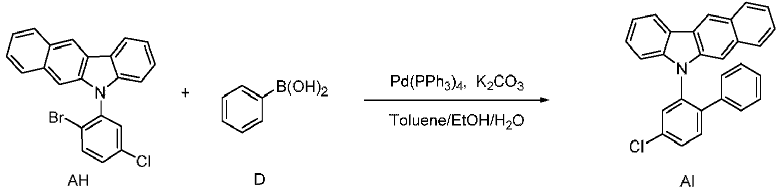

In a three-necked round-bottom flask, Intermediate AH (3.5 g, 8.6 mmol), Intermediate D (1.6 g, 12.9 mmol), Pd(PPh₃)₄ (0.5 g, 0.4 mmol), K₂CO₃ (2.4 g, 17.2 mmol), 80 mL of toluene, 20 mL of EtOH, and 20 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 4:1) to obtain Intermediate AI (3.2 g, 7.9 mmol) as a white solid with a yield of 91.9%.

### Step 3: Synthesis of Intermediate AJ

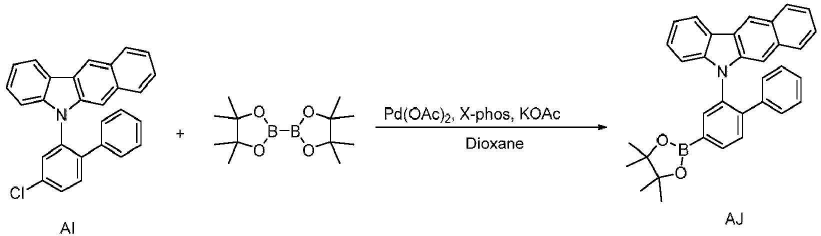

In a three-necked round-bottom flask, Intermediate AI (3.2 g, 7.9 mmol), bis(pinacolato)diboron (3.0 g, 11.9 mmol), Pd(OAc)₂ (0.09 g, 0.4 mmol), 2-dicyclohexylphosphino-2',4',6'-triisopropylbiphenyl (X-phos) (0.4 g, 0.8 mmol), KOAc (1.5 g, 15.8 mmol), and 50 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, and the filtrate was concentrated under reduced pressure to obtain a crude product. The crude product was purified through silica gel column chromatography (PE:DCM = 3:1) to obtain Intermediate AJ (3.5 g, 7.1 mmol) as a white solid with a yield of 89.9%.

### Step 4: Synthesis of Compound A-525

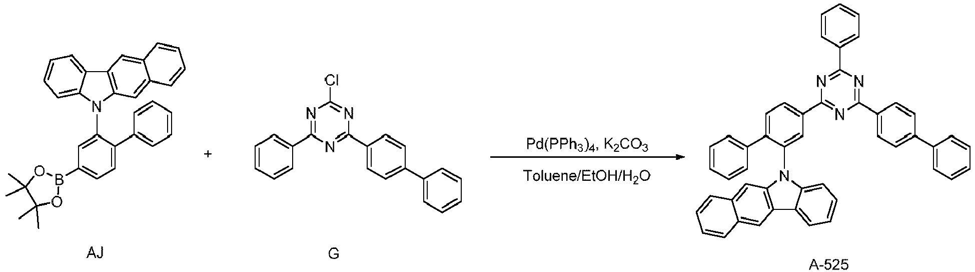

In a three-necked round-bottom flask, Intermediate AJ (3.5 g, 7.1 mmol), Intermediate G (2.6 g, 7.7 mmol), Pd(PPh₃)₄ (0.41 g, 0.35 mmol), K₂CO₃ (2.0 g, 14.2 mmol), 80 mL of toluene, 20 mL of EtOH, and 20 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was purified through silica gel column chromatography (PE:DCM = 3:1) to obtain a light yellow solid (3.0 g, 4.4 mmol) with a yield of 62.0%. The product was confirmed as the target product A-525 with a molecular weight of 676.3.

### Synthesis Example 12: Synthesis of Compound A-528

### Step 1: Synthesis of Intermediate AL

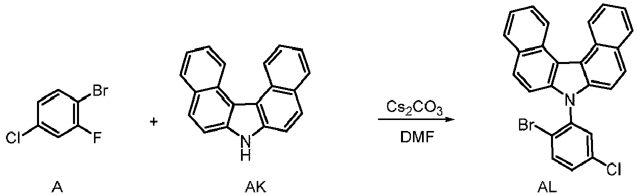

In a three-necked round-bottom flask, Intermediate A (2.2 g, 10.5 mmol), Intermediate AK (2.8 g, 10.5 mmol), cesium carbonate (Cs₂CO₃) (10.0 g, 31.5 mmol), and 50 mL of A A-dimethylformamide (DMF) were added in sequence. Under N₂ protection, the system was heated to 140 °C. After the reaction was completed as confirmed by TLC, heating was stopped and the system was cooled to room temperature. The reaction solution was poured into a large amount of water and extracted with ethyl acetate. The organic phases were collected and concentrated under reduced pressure to obtain a crude product. The crude product was subjected to silica gel column chromatography (PE:DCM = 3:1) to obtain Intermediate AL (3.0 g, 6.6 mmol) as a white solid with a yield of 62.9%.

### Step 2: Synthesis of Intermediate AM

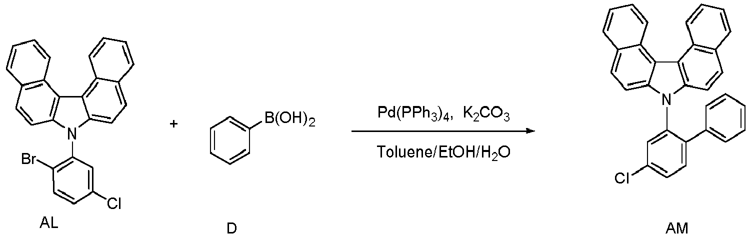

In a three-necked round-bottom flask, Intermediate AL (3.0 g, 6.6 mmol), Intermediate D (0.96 g, 7.9 mmol), Pd(PPh₃)₄ (0.76 g, 0.66 mmol), K₂CO₃ (2.7 g, 19.8 mmol), 24 mL of toluene, 6 mL of EtOH, and 6 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 4:1) to obtain Intermediate AM (2.2 g, 4.8 mmol) as a white solid with a yield of 72.7%.

### Step 3: Synthesis of Intermediate AN

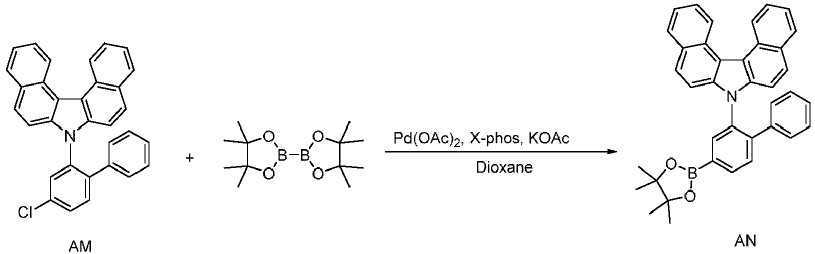

In a three-necked round-bottom flask, Intermediate AM (2.2 g, 4.8 mmol), bis(pinacolato)diboron (1.85 g, 7.3 mmol), Pd(OAc)₂ (0.11 g, 0.49 mmol), 2-dicyclohexylphosphino-2',4',6'-triisopropylbiphenyl (X-phos) (0.46 g, 0.97 mmol), KOAc (1.4 g, 14.6 mmol), and 25 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, and the filtrate was concentrated under reduced pressure to obtain a crude product. The crude product was purified through silica gel column chromatography (PE:DCM = 2:1) to obtain Intermediate AN (2.4 g, 4.4 mmol) as a white solid with a yield of 91.7%.

### Step 4: Synthesis of Compound A-528

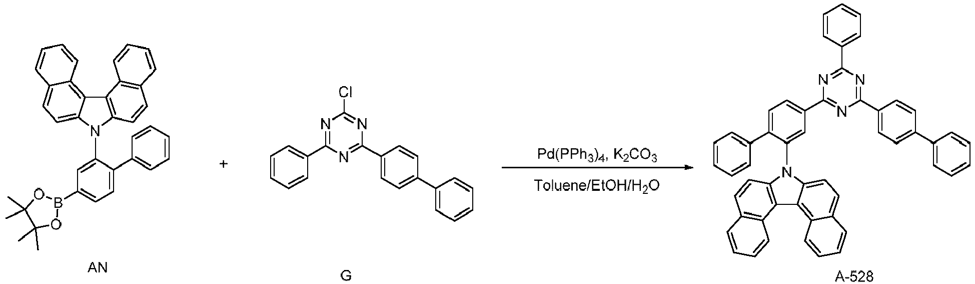

In a three-necked round-bottom flask, Intermediate AN (2.4 g, 4.4 mmol), Intermediate G (1.4 g, 4.2 mmol), Pd(PPh₃)₄ (0.48 g, 0.35 mmol), K₂CO₃ (1.7 g, 12.0 mmol), 20 mL of toluene, 5 mL of EtOH, and 5 mL of H₂O were added in sequence. Under N₂ protection, the system was heated to reflux overnight. After the reaction was completed as confirmed by TLC, heating was stopped and the system was cooled to room temperature. A large amount of solid was precipitated from the reaction solution and filtered. The obtained solid was washed with water and ethanol in sequence to obtain a crude product. The crude product was purified through silica gel column chromatography (PE:DCM = 6:1) to obtain a white solid (1.0 g, 1.4 mmol) with a yield of 31.8%. The product was confirmed as the target product A-528 with a molecular weight of 726.3.

### Synthesis Example 13: Synthesis of Compound A-65

### Step 1: Synthesis of Compound A-65

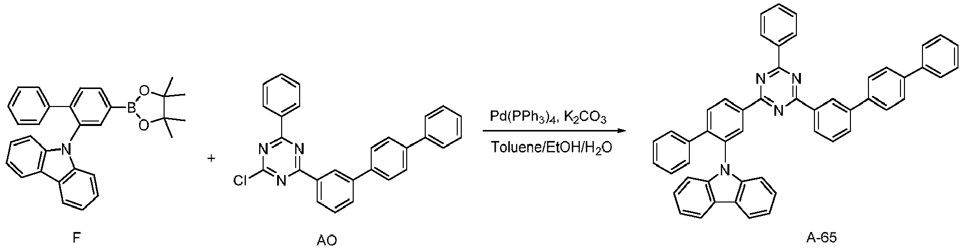

In a three-necked round-bottom flask, Intermediate F (6.68 g, 15.0 mmol), Intermediate AO (6.3 g, 15.0 mmol), Pd(PPh₃)₄ (0.52 g, 0.45 mmol), K₂CO₃ (4.15 g, 30.0 mmol), 72 mL of toluene, 18 mL of EtOH, and 18 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was subjected to silica gel column chromatography (PE:DCM = 10:1 to 2:1) to obtain a light yellow solid (8.3 g, 11.8 mmol) with a yield of 78.7%. The product was confirmed as the target product A-65 with a molecular weight of 702.3.

### Synthesis Example 14: Synthesis of Compound A-77

### Step 1: Synthesis of Intermediate AP

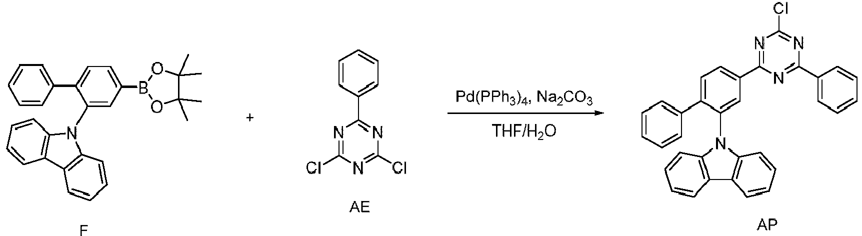

In a three-necked round-bottom flask, Intermediate F (17.8 g, 40.0 mmol), Intermediate AE (11.8 g, 52.0 mmol), Pd(PPh₃)₄ (0.92 g, 0.80 mmol), and Na₂CO₃ (8.48 g, 80.0 mmol ) were added to a mixed solvent of THF (256 mL) and H₂O (64 mL). Under N₂ protection, the system was heated to reflux. After the reaction was completed as confirmed by TLC after 7 h, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 6:1 to 3:1) to obtain Intermediate AP (12.4 g, 24.4 mmol) as a yellow solid with a yield of 60.9%.

### Step 2: Synthesis of Compound A-77

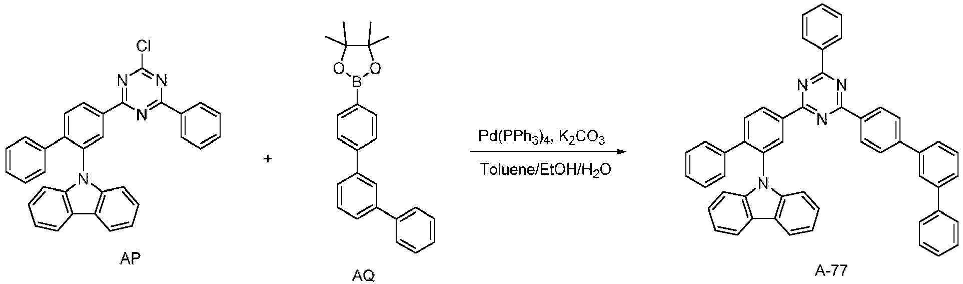

In a three-necked round-bottom flask, Intermediate AP (4.07 g, 8.0 mmol), Intermediate AQ (2.85 g, 8.0 mmol), Pd(PPh₃)₄ (0.28 g, 0.24 mmol), K₂CO₃ (2.21 g, 16.0 mmol), 40 mL of toluene, 10 mL of EtOH, and 10 mL of H₂O were added in sequence. Under N₂ protection, the system was heated to reflux overnight. After the reaction was completed as confirmed by TLC, heating was stopped and the system was cooled to room temperature. The reaction solution was suction filtered under reduced pressure, and the obtained solid was washed multiple times with water and methanol in sequence to obtain a crude product. The crude product was subjected to silica gel column chromatography (PE:DCM = 10:1 to 2:1) to obtain a light yellow solid (4.3 g, 6.1 mmol) with a yield of 76.5%. The product was confirmed as the target product A-77 with a molecular weight of 702.3.

### Synthesis Example 15: Synthesis of Compound A-434

### Step 1: Synthesis of Compound A-434

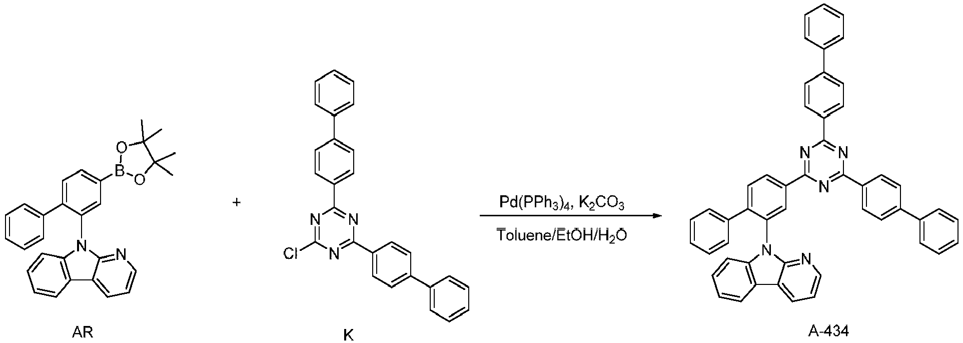

In a three-necked round-bottom flask, Intermediate AR (1.8 g, 4.0 mmol), Intermediate K (1.7 g, 4.0 mmol), Pd(PPh₃)₄ (0.14 g, 0.12 mmol), K₂CO₃ (1.1 g, 8.0 mmol), 24 mL of toluene, 6 mL of EtOH, and 6 mL of H₂O were added in sequence. Under N₂ protection, the system was heated to reflux overnight. After the reaction was completed as confirmed by TLC, heating was stopped and the system was cooled to room temperature. The reaction solution was suction filtered under reduced pressure, and the obtained solid was washed with water and ethanol in sequence to obtain a crude product. The crude product was recrystallized from a mixed solvent of toluene and acetonitrile to obtain a light yellow solid (2.2 g, 3.1 mmol) with a yield of 78.1%. The product was confirmed as the target product A-434 with a molecular weight of 703.3.

### Synthesis Example 16: Synthesis of Compound A-95

### Step 1: Synthesis of Compound A-95

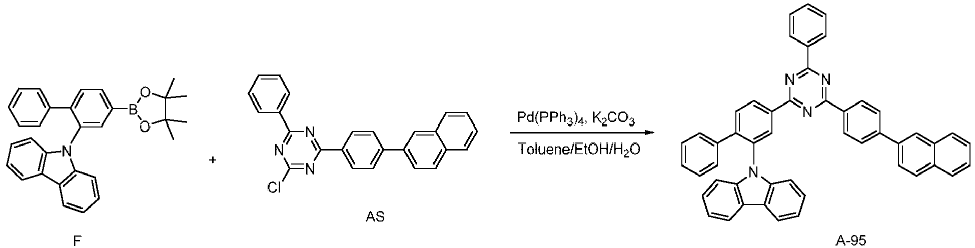

In a three-necked round-bottom flask, Intermediate F (5.0 g, 11.2 mmol), Intermediate AS (4.4 g, 11.2 mmol), Pd(PPh₃)₄ (0.26 g, 0.23 mmol), K₂CO₃ (3.1 g, 22.4 mmol), 120 mL of toluene, 30 mL of EtOH, and 30 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with ethyl acetate, and the organic phases were combined, dried over anhydrous Na₂SO₄, filtered, and concentrated under reduced pressure to obtain a crude product. The crude product was subjected to silica gel column chromatography (PE:DCM = 4:1) to obtain a light yellow solid (5.0 g, 7.4 mmol) with a yield of 66.1%. The product was confirmed as the target product A-95 with a molecular weight of 676.3.

### Synthesis Example 17: Synthesis of Compound A-582

### Step 1: Synthesis of Compound A-582

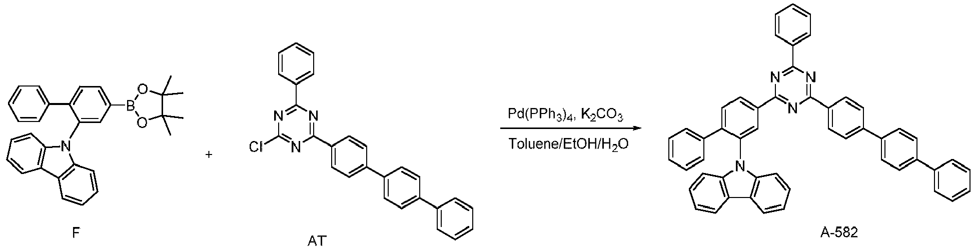

In a three-necked round-bottom flask, Intermediate F (4.0 g, 9.0 mmol), Intermediate AT (3.78 g, 9.0 mmol), Pd(PPh₃)₄ (0.31 g, 0.27 mmol), K₂CO₃ (2.49 g, 18.0 mmol), 48 mL of toluene, 12 mL of EtOH, and 12 mL of H₂O were added in sequence. Under N₂ protection, the system was heated to reflux overnight. After the reaction was completed as confirmed by TLC, heating was stopped and the system was cooled to room temperature. The reaction solution was suction filtered under reduced pressure, and the obtained solid was washed multiple times with water and methanol in sequence to obtain a crude product. The crude product was subjected to silica gel column chromatography (PE:DCM = 10:1 to 5:2) to obtain a light yellow solid (4.6 g, 6.54 mmol) with a yield of 72.7%. The product was confirmed as the target product A-582 with a molecular weight of 702.3.

### Synthesis Example 18: Synthesis of Compound A-583

### Step 1: Synthesis of Intermediate AV

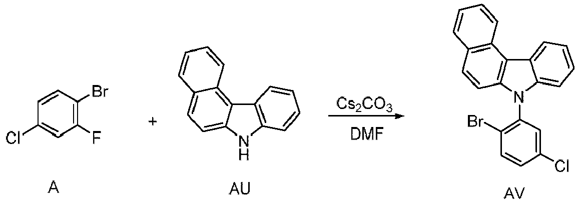

In a three-necked round-bottom flask, Intermediate A (4.8 g, 22.9 mmol), Intermediate AU (5.0 g, 23.0 mmol), cesium carbonate (18.6 g, 57.5 mmol), and 100 mL of *N*,*N*-dimethylformamide (DMF) were added in sequence. Under N₂ protection, the system was heated to 140 °C and reacted. After the reaction was completed as confirmed by TLC, heating was stopped and the system was cooled to room temperature. The reaction solution was poured into a large amount of water and extracted with DCM. The organic phases were collected and concentrated under reduced pressure to obtain a crude product. The crude product was subjected to silica gel column chromatography (PE:DCM = 5:1) to obtain Intermediate AV (5.5 g, 13.6 mmol) as a white solid with a yield of 59.0%.

### Step 2: Synthesis of Intermediate AW

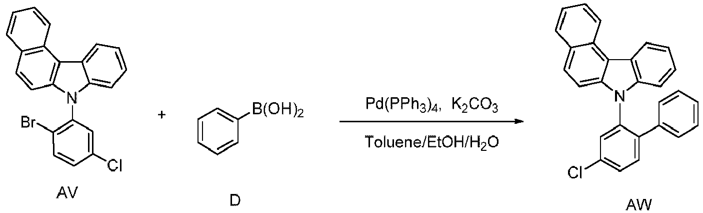

In a three-necked round-bottom flask, Intermediate AV (5.5 g, 13.6 mmol), Intermediate D (1.8 g, 14.9 mmol), Pd(PPh₃)₄ (1.6 g, 1.4 mmol), K₂CO₃ (4.3 g, 31.1 mmol), 48 mL of toluene, 12 mL of EtOH, and 12 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 5:1) to obtain Intermediate AW (4.3 g, 10.7 mmol) as a white solid with a yield of 78.7%.

### Step 3: Synthesis of Intermediate AX

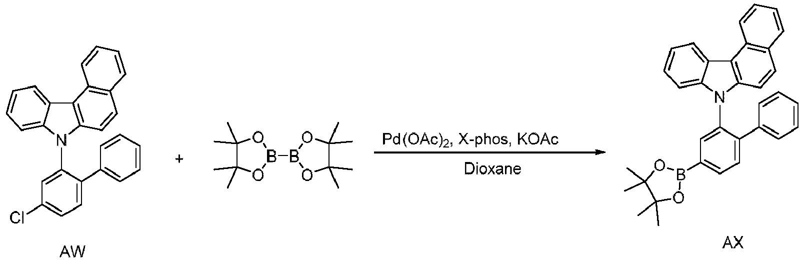

In a three-necked round-bottom flask, Intermediate AW (4.3 g, 10.7 mmol), bis(pinacolato)diboron (4.0 g, 15.8 mmol), Pd(OAc)₂ (0.22 g, 1.0 mmol), 2-dicyclohexylphosphino-2',4',6'-triisopropylbiphenyl (X-phos) (1.0 g, 2.1 mmol), KOAc (3.1 g, 32.1 mmol), and 50 mL of 1,4-dioxane were added in sequence. Under N₂ protection, the system was heated to reflux overnight. After the reaction was completed as confirmed by TLC, heating was stopped and the system was cooled to room temperature. The reaction system was filtered through Celite, and the filtrate was concentrated under reduced pressure to obtain a crude product. The crude product was purified through silica gel column chromatography (PE:DCM = 2:1) to obtain Intermediate AX (3.8 g, 7.7 mmol) as a white solid with a yield of 72.0%.

### Step 4: Synthesis of Compound A-583

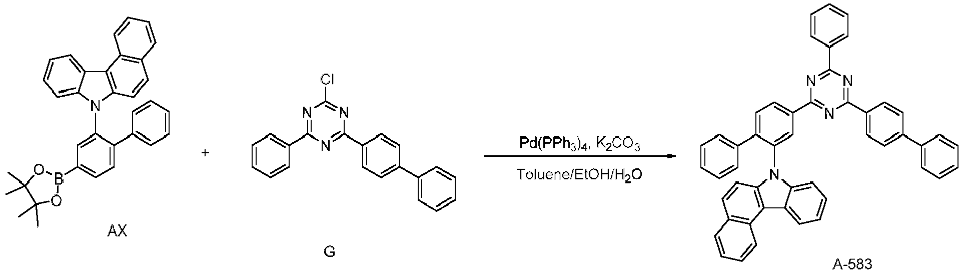

In a three-necked round-bottom flask, Intermediate AX (3.8 g, 7.7 mmol), Intermediate G (3.1 g, 7.7 mmol), Pd(PPh₃)₄ (0.89 g, 0.8 mmol), K₂CO₃ (3.2 g, 23 mmol), 28 mL of toluene, 7 mL of EtOH, and 7 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with DCM, and the organic phases were combined, dried over anhydrous Na₂SO₄, filtered, and concentrated under reduced pressure to obtain a crude product. The crude product was purified through silica gel column chromatography (PE:DCM = 5:1) to obtain a light yellow solid (2.1 g, 3.1 mmol) with a yield of 40.3%. The product was confirmed as the target product A-583 with a molecular weight of 676.3.

### Synthesis Example 19: Synthesis of Compound A-584

### Step 1: Synthesis of Compound A-584

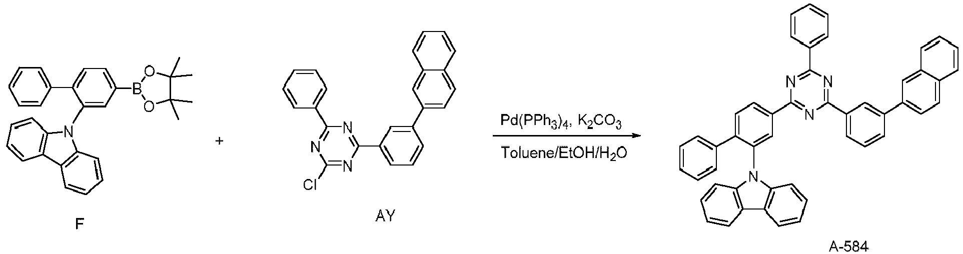

In a three-necked round-bottom flask, Intermediate F (4.1 g, 9.0 mmol), Intermediate AY (3.5 g, 9.0 mmol), Pd(PPh₃)₄ (0.21 g, 0.17 mmol), K₂CO₃ (2.5 g, 18.0 mmol), 120 mL of toluene, 30 mL of EtOH, and 30 mL of H₂O were added in sequence. Under N₂ protection, the system was heated to reflux overnight. After the reaction was completed as confirmed by TLC, heating was stopped and the system was cooled to room temperature. The reaction solution was separated into layers, the aqueous phase was extracted with ethyl acetate, and the organic phases were combined, dried over anhydrous Na₂SO₄, filtered, and concentrated under reduced pressure to obtain a crude product. The crude product was subjected to silica gel column chromatography (PE:DCM = 4:1) to obtain a light yellow solid (3.5 g, 5.2 mmol) with a yield of 57.5%. The product was confirmed as the target product A-584 with a molecular weight of 676.3.

Those skilled in the art will appreciate that the above preparation methods are merely exemplary. Those skilled in the art can obtain other compound structures of the present disclosure through the modifications of the preparation methods.

The method for preparing an electroluminescent device is not limited. The preparation methods in the following examples are merely examples and not to be construed as limitations. Those skilled in the art can make reasonable improvements on the preparation methods in the following examples based on the related art. Exemplarily, the proportions of various materials in an emissive layer are not particularly limited. Those skilled in the art can reasonably select the proportions within a certain range based on the related art. For example, taking the total weight of the materials in the emissive layer as reference, a host material may account for 80% to 99% and a light-emitting material may account for 1% to 20%; or the host material may account for 90% to 99% and the light-emitting material may account for 1% to 10%; or the host material may account for 95% to 99% and the light-emitting material may account for 1% to 5%. Further, the host material may include one material or two materials, where a ratio of two host materials may be 100:0 to 1:99; or the ratio may be 80:20 to 20:80; or the ratio may be 60:40 to 40:60. In the examples of the device, the characteristics of the device were also tested using conventional equipment in the art (including, but not limited to, evaporator produced by ANGSTROM ENGINEERING, optical testing system produced by SUZHOU FSTAR, life testing system produced by SUZHOU FSTAR, and ellipsometer produced by BEIJING ELLITOP, etc.) by methods well-known to the persons skilled in the art.

### Device Example

### Device Example 1

Firstly, a glass substrate having an indium tin oxide (ITO) anode with a thickness of 80 nm was cleaned and then treated with oxygen plasma and UV ozone. After the treatment, the substrate was dried in a glovebox to remove moisture. Then, the substrate was mounted on a substrate holder and placed in a vacuum chamber. Organic layers specified below were sequentially deposited through vacuum thermal evaporation on the ITO anode at a rate of 0.2 to 2 Angstroms per second and a vacuum degree of about 10⁻⁸ Torr. Compound HI was used as a hole injection layer (HIL). Compound HT was used as a hole transporting layer (HTL). Compound PH-23 was used as an electron blocking layer (EBL). Compound GD23 was doped with Compound PH-23 and Compound A-27 of the present disclosure (Compound PH-23:Compound A-27:Compound GD23 = 69:23:8) and co-deposited for use as an emissive layer (EML). Compound HB was used as a hole blocking layer (HBL). On the HBL, Compound ET and 8-hydroxyquinolinolato-lithium (Liq) were co-deposited for use as an electron transporting layer (ETL). Finally, 8-hydroxyquinolinolato-lithium (Liq) was deposited for use as an electron injection layer with a thickness of 1 nm and Al was deposited for use as a cathode with a thickness of 120 nm. The device was transferred back to the glovebox and encapsulated with a glass lid to complete the device.

### Device Comparative Example 1

Device Comparative Example 1 was prepared by the same method as Device Example 1 except that in the EML, Compound A-27 was replaced with Compound C-1.

### Device Comparative Example 2

Device Comparative Example 2 was prepared by the same method as Device Example 1 except that in the EML, Compound A-27 was replaced with Compound C-2.

### Device Comparative Example 3

Device Comparative Example 3 was prepared by the same method as Device Example 1 except that in the EML, Compound A-27 was replaced with Compound C-3.

### Device Comparative Example 4

Device Comparative Example 4 was prepared by the same method as Device Example 1 except that in the EML, Compound A-27 was replaced with Compound C-4.

### Device Comparative Example 5

Device Comparative Example 5 was prepared by the same method as Device Example 1 except that in the EML, Compound A-27 was replaced with Compound C-5.

### Device Comparative Example 6

Device Comparative Example 6 was prepared by the same method as Device Example 1 except that in the EML, Compound A-27 was replaced with Compound C-6.

### Device Comparative Example 7

Device Comparative Example 7 was prepared by the same method as Device Example 1 except that in the EML, Compound A-27 was replaced with Compound C-7.

### Device Comparative Example 8

Device Comparative Example 8 was prepared by the same method as Device Example 1 except that in the EML, Compound A-27 was replaced with Compound C-8.

### Device Comparative Example 9

Device Comparative Example 9 was prepared by the same method as Device Example 1 except that in the EML, Compound A-27 was replaced with Compound C-9.

### Device Example 2

Device Example 2 was prepared by the same method as Device Example 1 except that in the EML, Compound A-27 was replaced with Compound A-28.

### Device Example 3

Device Example 3 was prepared by the same method as Device Example 1 except that in the EML, Compound A-27 was replaced with Compound A-49.

### Device Example 4

Device Example 4 was prepared by the same method as Device Example 1 except that in the EML, Compound A-27 was replaced with Compound A-56.

### Device Example 5

Device Example 5 was prepared by the same method as Device Example 1 except that in the EML, Compound A-27 was replaced with Compound A-163.

### Device Example 6

Device Example 6 was prepared by the same method as Device Example 1 except that in the EML, Compound A-27 was replaced with Compound A-273 and PH-23:A-273:GD23 = 64:28:8.

Detailed structures and thicknesses of layers of the devices are shown in the following table. A layer using more than one material is obtained by doping different compounds at their weight ratio as recorded.

**Table 1 Partial device structures of Device Examples 1 to 6 and Comparative Examples 1 to 9**

| Device ID | HIL | HTL | EBL | EML | HBL | ETL |
|---|---|---|---|---|---|---|
| Example 1 | Compound HI (100 Å) | Compound HT (350 Å) | Compound PH-23 (50 Å) | Compound PH-23:Compound A-27:Compound GD23 (69:23:8) (400 Å) | Compound HB (50 Å) | Compound ET:Liq (40:60) (350 Å) |
| Comparative Example 1 | Compound HI (100 Å) | Compound HT (350 Å) | Compound PH-23 (50 Å) | Compound PH-23:Compound C-1:Compound GD23 (69:23:8) (400 Å) | Compound HB (50 Å) | Compound ET:Liq (40:60) (350 Å) |
| Comparative Example 2 | Compound HI (100 Å) | Compound HT (350 Å) | Compound PH-23 (50 Å) | Compound PH-23:Compound C-2:Compound GD23 (69:23:8) (400 Å) | Compound HB (50 Å) | Compound ET:Liq (40:60) (350 Å) |
| Comparative Example 3 | Compound HI (100 A) | Compound HT (350 Å) | Compound PH-23 (50 Å) | Compound PH-23: Compound C-3:Compound GD23 (69:23:8) (400 Å) | Compound HB (50 Å) | Compound ET:Liq (40:60) (350 Å) |
| Comparative Example 4 | Compound HI (100 Å) | Compound HT (350 A) | Compound PH-23 (50 Å) | Compound PH-23:Compound C-4:Compound GD23 (69:23:8) (400 Å) | Compound HB (50 Å) | Compound ET:Liq (40:60) (350 Å) |
| Comparative Example 5 | Compound HI (100 Å) | Compound HT (350 Å) | Compound PH-23 (50 Å) | Compound PH-23:Compound C-5:Compound GD23 (69:23:8) (400 Å) | Compound HB (50 Å) | Compound ET:Liq (40:60) (350 Å) |
| Comparative Example 6 | Compound HI (100 Å) | Compound HT (350 Å) | Compound PH-23 (50 Å) | Compound PH-23:Compound C-6:Compound GD23 (69:23:8) (400 Å) | Compound HB (50 Å) | Compound ET:Liq (40:60) (350 A) |
| Comparative Example 7 | Compound HI (100 Å) | Compound HT (350 Å) | Compound PH-23 (50 Å) | Compound PH-23 :Compound C-7:Compound GD23 (69:23:8) (400 Å) | Compound HB (50 Å) | Compound ET:Liq (40:60) (350 Å) |
| Comparative Example 8 | Compound HI (100 Å) | Compound HT (350 Å) | Compound PH-23 (50 A) | Compound PH-23:Compound C-8:Compound GD23 (69:23:8) (400 Å) | Compound HB (50 Å) | Compound ET:Liq (40:60) (350 Å) |
| Comparative Example 9 | Compound HI (100 Å) | Compound HT (350 Å) | Compound PH-23 (50 Å) | Compound PH-23: Compound C-9:Compound GD23 (69:23:8) (400 Å) | Compound HB (50 Å) | Compound ET:Liq (40:60) (350 Å) |
| Example 2 | Compound HI (100 Å) | Compound HT (350 Å) | Compound PH-23 (50 Å) | Compound PH-23:Compound A-2 8: Compound GD23 (69:23:8) (400 Å) | Compound HB (50 Å) | Compound ET:Liq (40:60) (350 Å) |
| Example 3 | Compound HI (100 Å) | Compound HT (350 Å) | Compound PH-23 (50 Å) | Compound PH-23:Compound A-49:Compound GD23 (69:23:8) (400 Å) | Compound HB (50 Å) | Compound ET:Liq (40:60) (350 Å) |
| Example 4 | Compound HI (100 Å) | Compound HT (350 Å) | Compound PH-23 (50 Å) | Compound PH-23:Compound A-56:Compound GD23 (69:23:8) (400 Å) | Compound HB (50 Å) | Compound ET:Liq (40:60) (350 Å) |
| Example 5 | Compound HI (100 Å) | Compound HT (350 Å) | Compound PH-23 (50 Å) | Compound PH-23:Compound A-163:Compound GD23 (69:23:8) (400 Å) | Compound HB (50 Å) | Compound ET:Liq (40:60) (350 Å) |
| Example 6 | Compound HI (100 Å) | Compound HT (350 Å) | Compound PH-23 (50 Å) | Compound PH-23:Compound A-273:Compound GD23 (64:28:8) (400 Å) | Compound HB (50 Å) | Compound ET:Liq (40:60) (350 Å) |

The materials used in the devices have the following structures:

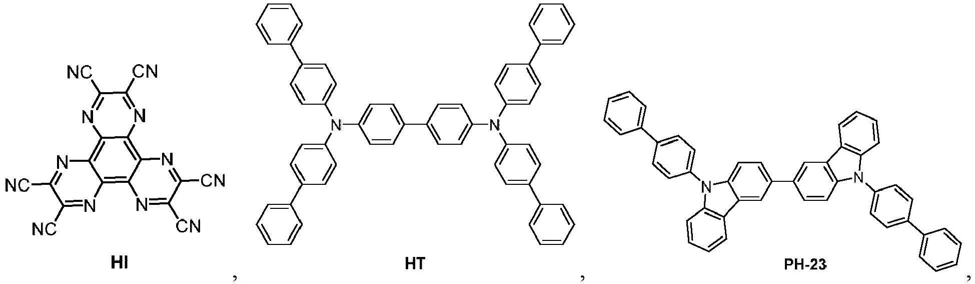

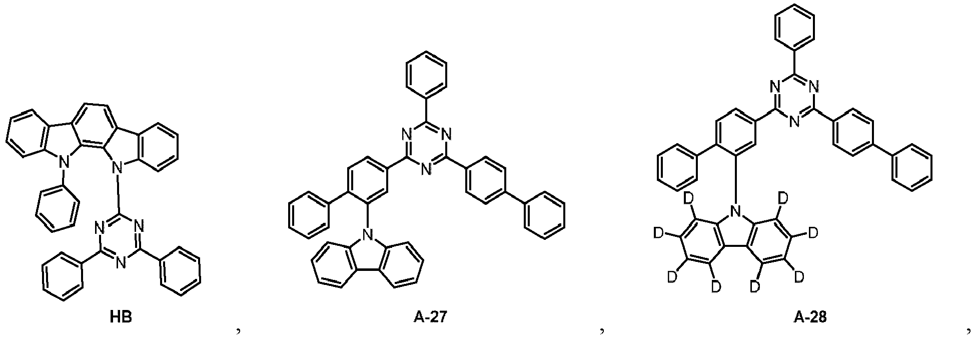

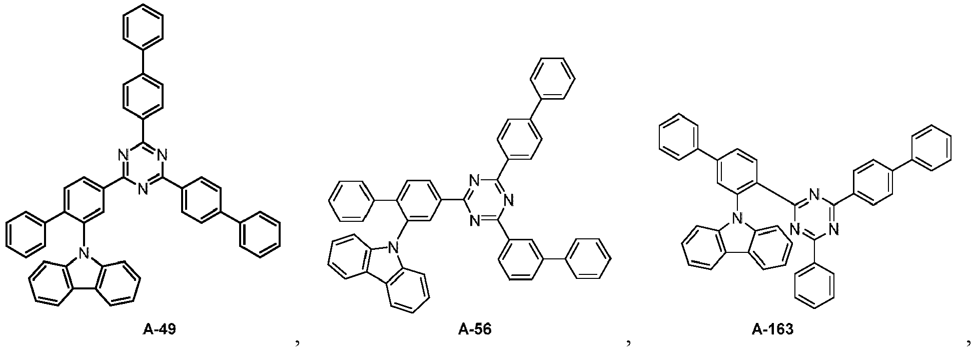

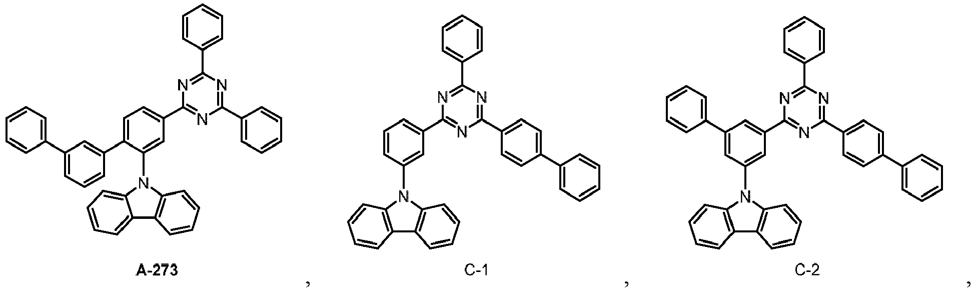

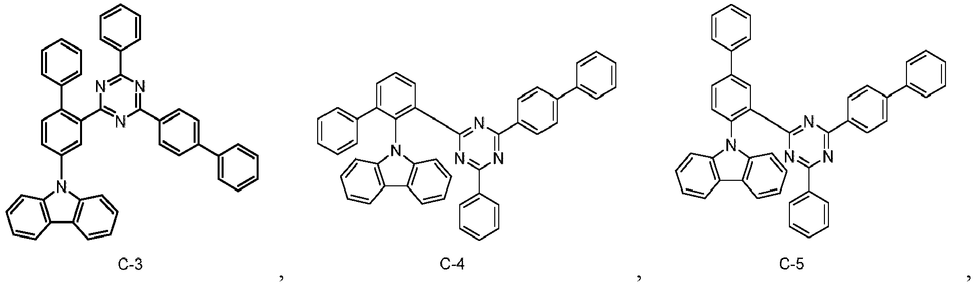

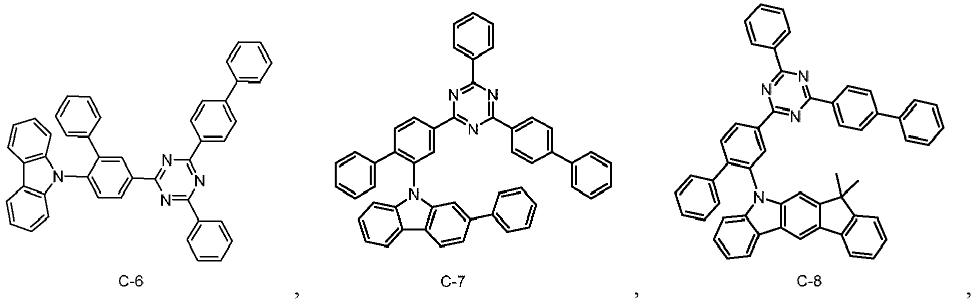

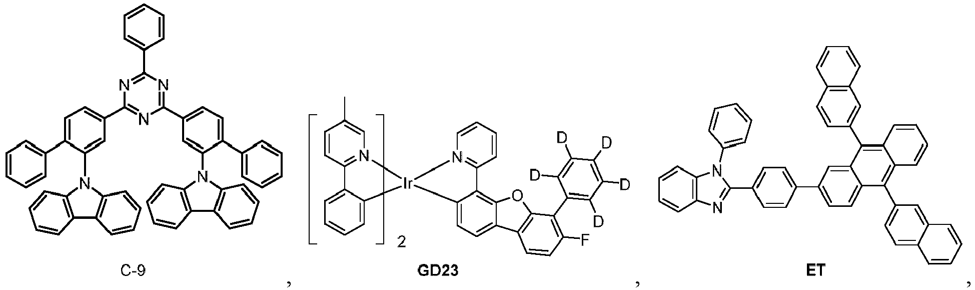

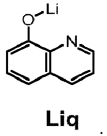

Table 2 shows the CIE data, driving voltage, external quantum efficiency (EQE), and current efficiency (CE) measured at a constant current of 15 mA/cm² and the device lifetime (LT97) measured at a constant current of 80 mA/cm².

**Table 2 Device data of Examples 1 to 6 and Comparative Examples 1 to 9**

| Device ID | EML | CIE (x, y) | Driving Voltage (V) | EQE (%) | CE (cd/A) | LT97 (h) |
|---|---|---|---|---|---|---|
| Example 1 | PH-23:A-27:GD23 (69:23:8) | (0.357, 0.619) | 4.2 | 21.9 | 83 | 52.2 |
| Comparative Example 1 | PH-23:C-1:GD23 (69:23:8) | (0.356, 0.619) | 4.4 | 21.7 | 83 | 44.9 |
| Comparative Example 2 | PH-23:C-2:GD23 (69:23:8) | (0.357, 0.619) | 4.4 | 21.6 | 83 | 38.2 |
| Comparative Example 3 | PH-23:C-3:GD23 (69:23:8) | (0.351, 0.623) | 4.2 | 21.7 | 83 | 0.05 |
| Comparative Example 4 | PH-23:C-4:GD23 (69:23:8) | (0.353, 0.622) | 4.7 | 21.7 | 83 | 36.7 |
| Comparative Example 5 | PH-23:C-5:GD23 (69:23:8) | (0.353, 0.621) | 4.4 | 21.9 | 84 | 40.0 |
| Comparative Example 6 | PH-23:C-6:GD23 (69:23:8) | (0.354, 0.620) | 4.3 | 21.8 | 83 | 42.3 |
| Comparative Example 7 | PH-23:C-7:GD23 (69:23:8) | (0.357, 0.618) | 4.4 | 21.8 | 83 | 28.2 |
| Comparative Example 8 | PH-23:C-8:GD23 (69:23:8) | (0.354, 0.621) | 4.4 | 21.3 | 82 | 30.0 |
| Comparative Example 9 | PH-23:C-9:GD23 (69:23:8) | (0.357, 0.619) | 4.6 | 20.8 | 80 | 37.2 |
| Example 2 | PH-23:A-28:GD23 (69:23:8) | (0.353, 0.622) | 3.9 | 22.0 | 84 | 54.8 |
| Example 3 | PH-23:A-49:GD23 (69:23:8) | (0.357, 0.619) | 3.8 | 21.6 | 82 | 56.2 |
| Example 4 | PH-23:A-56:GD23 (69:23:8) | (0.355, 0.620) | 4.0 | 21.8 | 83 | 52.8 |
| Example 5 | PH-23:A-163:GD2 3 (69:23:8) | (0.355, 0.620) | 4.4 | 21.9 | 84 | 55.6 |
| Example 6 | PH-23:A-273:GD2 3 (64:28:8) | (0.355, 0.620) | 4.0 | 21.9 | 84 | 55.9 |

### Discussion:

Example 1 and Comparative Example 1 used Compound A-27 of the present disclosure and Compound C-1 not provided in the present disclosure, respectively. A difference between Compound A-27 and Compound C-1 lay in whether a phenyl substituent existed on the bridging group, phenylene, joined to carbazole and triazine. Compared with Comparative Example 1, Example 1 had similar EQE and CE, a reduced driving voltage, and a device lifetime increased by 16.3%. This indicates that compared with a compound with no aryl substituent on the bridging group, phenylene, joined to carbazole and triazine, the compound of the present disclosure having the structure of Formula 1, can significantly improve device performance and especially improve a device lifetime when applied to an electroluminescent device.

Example 1, Comparative Example 2, and Comparative Example 3 used Compound A-27 of the present disclosure and Compounds C-2 and C-3 not provided in the present disclosure, respectively. Differences between Compound A-27 and Compounds C-2 and C-3 lay in different positions of the phenyl substituent on the bridging group, phenylene, joined to carbazole and triazine. In Compound A-27, phenyl was at a para position relative to triazine; in C-2, phenyl was at a meta position relative to triazine; and in C-3, phenyl was at an ortho position relative to triazine. Compared with Comparative Example 2, Example 1 had similar EQE and CE, a reduced driving voltage, and a device lifetime increased by 36.6%. Compared with Comparative Example 3, Example 1 had similar driving voltage, EQE, and CE and a device lifetime greatly increased by 1043 times. This indicates that compared with a compound with a phenyl substitution at another position of the bridging group, phenylene, joined to carbazole and triazine, the compound of the present disclosure having the structure of Formula 1, can significantly improve device performance and especially improve a device lifetime to an unexpected degree when applied to an electroluminescent device.

Example 5, Comparative Example 4, and Comparative Example 5 used Compound A-163 of the present disclosure and Compounds C-4 and C-5 not provided in the present disclosure, respectively. In Compound A-163, carbazole was joined at an ortho position relative to triazine, and differences between Compound A-163 and C-4 and C-5 lay in different positions of the phenyl substituent on the bridging group, phenylene, joined to carbazole and triazine. In Compound A-163, phenyl was at a para position relative to triazine; and in C-4 and C-5, phenyl was at a meta position relative to triazine. Compared with Comparative Example 4, Example 5 had a driving voltage reduced by 0.3 V, improved EQE and CE, and a device lifetime greatly increased by 51.5%. Compared with Comparative Example 5, Example 5 had the same driving voltage, EQE, and CE and a device lifetime greatly increased by 39.0%. Similarly, this indicates that compared with a compound with a phenyl substitution at another position of the bridging group, phenylene, joined to carbazole and triazine, the compound of the present disclosure having the structure of Formula 1, can significantly improve device performance and especially improve a device lifetime to an unexpected degree when applied to an electroluminescent device.

Example 1 and Comparative Example 6 used Compound A-27 of the present disclosure and Compound C-6 not provided in the present disclosure, respectively. A difference between Compounds A-27 and C-6 only lay in different positions of carbazole relative to triazine. In Compound A-27, carbazole was at a meta position relative to triazine; and in C-6, carbazole was at a para position relative to triazine. Compared with Comparative Example 6, Example 1 had a slightly reduced driving voltage, similar EQE and CE, and a device lifetime increased by 23.4%. This indicates that compared with a compound with carbazole at a para position relative to triazine, the compound of the present disclosure having the structure of Formula 1, can significantly improve device performance and especially improve a device lifetime when applied to an electroluminescent device.

Example 1 and Comparative Example 7 use Compound A-27 of the present disclosure and Compound C-7 not provided in the present disclosure, respectively. A difference between Compounds A-27 and C-7 only lay in whether a phenyl substitution existed on carbazole. Compared with Comparative Example 7, Example 1 had similar EQE and CE, a reduced driving voltage, and a device lifetime greatly increased by 85.1%. This indicates that compared with a compound with a phenyl substitution on carbazole, the compound of the present disclosure having the structure of Formula 1, can significantly improve device performance and especially improve a device lifetime when applied to an electroluminescent device.

Example 1 and Comparative Example 8 used Compound A-27 of the present disclosure and Compound C-8 not provided in the present disclosure, respectively. A difference between Compounds A-27 and C-8 only lay in whether carbazole was further fused with a 5-membered ring. Compared with Comparative Example 8, Example 1 had a reduced driving voltage, improved EQE and CE, and in particular a device lifetime greatly increased by 74.0%. This indicates that compared with a compound with carbazole further fused with a 5-membered ring, the compound of the present disclosure having the structure of Formula 1, can significantly improve device performance and especially improve a device lifetime when applied to an electroluminescent device.

Example 1 and Comparative Example 9 used Compound A-27 of the present disclosure and Compound C-9 not provided in the present disclosure, respectively. A difference between Compounds A-27 and C-9 only lay in the number of phenylene carbazole joined to triazine. In Compound A-27, one and only one phenylene carbazole existed on triazine, and in Compound C-9, two phenylene carbazole existed on triazine. Compared with Comparative Example 9, Example 1 had a driving voltage reduced by 0.4 V, improved EQE and CE, and a device lifetime increased by 40.3%. This indicates that compared with a compound with two phenylene carbazole substitutions on triazine, the compound of the present disclosure having the structure of Formula 1, can significantly improve device performance and especially improve a device lifetime when applied to an electroluminescent device.

Examples 2 to 6 used compounds of the present disclosure having different structures of Formula 1 and achieved the performance similar to or better than that of Example 1 in voltage, CE, EQE, and lifetime. These examples further prove the superiority of the compound of the present disclosure having the structure of Formula 1.

### Device Example 7

Device Example 7 was prepared by the same method as Device Example 3 except that in the EML, Compound PH-23 was replaced with Compound PH-24.

### Device Example 8

Device Example 8 was prepared by the same method as Device Example 7 except that in the EML, Compound A-49 was replaced with Compound A-50.

### Device Example 9

Device Example 9 was prepared by the same method as Device Example 7 except that in the EML, Compound PH-24 was replaced with Compound PH-27 and PH-27:A-49:GD23 = 64:28:8.

### Device Example 10

Device Example 10 was prepared by the same method as Device Example 9 except that in the EML, Compound A-49 was replaced with Compound A-185.

### Device Example 11

Device Example 11 was prepared by the same method as Device Example 9 except that in the EML, Compound A-49 was replaced with Compound A-192.

### Device Example 20

Device Example 20 was prepared by the same method as Device Example 1 except that in the EML, Compound PH-23 was replaced with Compound PH-104 and PH-104:A-27:GD23 = 64:28:8.

### Device Example 21

Device Example 21 was prepared by the same method as Device Example 20 except that in the EML, Compound PH-104 was replaced with Compound PH-105.

### Device Example 22

Device Example 22 was prepared by the same method as Device Example 20 except that in the EML, Compound PH-104 was replaced with Compound PH-109.

Detailed structures and thicknesses of layers of the devices are shown in the following table. A layer using more than one material is obtained by doping different compounds at their weight ratio as recorded.

**Table 3 Partial device structures of Device Examples 7 to 11 and Examples 20 to 22**

| Device ID | HIL | HTL | EBL | EML | HBL | ETL |
|---|---|---|---|---|---|---|
| Example 7 | Compound HI (100 Å) | Compound HT (350 Å) | Compound PH-23 (50 Å) | Compound PH-24:Compound A-49:Compound GD23 (69:23:8) (400 Å) | Compound HB (50 Å) | Compound ET:Liq (40:60) (350 Å) |
| Example 8 | Compound HI (100 Å) | Compound HT (350 Å) | Compound PH-23 (50 Å) | Compound PH-24:Compound A-50:Compound GD23 (69:23:8) (400 Å) | Compound HB (50 Å) | Compound ET:Liq (40:60) (350 Å) |
| Example 9 | Compound HI (100 Å) | Compound HT (350 Å) | Compound PH-23 (50 Å) | Compound PH-27:Compound A-49:Compound GD23 (64:28:8) (400 Å) | Compound HB (50 Å) | Compound ET:Liq (40:60) (350 Å) |
| Example 10 | Compound HI (100 Å) | Compound HT (350 Å) | Compound PH-23 (50 Å) | Compound PH-27:Compound A-185:Compound GD23 (64:28:8) (400 Å) | Compound HB (50 Å) | Compound ET:Liq (40:60) (350 Å) |
| Example 11 | Compound HI (100 Å) | Compound HT (350 Å) | Compound PH-23 (50 Å) | Compound PH-27:Compound A-192:Compound GD23 (64:28:8) (400 Å) | Compound HB (50 Å) | Compound ET:Liq (40:60) (350 Å) |
| Example 20 | Compound HI (100 Å) | Compound HT (350 Å) | Compound PH-23 (50 Å) | Compound PH-104:Compound A-27:Compound GD23 (64:28:8) (400 Å) | Compound HB (50 Å) | Compound ET:Liq (40:60) (350 Å) |
| Example 21 | Compound HI (100 Å) | Compound HT (350 Å) | Compound PH-23 (50 Å) | Compound PH-105:Compound A-27:Compound GD23 (64:28:8) (400 Å) | Compound HB (50 Å) | Compound ET:Liq (40:60) (350 Å) |
| Example 22 | Compound HI (100 Å) | Compound HT (350 Å) | Compound PH-23 (50 Å) | Compound PH-109:Compound A-27:Compound GD23 (64:28:8) (400 Å) | Compound HB (50 Å) | Compound ET:Liq (40:60) (350 Å) |

The new materials used in the devices have the following structures:

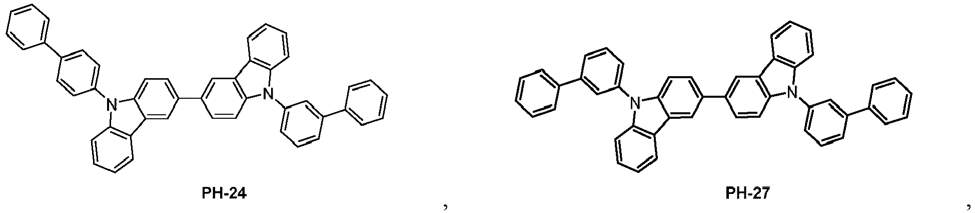

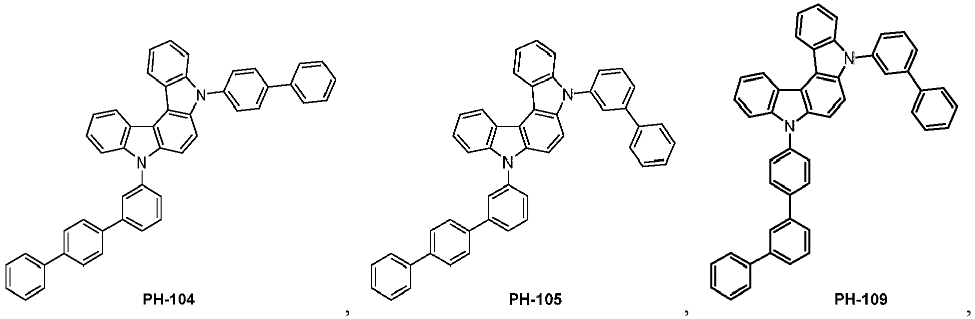

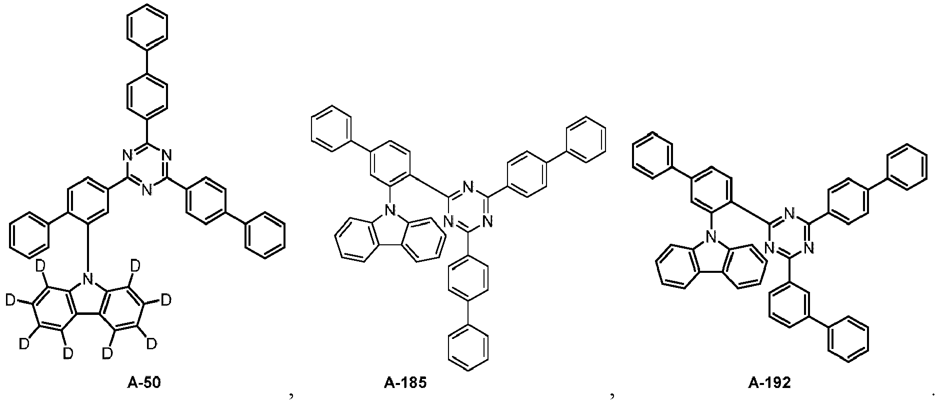

Table 4 shows the CIE data, driving voltage, external quantum efficiency (EQE), and current efficiency (CE) measured at a constant current of 15 mA/cm² and the device lifetime (LT97) measured at a constant current of 80 mA/cm².

**Table 4 Device data of Examples 7 to 11 and Examples 20 to 22**

| Device ID | EML | CIE (x, y) | Driving Voltage (V) | EQE (%) | CE (cd/A) | LT97 (h) |
|---|---|---|---|---|---|---|
| Example 7 | PH-24:A-49:GD23 (69:23:8) | (0.356, 0.619) | 4.1 | 21.8 | 83 | 57.0 |
| Example 8 | PH-24:A-50:GD23 (69:23:8) | (0.353, 0.621) | 4.1 | 21.7 | 83 | 57.8 |
| Example 9 | PH-27:A-49:GD23 (64:28:8) | (0.352, 0.622) | 4.0 | 21.5 | 82 | 54.0 |
| Example 10 | PH-27:A-185:GD2 3 (64:28:8) | (0.351, 0.623) | 4.1 | 21.7 | 83 | 56.5 |
| Example 11 | PH-27:A-192:GD2 3 (64:28:8) | (0.355, 0.620) | 4.2 | 21.9 | 84 | 54.9 |
| Example 20 | PH-104:A-27:GD2 3 (64:28:8) | (0.356, 0.620) | 3.8 | 21.6 | 83 | 55.7 |
| Example 21 | PH-105:A-27:GD2 3 (64:28:8) | (0.356, 0.620) | 4.0 | 22.0 | 84 | 60.2 |
| Example 22 | PH-109:A-27:GD2 3 (64:28:8) | (0.352, 0.623) | 3.8 | 22.2 | 85 | 53.8 |

### Discussion:

Examples 7 to 11 and Examples 20 to 22 used different compounds of the present disclosure in combination with different second host compounds, respectively and the devices all achieved high efficiency and long lifetimes, indicating that the compounds of the present disclosure can be combined with a wide variety of second host compounds and achieve good device performance.

### Device Example 12

Device Example 12 was prepared by the same method as Device Example 7 except that in the EML, Compound GD23 was replaced with Compound GD15 and PH-24:A-49:GD15 = 56:38:6.

### Device Example 13

Device Example 13 was prepared by the same method as Device Example 12 except that in the EML, Compound GD15 was replaced with Compound GD2 and PH-24:A-49:GD2 = 61:33:6.

### Device Example 14

Device Example 14 was prepared by the same method as Device Example 13 except that in the EML, Compound A-49 was replaced with Compound A-50.

### Device Example 15

Device Example 15 was prepared by the same method as Device Example 9 except that in the EML, Compound GD23 was replaced with Compound GD2 and PH-27:A-49:GD2 = 66:28:6.

### Device Example 16

Device Example 16 was prepared by the same method as Device Example 15 except that in the EML, Compound A-49 was replaced with Compound A-185.

Detailed structures and thicknesses of layers of the devices are shown in the following table. A layer using more than one material is obtained by doping different compounds at their weight ratio as recorded.

**Table 5 Partial device structures of Device Examples 12 to 16**

| Device ID | HIL | HTL | EBL | EML | HBL | ETL |
|---|---|---|---|---|---|---|
| Example 12 | Compound HI (100 Å) | Compound HT (350 Å) | Compound PH-23 (50 Å) | Compound PH-24:Compound A-49:Compound GD15 (56:38:6) (400 Å) | Compound HB (50 Å) | Compound ET:Liq (40:60) (350 Å) |
| Example 13 | Compound HI (100 Å) | Compound HT (350 Å) | Compound PH-23 (50 Å) | Compound PH-24:Compound A-49:Compound GD2 (61:33:6) (400 Å) | Compound HB (50 Å) | Compound ET:Liq (40:60) (350 Å) |
| Example 14 | Compound HI (100 Å) | Compound HT (350 Å) | Compound PH-23 (50 Å) | Compound PH-24:Compound A-50:Compound GD2 (61:33:6) (400 Å) | Compound HB (50 Å) | Compound ET:Liq (40:60) (350 Å) |
| Example 15 | Compound HI (100 Å) | Compound HT (350 Å) | Compound PH-23 (50 Å) | Compound PH-27:Compound A-49:Compound GD2 (66:28:6) (400 Å) | Compound HB (50 Å) | Compound ET:Liq (40:60) (350 Å) |
| Example 16 | Compound HI (100 Å) | Compound HT (350 Å) | Compound PH-23 (50 Å) | Compound PH-27:Compound A-185:Compound GD2 (66:28:6) (400 Å) | Compound HB (50 Å) | Compound ET:Liq (40:60) (350 Å) |

The new materials used in the devices have the following structures:

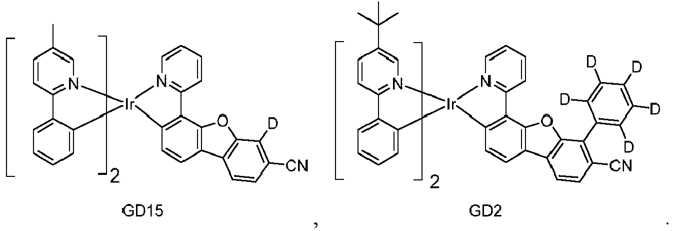

Table 6 shows the CIE data, driving voltage, external quantum efficiency (EQE), and current efficiency (CE) measured at a constant current of 15 mA/cm² and the device lifetime (LT97) measured at a constant current of 80 mA/cm².

**Table 6 Device data of Examples 12 to 16**

| Device ID | EML | CIE (x, y) | Driving Voltage (V) | EQE (%) | CE (cd/A) | LT97 (h) |
|---|---|---|---|---|---|---|
| Example 12 | PH-24:A-49:GD15 (56:38:6) | (0.346, 0.630) | 3.6 | 22.4 | 87 | 47.7 |
| Example 13 | PH-24:A-49:GD2 (61:33:6) | (0.344, 0.633) | 3.5 | 23.7 | 93 | 57.5 |
| Example 14 | PH-24:A-50:GD2 (61:33:6) | (0.344, 0.633) | 3.5 | 23.6 | 93 | 56.8 |
| Example 15 | PH-27:A-49: GD2 (66:28:6) | (0.341, 0.635) | 3.6 | 23.8 | 94 | 63.0 |
| Example 16 | PH-27:A-185:GD2 (66:28:6) | (0.339, 0.637) | 3.7 | 24.0 | 95 | 65.3 |

### Discussion:

Examples 12 to 16 used different compounds of the present disclosure in combination with different green phosphorescent dopants, respectively and the devices all exhibited extremely low driving voltages, high efficiency, and long lifetimes, indicating that the compound of the present disclosure having the structure of Formula 1 can be combined with a wide variety of green phosphorescent dopants and achieve good device performance.

### Device Example 23

Firstly, a glass substrate having an indium tin oxide (ITO) anode with a thickness of 80 nm was cleaned and then treated with oxygen plasma and UV ozone. After the treatment, the substrate was dried in a glovebox to remove moisture. Then, the substrate was mounted on a substrate holder and placed in a vacuum chamber. Organic layers specified below were sequentially deposited through vacuum thermal evaporation on the ITO anode at a rate of 0.1 to 2 Angstroms per second and a vacuum degree of about 10⁻⁸ Torr. Compound HT and Compound HT1 were co-deposited for use as a hole injection layer (HIL) with a thickness of 100 Å, where the weight ratio of Compound HT:Compound HT1 was 97:3. Compound HT was used as a hole transporting layer (HTL) with a thickness of 350 Å. Compound PH-23 was used as an electron blocking layer (EBL) with a thickness of 50 Å. Compound GD23 was doped with Compound PH-24 and Compound A-65 of the present disclosure and co-deposited for use as an emissive layer (EML) with a thickness of 400 Å, where the weight ratio of Compound PH-24:Compound A-65:Compound GD23 was 64:28:8. Compound HB was used as a hole blocking layer (HBL) with a thickness of 50 Å. On the HBL, Compound ET and 8-hydroxyquinolinolato-lithium (Liq) were co-deposited for use as an electron transporting layer (ETL) with a thickness of 350 Å. Finally, 8-hydroxyquinolinolato-lithium (Liq) was deposited for use as an electron injection layer with a thickness of 1 nm and Al was deposited for use as a cathode with a thickness of 1200 Å. The device was transferred back to the glovebox and encapsulated with a glass lid to complete the device.

### Device Example 24

Device Example 24 was prepared by the same method as Device Example 23 except that in the EML, Compound PH-24 was replaced with Compound PH-121, Compound A-65 was replaced with Compound A-56, Compound GD23 was replaced with Compound GD2, and PH-121:A-56:GD2 = 66:28:6.

### Device Example 25

Device Example 25 was prepared by the same method as Device Example 24 except that in the EML, Compound A-56 was replaced with Compound A-77.

### Device Example 26

Device Example 26 was prepared by the same method as Device Example 24 except that in the EBL, Compound PH-23 was replaced with Compound PH-1, and in the EML, Compound GD2 was replaced with Compound GD66 and PH-121:A-56:GD66 = 64:28:8.

### Device Example 27

Device Example 27 was prepared by the same method as Device Example 26 except that in the HIL, Compound HT and Compound HT1 were replaced with Compound HI, in the EBL, Compound PH-1 was replaced with Compound PH-23, and in the EML, Compound PH-121 was replaced with Compound PH-24, Compound A-56 was replaced with Compound A-50, and PH-24:A-50:GD66 = 70:24:6.

### Device Example 28

Device Example 28 was prepared by the same method as Device Example 26 except that in the EML, Compound PH-121 was replaced with Compound PH-104 and Compound A-56 was replaced with Compound A-49.

### Device Example 29

Device Example 29 was prepared by the same method as Device Example 26 except that in the EML, Compound GD66 was replaced with Compound GD77.

### Device Example 30

Device Example 30 was prepared by the same method as Device Example 27 except that in the HIL, Compound HI was replaced with Compound HT and Compound HT1 (where the weight ratio of Compound HT:Compound HT1 was 97:3), and in the EML, Compound GD66 was replaced with Compound GD77 and PH-24:A-50:GD77 = 64:28:8.

### Device Example 31

Device Example 31 was prepared by the same method as Device Example 28 except that in the EML, Compound GD66 was replaced with Compound GD77.

### Device Example 33

Device Example 33 was prepared by the same method as Device Example 31 except that in the EML, Compound PH-104 was replaced with Compound PH-130.

### Device Example 34

Device Example 34 was prepared by the same method as Device Example 33 except that in the EML, Compound GD77 was replaced with Compound GD66.

### Device Example 35

Device Example 35 was prepared by the same method as Device Example 33 except that in the EML, Compound GD77 was replaced with Compound GD78 and PH-130:A-49:GD78 = 66:28:6.

Detailed structures and thicknesses of layers of the devices are shown in the following table. A layer using more than one material is obtained by doping different compounds at their weight ratio as recorded.

**Table 11 Partial device structures of Device Examples 23 to 31 and Examples 33 to 35**

| Device ID | HIL | HTL | EBL | EML | HBL | ETL |
|---|---|---|---|---|---|---|
| Example 23 | Compound HT: Compound HT1 (97:3) (100 Å) | Compound HT (350 Å) | Compound PH-23 (50 Å) | Compound PH-24:Compound A-65:Compound GD23 (64:28:8) (400 Å) | Compound HB (50 Å) | Compound ET:Liq (40:60) (350 Å) |
| Example 24 | Compound HT: Compound HT1 (97:3) (100 Å) | Compound HT (350 Å) | Compound PH-23 (50 Å) | Compound PH-121:Compound A-56:Compound GD2 (66:28:6) (400 Å) | Compound HB (50 Å) | Compound ET:Liq (40:60) (350 Å) |
| Example 25 | Compound HT: Compound HT1 (97:3) (100 Å) | Compound HT (350 Å) | Compound PH-23 (50 Å) | Compound PH-121:Compound A-77:Compound GD2 (66:28:6) (400 Å) | Compound HB (50 Å) | Compound ET:Liq (40:60) (350 Å) |
| Example 26 | Compound HT: Compound HT1 (97:3) (100 Å) | Compound HT (350 Å) | Compound PH-1 (50 Å) | Compound PH-121:Compound A-56:Compound GD66 (64:28:8) (400 Å) | Compound HB (50 Å) | Compound ET:Liq (40:60) (350 Å) |
| Example 27 | Compound HI (100 Å) | Compound HT (350 Å) | Compound PH-23 (50 Å) | Compound PH-24:Compound A-50:Compound GD66 (70:24:6) (400 Å) | Compound HB (50 Å) | Compound ET:Liq (40:60) (350 Å) |
| Example 28 | Compound HT: Compound HT1 (97:3) (100 Å) | Compound HT (350 Å) | Compound PH-1 (50 Å) | Compound PH-104:Compound A-49:Compound GD66 (64:28:8) (400 Å) | Compound HB (50 Å) | Compound ET:Liq (40:60) (350 Å) |
| Example 29 | Compound HT: Compound HT1 (97:3) (100 Å) | Compound HT (350 Å) | Compound PH-1 (50 Å) | Compound PH-121:Compound A-5 6:Compound GD77 (64:28:8) (400 Å) | Compound HB (50 Å) | Compound ET:Liq (40:60) (350 Å) |
| Example 30 | Compound HT:Compound HT1 (97:3) (100 Å) | Compound HT (350 Å) | Compound PH-23 (50 Å) | Compound PH-24:Compound A-50:Compound GD77 (64:28:8) (400 Å) | Compound HB (50 Å) | Compound ET:Liq (40:60) (350 Å) |
| Example 31 | Compound HT:Compound HT1 (97:3) (100 Å) | Compound HT (350 Å) | Compound PH-1 (50 Å) | Compound PH-104:Compound A-49:Compound GD77 (64:28:8) (400 Å) | Compound HB (50 Å) | Compound ET:Liq (40:60) (350 Å) |
| Example 33 | Compound HT:Compound HT1 (97:3) (100 Å) | Compound HT (350 Å) | Compound PH-1 (50 Å) | Compound PH-130:Compound A-49:Compound GD77 (64:28:8) (400 Å) | Compound HB (50 Å) | Compound ET:Liq (40:60) (350 Å) |
| Example 34 | Compound HT:Compound HT1 (97:3) (100 Å) | Compound HT (350 Å) | Compound PH-1 (50 Å) | Compound PH-130:Compound A-49:Compound GD66 (64:28:8) (400 Å) | Compound HB (50 Å) | Compound ET:Liq (40:60) (350 Å) |
| Example 35 | Compound HT:Compound HT1 (97:3) (100 Å) | Compound HT (350 Å) | Compound PH-1 (50 Å) | Compound PH-130:Compound A-49:Compound GD78 (66:28:6) (400 Å) | Compound HB (50 Å) | Compound ET:Liq (40:60) (350 Å) |

The new materials used in the devices have the following structures:

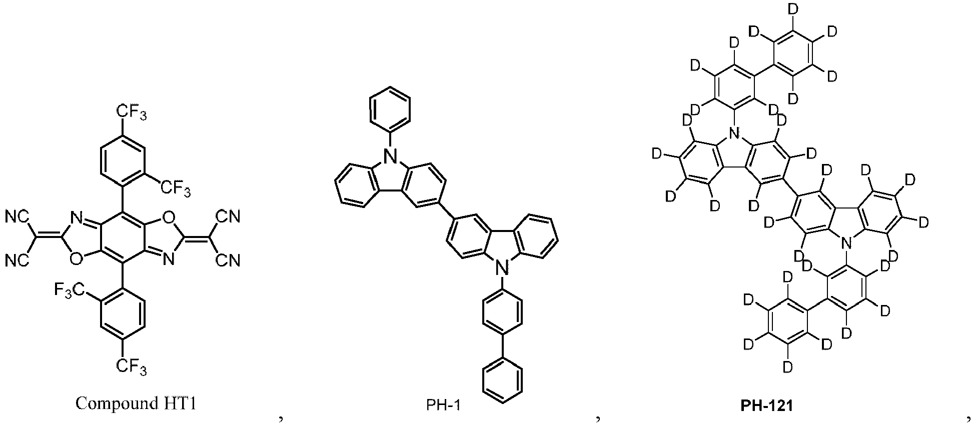

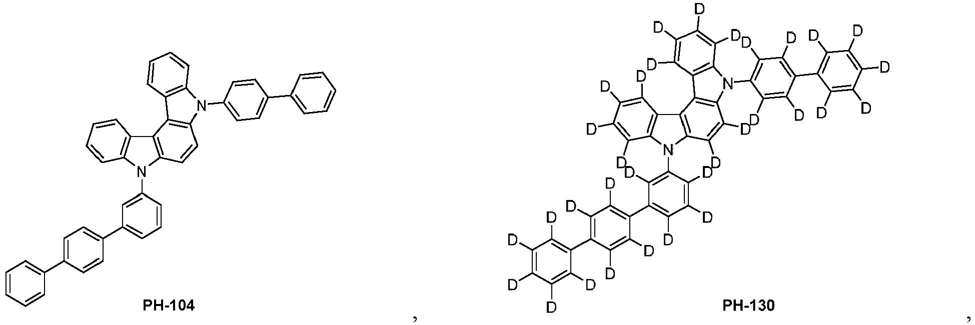

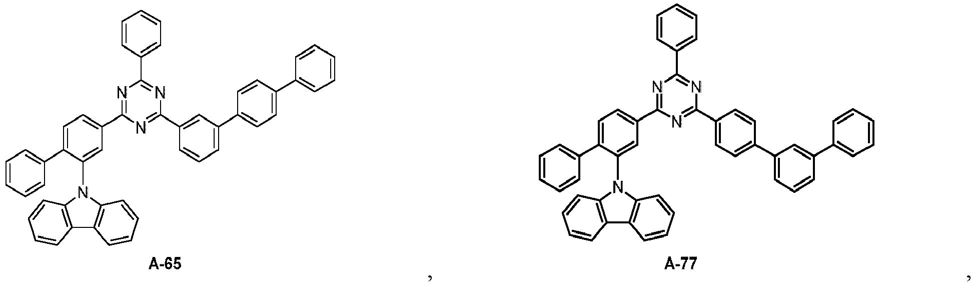

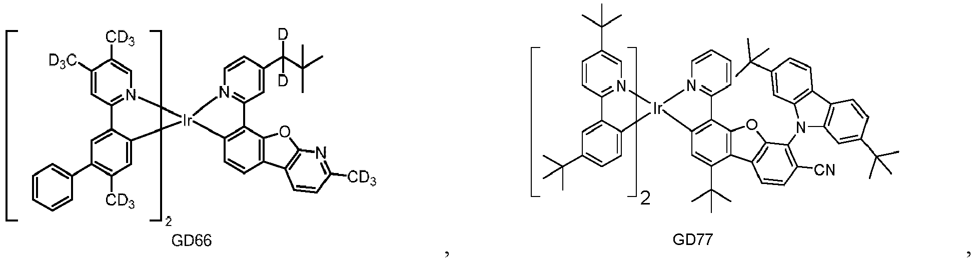

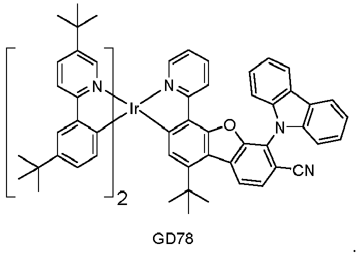

Table 12 shows the CIE data, driving voltages, external quantum efficiency (EQE), and current efficiency (CE) of Examples 23 to 25 measured at a constant current of 15 mA/cm² and the device lifetime (LT97) measured at a constant current of 80 mA/cm².

**Table 12 Device data of Examples 23 to 25**

| Device ID | EML | CIE (x, y) | Driving Voltage (V) | EQE (%) | CE (cd/A) | LT97 (h) |
|---|---|---|---|---|---|---|
| Example 23 | PH-24:A-65:GD23 (64:28:8) | (0.350, 0.624) | 4.4 | 22.7 | 87 | 66.9 |
| Example 24 | PH-121:A-56:GD2 (66:28:6) | (0.341, 0.635) | 3.7 | 24.2 | 95 | 65.0 |
| Example 25 | PH-121:A-77:GD2 (66:28:6) | (0.340, 0.636) | 3.7 | 24.2 | 96 | 65.7 |

Examples 23 to 25 use compounds of the present disclosure having different structures of Formula 1 in combination with different second host compounds and green phosphorescent dopants, respectively and the devices all achieve relatively low driving voltages, high efficiency, and long lifetimes. These examples further prove the superiority of the compound of the present disclosure having the structure of Formula 1.

Table 13 shows the CIE data, driving voltages, external quantum efficiency (EQE), and current efficiency (CE) of Examples 26 to 31 and Example 33 measured at a constant current of 15 mA/cm².

**Table 13 Device data of Examples 26 to 31 and Example 33**

| Device ID | EML | CIE (x, y) | Driving Voltage (V) | EQE (%) | CE (cd/A) |
|---|---|---|---|---|---|
| Example 26 | PH-121:A-56: GD66 (64:28:8) | (0.341, 0.629) | 4.5 | 23.4 | 89 |
| Example 27 | PH-24:A-50: GD66 (70:24:6) | (0.341, 0.629) | 4.4 | 22.6 | 85 |
| Example 28 | PH-104:A-49: GD66 (64:28:8) | (0.340, 0.630) | 4.0 | 23.3 | 89 |
| Example 29 | PH-121:A-56: GD77 (64:28:8) | (0.301, 0.661) | 4.4 | 26.8 | 105 |
| Example 30 | PH-24:A-50:GD77 (64:28:8) | (0.308, 0.658) | 4.2 | 26.2 | 103 |
| Example 31 | PH-104:A-49: GD77 (64:28:8) | (0.299, 0.663) | 3.8 | 27.1 | 107 |
| Example 33 | PH-130:A-49:GD7 7 (64:28:8) | (0.341, 0.635) | 3.7 | 24.2 | 95 |

Examples 26 to 31 and Example 33 use compounds of the present disclosure having different structures of Formula 1 in combination with different second host compounds and green phosphorescent dopants, respectively and the devices all achieve relatively low driving voltages and high efficiency. These examples further prove the superiority of the compound of the present disclosure having the structure of Formula 1.

Table 14 shows the CIE data, driving voltages, external quantum efficiency (EQE), and current efficiency (CE) of Examples 34 and 35 measured at a constant current of 15 mA/cm² and the device lifetime (LT97) measured at a constant current of 80 mA/cm².

**Table 14 Device data of Examples 34 and 35**

| Device ID | EML | CIE (x, y) | Driving Voltage (V) | EQE (%) | CE (cd/A) | LT97 (h) |
|---|---|---|---|---|---|---|
| Example 34 | PH-130:A-49:GD 66 (64:28:8) | (0.306, 0.658) | 3.9 | 26.6 | 105 | 55.0 |
| Example 35 | PH-130:A-49:GD 78 (66:28:6) | (0.314, 0.652) | 3.6 | 24.4 | 96 | 57.0 |

Examples 34 and 35 use the compound of Formula 1 of the present disclosure in combination with different second host compounds and green phosphorescent dopants, respectively and the devices all achieve relatively low voltages, high efficiency, and long lifetimes. These examples further prove the superiority of the compound of the present disclosure having the structure of Formula 1.

### Device Example 17

Firstly, a glass substrate having an indium tin oxide (ITO) anode with a thickness of 120 nm was cleaned and then treated with oxygen plasma and UV ozone. After the treatment, the substrate was dried in a nitrogen-filled glovebox to remove moisture and then mounted on a substrate holder and placed in a vacuum chamber. Organic layers specified below were sequentially deposited through vacuum thermal evaporation on the ITO anode at a rate of 0.01 to 5 Å/s and at a vacuum degree of about 10⁻⁸ Torr. Compound HT and Compound HT1 were co-deposited for use as a hole injection layer (HIL) (100 Å). Compound HT was used as a hole transporting layer (HTL) (400 Å). Compound HT2 was used as an electron blocking layer (EBL) (50 Å). Compound RD28 was doped with Compound PH-44 and Compound A-528 of the present disclosure (Compound PH-44:Compound A-528:Compound RD28 = 49:49:2) and co-deposited for use as an emissive layer (EML) (400 Å). Compound HB was used as a hole blocking layer (HBL) (50 Å). On the HBL, Compound ET and 8-hydroxyquinolinolato-lithium (Liq) were co-deposited for use as an electron transporting layer (ETL) (350 Å). Finally, 8-hydroxyquinolinolato-lithium (Liq) was deposited for use as an electron injection layer (EIL) with a thickness of 10 Å and Al was deposited for use as a cathode with a thickness of 1200 Å. The device was transferred back to the glovebox and encapsulated with a glass lid to complete the device.

### Device Example 18

Device Example 18 was prepared by the same method as Device Example 17 except that in the EML, Compound PH-44 was replaced with Compound PH-81 and PH-81:A-528:RD28 = 88:10:2.

### Device Example 32

Device Example 32 was prepared by the same method as Device Example 17 except that in the EML, Compound A-528 was replaced with Compound A-95, Compound RD28 was replaced with Compound RD135, and Compound PH-44:A-95:RD135 = 39:59:2.

Detailed structures and thicknesses of layers of the devices are shown in the following table. A layer using more than one material is obtained by doping different compounds at their weight ratio as recorded.

**Table 7 Partial device structures of Device Examples 17 and 18 and Example 32**

| Device ID | HIL | HTL | EBL | EML | HBL | ETL |
|---|---|---|---|---|---|---|
| Example 17 | Compound HT:Compound HT1 (97:3) (100 Å) | Compound HT (400 Å) | Compound HT2 (50 Å) | Compound PH-44:Compound A-528:Compound RD28 (49:49:2) (400 Å) | Compound HB (50 Å) | Compound ET:Liq (40:60) (350 Å) |
| Example 18 | Compound HT:Compound HT1 (97:3) (100 Å) | Compound HT (400 Å) | Compound HT2 (50 Å) | Compound PH-81:Compound A-528:Compound RD28 (88:10:2) (400 Å) | Compound HB (50 Å) | Compound ET:Liq (40:60) (350 Å) |
| Example 32 | Compound HT:Compound HT1 (97:3) (100 Å) | Compound HT (400 Å) | Compound HT2 (50 Å) | Compound PH-44:Compound A-95:Compound RD135 (39:59:2) (400 Å) | Compound HB (50 Å) | Compound ET:Liq (40:60) (350 Å) |

The new materials used in the devices have the following structures:

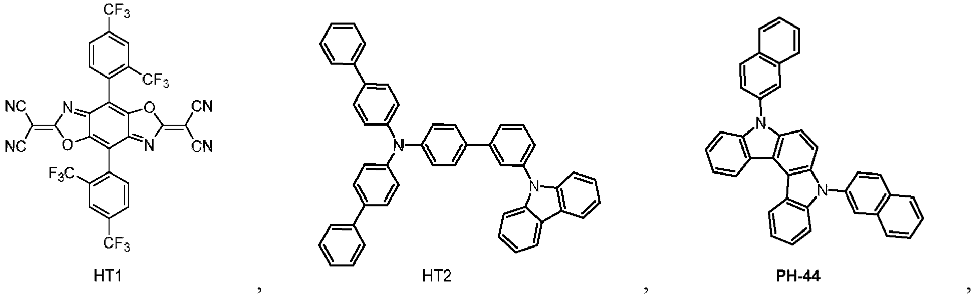

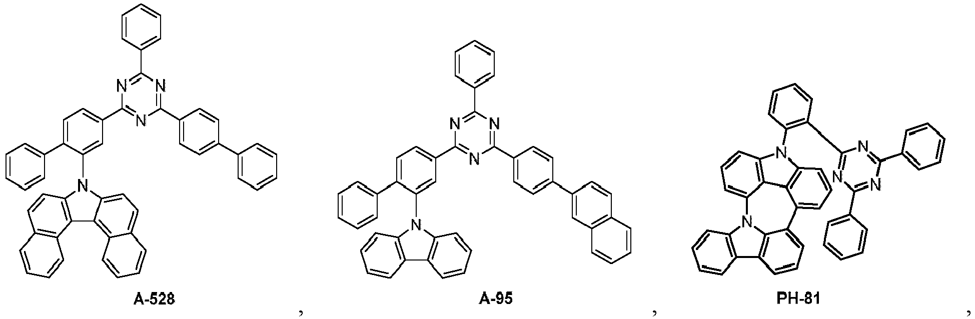

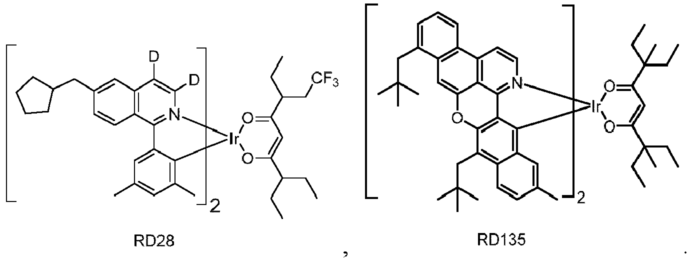

Table 8 shows the CIE data, driving voltage, external quantum efficiency (EQE), and current efficiency (CE) measured at a constant current of 15 mA/cm² and the device lifetime (LT97) measured at a constant current of 80 mA/cm².

**Table 8 Device data of Examples 17 and 18 and Example 32**

| Device ID | EML | CIE (x, y) | Driving Voltage (V) | EQE (%) | CE (cd/A) | LT97 (h) |
|---|---|---|---|---|---|---|
| Example 17 | PH-44:A-528:RD2 8 (49:49:2) | (0.677, 0.322) | 3.76 | 21.51 | 21.0 | 136.0 |
| Example 18 | PH-81:A-528:RD2 8 (88:10:2) | (0.678, 0.321) | 3.63 | 25.13 | 23.74 | 155.0 |
| Example 32 | PH-44:A-95:RD13 5 (39:59:2) | (0.683, 0.316) | 3.82 | 26.84 | 28.17 | 89.5 |

### Discussion:

Examples 17 and 18 used Compound A-528 of the present disclosure comprising carbazole fused with 6-membered rings in a red phosphorescent device, Example 32 used Compound A-95 of the present disclosure comprising triazine joined to a phenyl-naphthyl structure in a red phosphorescent device, and the devices all exhibited low driving voltages, high efficiency, and long lifetimes. This indicates that the compound of the present disclosure having the structure of Formula 1 is also a good red light host material.

In summary, when used as a host material of the light-emitting layer, the compound of the present disclosure improves the electron and hole transporting balance ability of the material. Compared with the use of the compound not provided in the present disclosure as the host material of the light-emitting layer, the use of the compound of the present disclosure can achieve a similar or reduced driving voltage, similar or improved device efficiency (EQE and CE), and a greatly increased device lifetime and can significantly improve the overall performance of the device. The compound of the present disclosure is of great help to the industry.

### Device Example 19

Firstly, a glass substrate having an indium tin oxide (ITO) anode with a thickness of 80 nm was cleaned and then treated with oxygen plasma and UV ozone. After the treatment, the substrate was dried in a glovebox to remove moisture. Then, the substrate was mounted on a substrate holder and placed in a vacuum chamber. Organic layers specified below were sequentially deposited through vacuum thermal evaporation on the ITO anode at a rate of 0.2 to 2 Angstroms per second and a vacuum degree of about 10⁻⁸ Torr. Compound HI was used as a hole injection layer (HIL). Compound HT was used as a hole transporting layer (HTL). Compound PH-23 was used as an electron blocking layer (EBL). Compound GD23 was doped with Compound PH-23 and Compound NH-1 and co-deposited for use as an emissive layer (EML). Compound HB was used as a hole blocking layer (HBL). On the HBL, Compound A-27 of the present disclosure and 8-hydroxyquinolinolato-lithium (Liq) were co-deposited for use as an electron transporting layer (ETL). Finally, 8-hydroxyquinolinolato-lithium (Liq) was deposited for use as an electron injection layer with a thickness of 1 nm and Al was deposited for use as a cathode with a thickness of 120 nm. The device was transferred back to the glovebox and encapsulated with a glass lid to complete the device.

### Device Comparative Example 10

Device Comparative Example 10 was prepared by the same method as Device Example 19 except that in the ETL, Compound A-27 was replaced with Compound ET.

Detailed structures and thicknesses of layers of the devices are shown in the following table. A layer using more than one material is obtained by doping different compounds at their weight ratio as recorded.

**Table 9 Partial device structures of Device Example 19 and Comparative Example 10**

| Device ID | HIL | HTL | EBL | EML | HBL | ETL |
|---|---|---|---|---|---|---|
| Example 19 | Compound HI (100 Å) | Compound HT (350 Å) | Compound PH-23 (50 Å) | Compound PH-23:Compound NH-1:Compound GD23 (46:46:8) (400 Å) | Compound HB (50 Å) | Compound A-27:Liq (40:60) (350 Å) |
| Comparative Example 10 | Compound HI (100 Å) | Compound HT (350 Å) | Compound PH-23 (50 Å) | Compound PH-23 : Compound NH-1:Compound GD23 (46:46:8) (400 Å) | Compound HB (50 Å) | Compound ET:Liq (40:60) (350 Å) |

The new material used in the devices has the following structure:

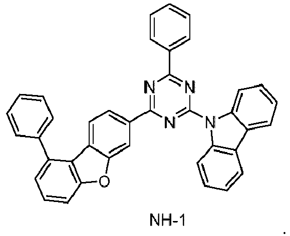

Table 10 shows the CIE data, driving voltage (V), and external quantum efficiency (EQE) measured at a constant current of 15 mA/cm² and the device lifetime (LT97) measured at a constant current of 80 mA/cm².

**Table 10 Device Data of Example 19 and Comparative Example 10**

| Device ID | ETL | CIE (x, y) | Driving Voltage (V) | EQE (%) | Lifetime LT97 (h) |
|---|---|---|---|---|---|
| Example 19 | A-27:Liq (40:60) | (0.357, 0.619) | 3.7 | 21.3 | 43.5 |
| Comparative Example 10 | ET:Liq (40:60) | (0.359, 0.617) | 3.7 | 20.9 | 42.8 |

### Discussion:

Example 19 and Comparative Example 10 used Compound A-27 of the present disclosure and Compound ET not provided in the present disclosure as an electron transporting material, respectively. Compared with Comparative Example 10, Example 19 had the same driving voltage, improved EQE, and an increased device lifetime. It is to be noted that Compound ET is a commercial electron transporting material at present. It can be seen that the compound of the present disclosure is also an excellent electron transporting material.

It should be understood that various embodiments described herein are merely embodiments and not intended to limit the scope of the present disclosure. Therefore, it is apparent to those skilled in the art that the present disclosure as claimed may include variations of specific embodiments and preferred embodiments described herein. Many of the materials and structures described herein may be replaced with other materials and structures without departing from the spirit of the present disclosure. It should be understood that various theories as to why the present disclosure works are not intended to be limitative.

## Claims

1. A compound having a structure of Formula 1: 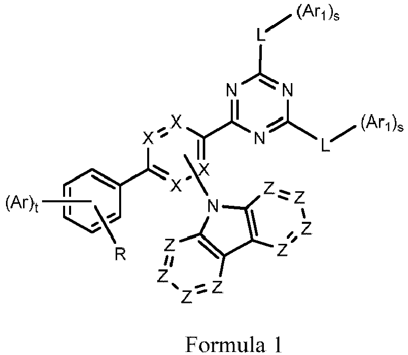 wherein
X is, at each occurrence identically or differently, selected from C, CRₓ, or N; Z is, at each occurrence identically or differently, selected from CR_{z} or N;
Ar is, at each occurrence identically or differently, selected from Formula 2, Formula 3, Formula 4, or a combination thereof, wherein Formula 2, Formula 3, and Formula 4 have the following structures, respectively: 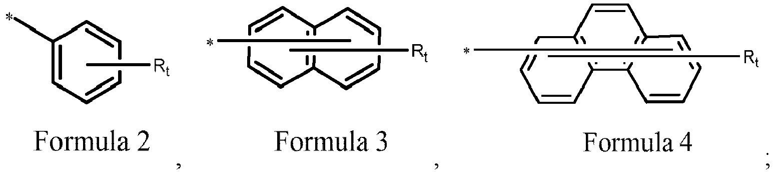 wherein
Rₜ in Formula 2, Formula 3, and Formula 4 represents, at each occurrence identically or differently, mono-substitution, multiple substitutions, or non-substitution;
"*" in the structures of Formula 2, Formula 3, and Formula 4 represents a position where the benzene ring with the substituent R in Formula 1, Formula 2, Formula 3, or Formula 4 is joined;
L is, at each occurrence identically or differently, selected from a single bond, Formula 5, Formula 6, Formula 7, or a combination thereof, wherein Formula 5, Formula 6, and Formula 7 have the following structures, respectively: 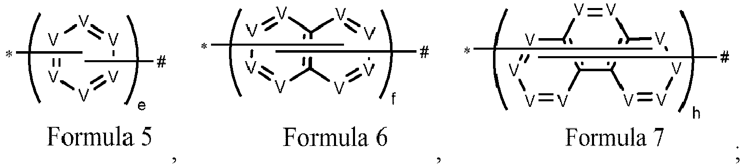
wherein V in Formula 5, Formula 6, and Formula 7 is, at each occurrence identically or differently, selected from C, CRᵥ, or N;
e, f, and h are, at each occurrence identically or differently, selected from 1, 2, or 3;
"*" in Formula 5, Formula 6, and Formula 7 represents a position where triazine shown in Formula 1, Formula 5, Formula 6, or Formula 7 is joined; and "#" represents a position where Ar₁ in Formula 1, Formula 5, Formula 6, or Formula 7 is joined;
Ar₁ is, at each occurrence identically or differently, selected from Formula 8, Formula 9, Formula 10, or a combination thereof, wherein Formula 8, Formula 9, and Formula 10 have the following structures, respectively: 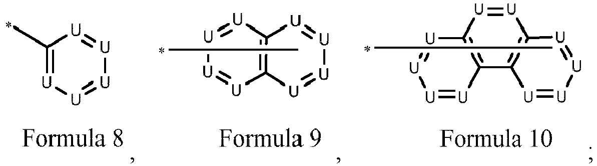
wherein U in Formula 8, Formula 9, and Formula 10 is, at each occurrence identically or differently, selected from C, CRᵤ, or N;
"*" in Formula 8, Formula 9, and Formula 10 represents a position where L in Formula 1, Formula 8, Formula 9, or Formula 10 is joined;
Rₓ, Rᵤ, Rᵥ, and Rₜ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R_{z} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
R represents, at each occurrence identically or differently, mono-substitution, multiple substitutions, or non-substitution;
R is, at each occurrence identically or differently, selected from the group consisting of: hydrogen, deuterium, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted amino having 0 to 20 carbon atoms, an acyl group, a carbonyl group, a carboxylic acid group, an ester group, a hydroxyl group, a sulfanyl group, a sulfinyl group, a sulfonyl group, a phosphino group, and combinations thereof;
t is selected from 0, 1, 2, 3, 4, or 5; s is, at each occurrence identically or differently, selected from 1 to a maximum number of available substitutions for L; and
in Formula 1, only adjacent substituents R, adjacent substituents Rₓ, and adjacent substituents R_{z} can be optionally joined to form a 6-membered ring.

2. The compound according to claim 1, wherein Ar is selected from the structure of Formula 2, and/or L is, at each occurrence identically or differently, selected from a single bond or the structure of Formula 5; and/or Ar₁ is, at each occurrence identically or differently, selected from the structure of Formula 8.

3. The compound according to claim 1 or 2, wherein X is, at each occurrence identically or differently, selected from C or CRₓ, and/or Z is, at each occurrence identically or differently, selected from CR_{z}, and/or U is, at each occurrence identically or differently, selected from C or CRᵤ, and/or V is, at each occurrence identically or differently, selected from C or CRᵥ.

4. The compound according to any one of claims 1 to 3, wherein t is selected from 0 or 1.

5. The compound according to claim 1, wherein Rₓ, Rᵤ, Rₜ, Rᵥ, and R_{z} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, and substituted or unsubstituted alkenyl having 2 to 20 carbon atoms.

6. The compound according to claim 1, wherein R is, at each occurrence identically or differently, selected from the group consisting of: hydrogen, deuterium, halogen, substituted or unsubstituted cycloalkyl having 3 to 20 ring carbon atoms, and substituted or unsubstituted alkenyl having 2 to 20 carbon atoms.

7. The compound according to claim 1, wherein the compound is selected from the group consisting of the following structures: 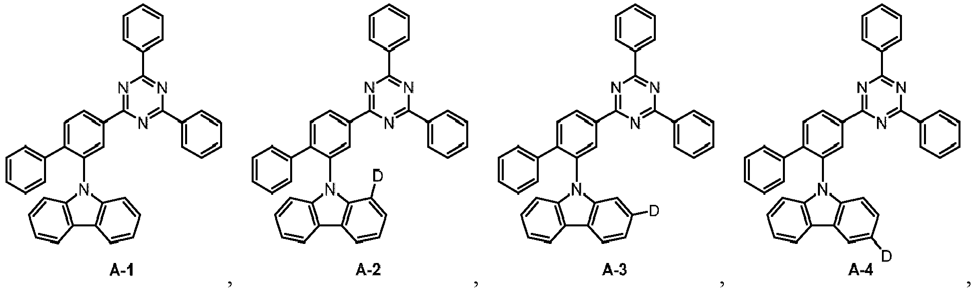 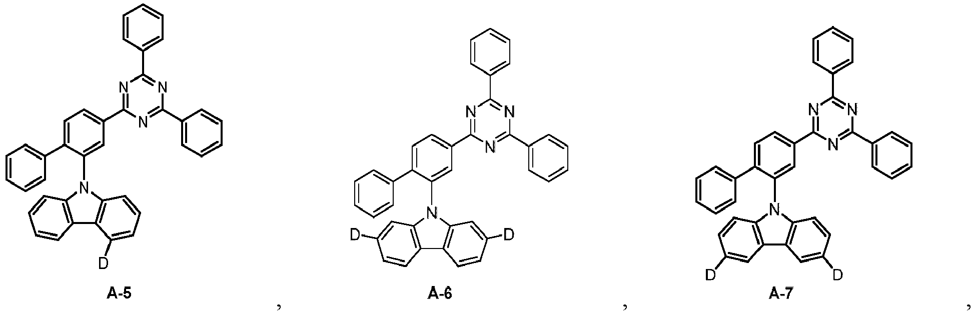 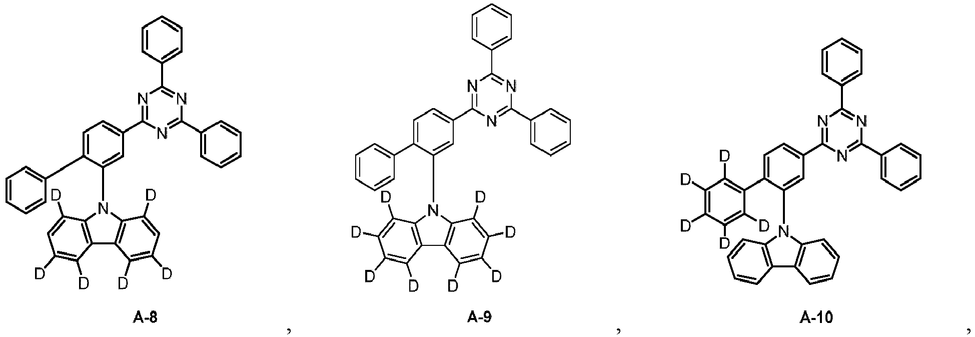 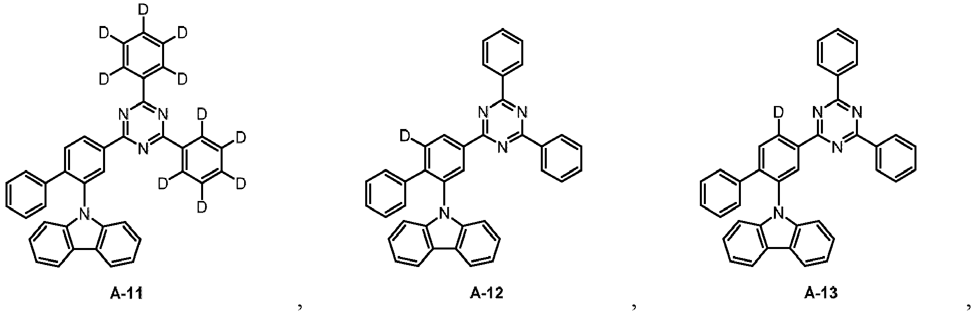 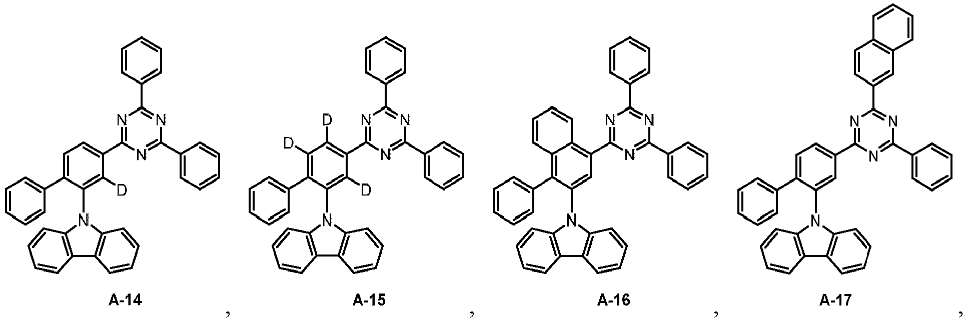 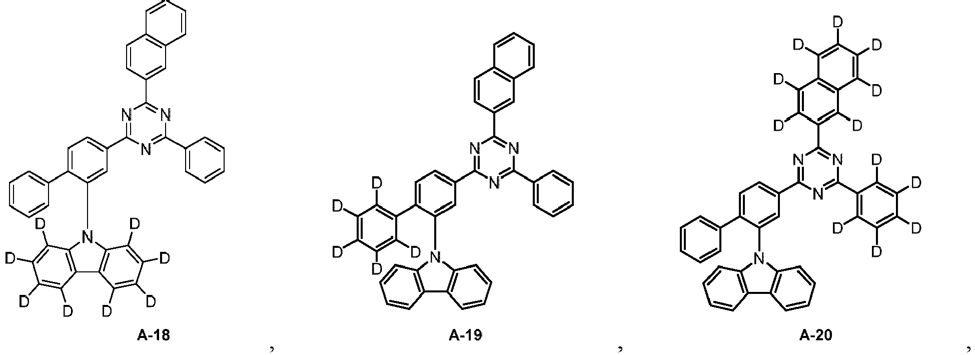 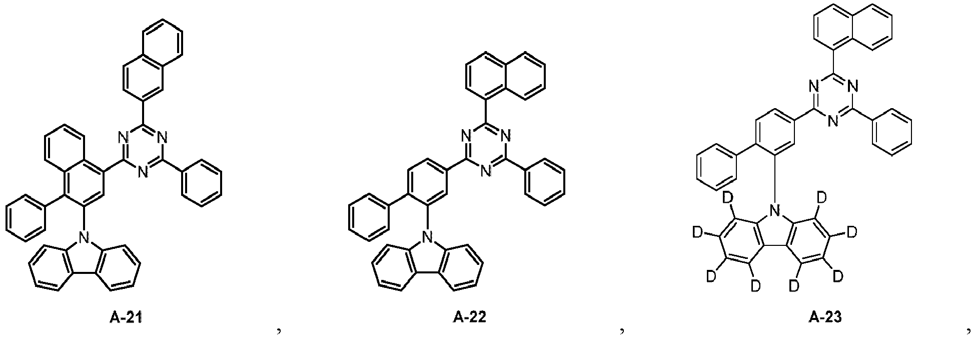 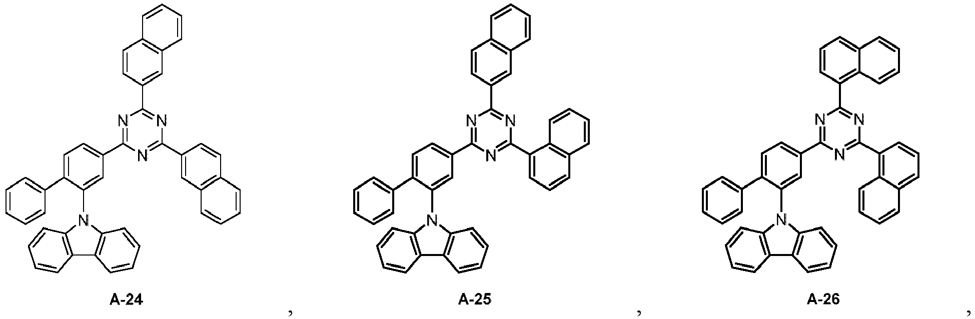 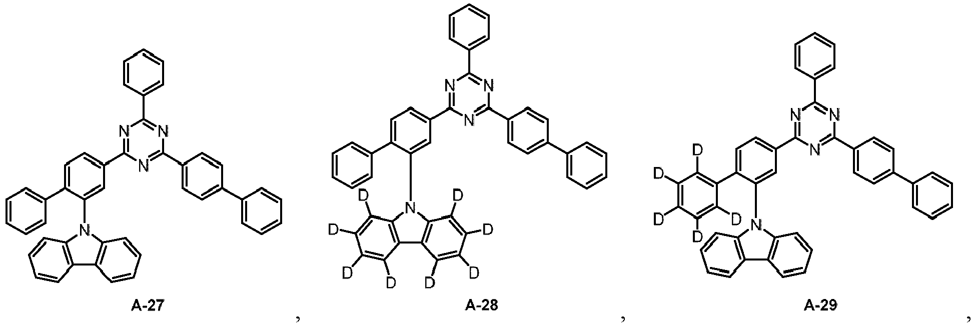 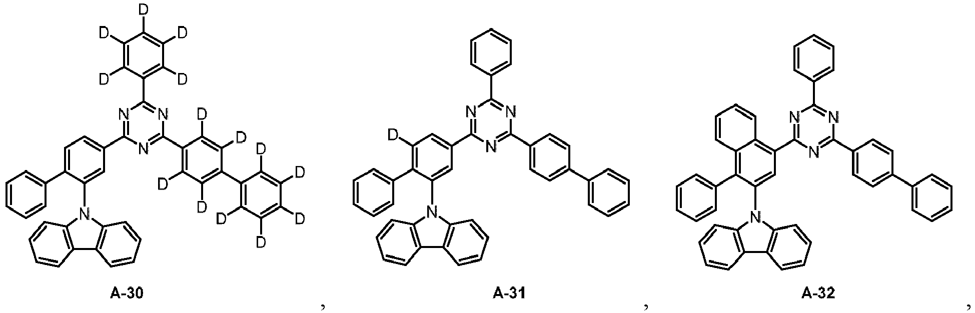 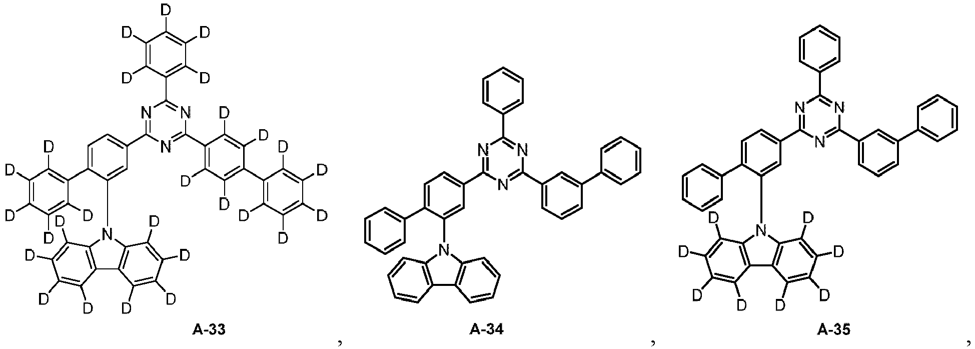 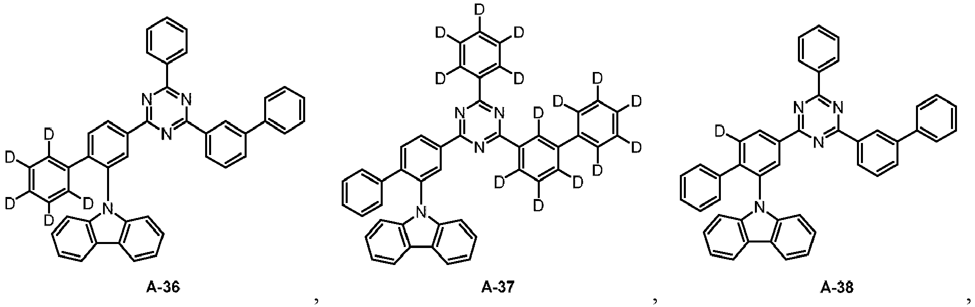 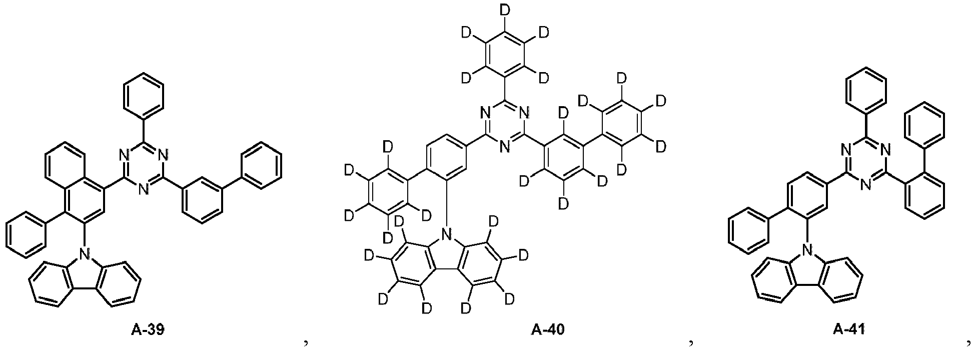 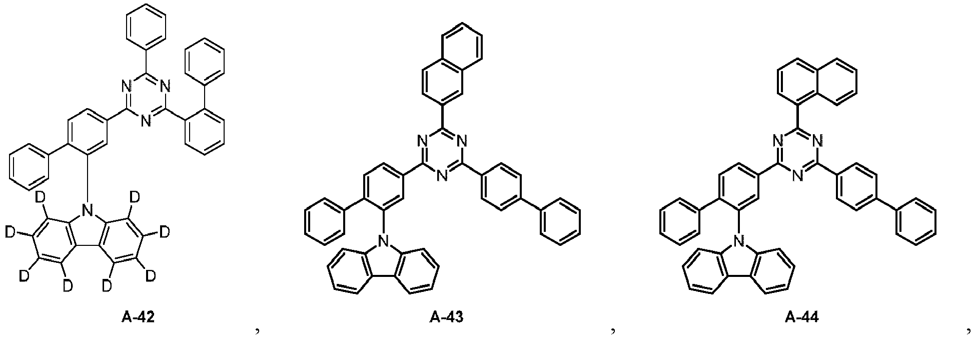 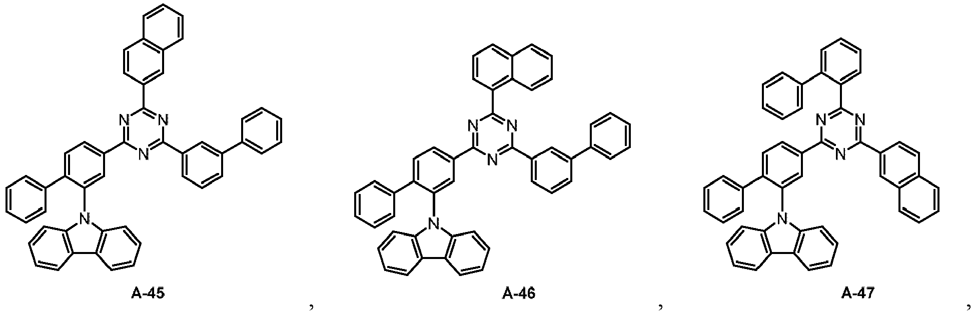 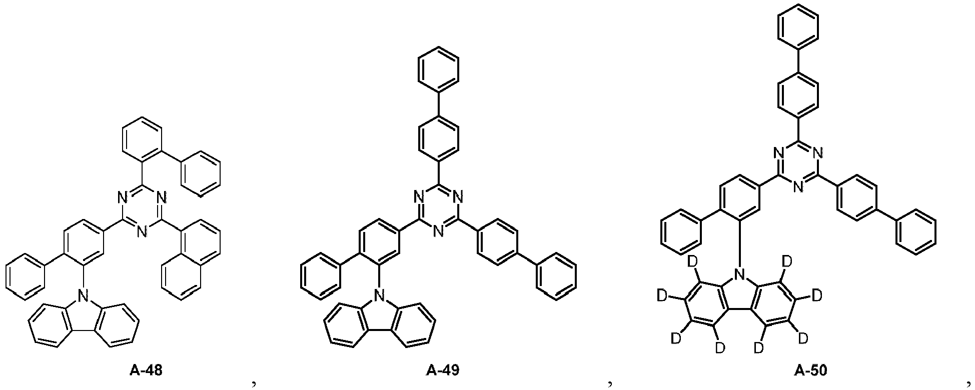 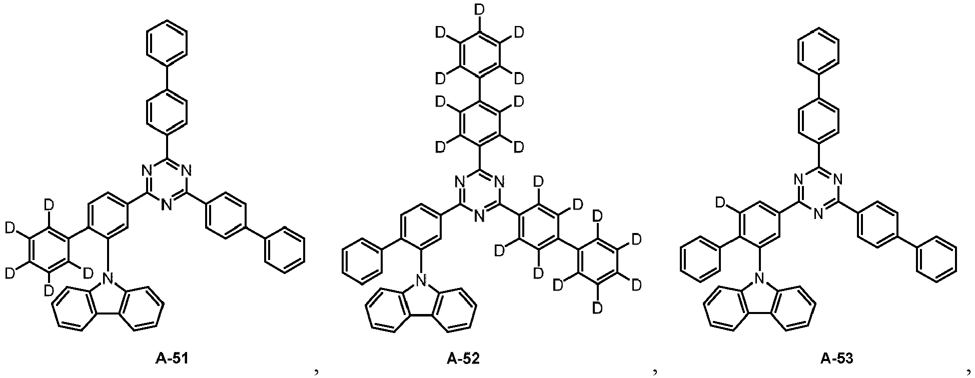 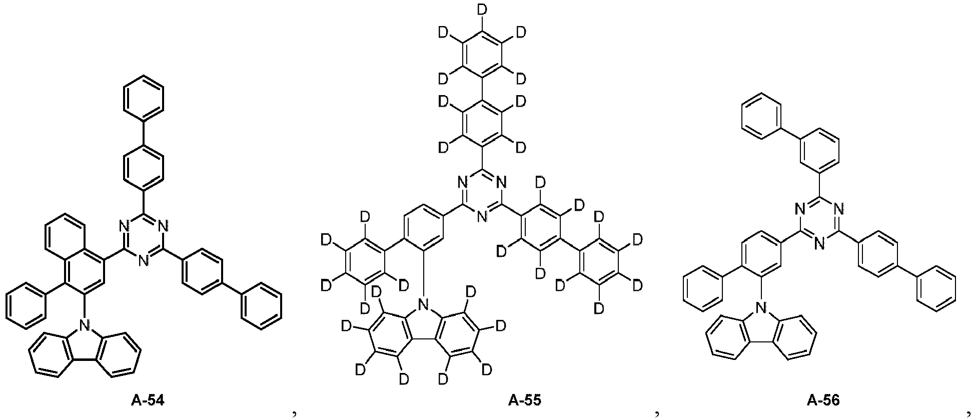 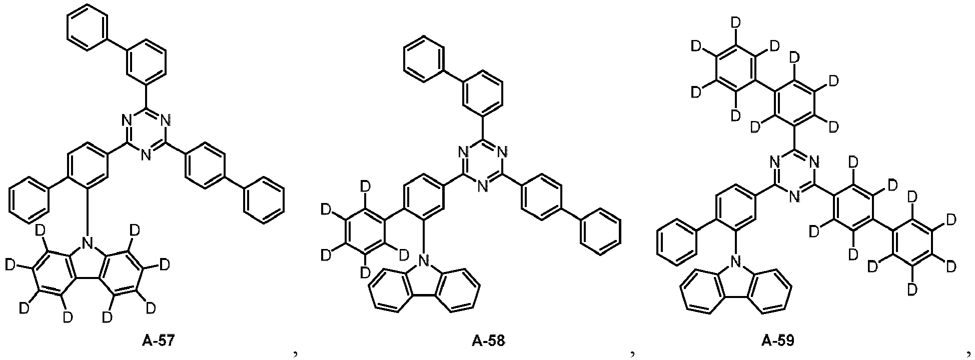 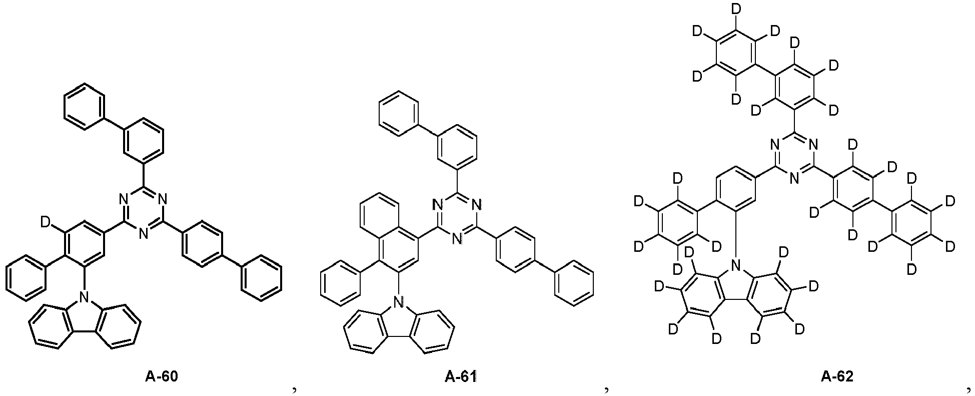 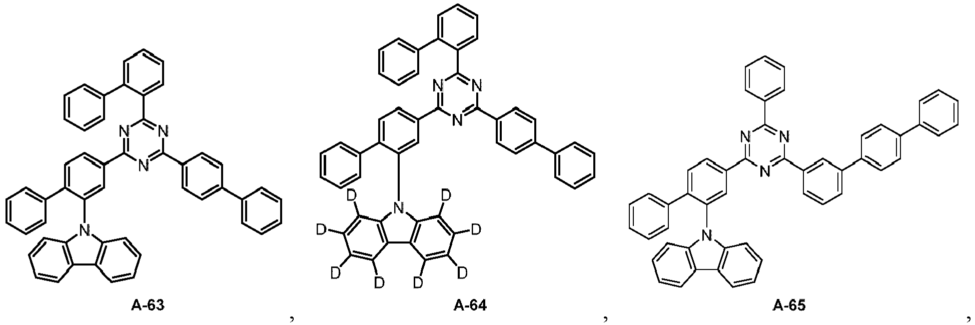 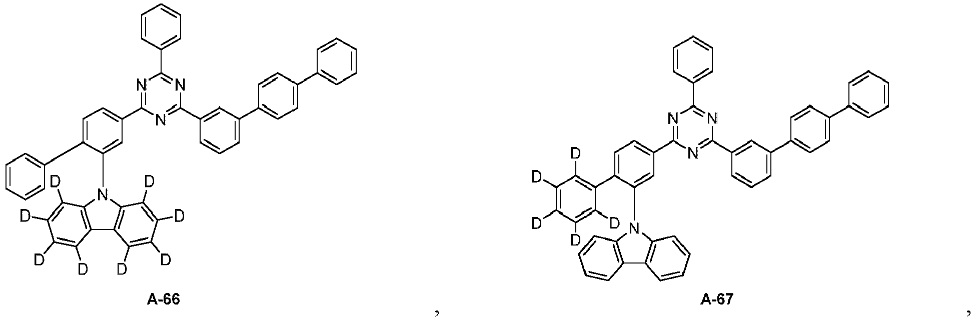 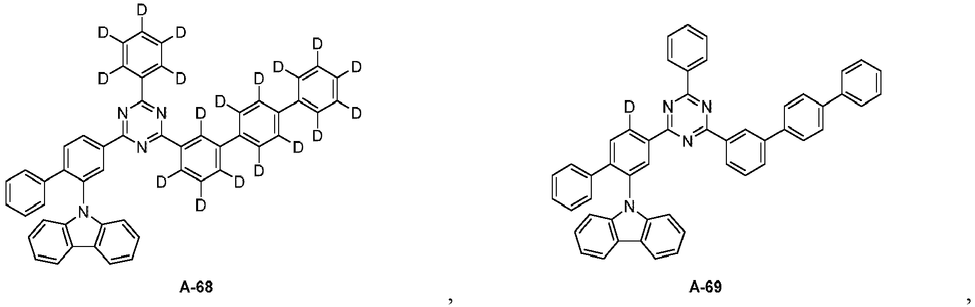 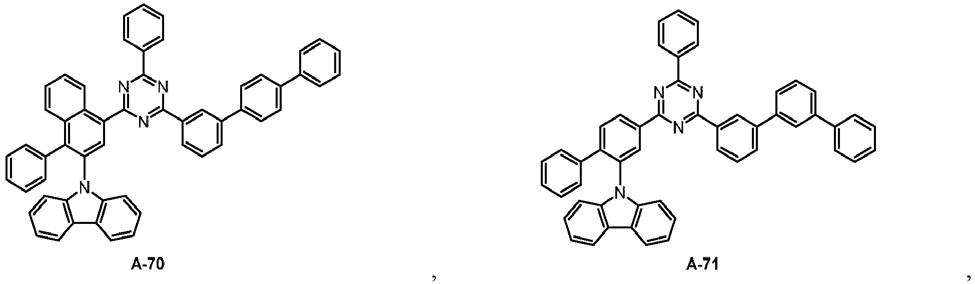 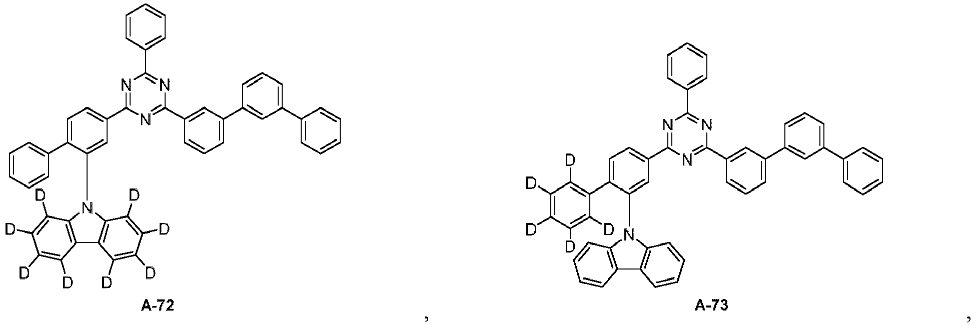 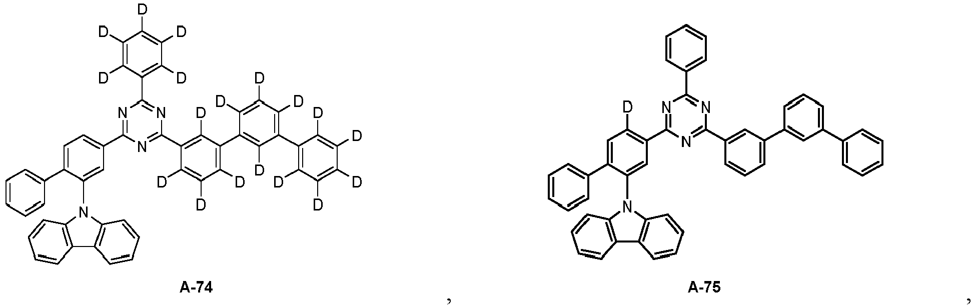 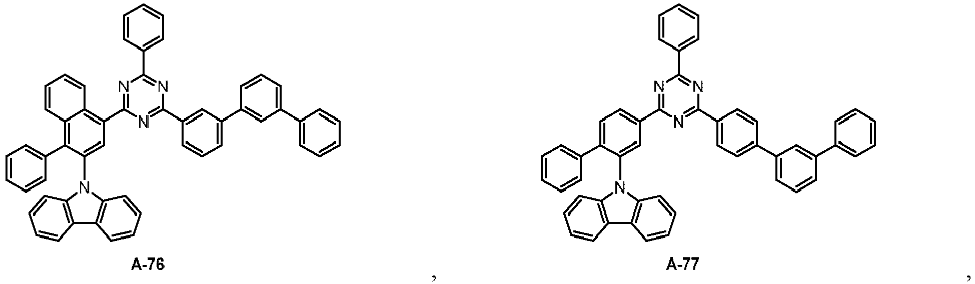 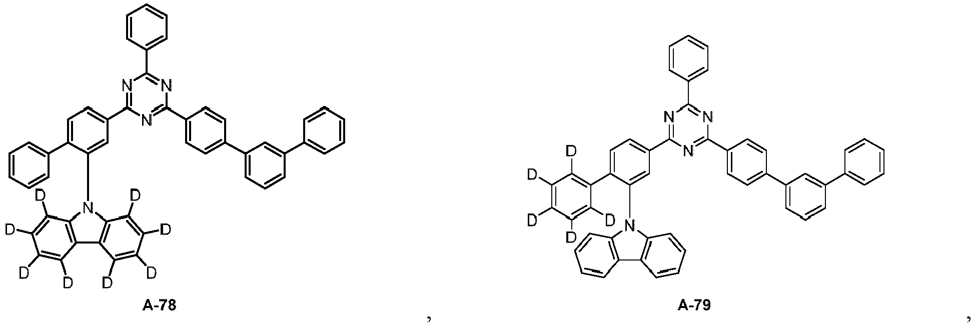 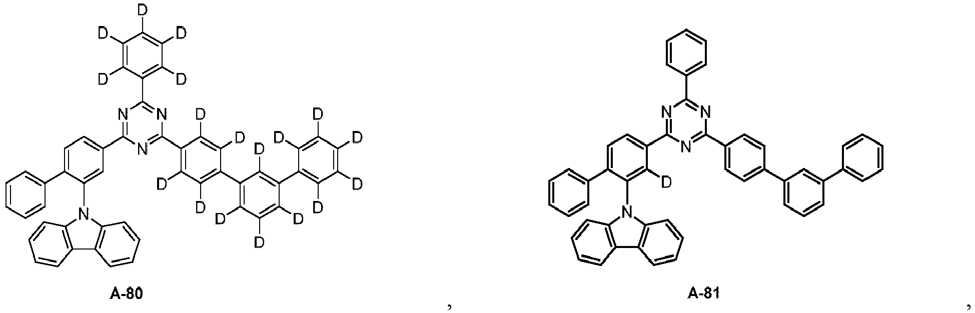 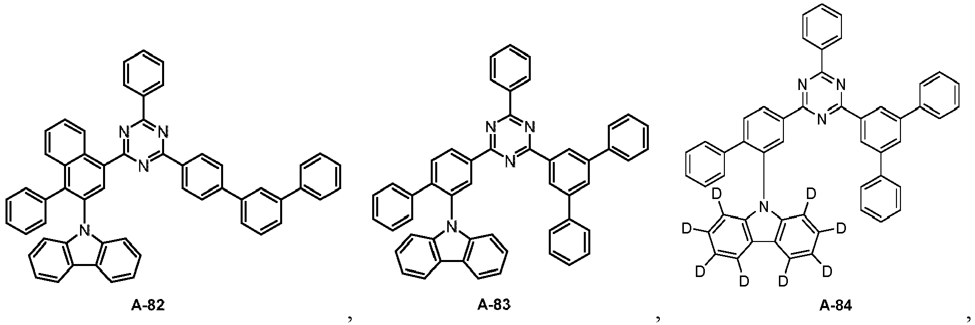 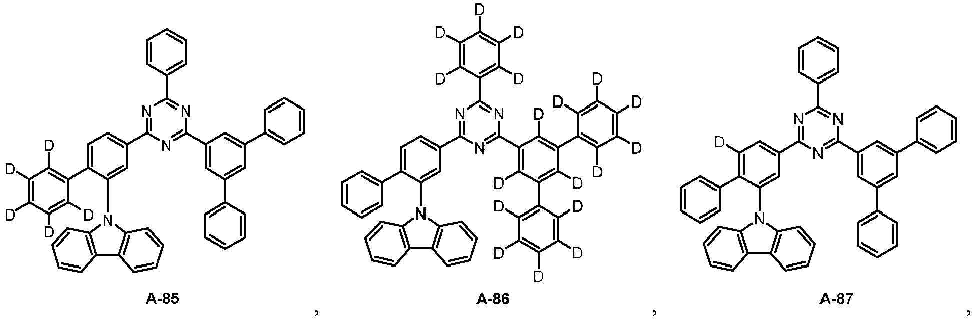 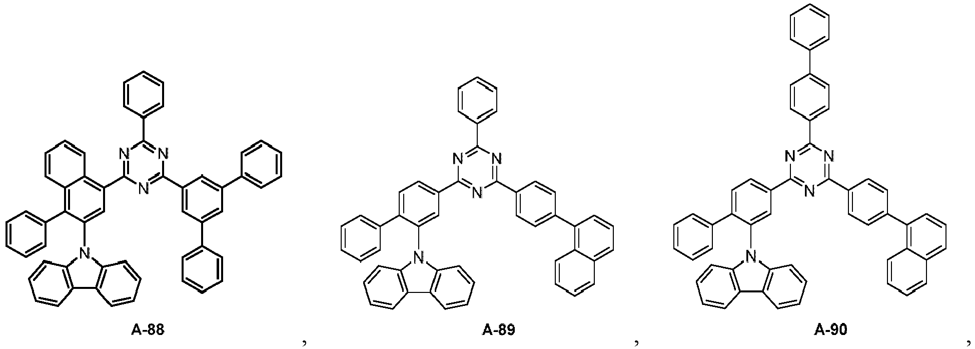 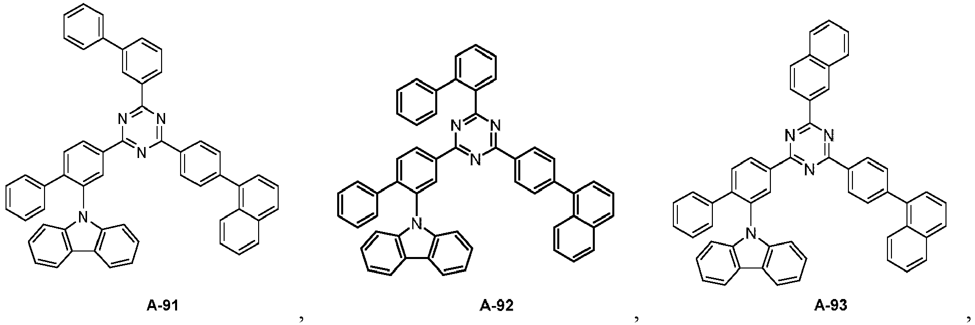 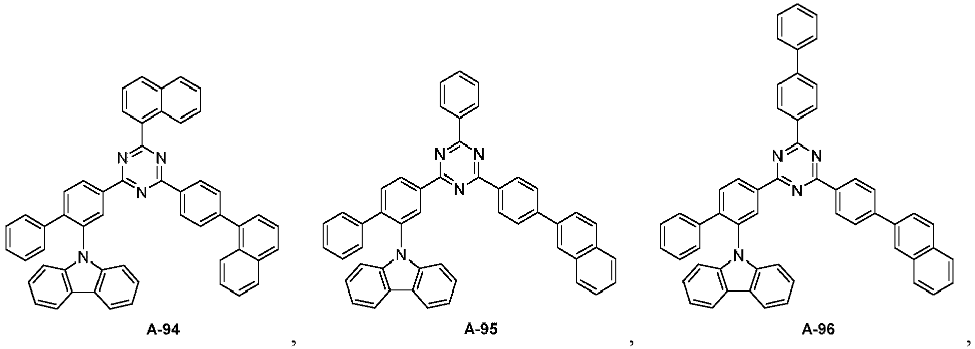 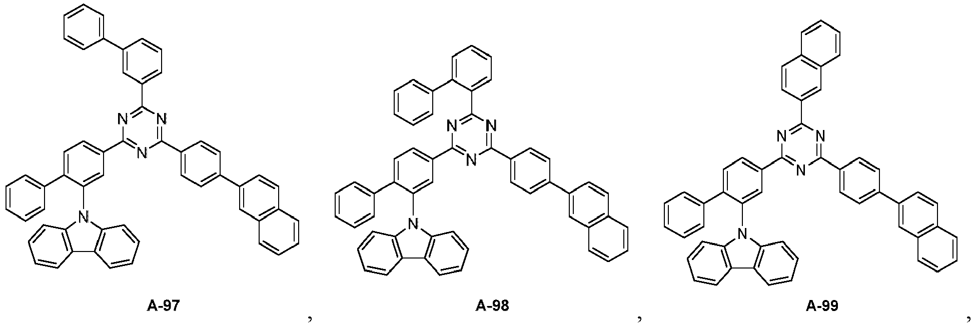 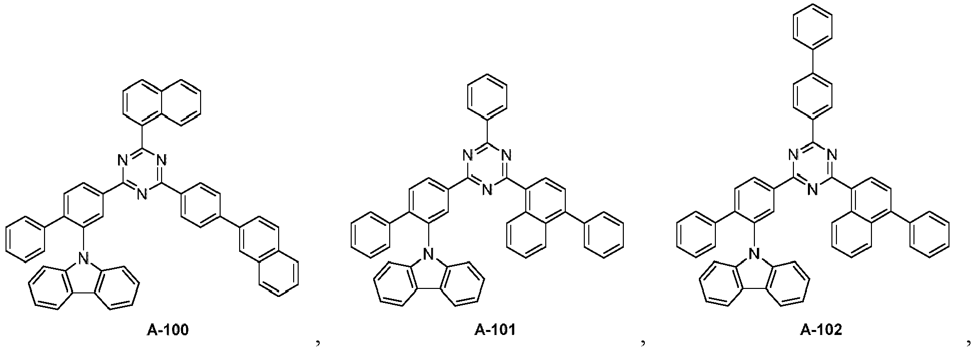 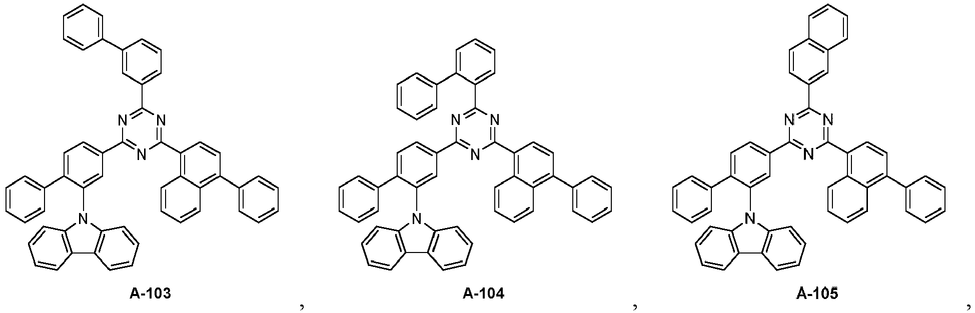 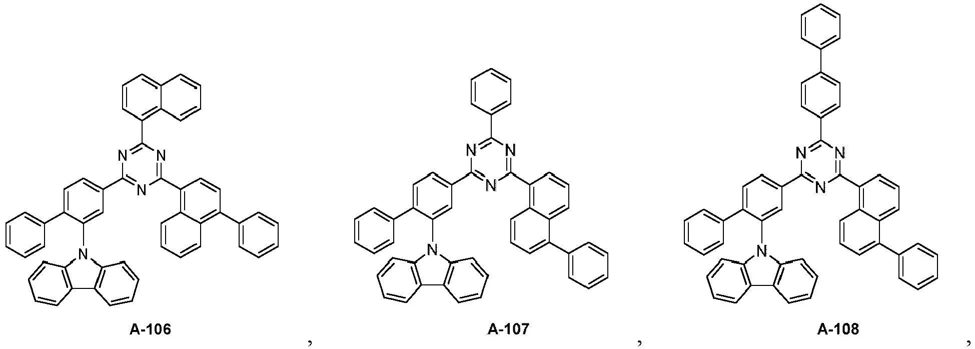 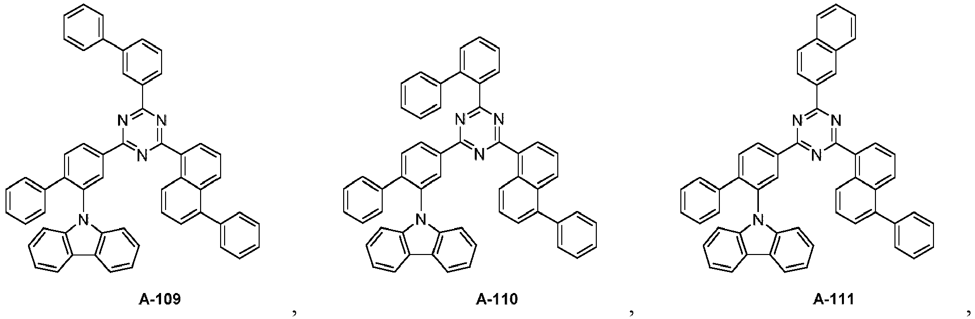 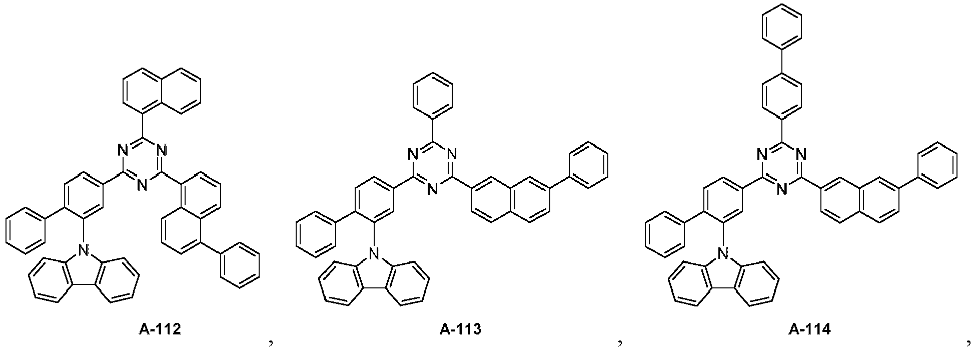 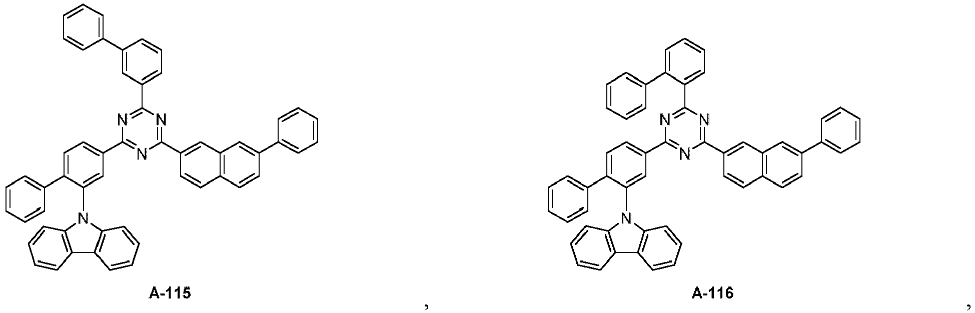 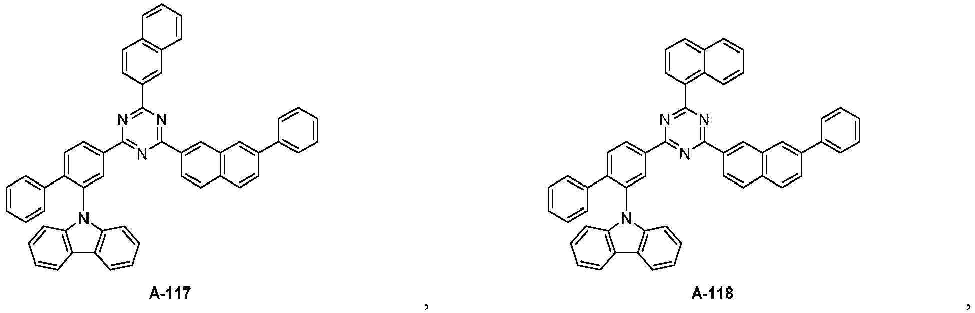 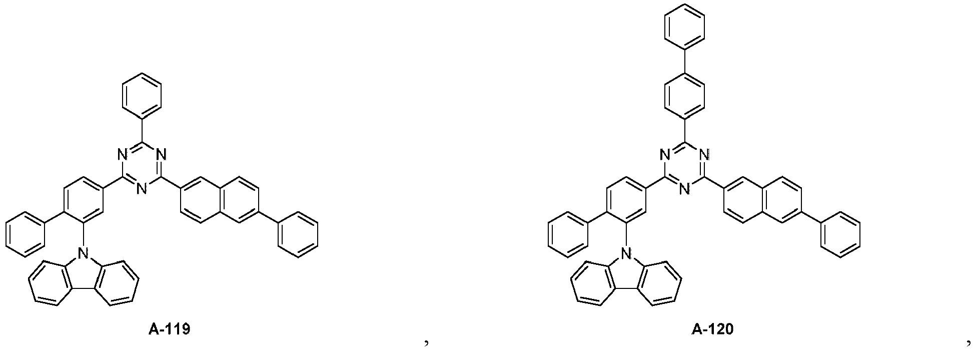 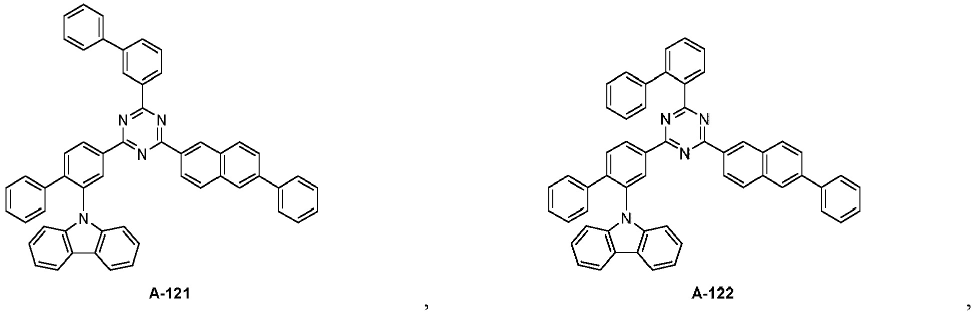 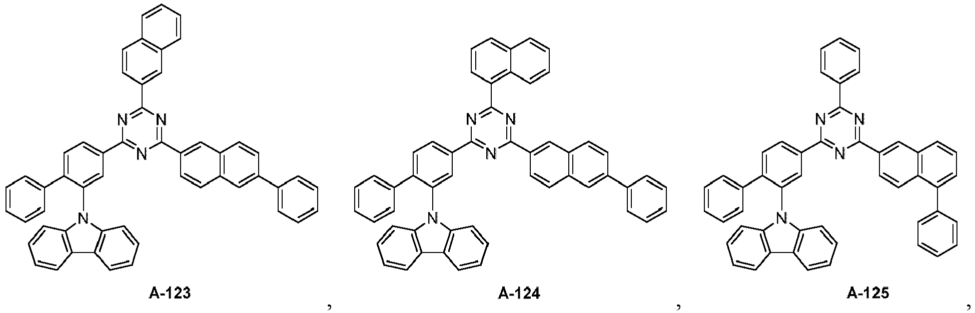 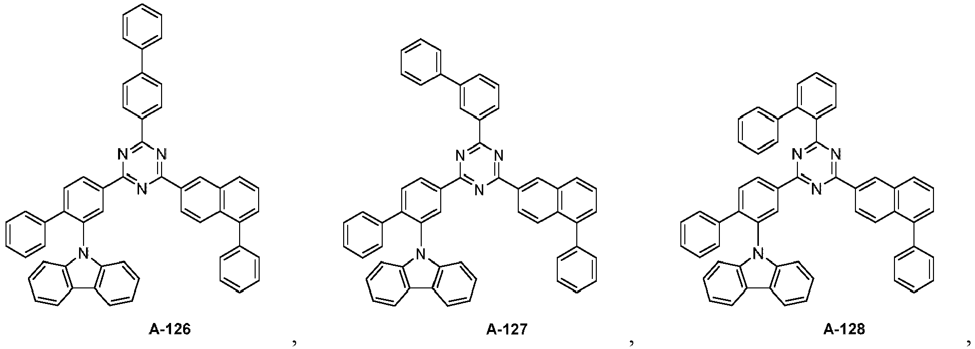 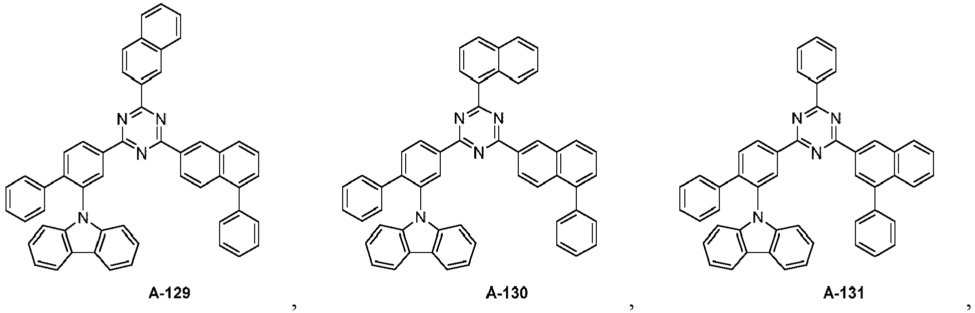 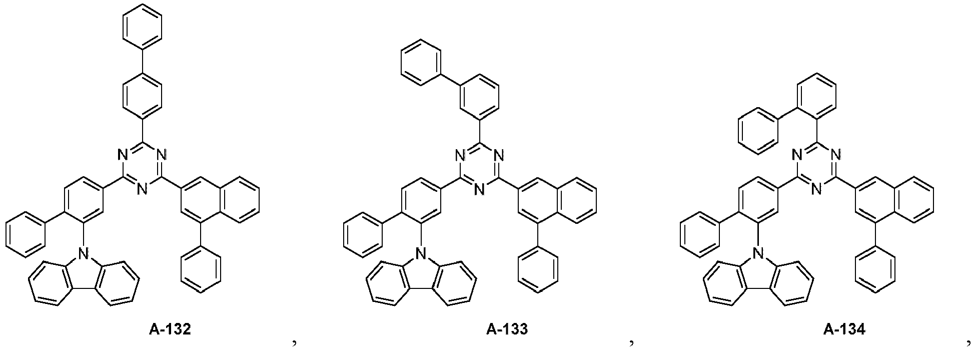 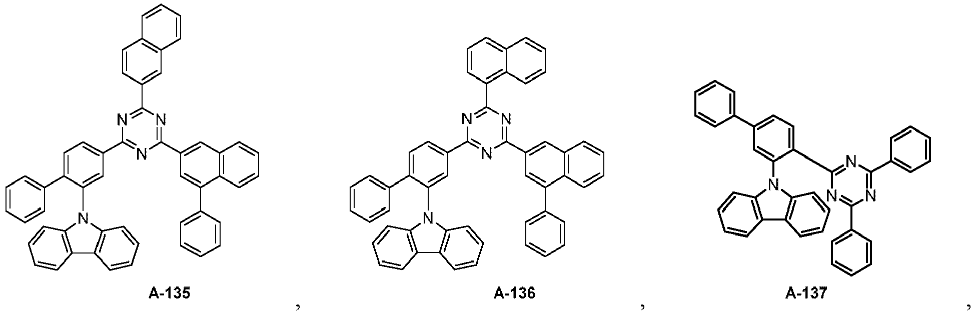 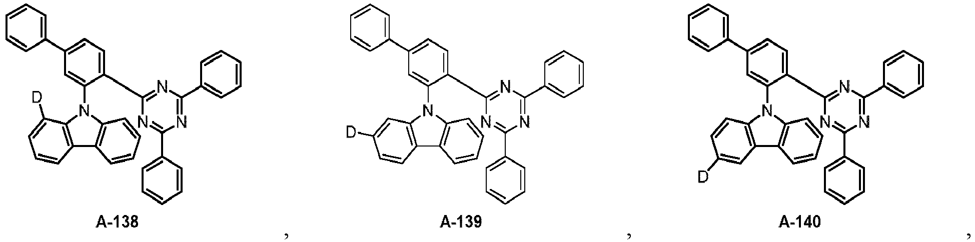 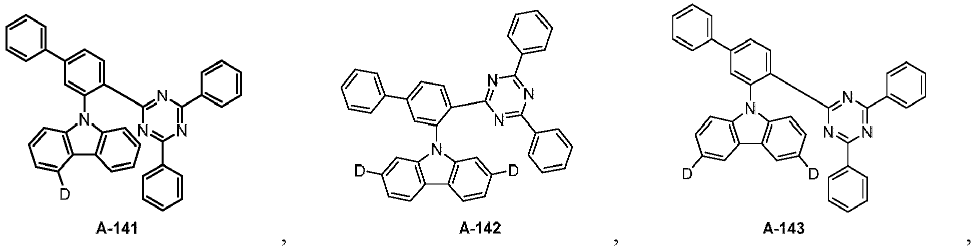 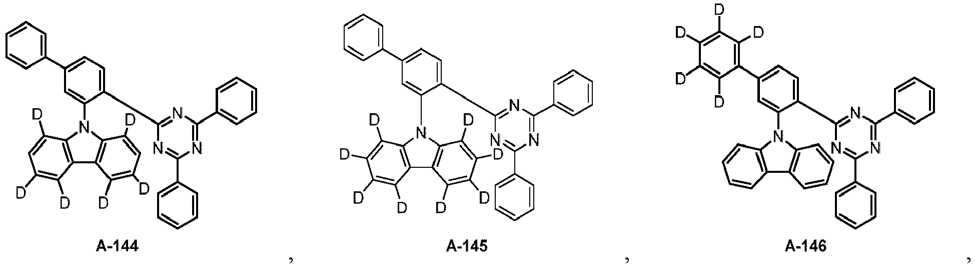 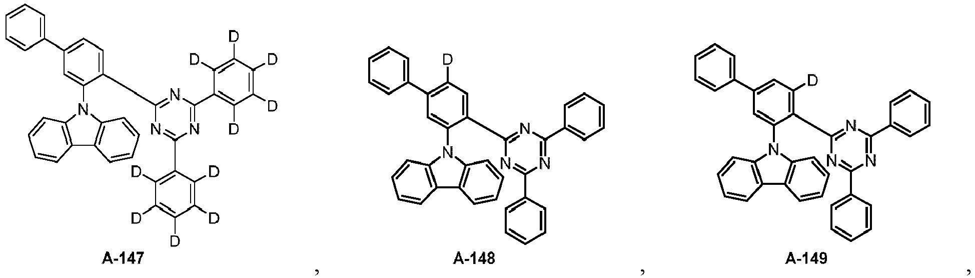 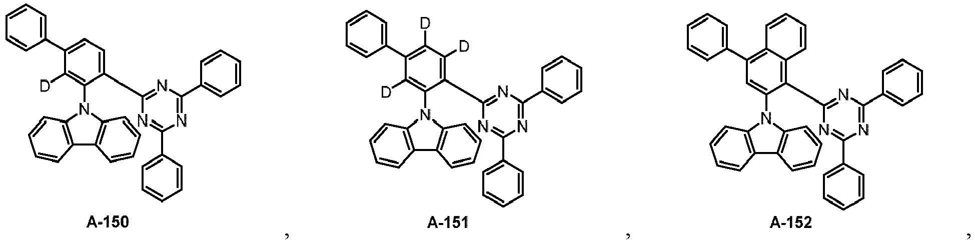 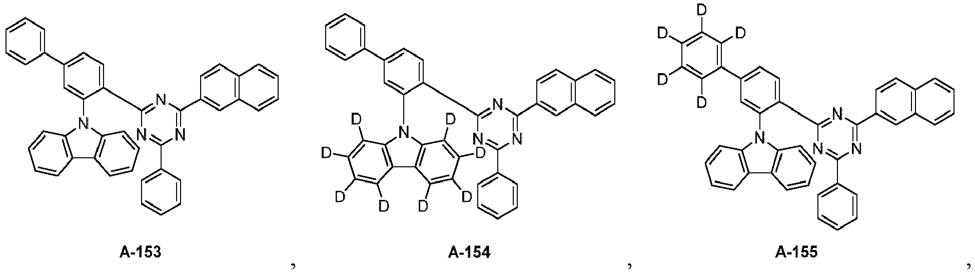 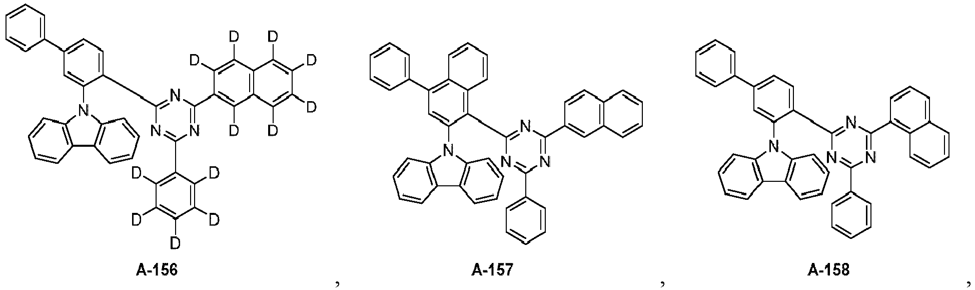 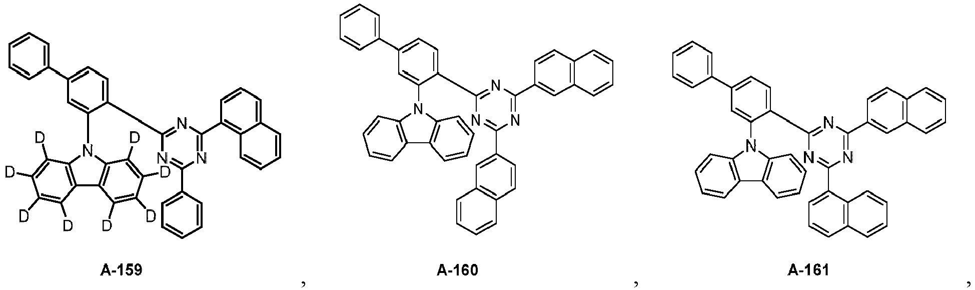 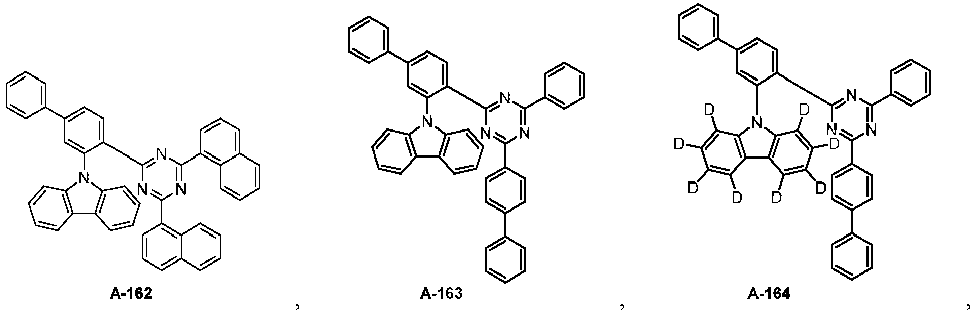 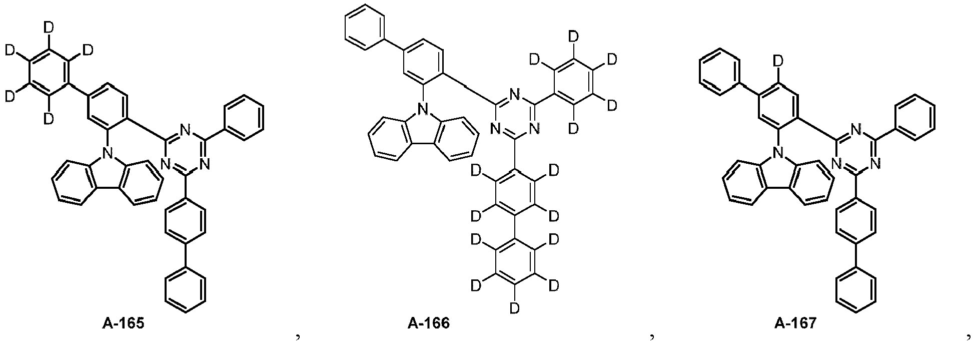 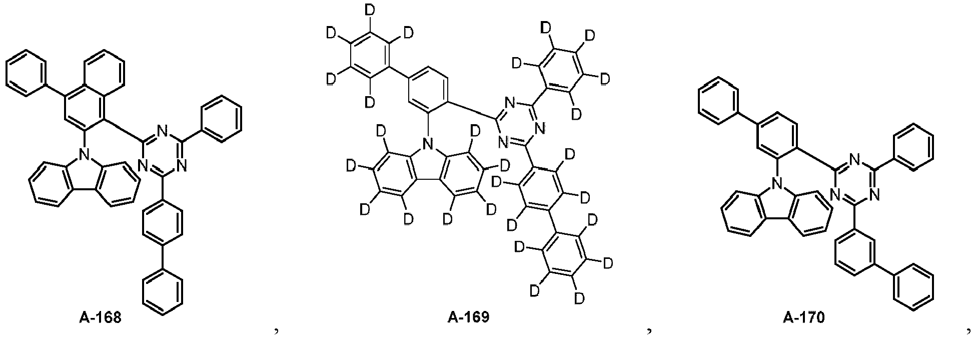 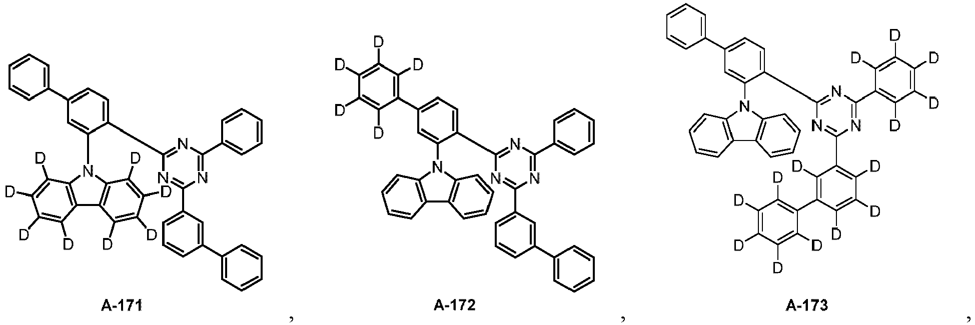 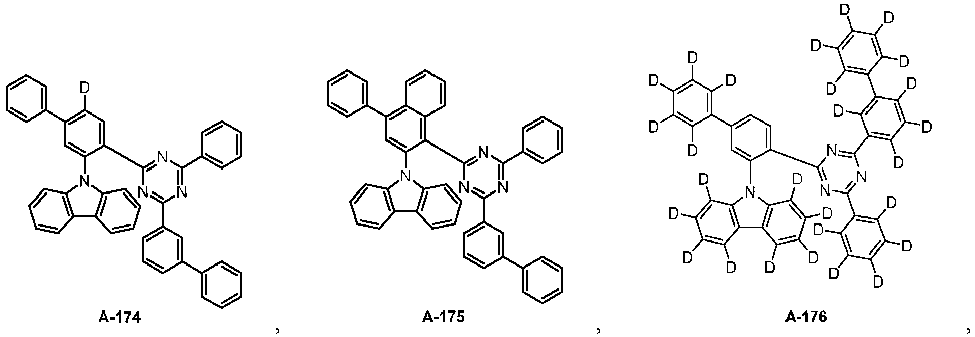 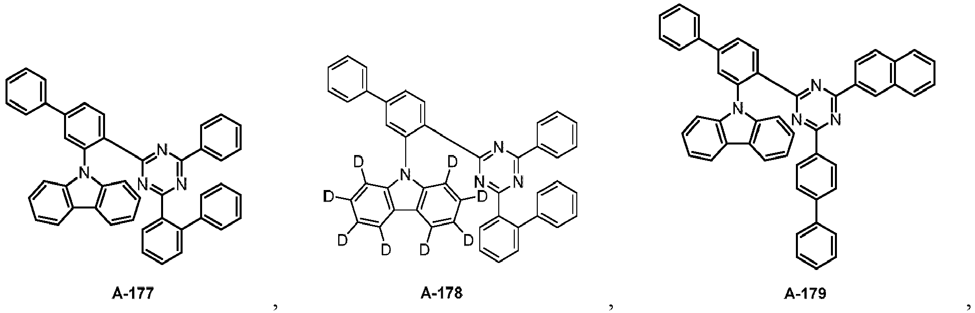 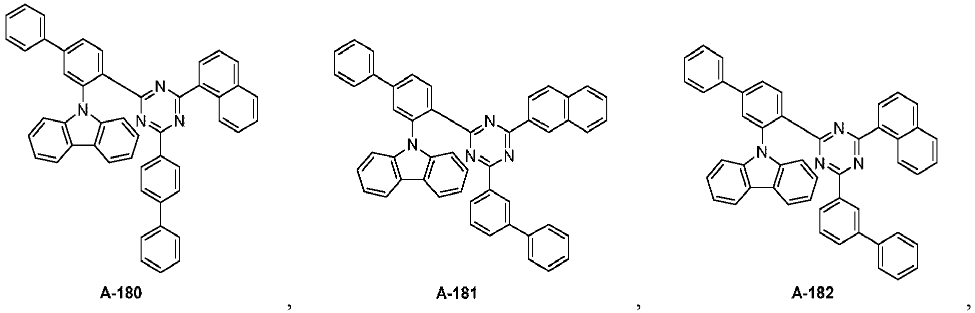 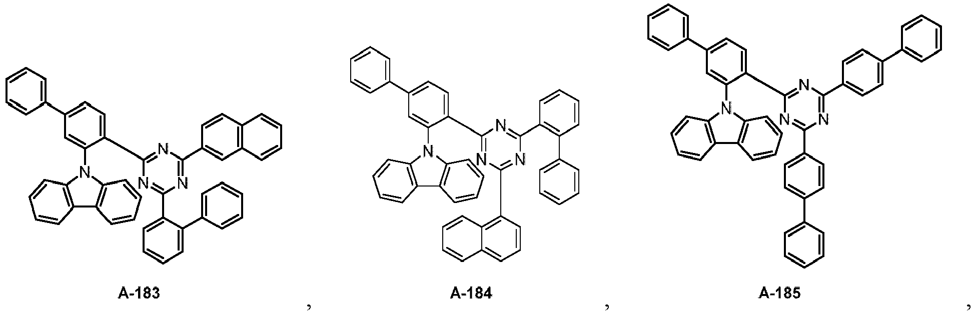 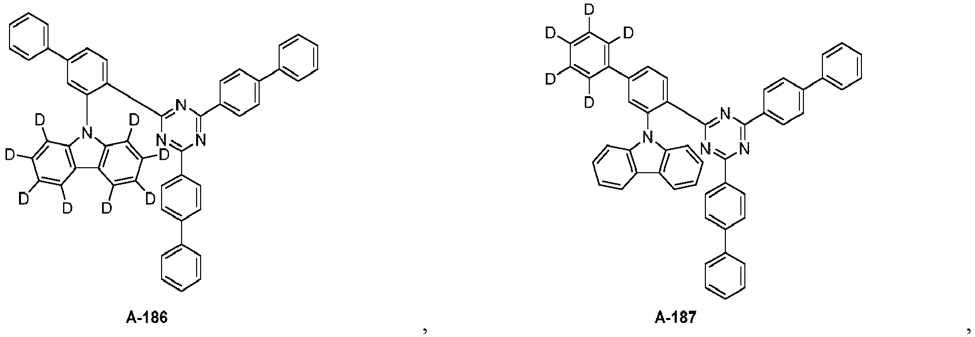 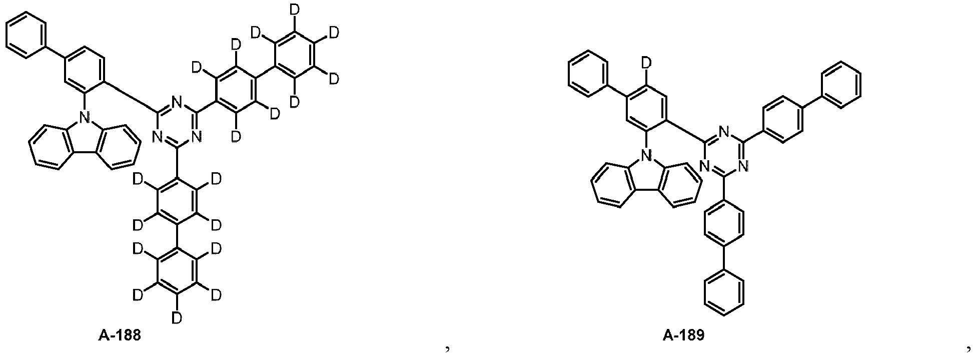 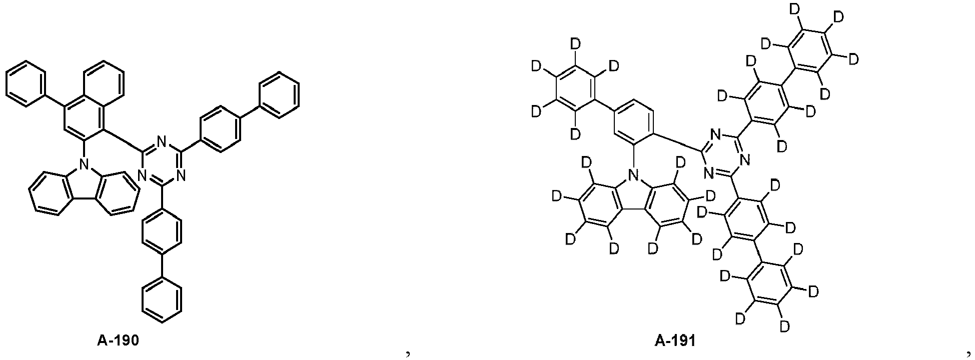 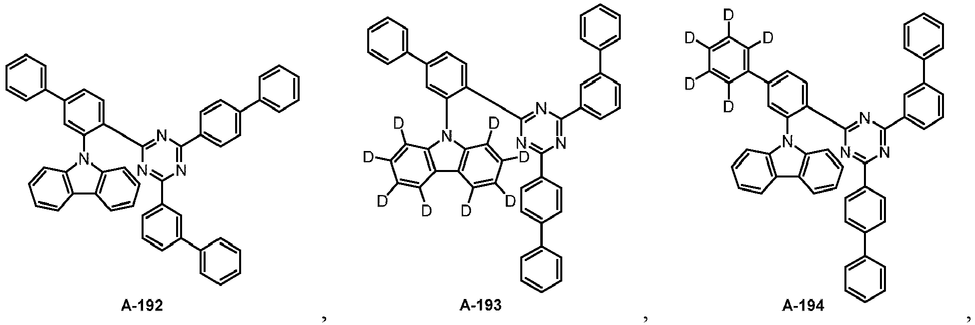 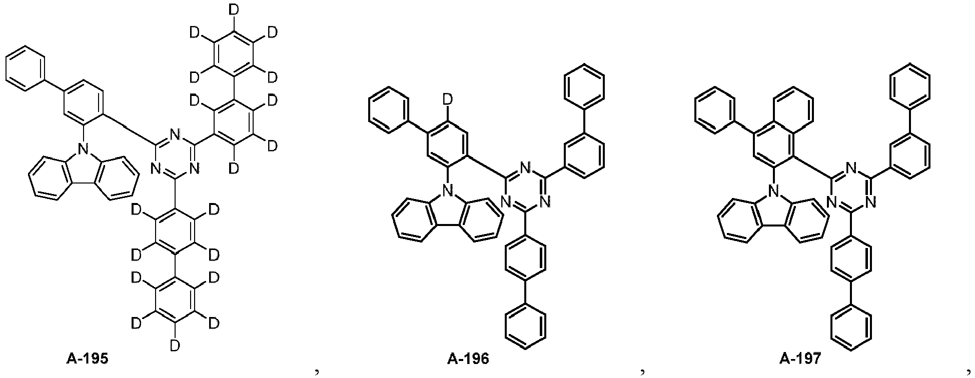 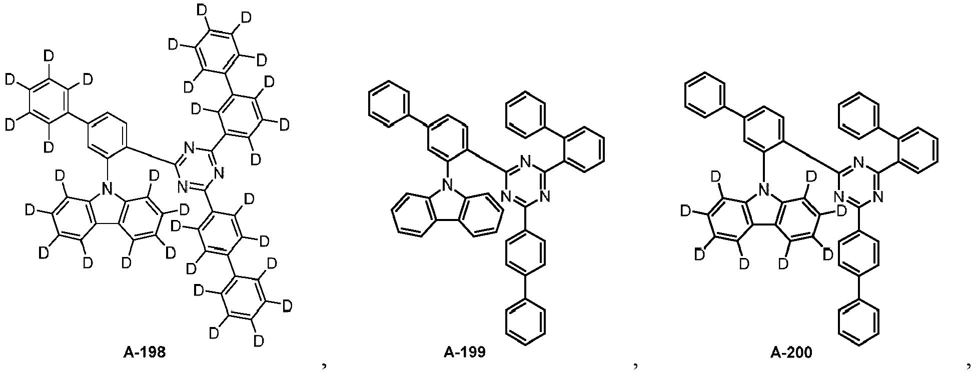 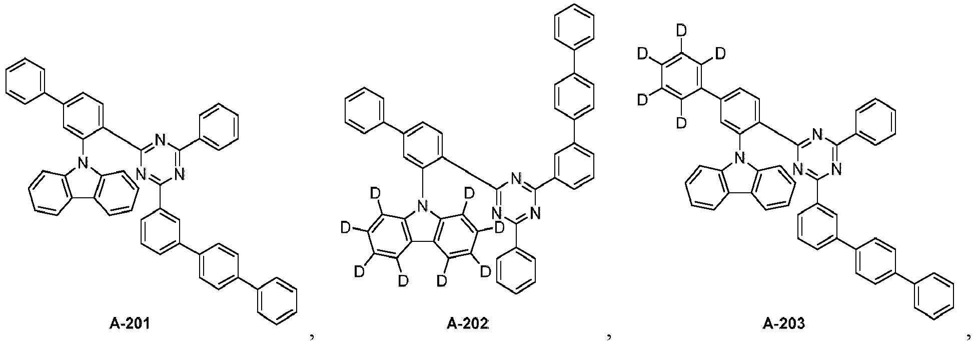 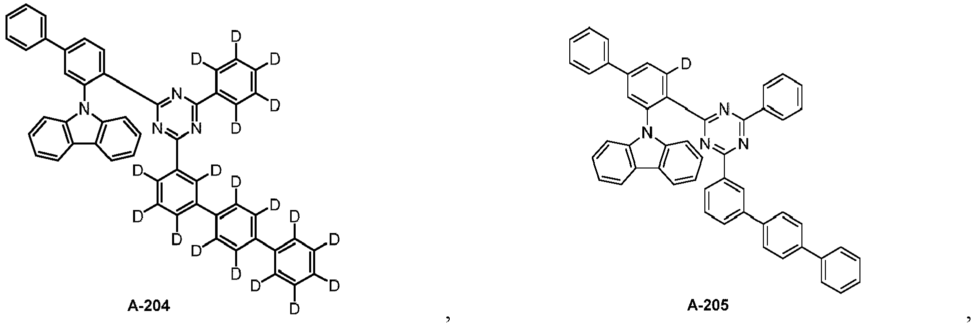 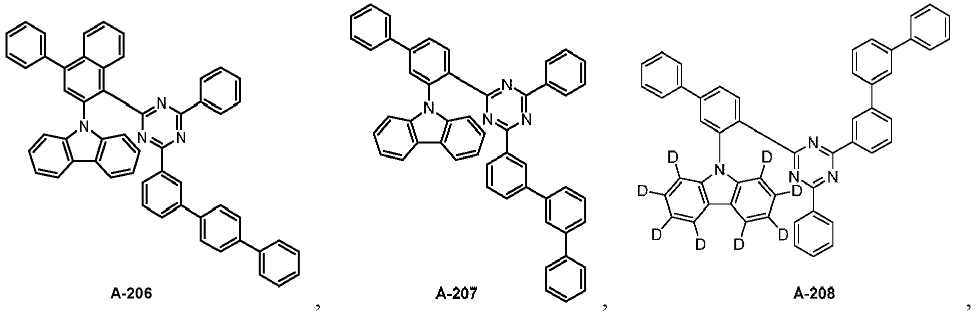 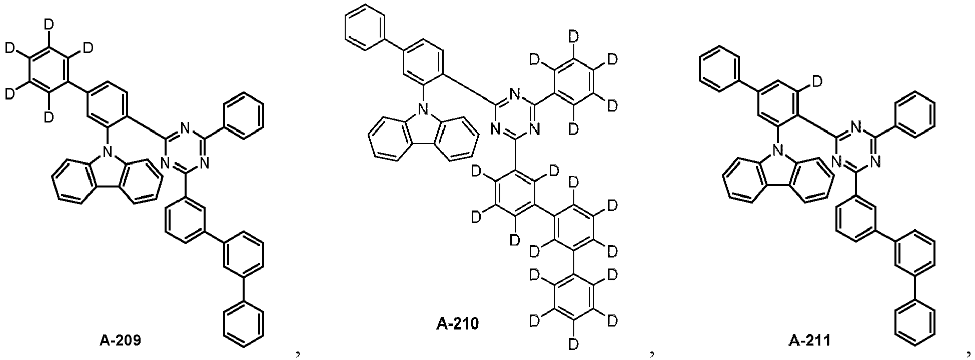 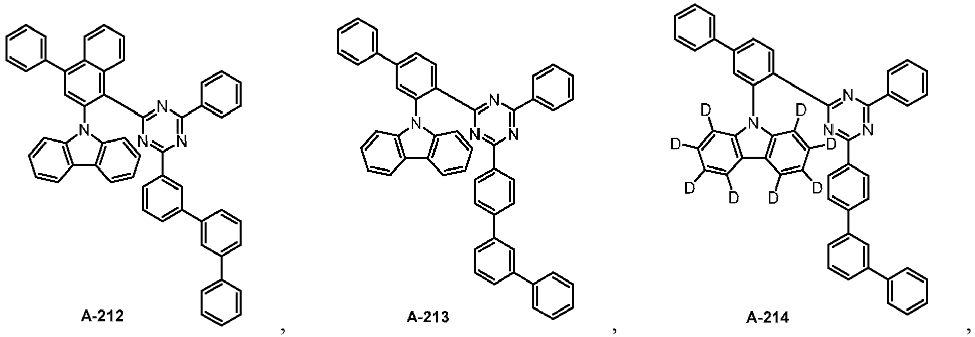 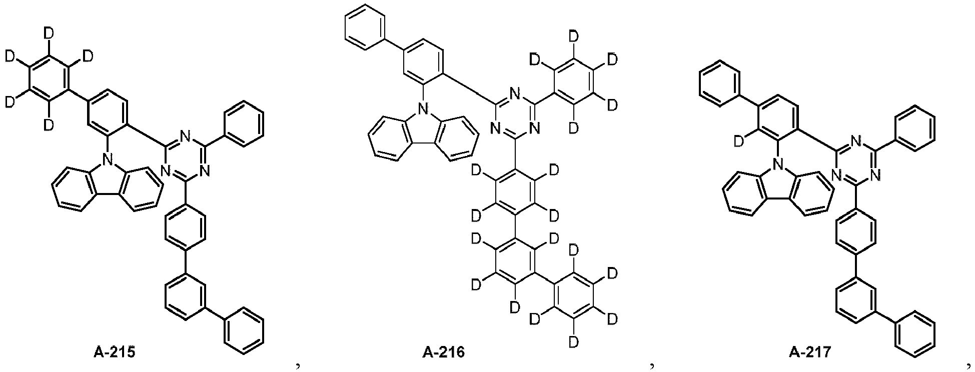 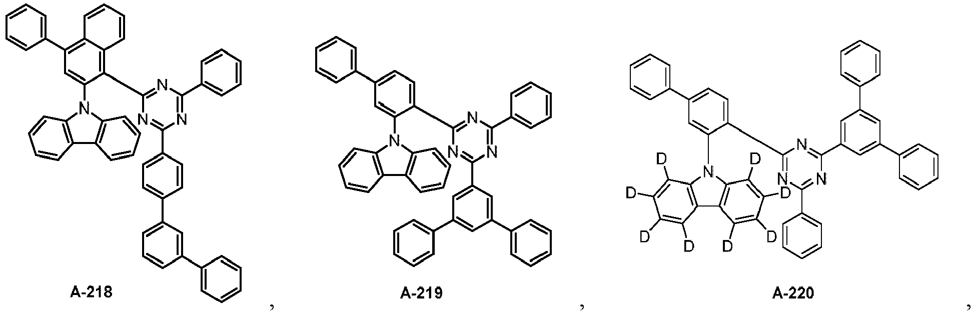 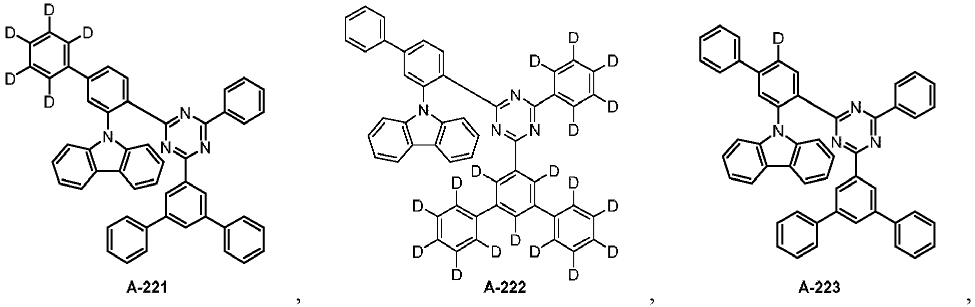 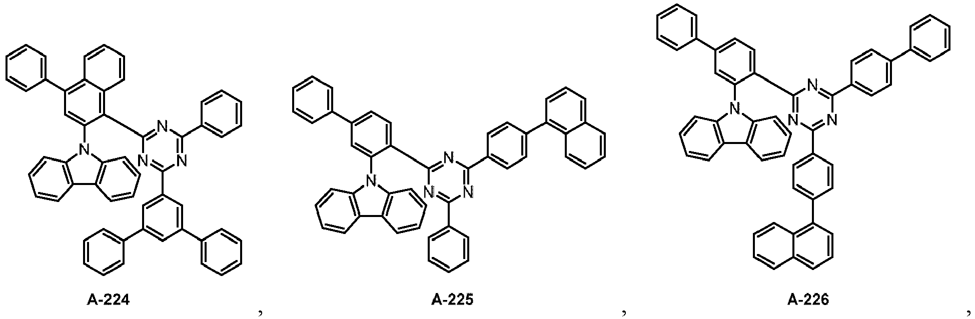 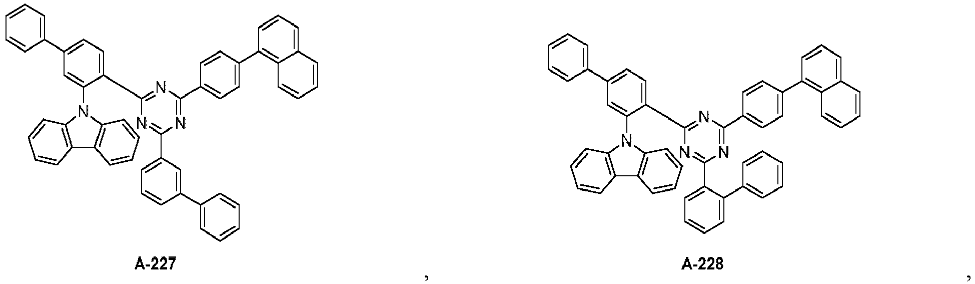 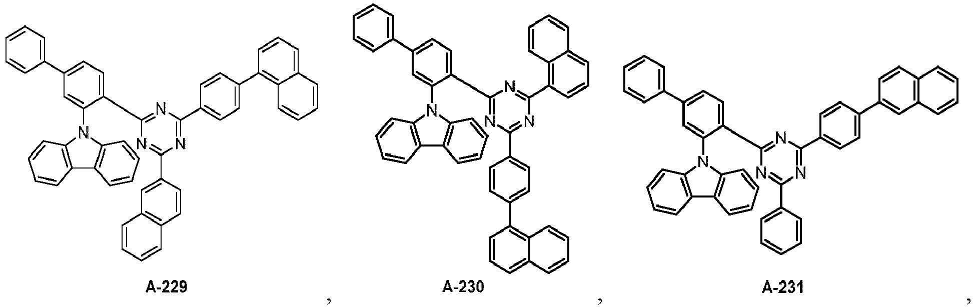 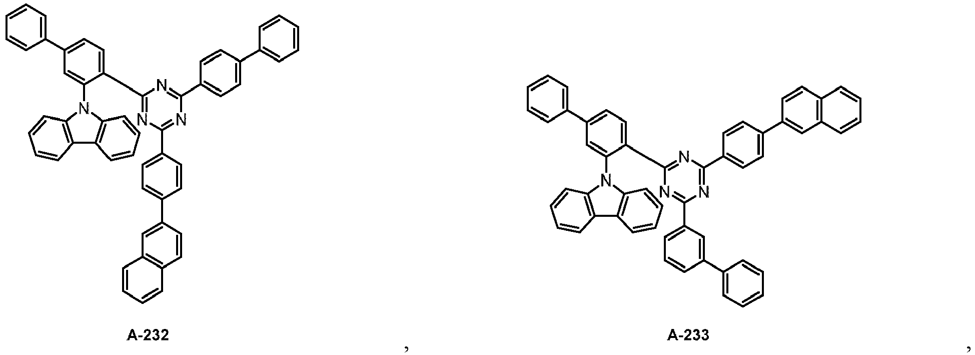 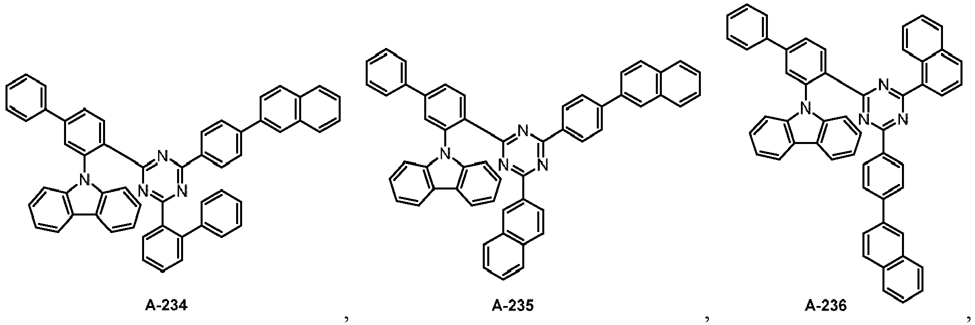 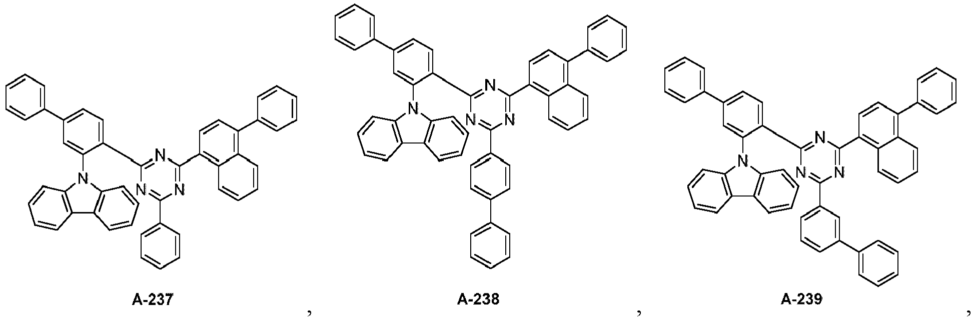 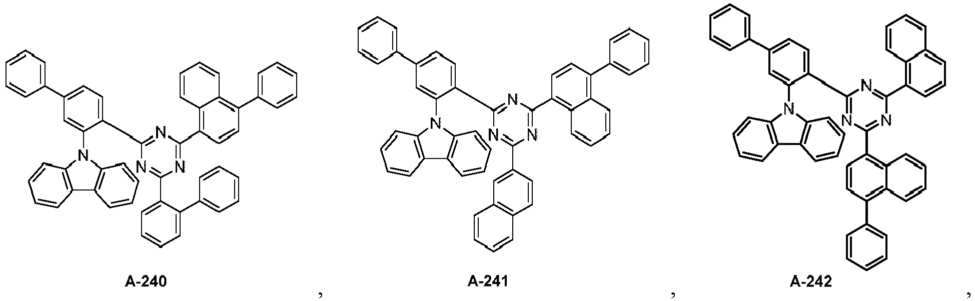 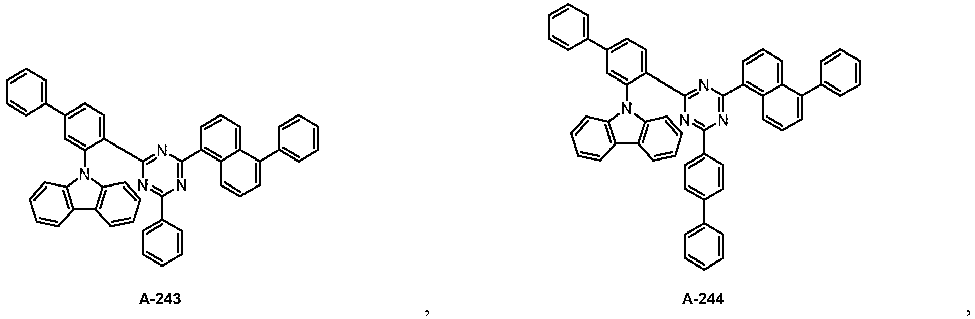 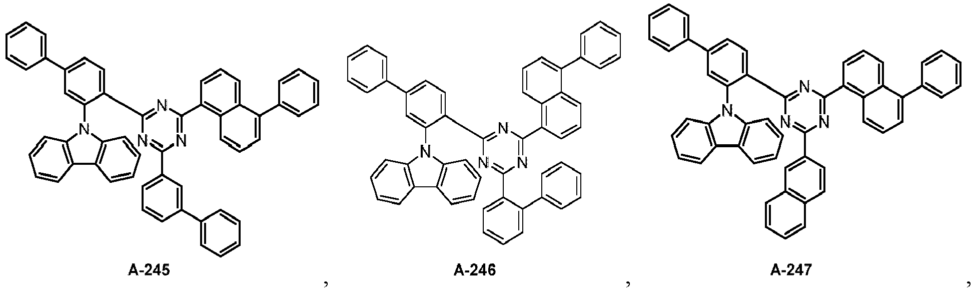 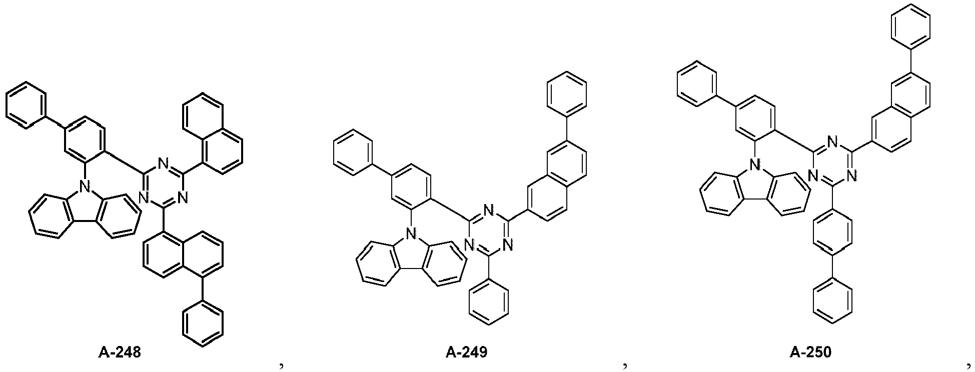 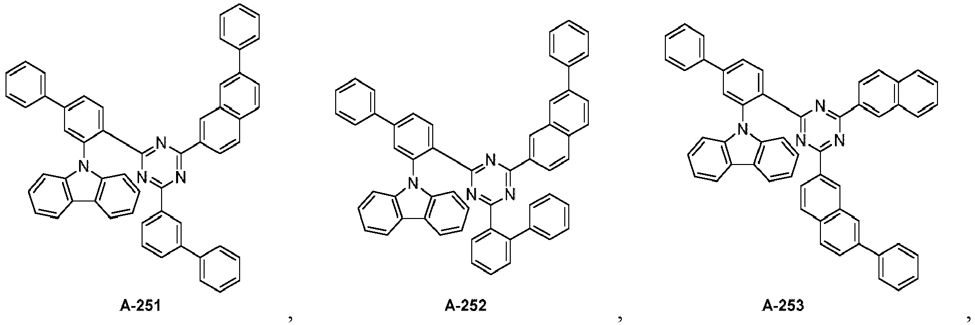 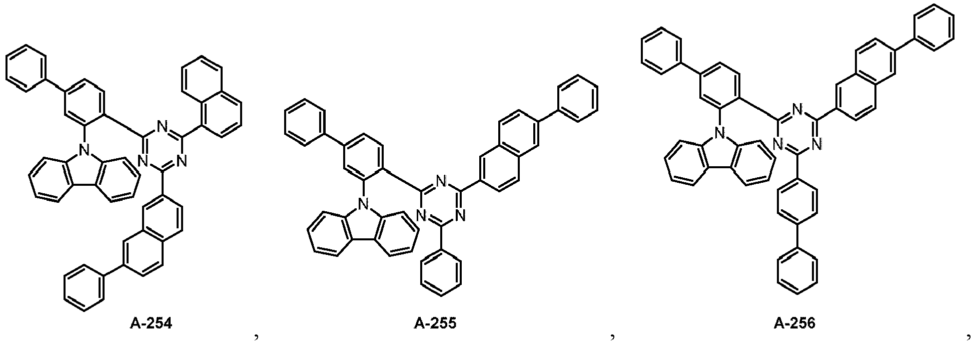 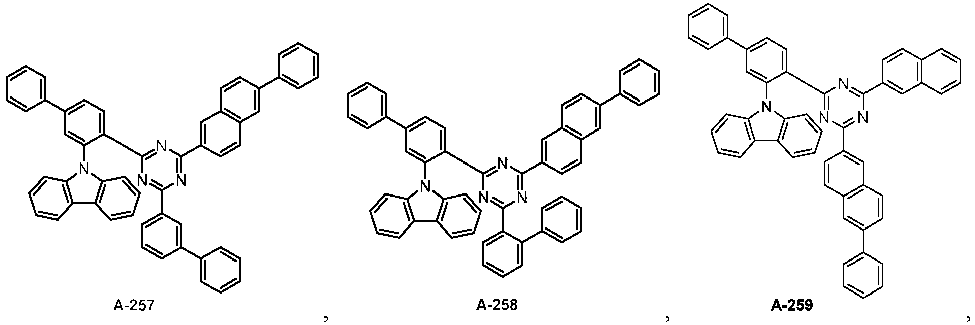 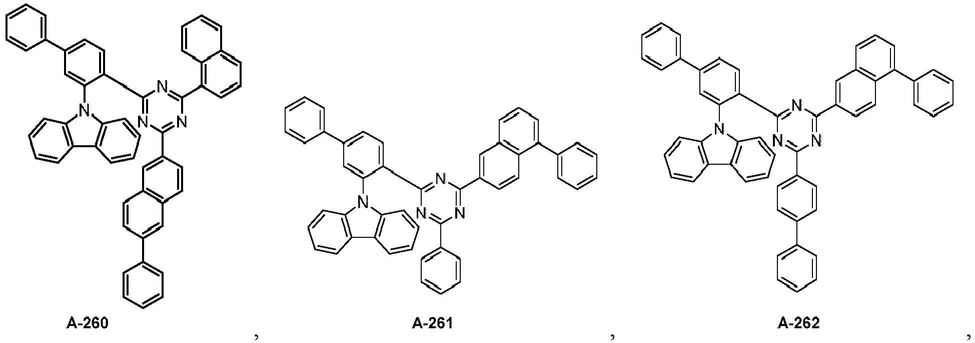 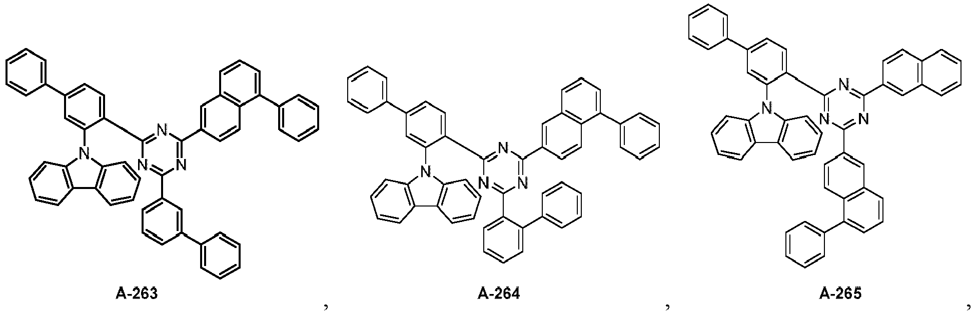 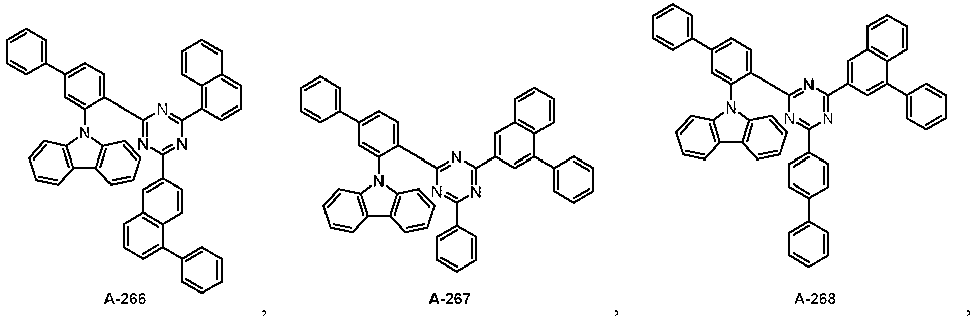 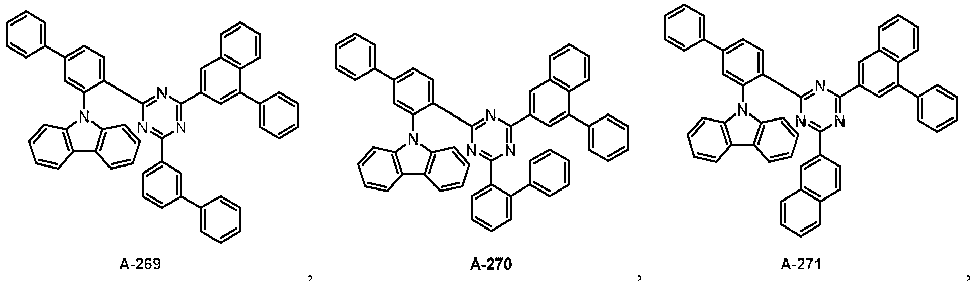 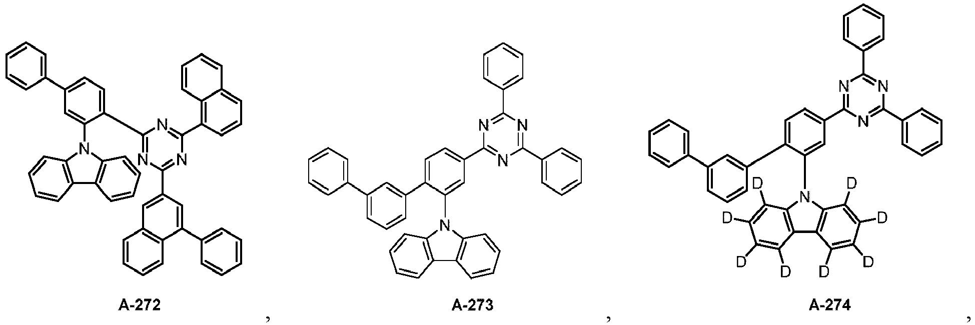 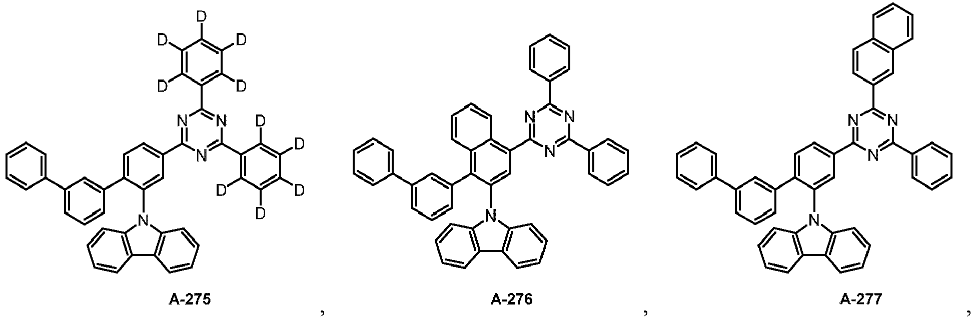 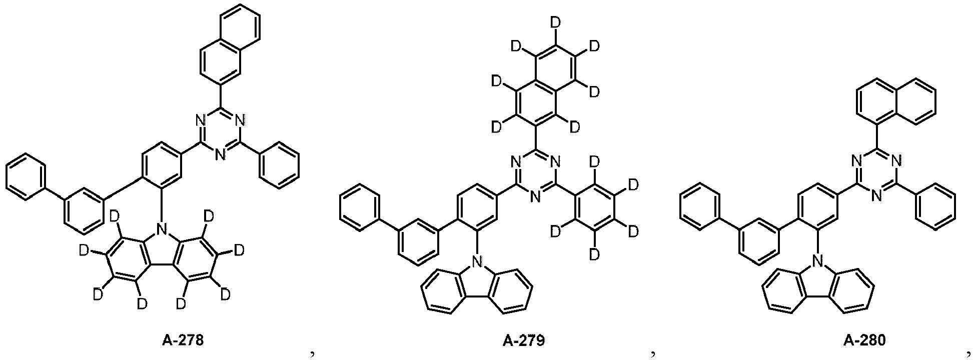 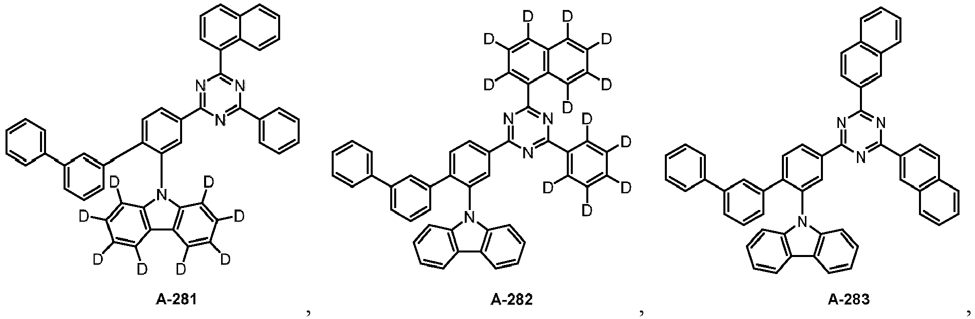 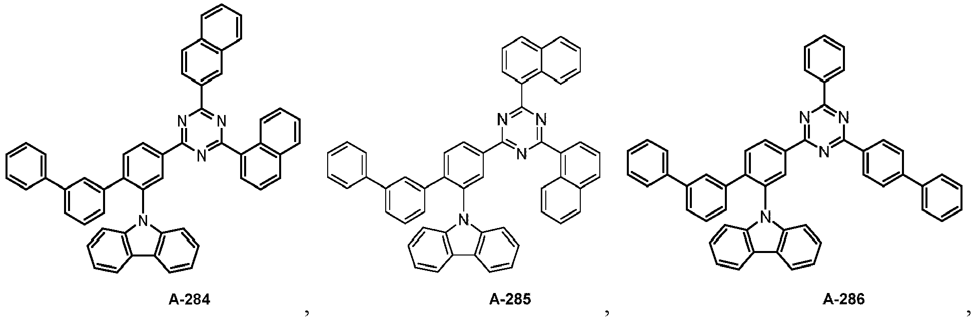 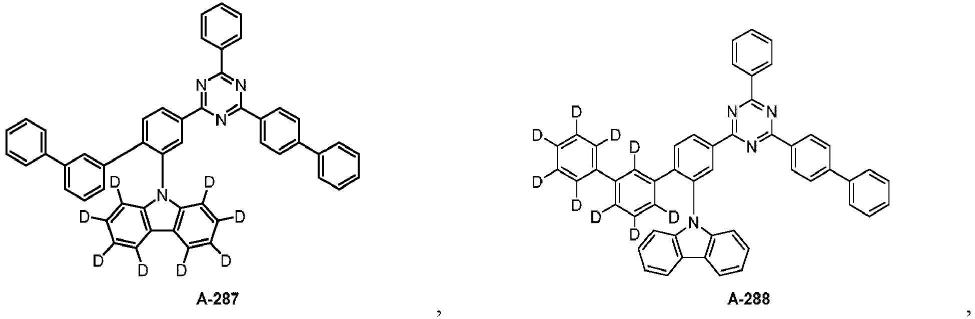 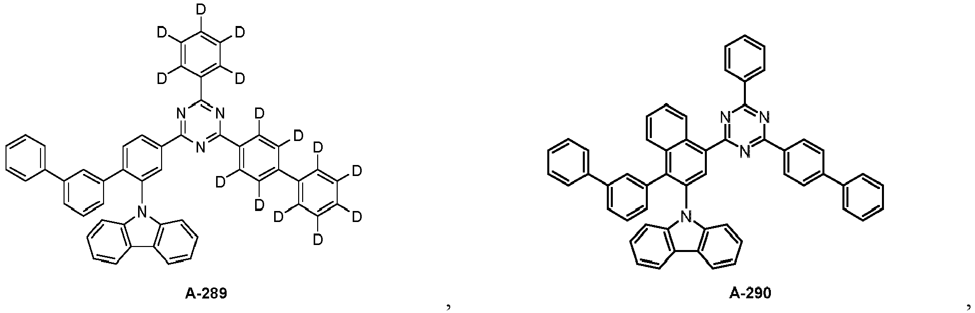 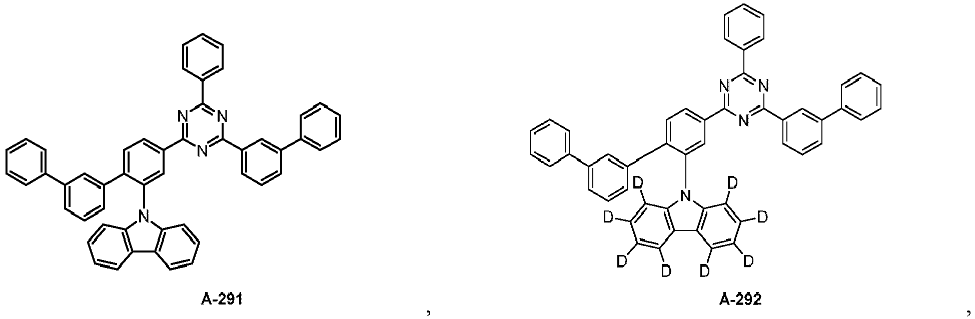 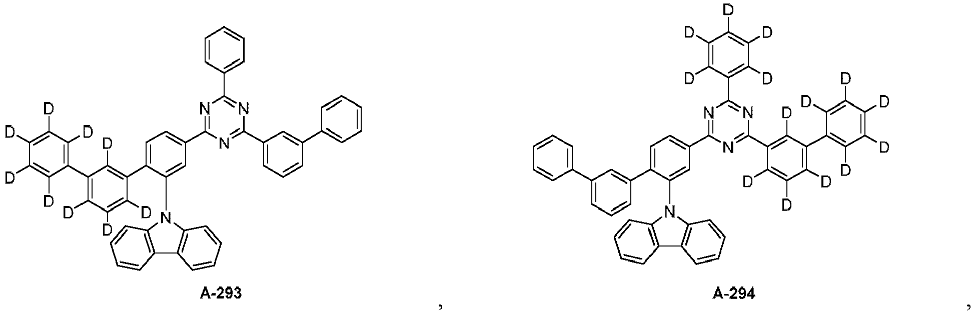 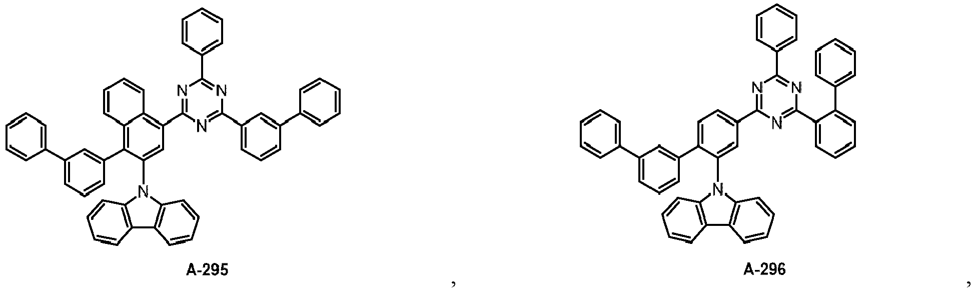 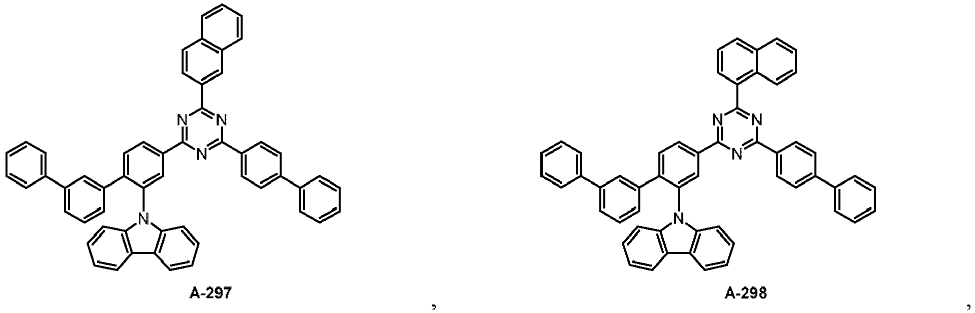 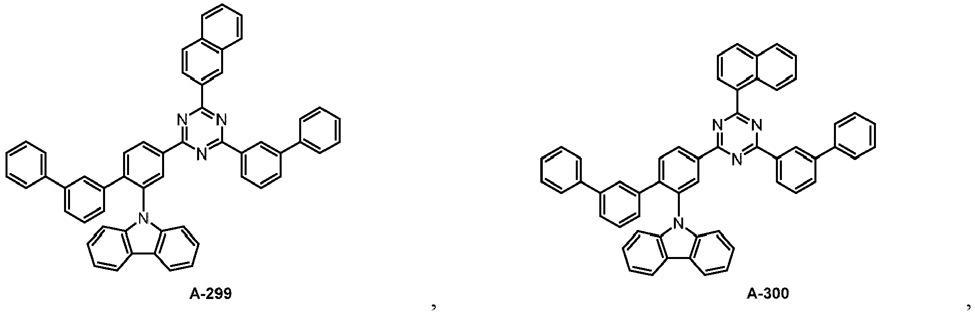 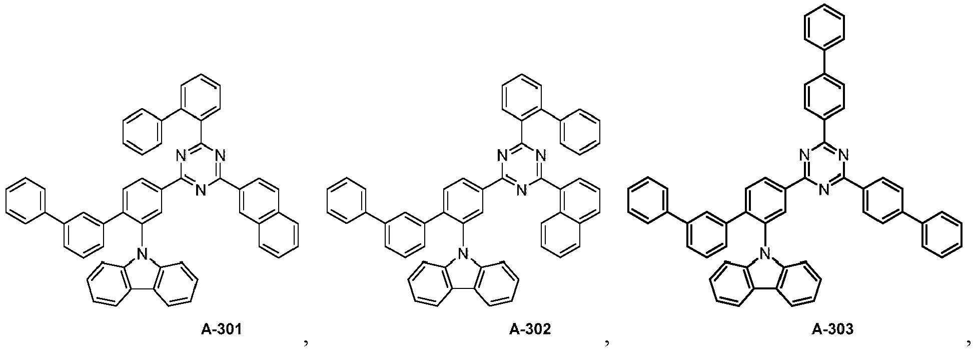 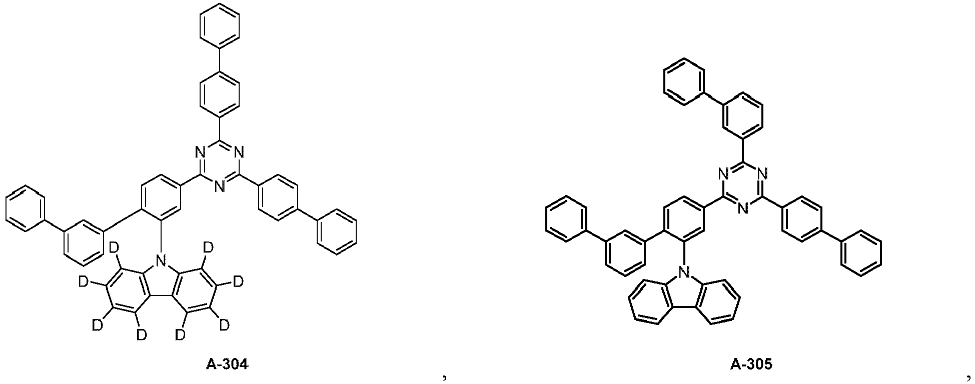 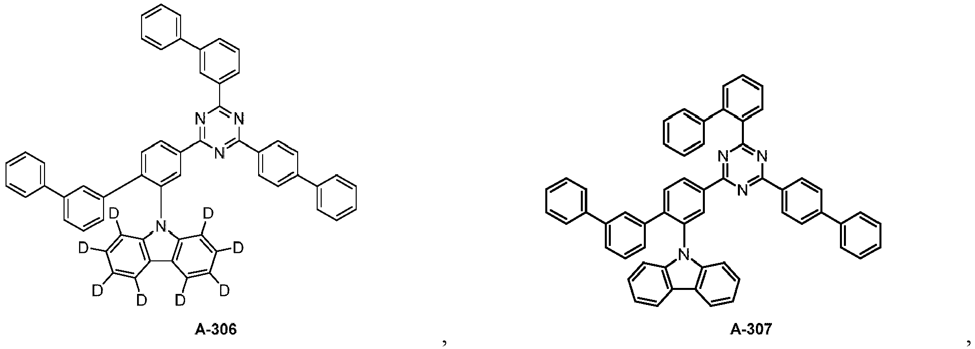 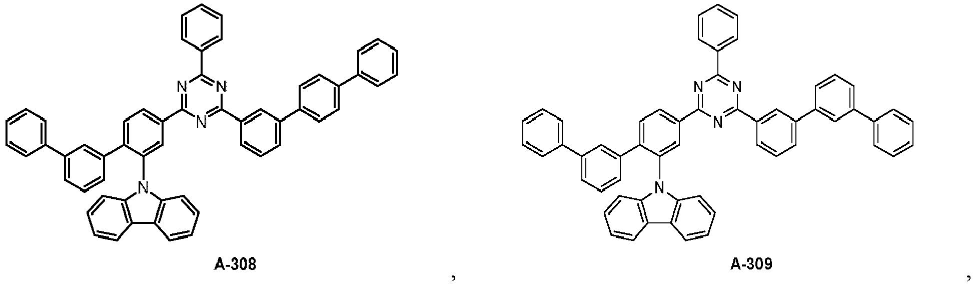 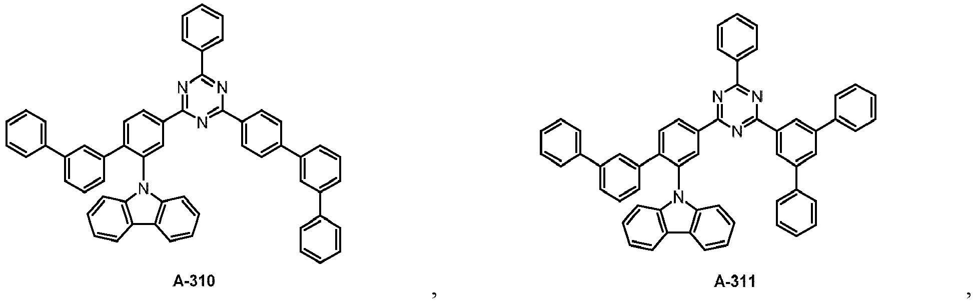 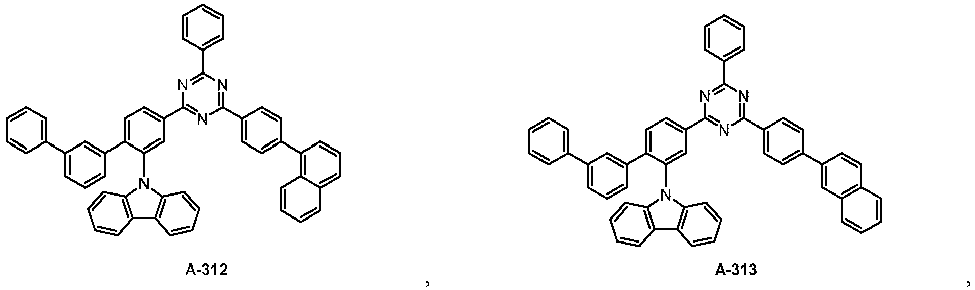 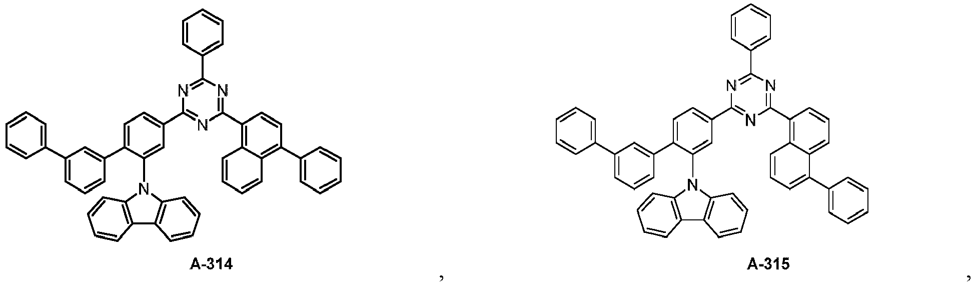 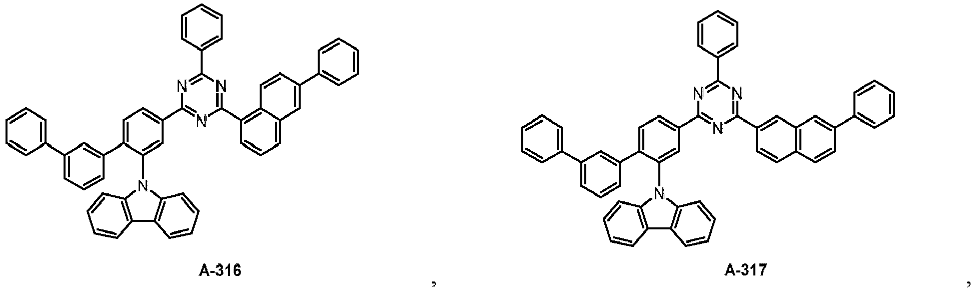 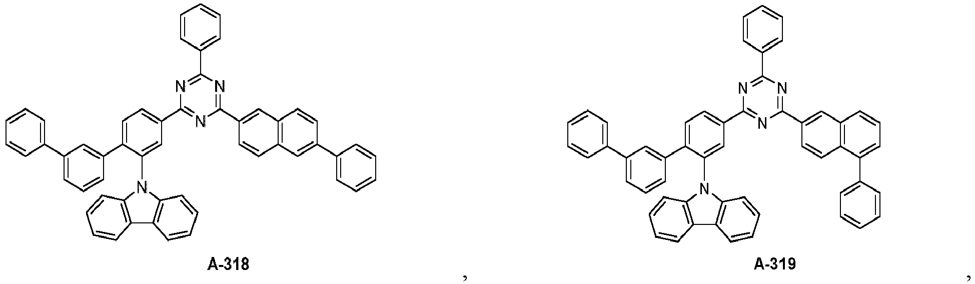 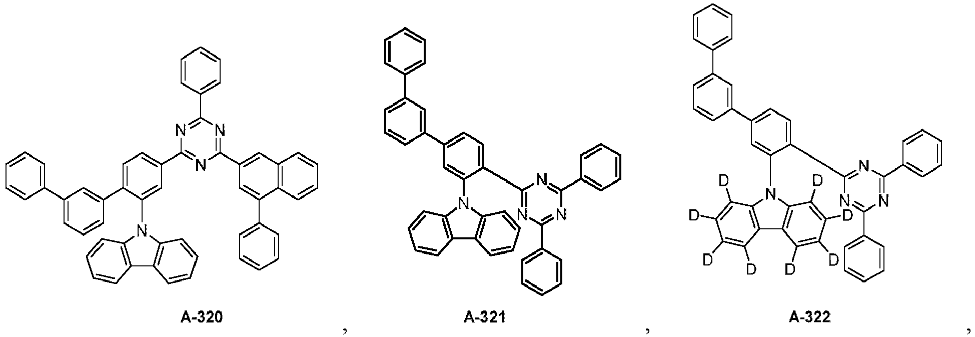 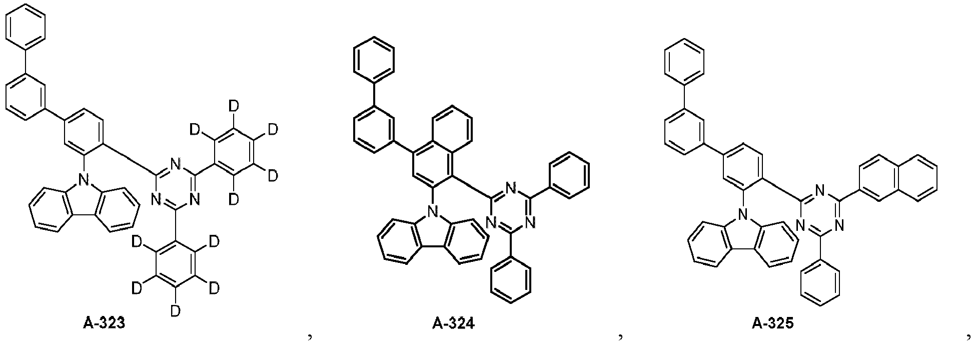 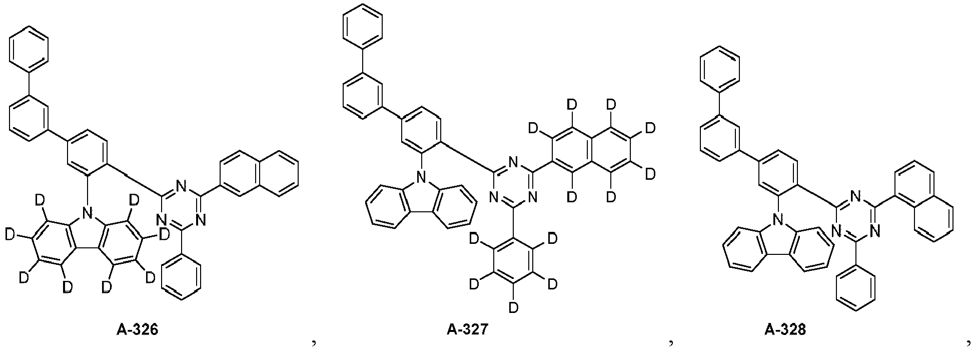 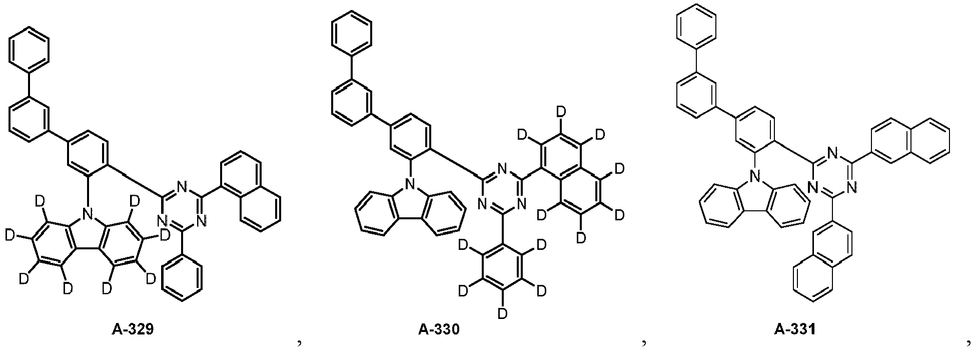 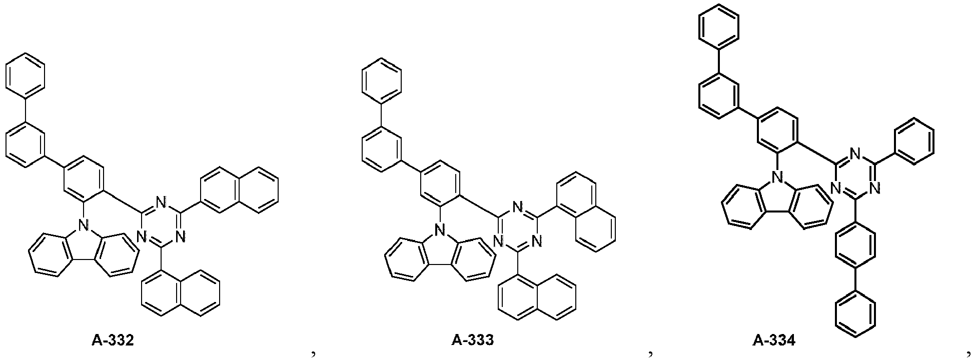 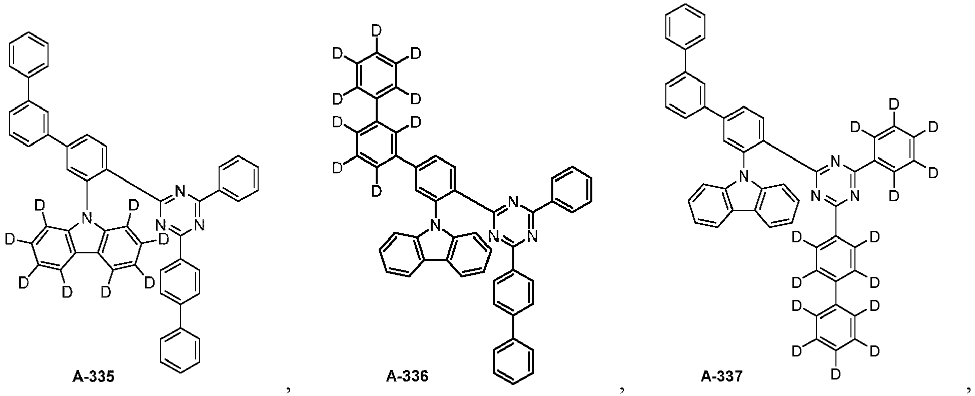 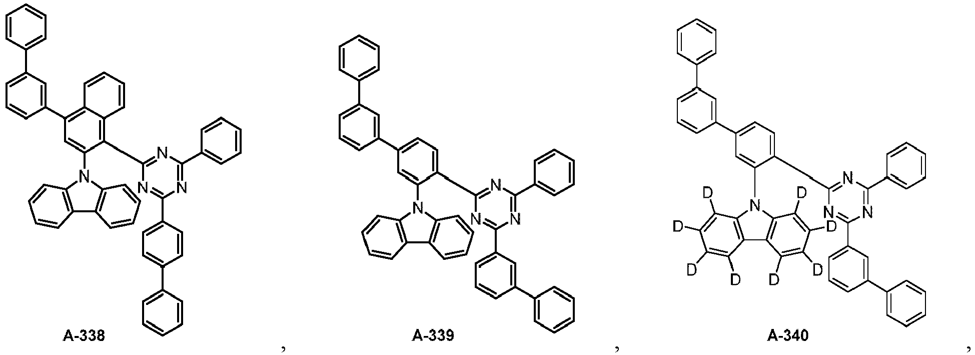 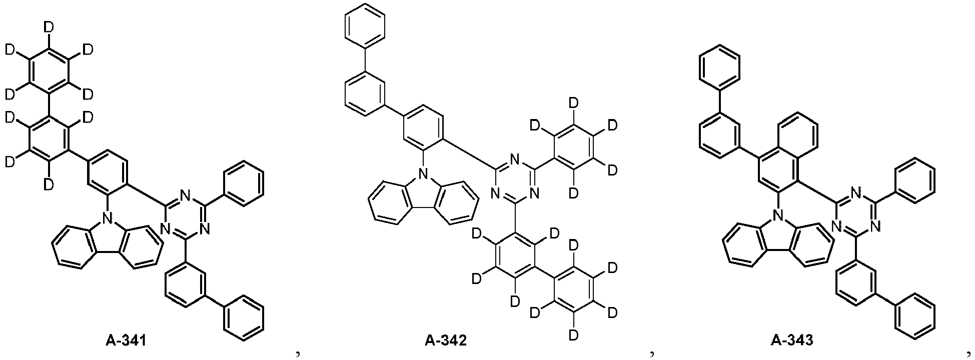 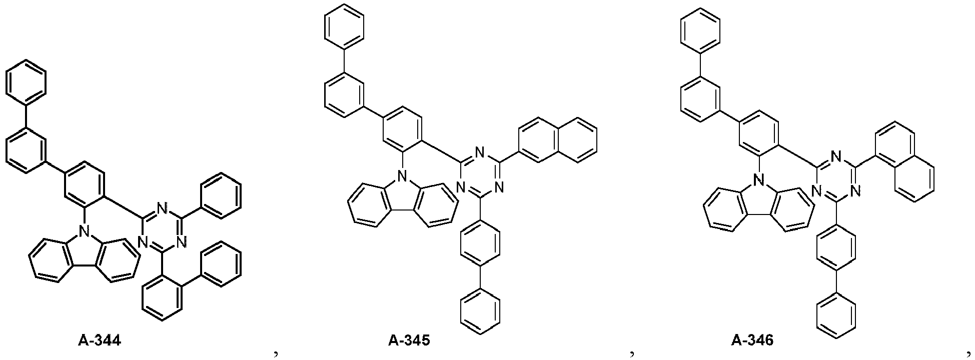 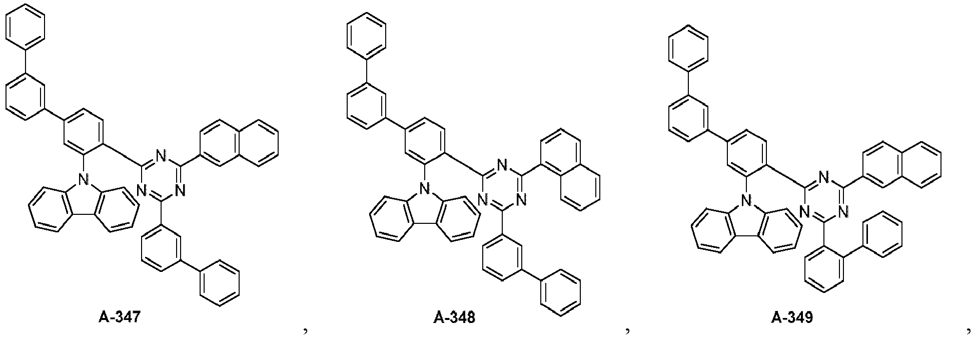 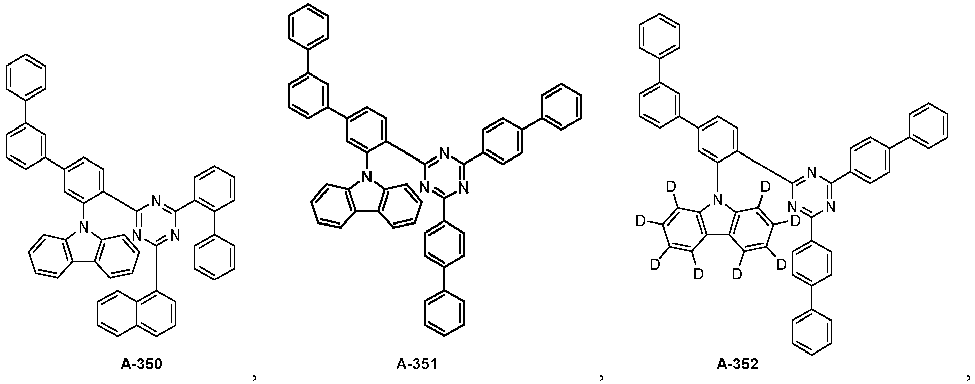 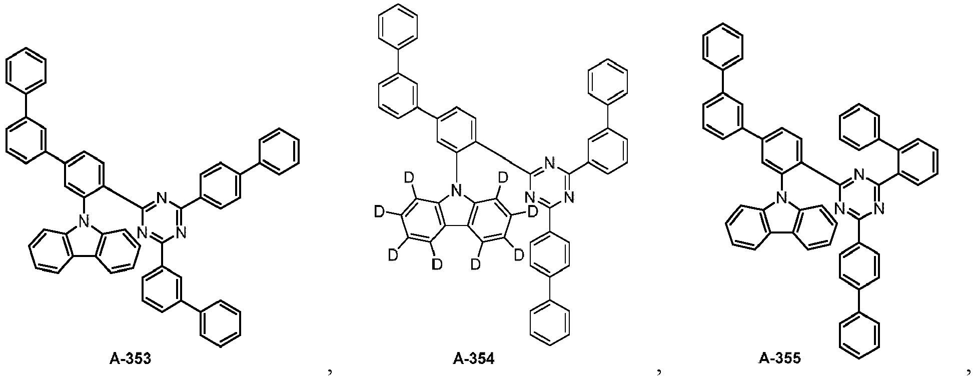 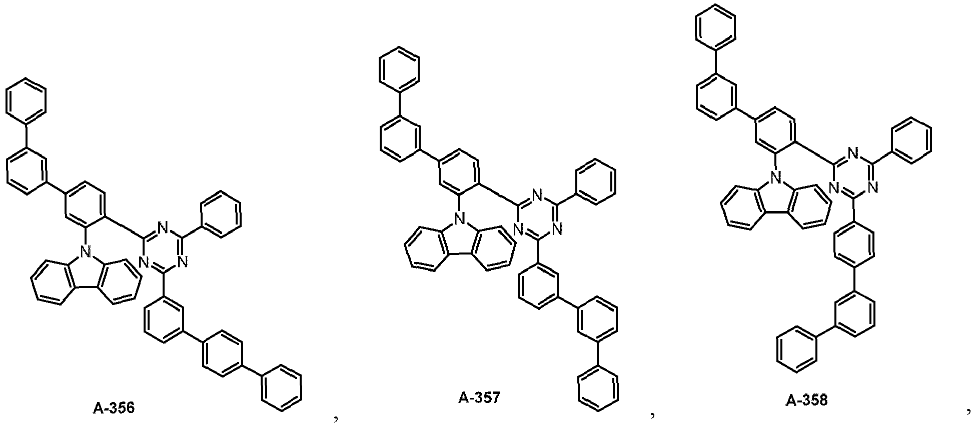 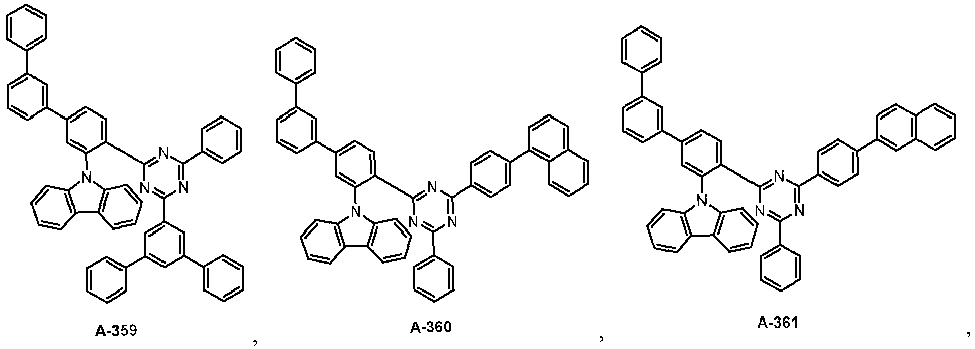 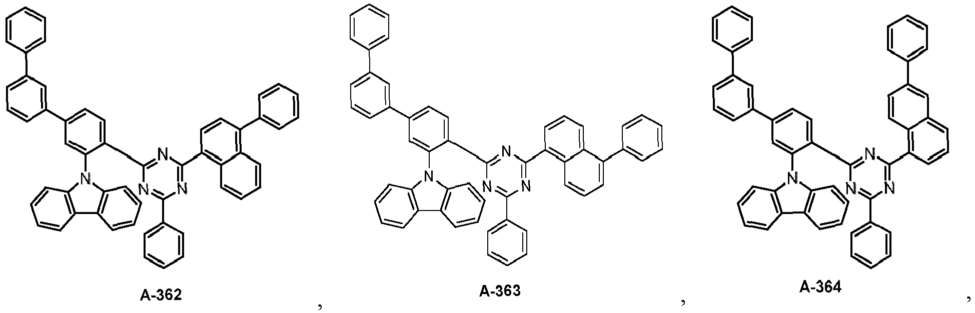 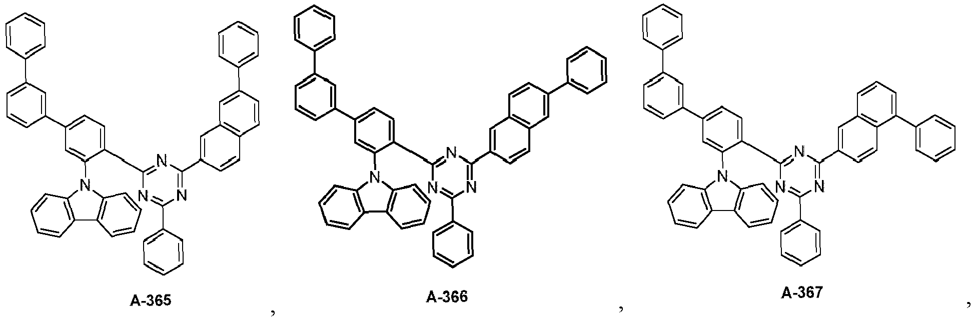 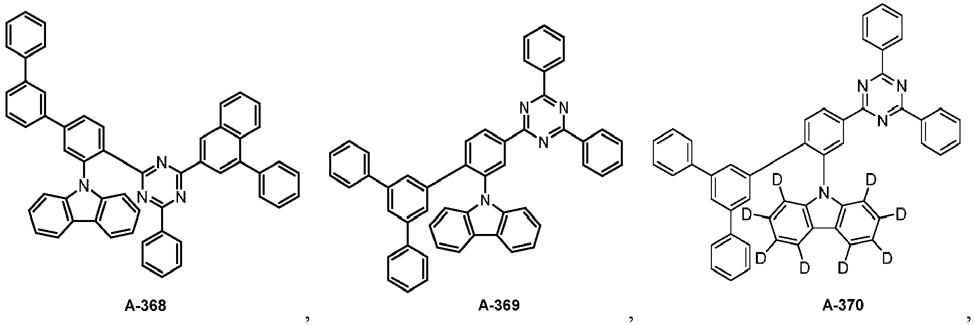 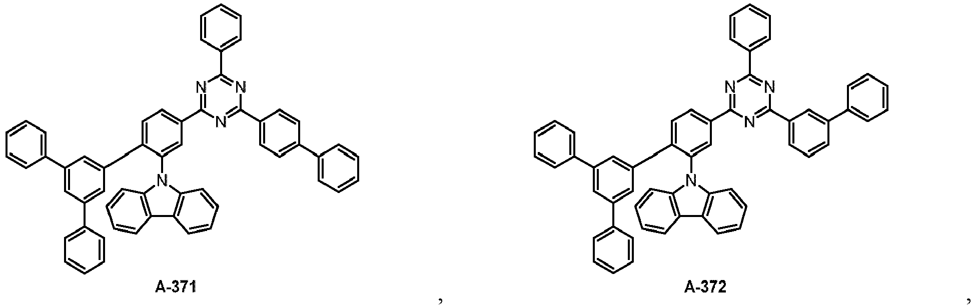 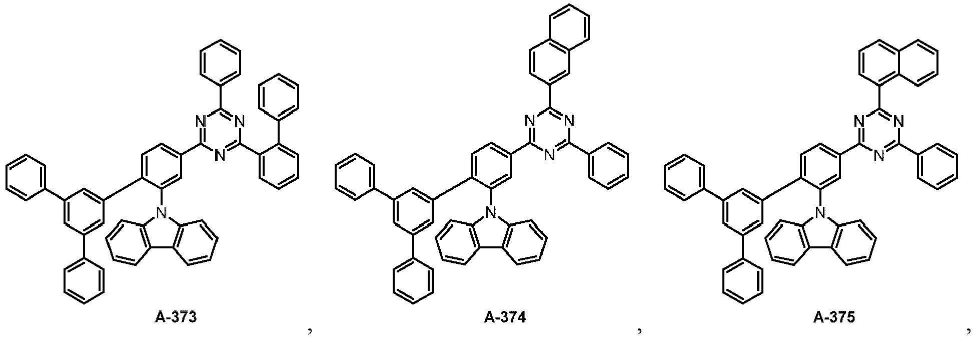 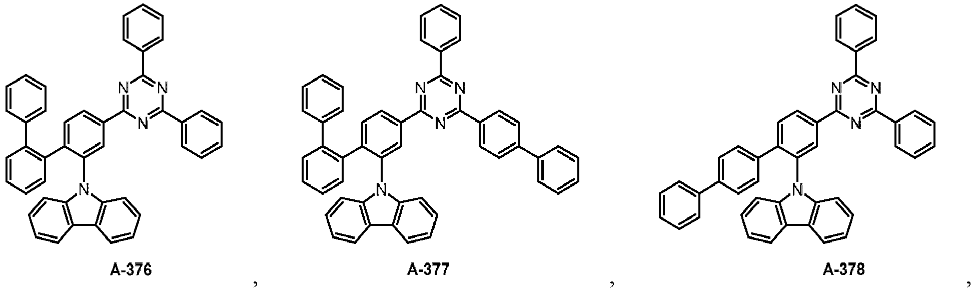 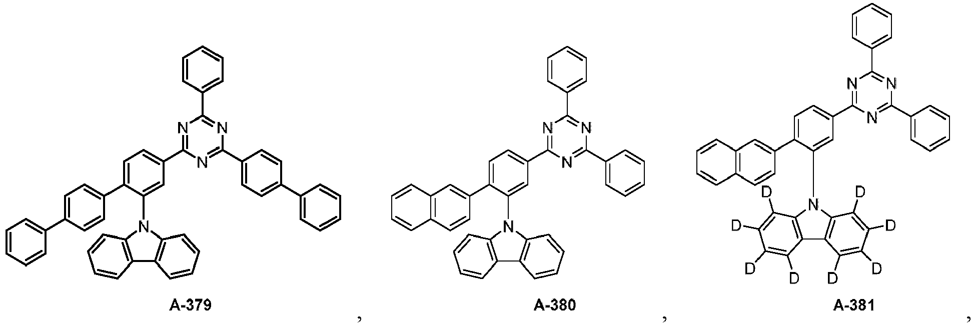 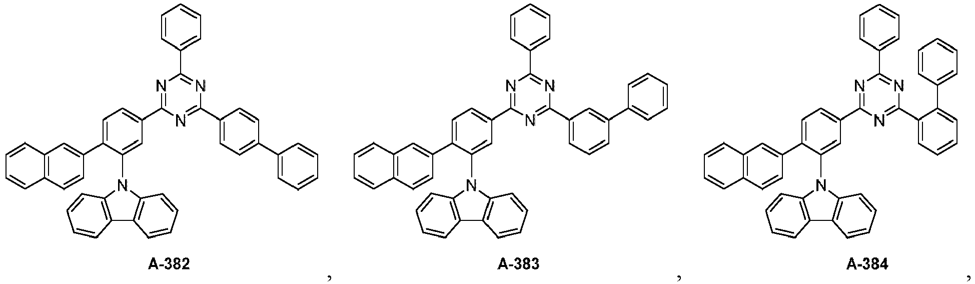 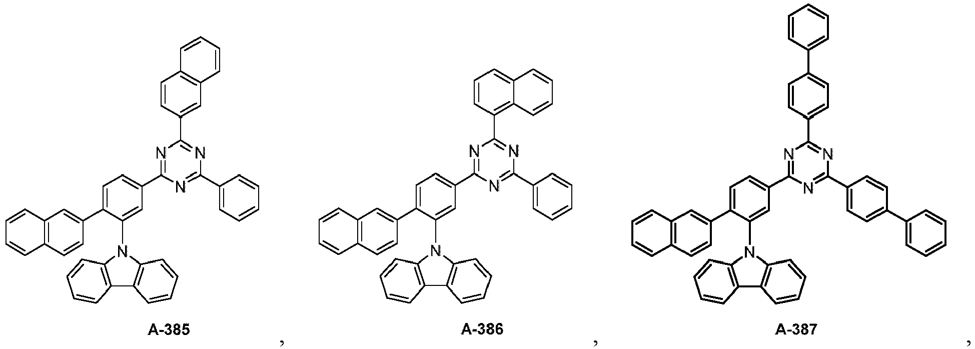 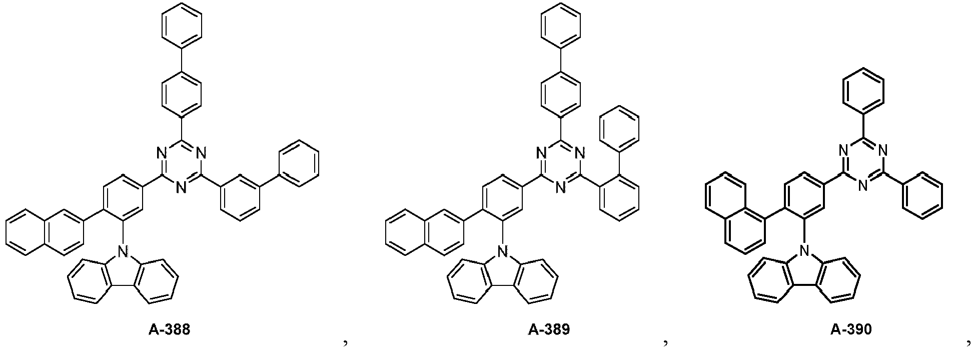 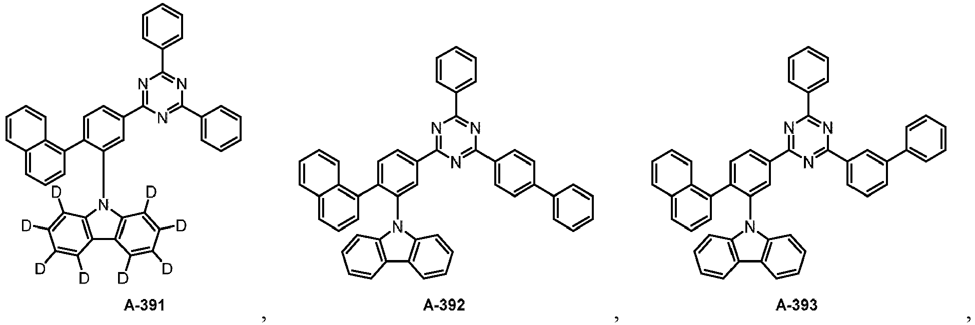 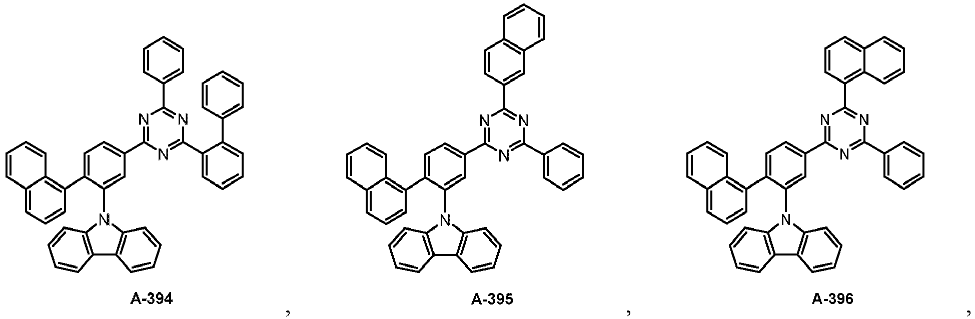 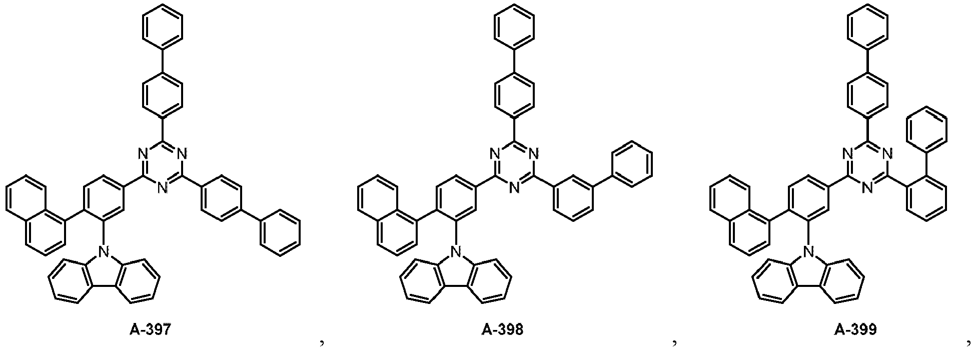 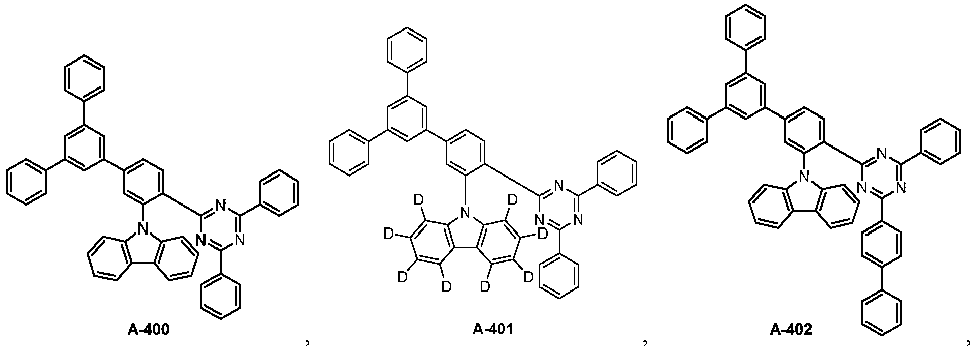 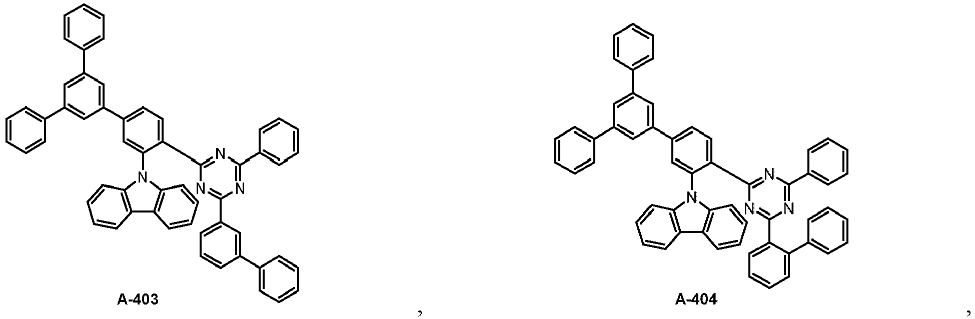 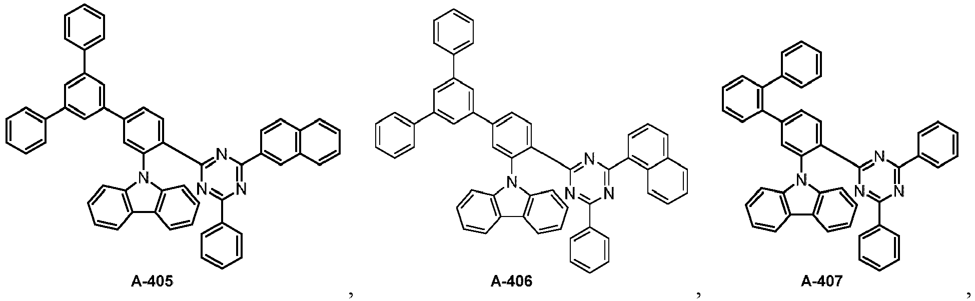 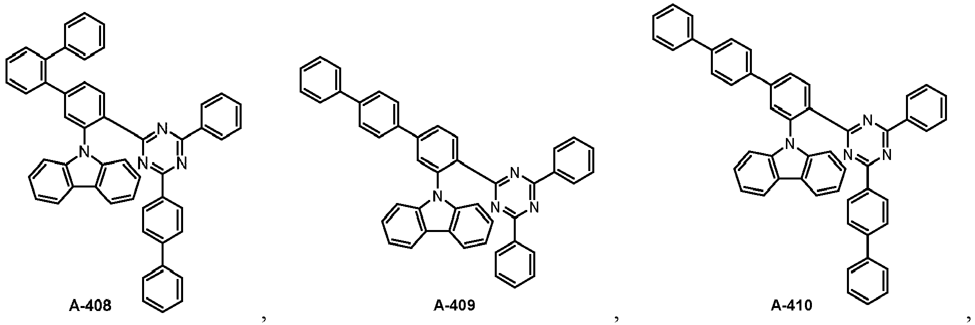 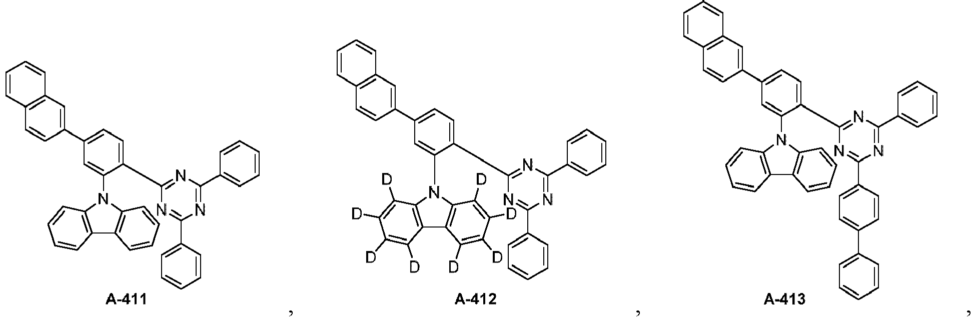 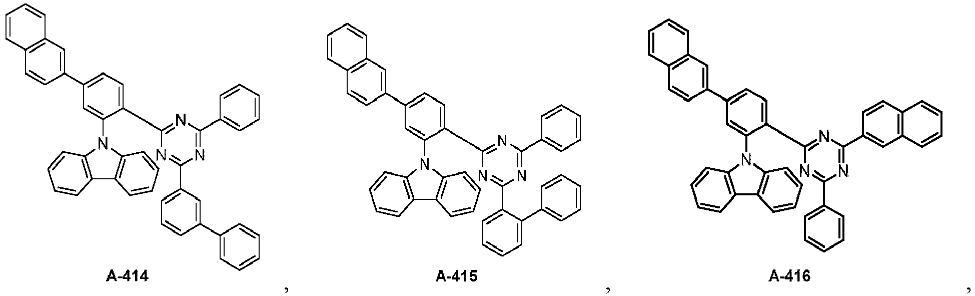 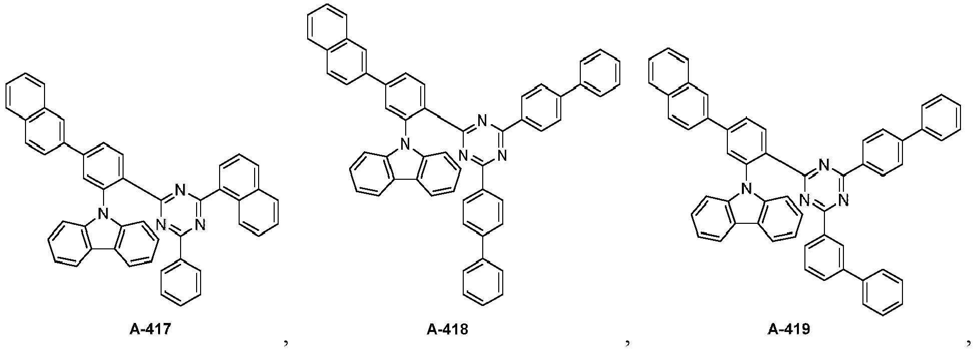 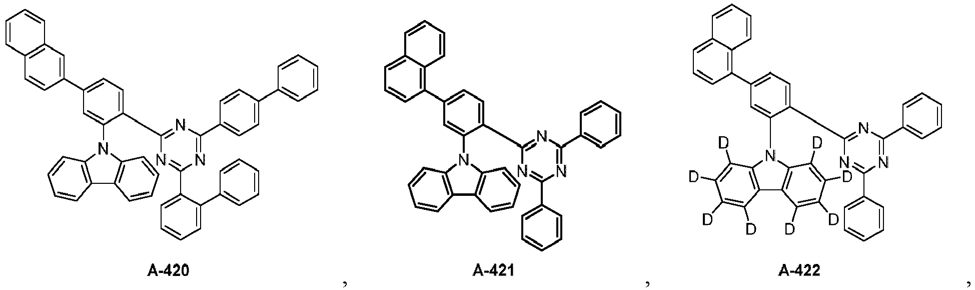 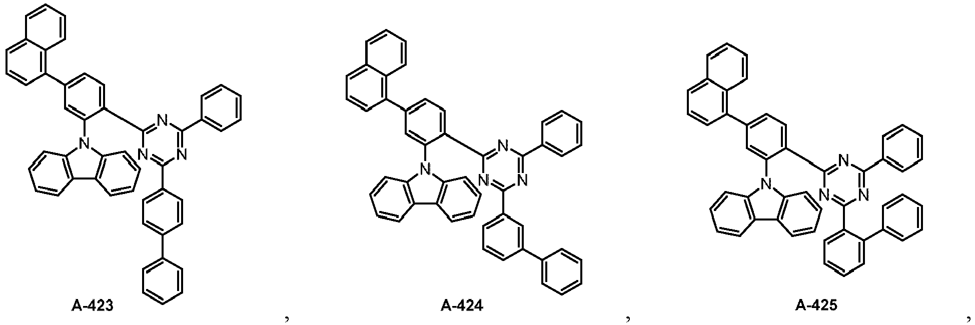 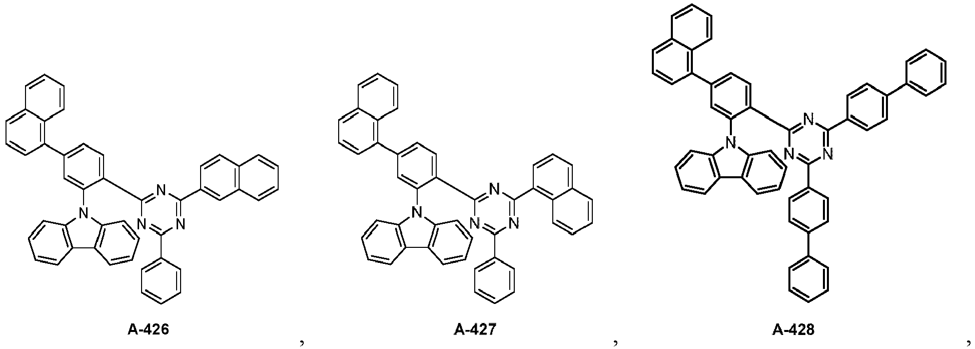 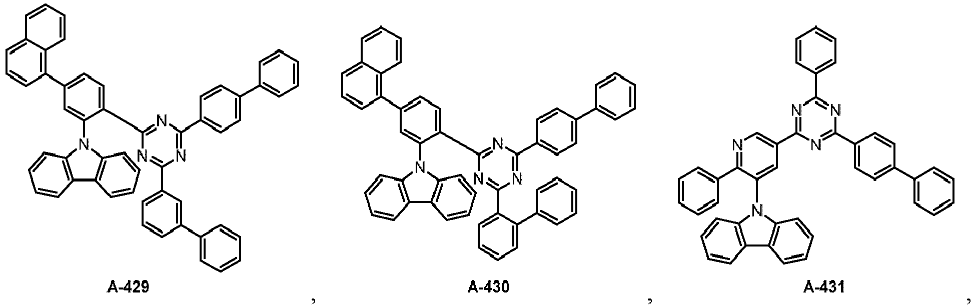 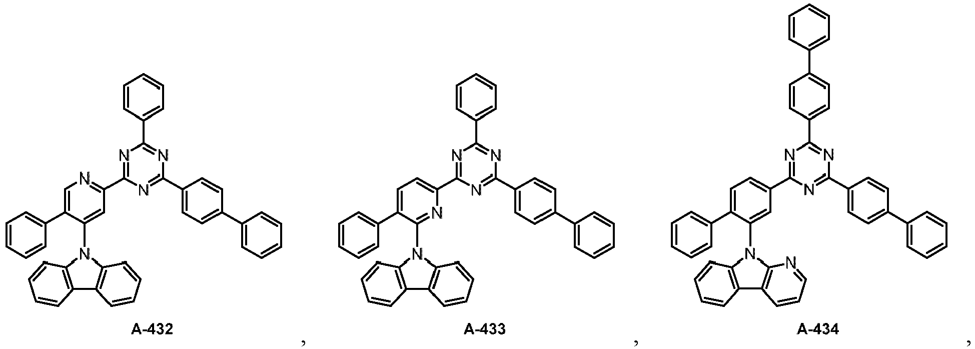 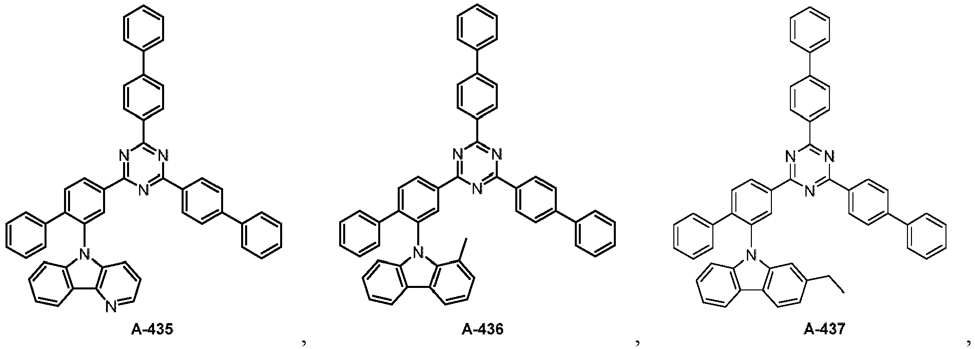 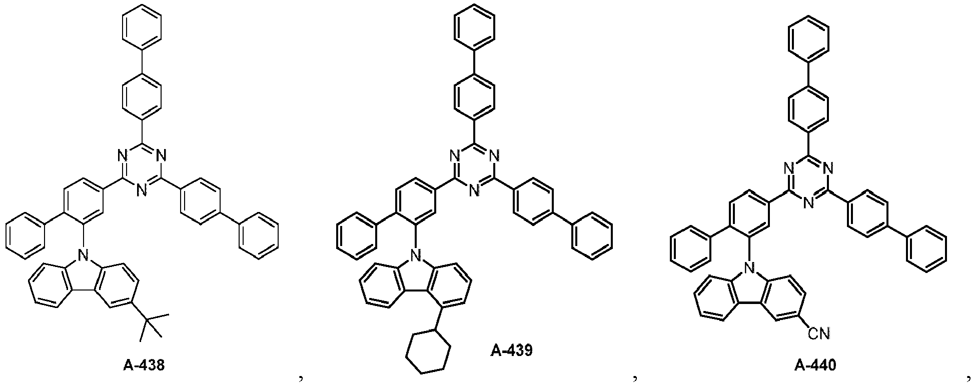 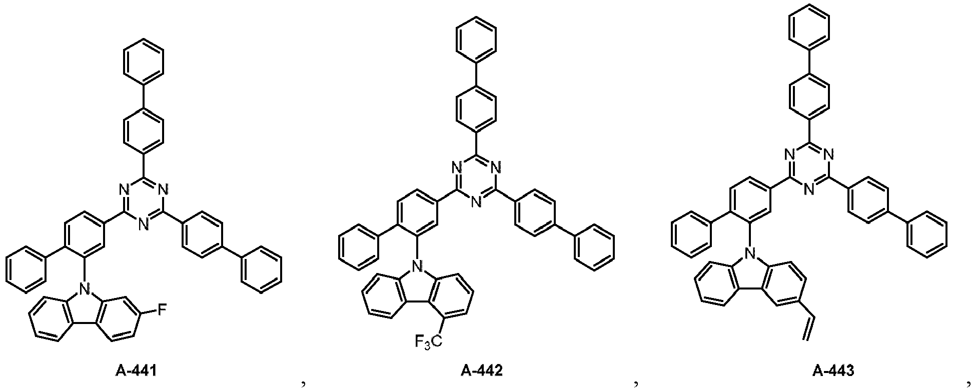 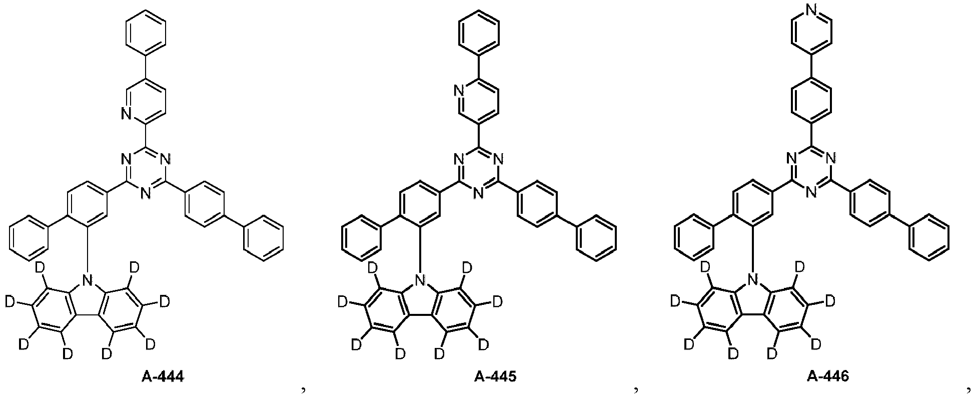 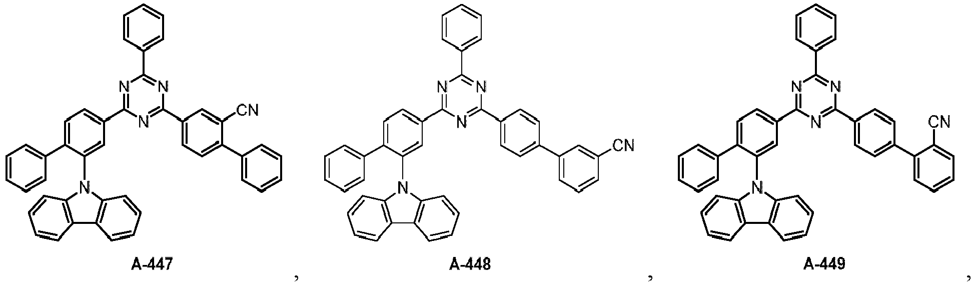 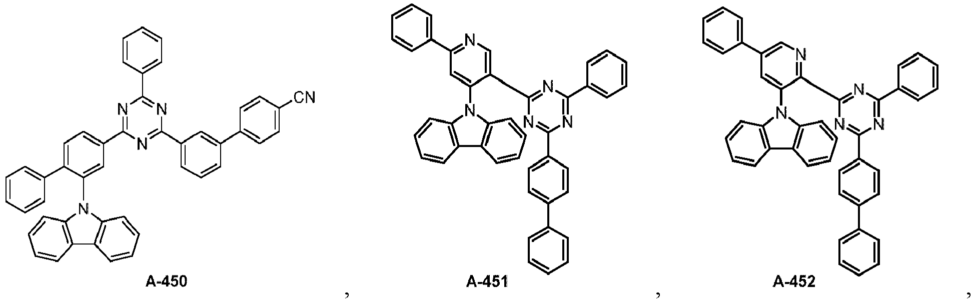 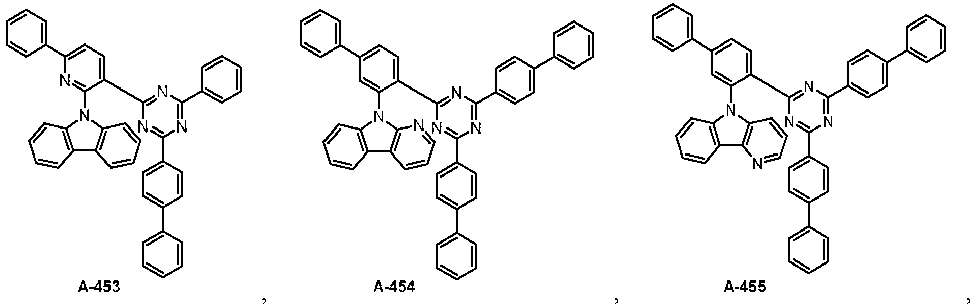 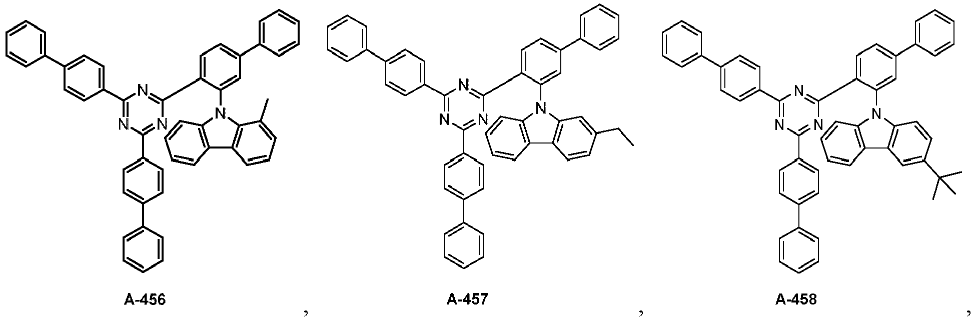 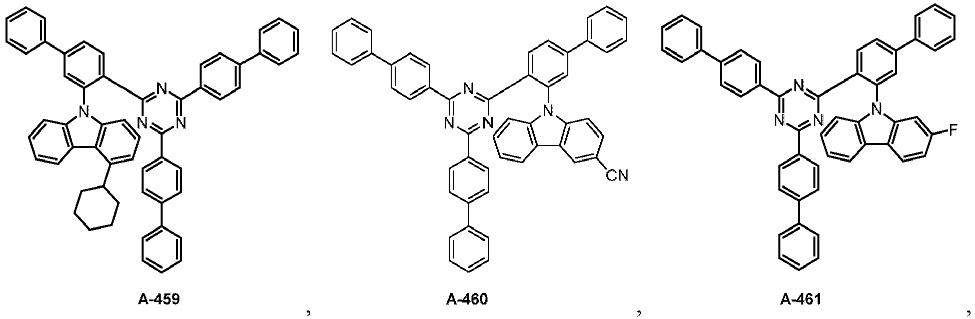 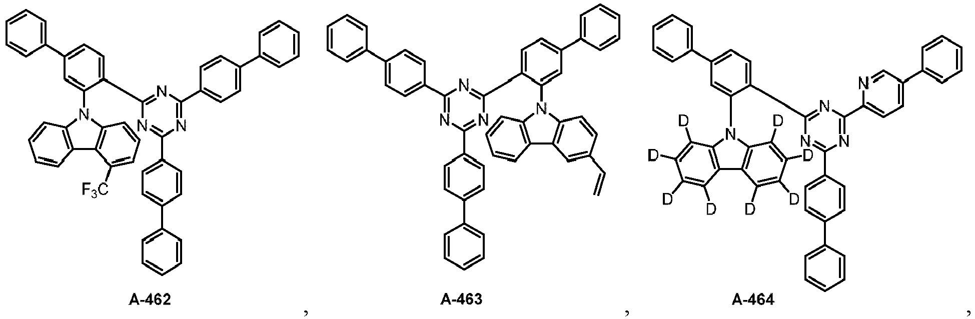 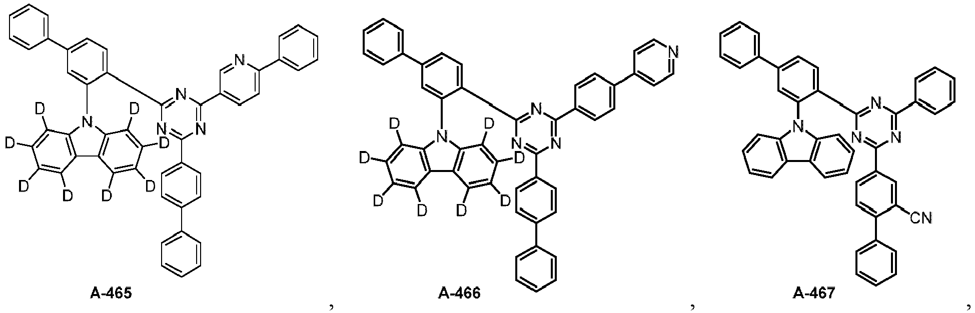 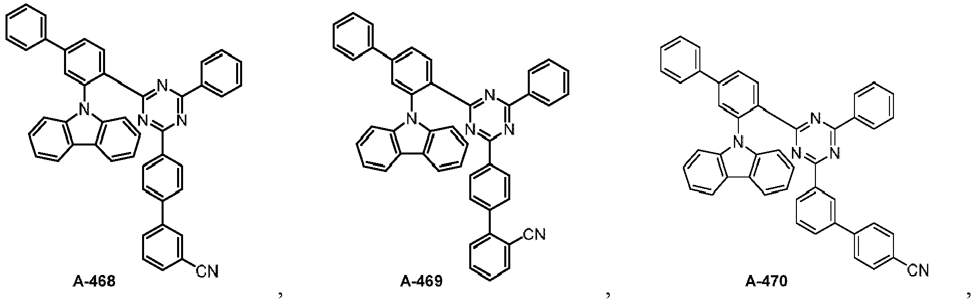 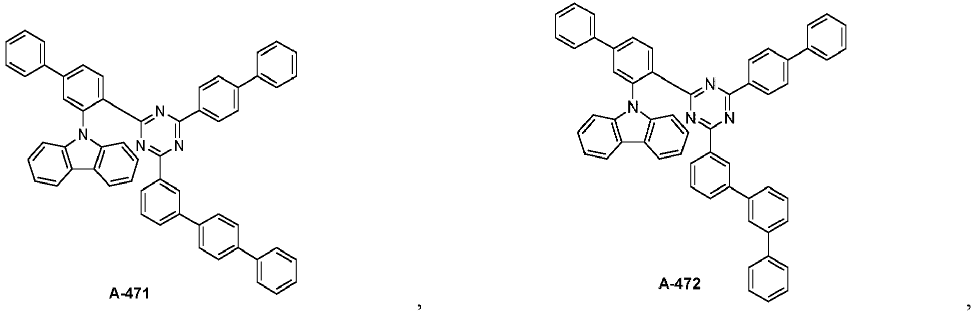 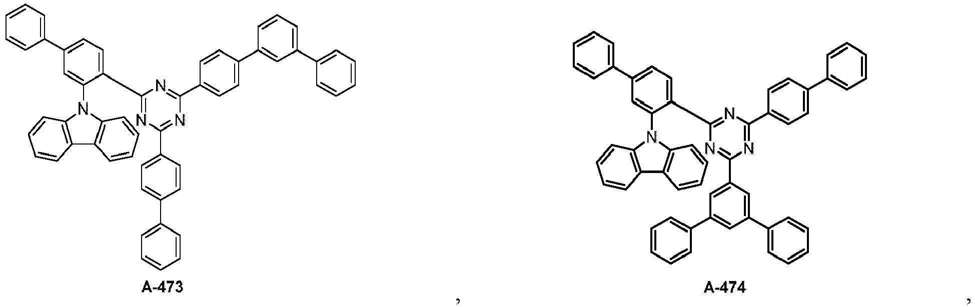 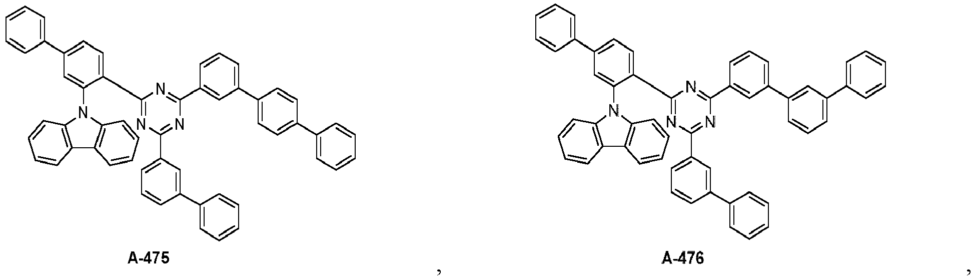 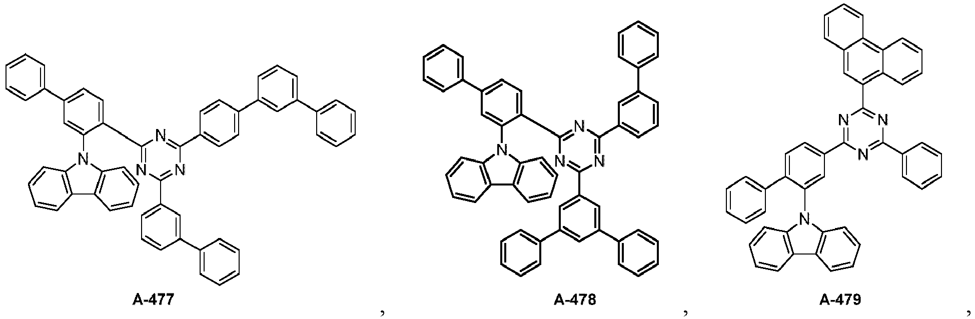 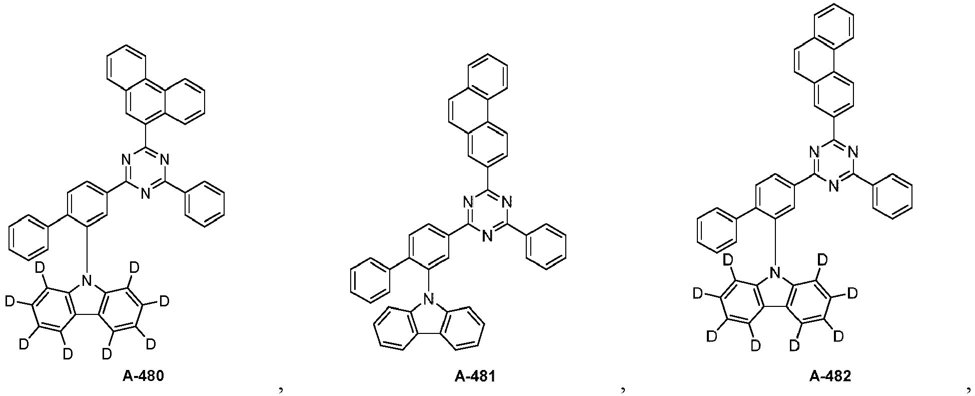 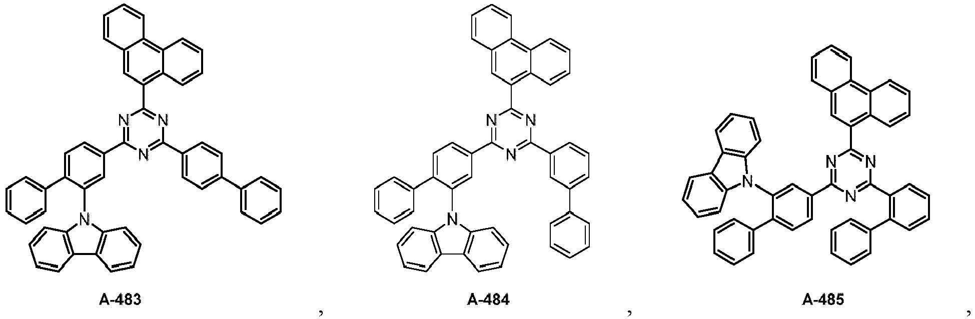 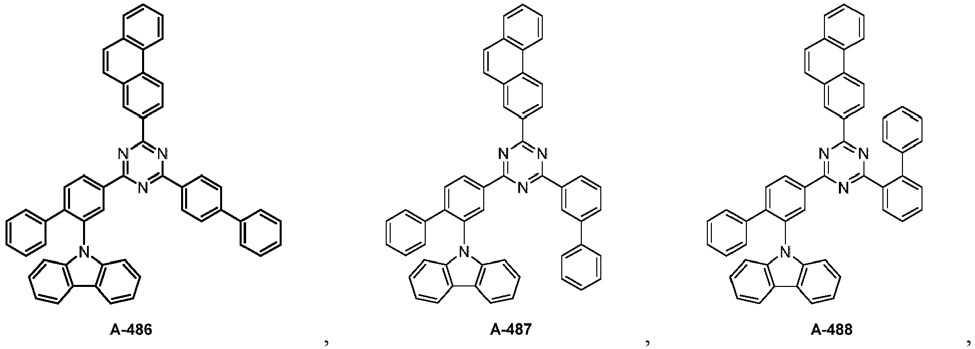 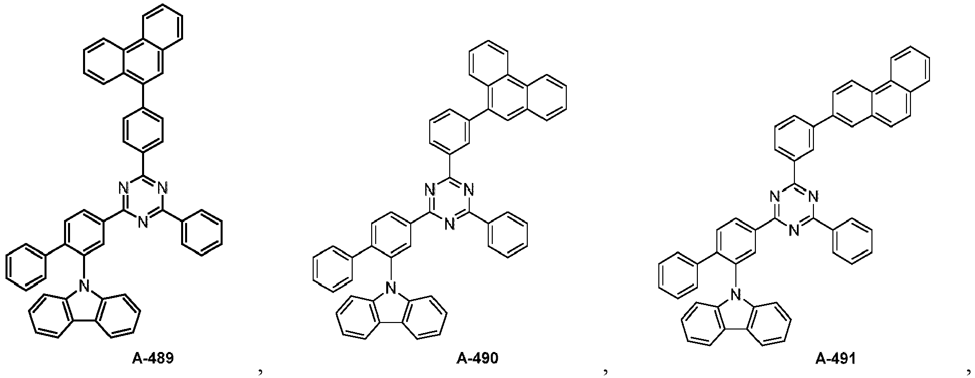 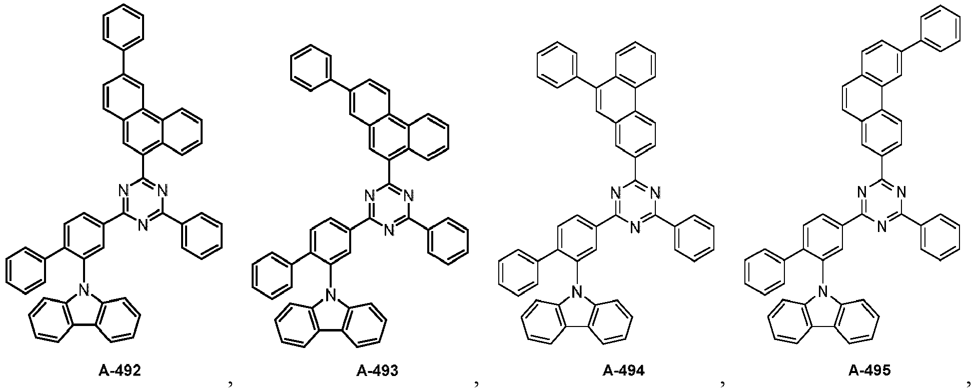 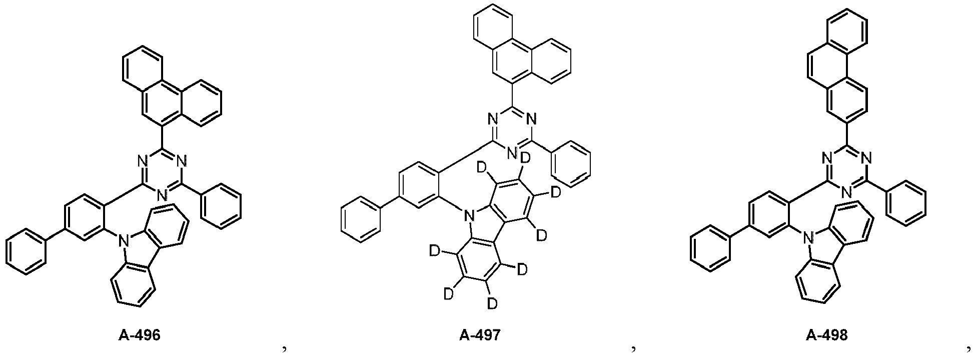 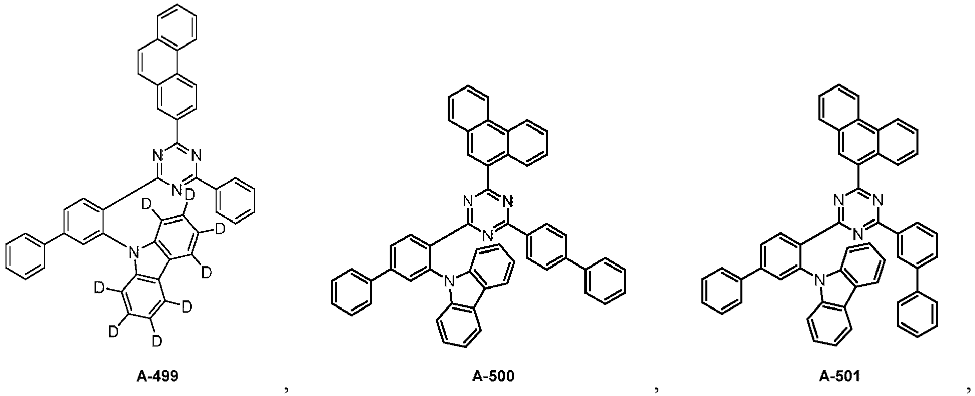 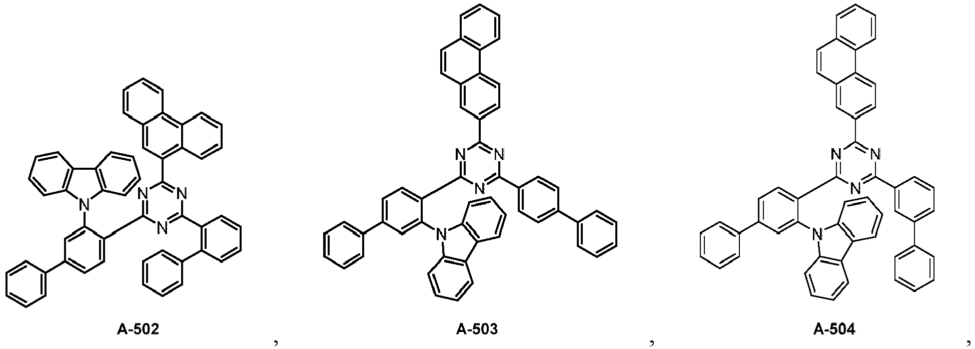 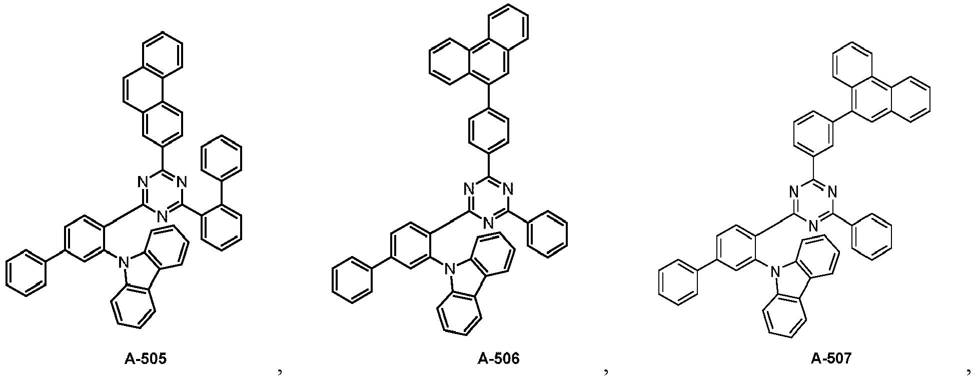 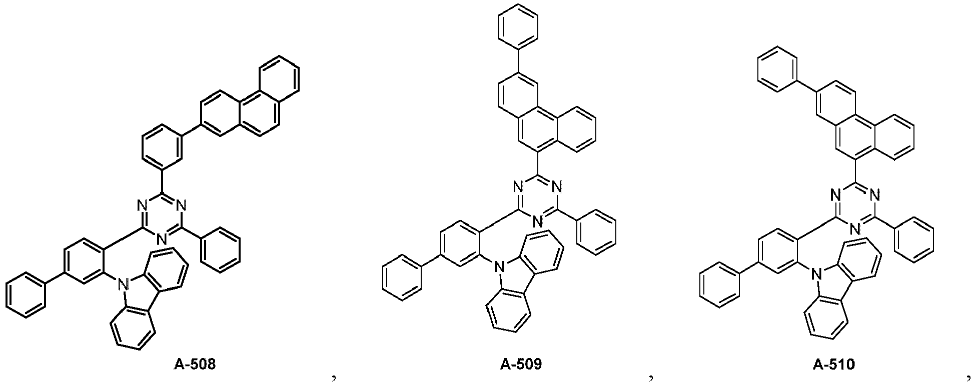 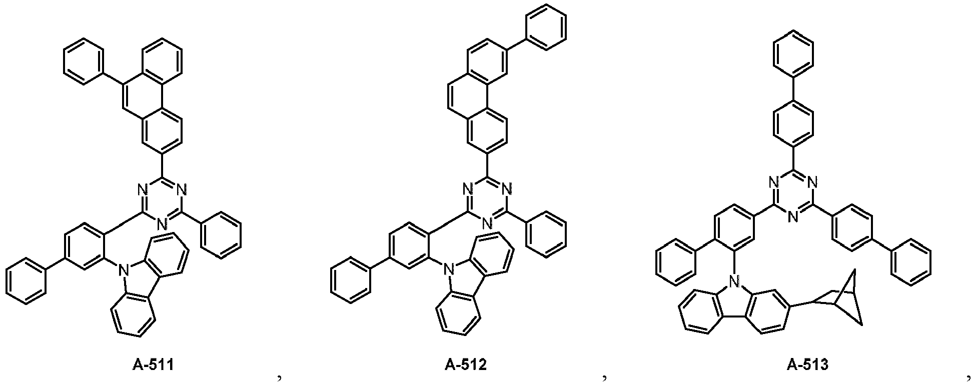 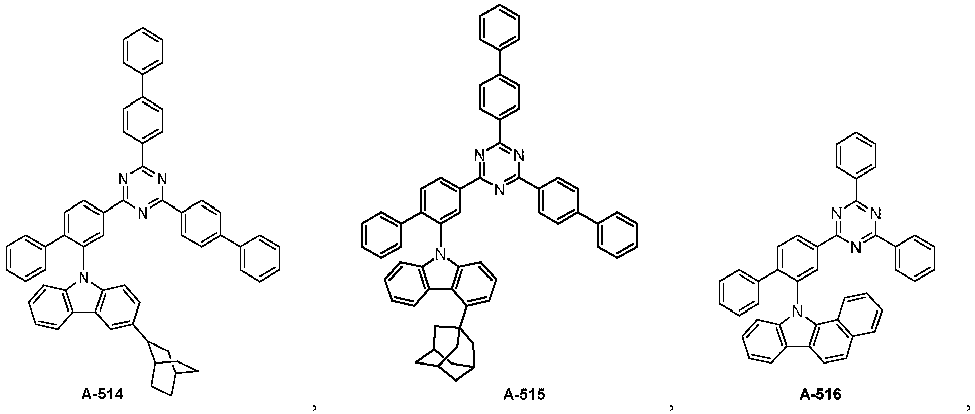 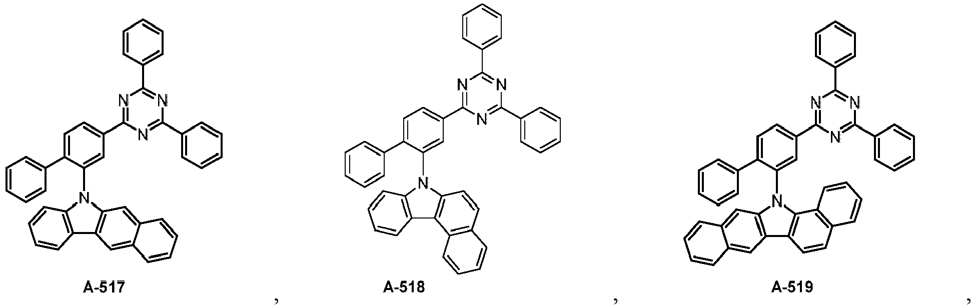 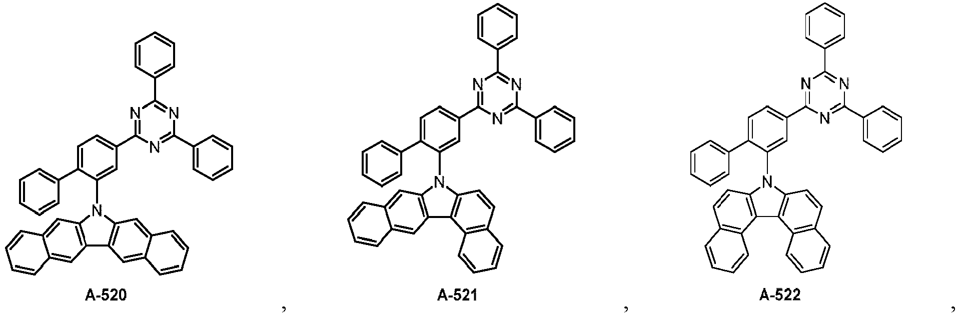 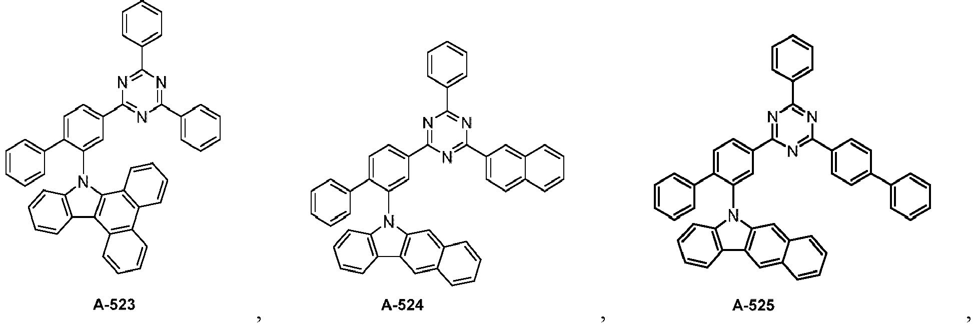 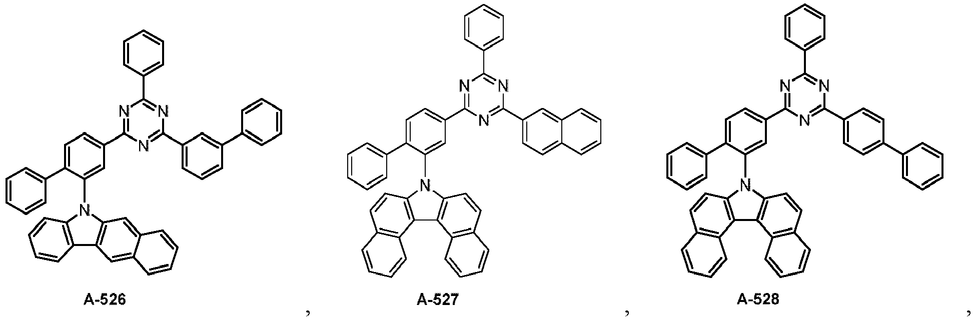 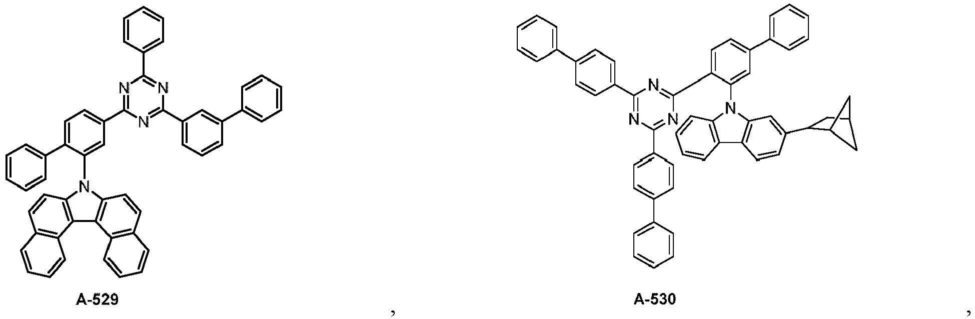 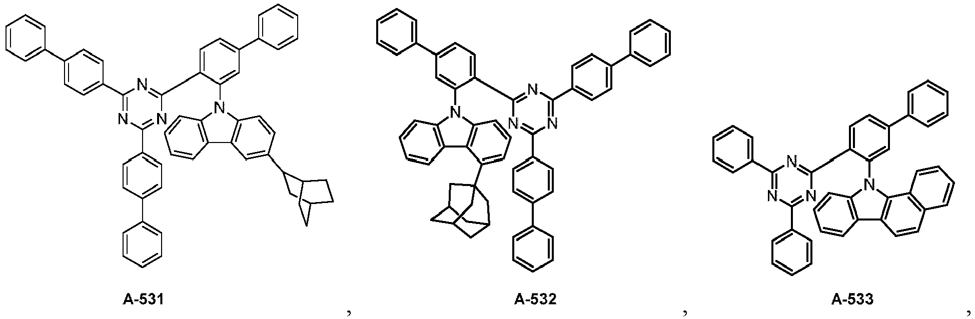 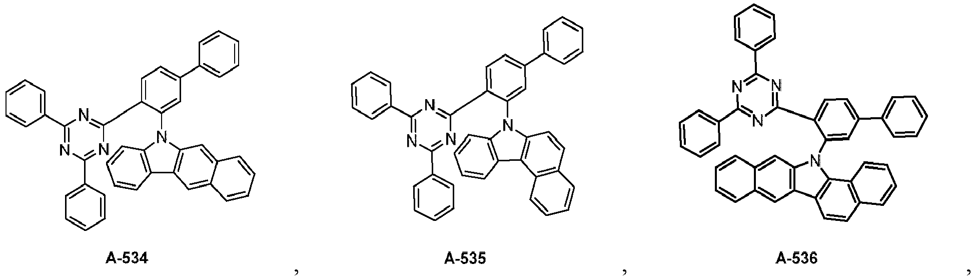 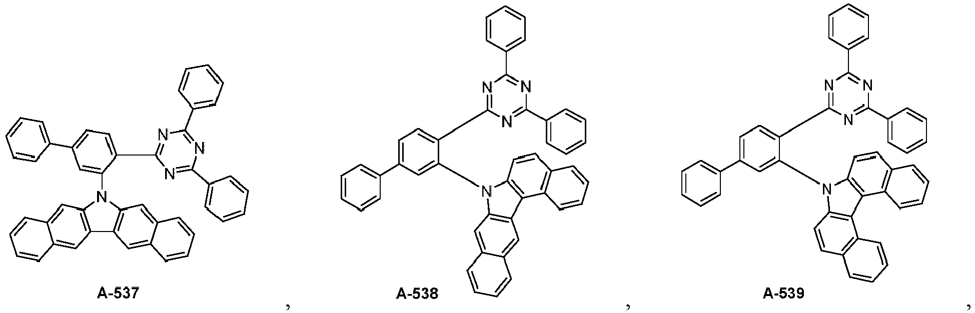 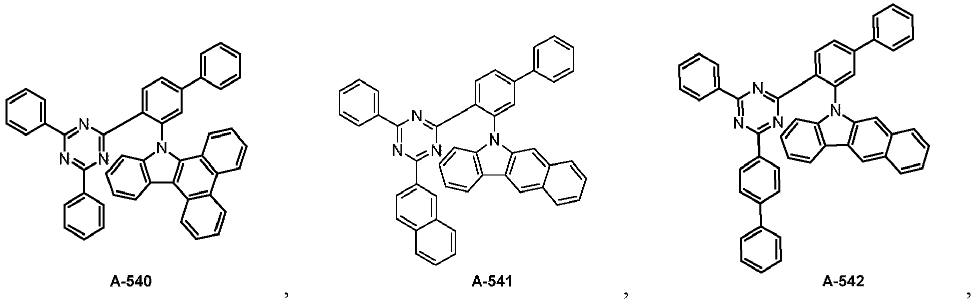 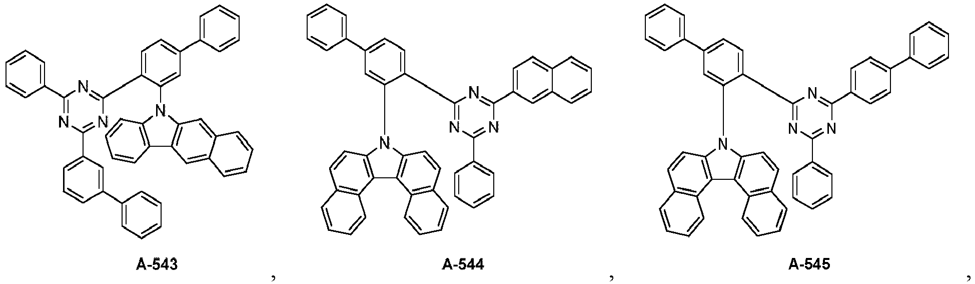 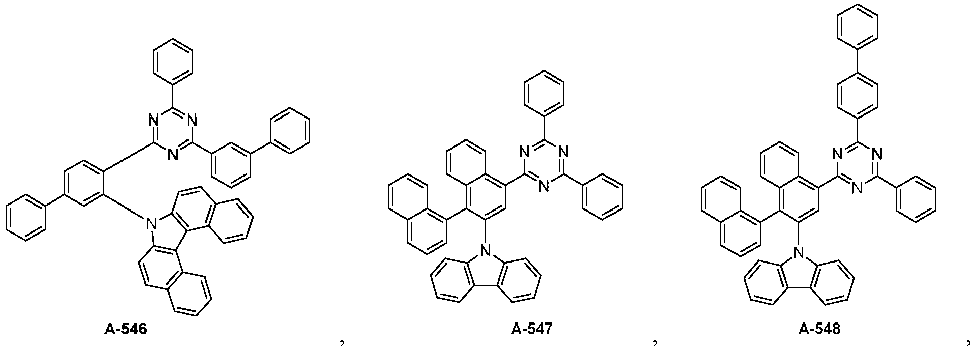 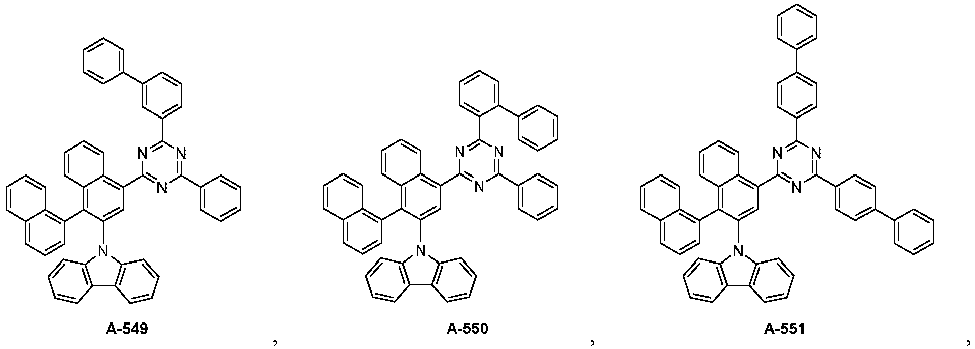 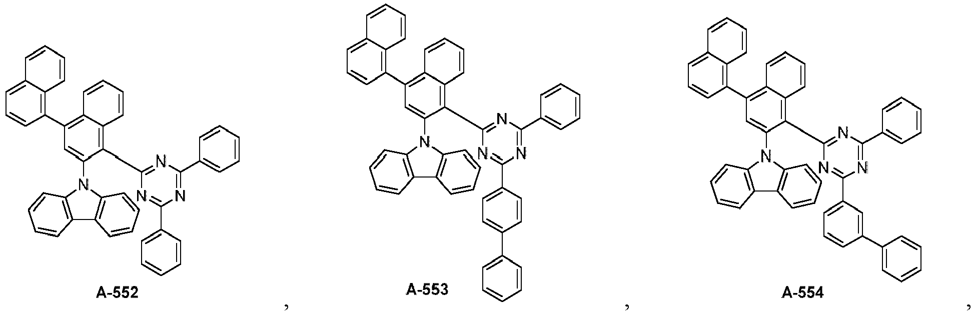 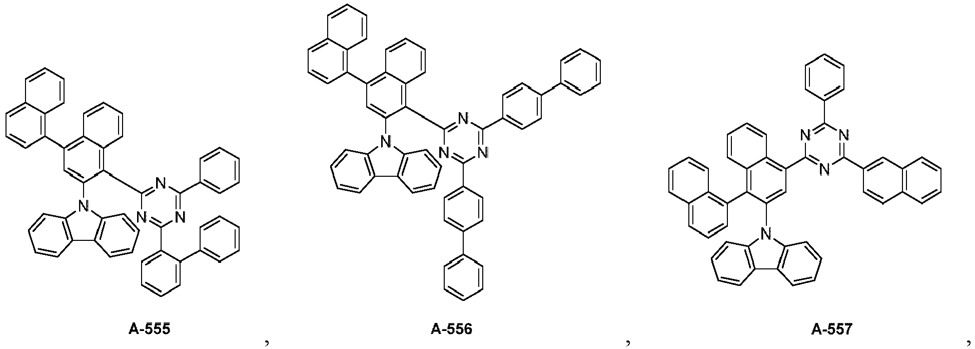 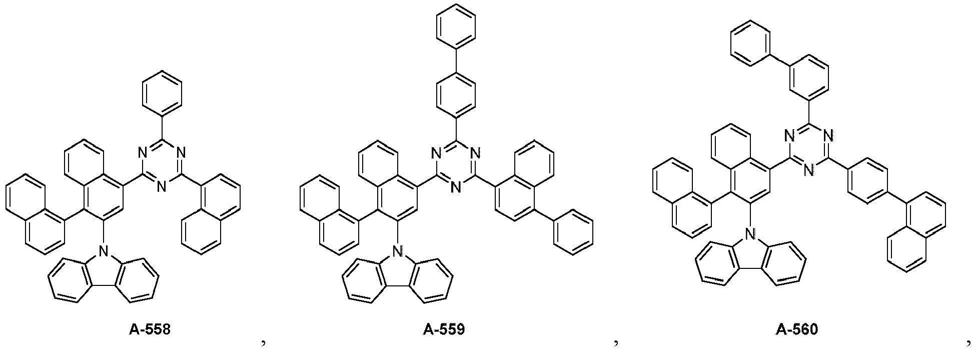 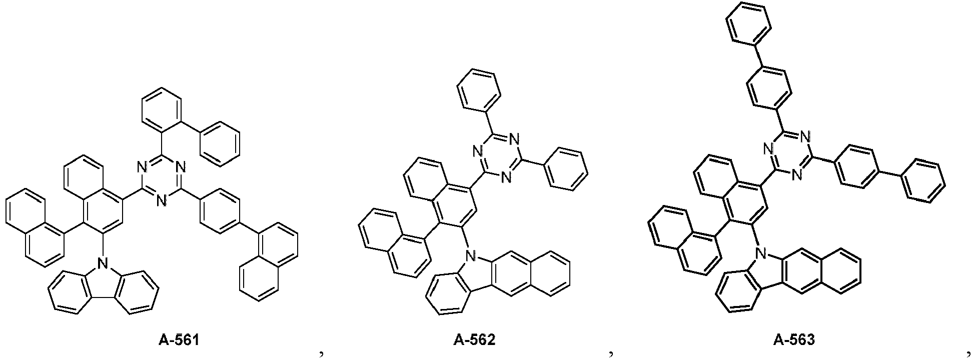 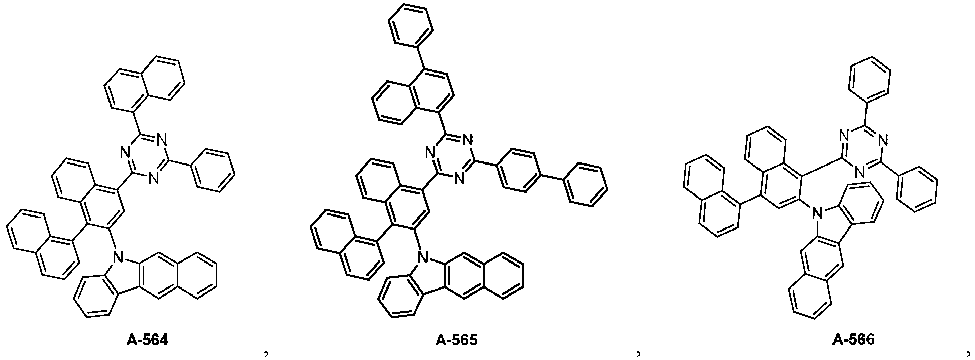 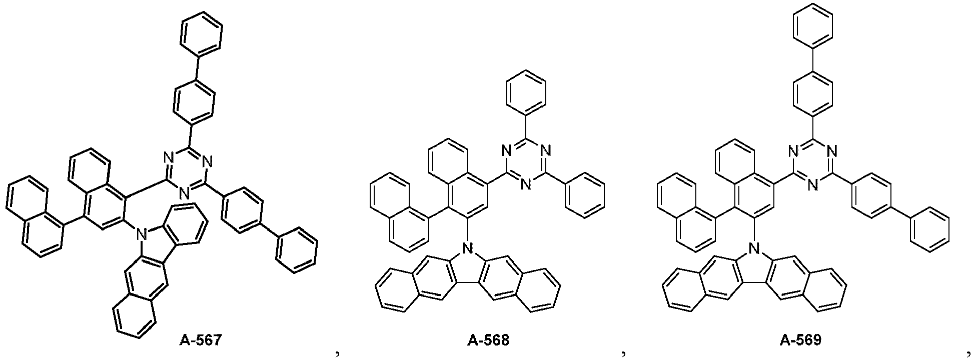 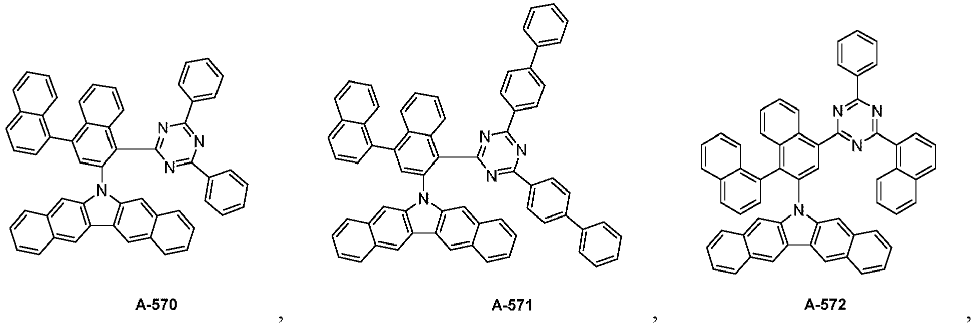 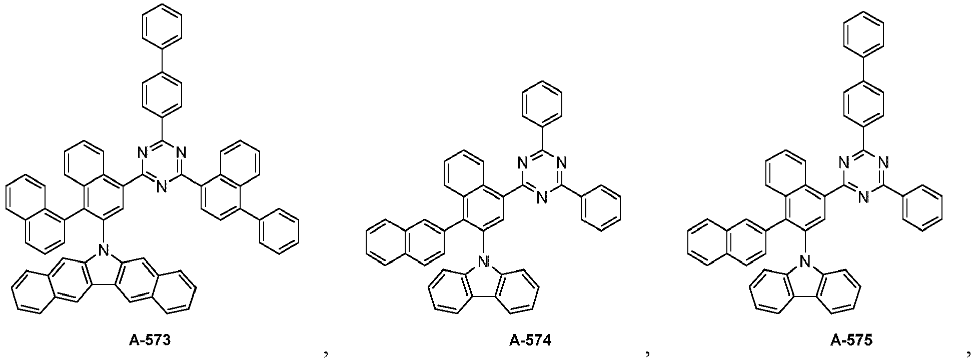 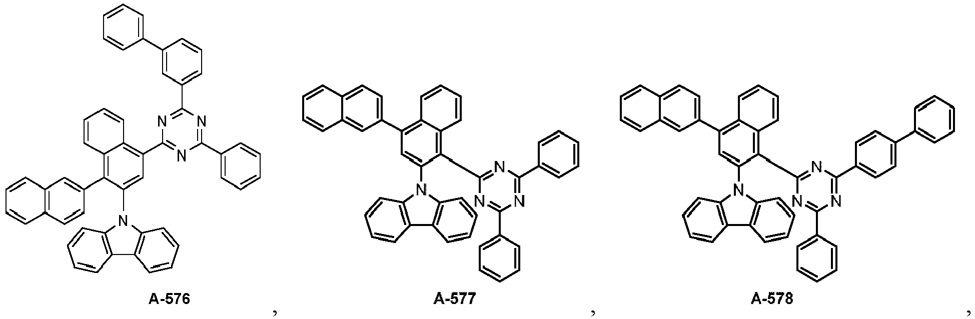 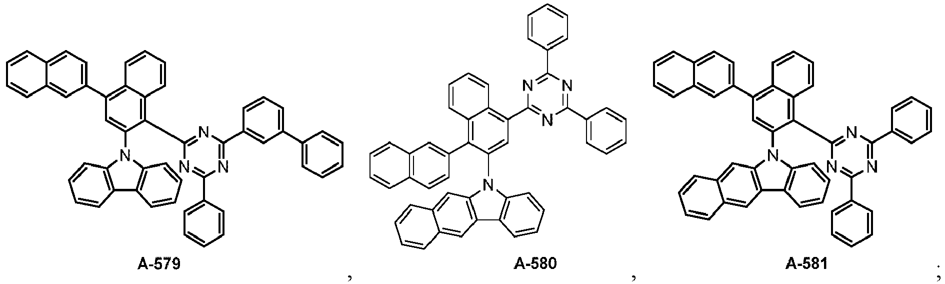 wherein optionally, hydrogens in Compound A-1 to Compound A-581 can be partially or fully substituted with deuterium.

8. An organic electroluminescent device, comprising:
an anode,
a cathode, and
an organic layer disposed between the anode and the cathode, wherein the organic layer comprises the compound according to any one of claims 1 to 7.

9. The device according to claim 8, wherein the organic layer is a light-emitting layer, and the compound is a host compound; or the organic layer is an electron transporting layer, and the compound is an electron transporting compound; or the organic layer is a hole blocking layer, and the compound is a hole blocking compound.

10. The device according to claim 9, wherein the light-emitting layer further comprises a first metal complex having a general formula of M(Lₐ)ₘ(L_{b})ₙ(L_{c})_{q}; wherein
the metal M is selected from a metal with a relative atomic mass greater than 40;
Lₐ, L_{b}, and L_{c} are a first ligand, a second ligand, and a third ligand coordinated to the metal M, respectively; Lₐ, L_{b}, and L_{c} may be the same or different;
the ligands Lₐ, L_{b}, and L_{c} can be optionally joined to form a multidentate ligand;
m is 1, 2, or 3, n is 0, 1, or 2, q is 0, 1, or 2, and m+n+q is equal to an oxidation state of the metal M; when m is greater than or equal to 2, multiple Lₐ may be the same or different; when n is 2, two L_{b} may be the same or different; when q is 2, two L_{c} may be the same or different;
the ligand Lₐ has a structure represented by Formula 11: 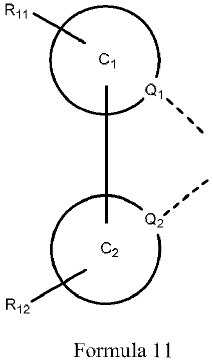
wherein the ring C₁ and the ring C₂ are, at each occurrence identically or differently, selected from an aromatic ring having 5 to 30 ring atoms, a heteroaromatic ring having 5 to 30 ring atoms, or a combination thereof;
Q₁ and Q₂ are, at each occurrence identically or differently, selected from C or N;
R₁₁ and R₁₂ represent, at each occurrence identically or differently, mono-substitution, multiple substitutions, or non-substitution;
R₁₁ and R₁₂ are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
adjacent substituents R₁₁, R₁₂ can be optionally joined to form a ring;
the ligands L_{b} and L_{c} are, at each occurrence identically or differently, selected from a monoanionic bidentate ligand;
preferably, the ligands L_{b} and L_{c} are, at each occurrence identically or differently, selected from the group consisting of the following structures: 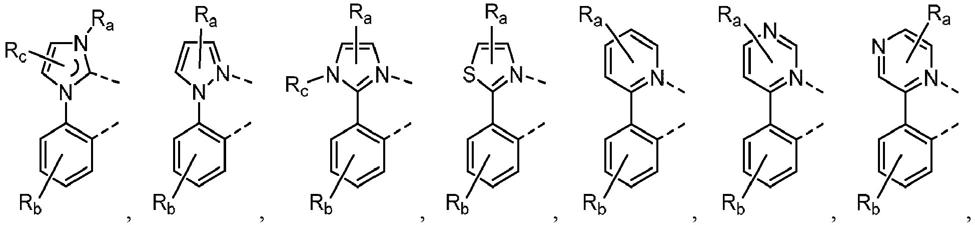 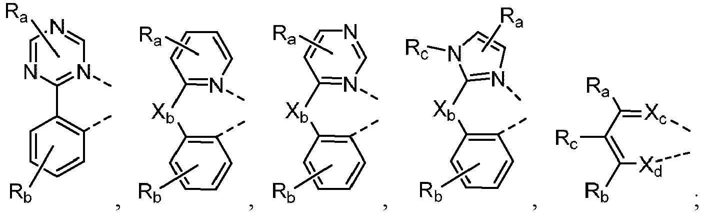 wherein
Rₐ and R_{b} represent, at each occurrence identically or differently, mono-substitution, multiple substitutions, or non-substitution;
X_{b} is, at each occurrence identically or differently, selected from the group consisting of: O, S, Se, NR_{N1}, and CR_{C1}R_{C2};
X_{c} and X_{d} are, at each occurrence identically or differently, selected from the group consisting of: O, S, Se, and NR_{N2};
Rₐ, R_{b}, R_{c}, R_{N1}, R_{N2}, R_{C1}, and R_{C2} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof; and
adjacent substituents Rₐ, R_{b}, R_{c}, R_{N1}, R_{N2}, R_{C1}, and R_{C2} can be optionally joined to form a ring;
preferably, the first metal complex has a general formula of Ir(Lₐ)ₘ(L_{b})₃₋ₘ and has a structure represented by Formula 11-1: 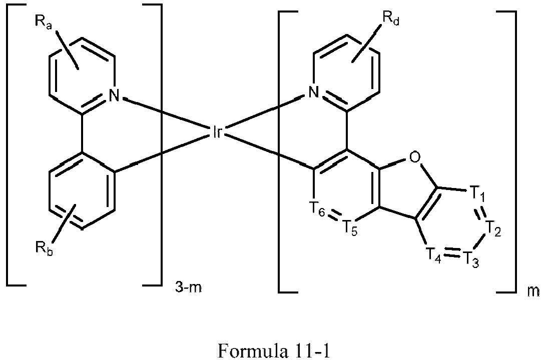 wherein
m is 0, 1, 2, or 3; when m is 2 or 3, multiple Lₐ are the same or different; when m is 0 or 1, multiple L_{b} are the same or different;
T₁ to T₆ are, at each occurrence identically or differently, selected from CR_{T} or N;
Rₐ, R_{b}, and R_{d} represent, at each occurrence identically or differently, mono-substitution, multiple substitutions, or non-substitution;
Rₐ, R_{b}, R_{d}, and R_{T} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a sulfanyl group, a hydroxyl group, a sulfinyl group, a sulfonyl group, a phosphino group, and combinations thereof;
adjacent substituents Rₐ, R_{b} can be optionally joined to form a ring; and
adjacent substituents R_{d}, R_{T} can be optionally joined to form a ring;
more preferably, the first metal complex is selected from the group consisting of the following structures: 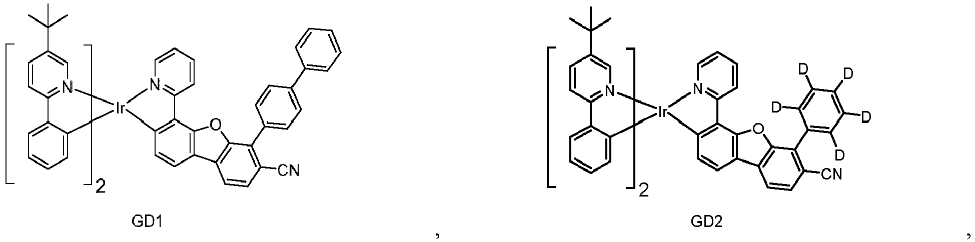 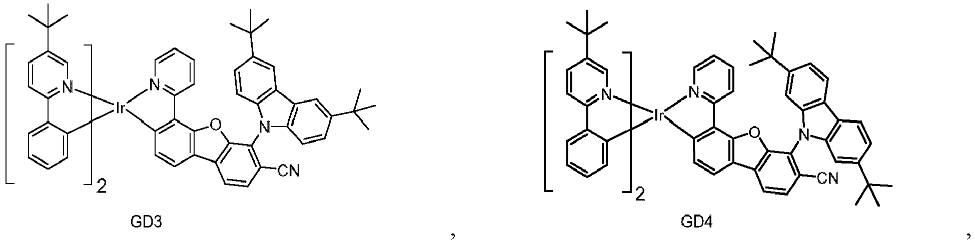 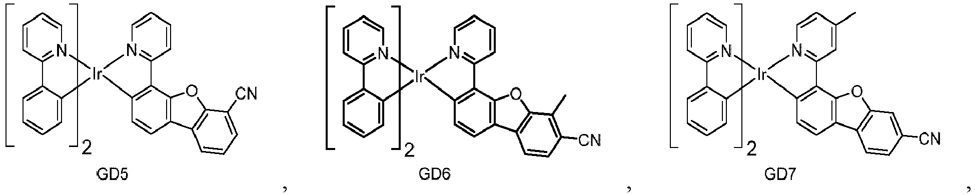 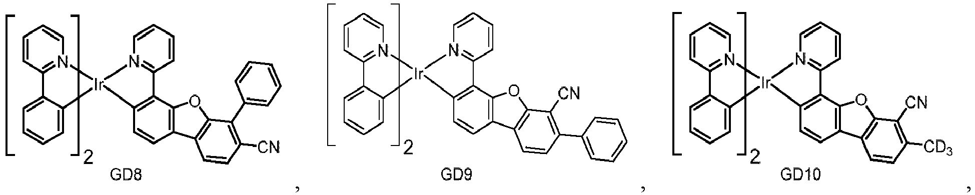 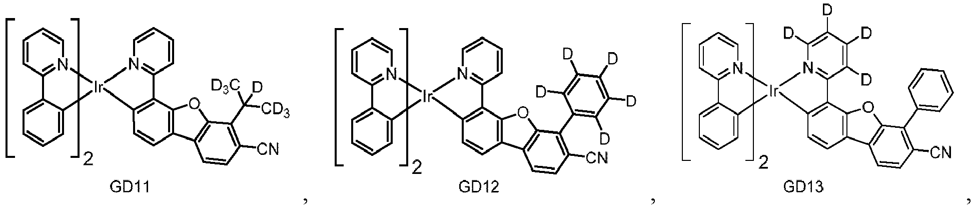 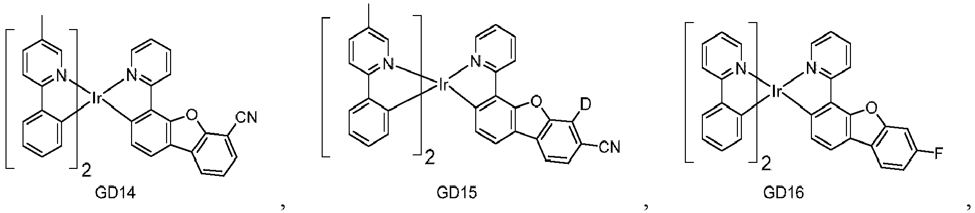 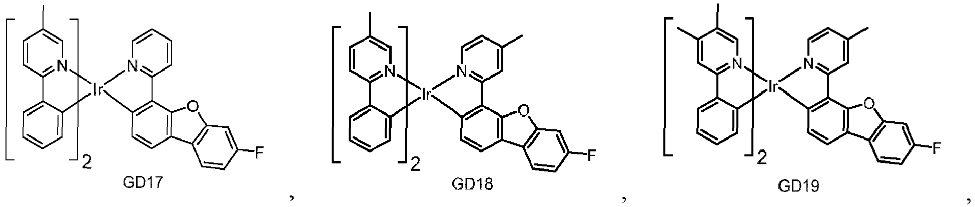 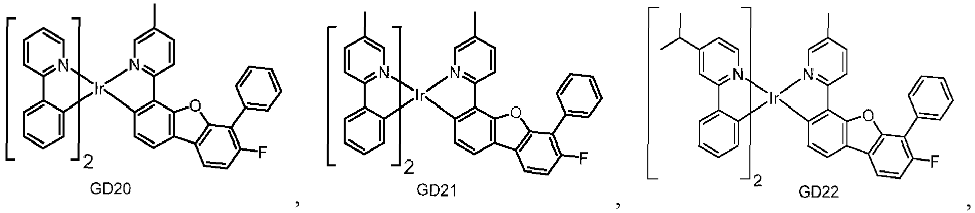 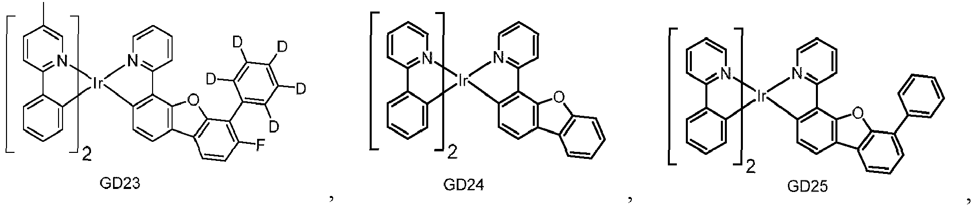 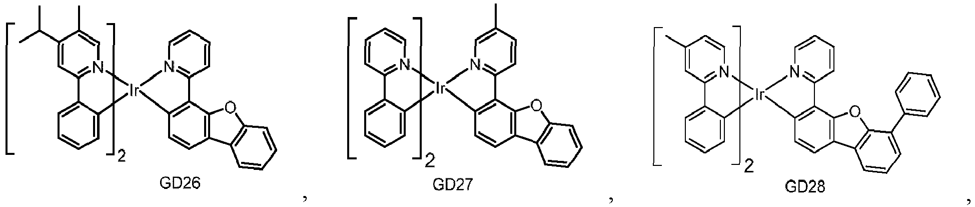 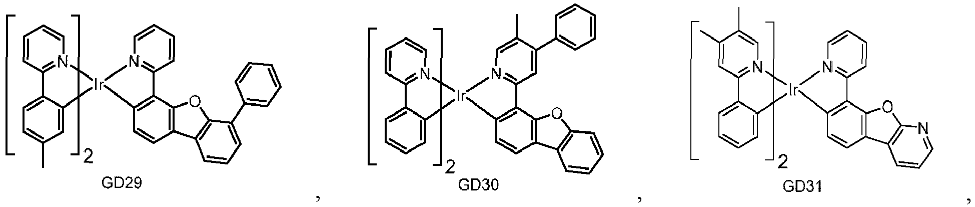 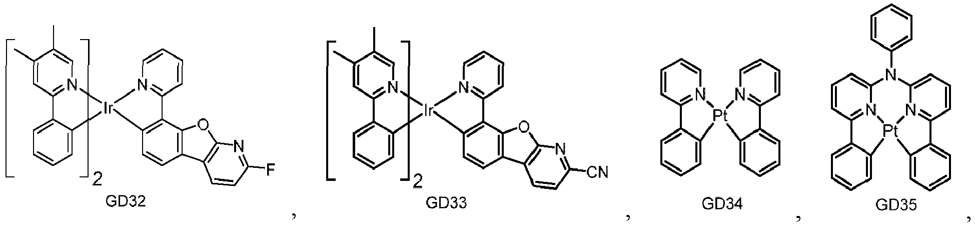 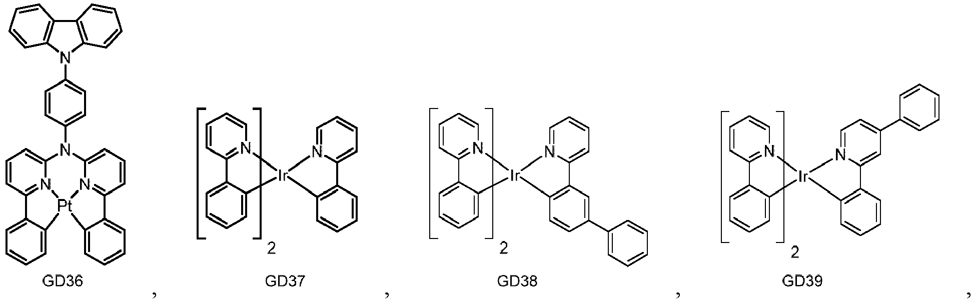 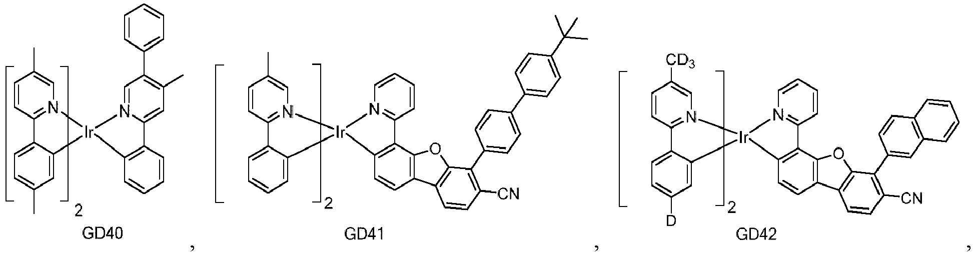 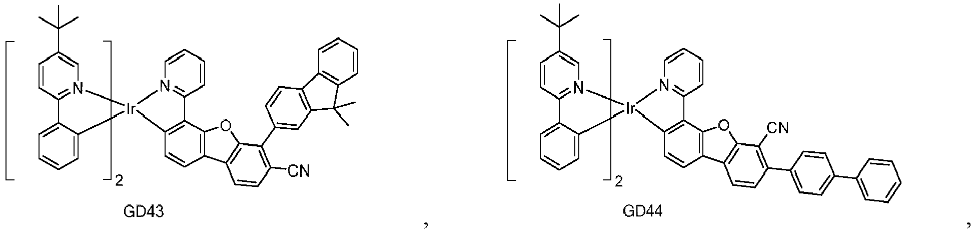 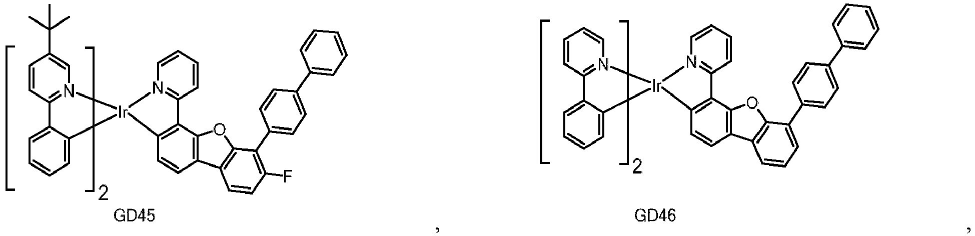 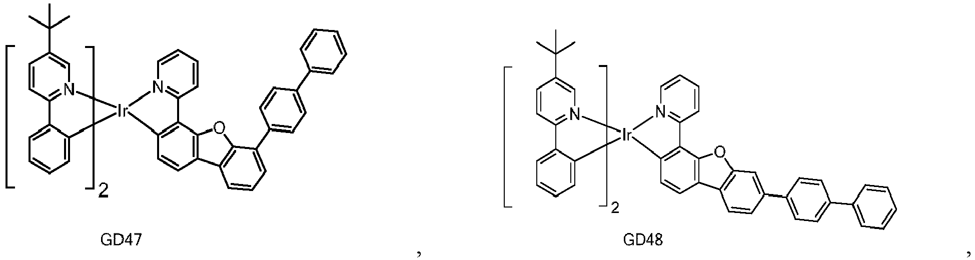 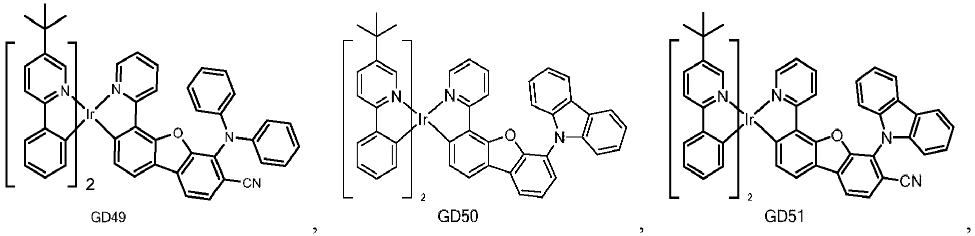 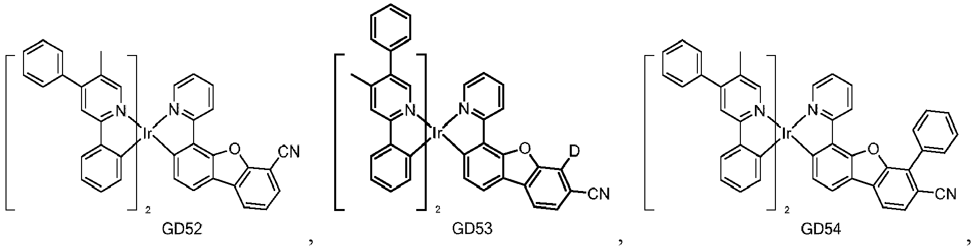 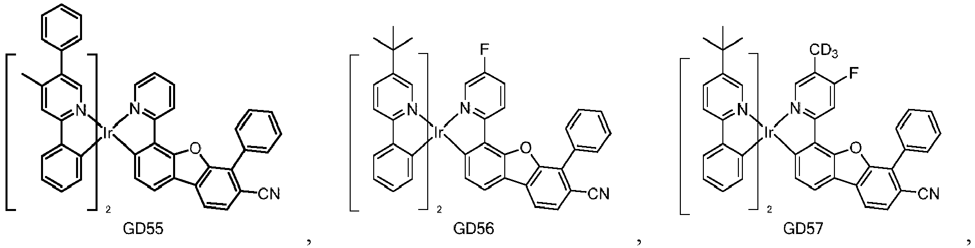 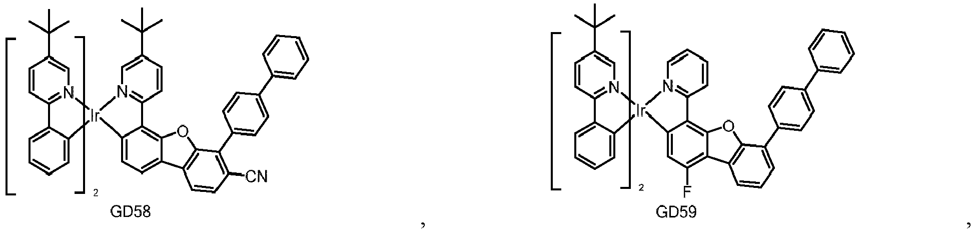 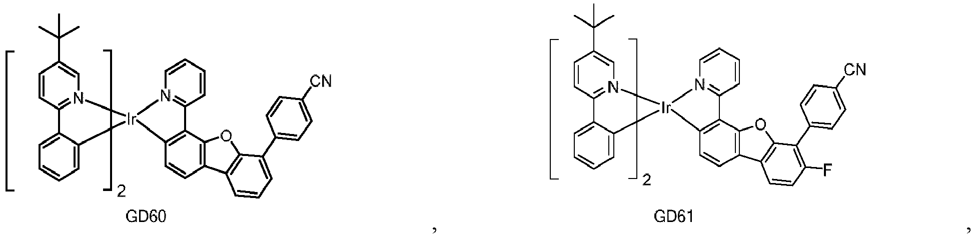 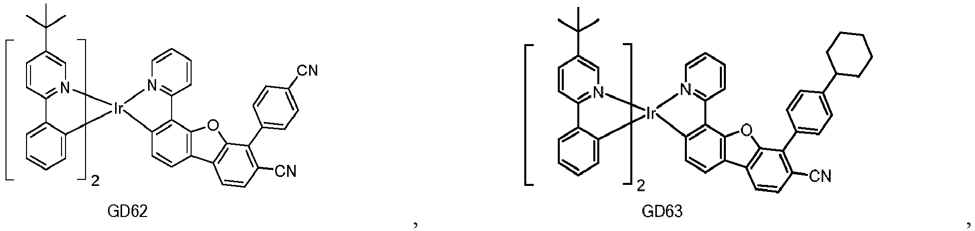 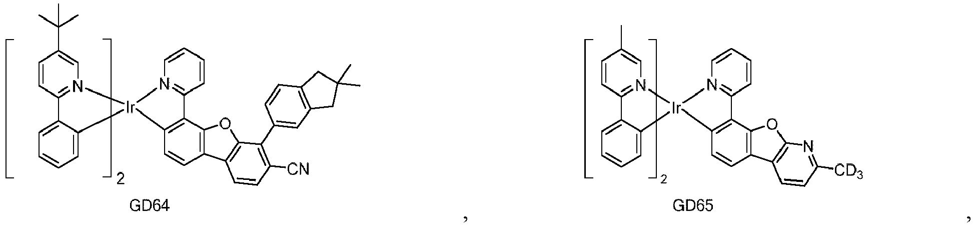 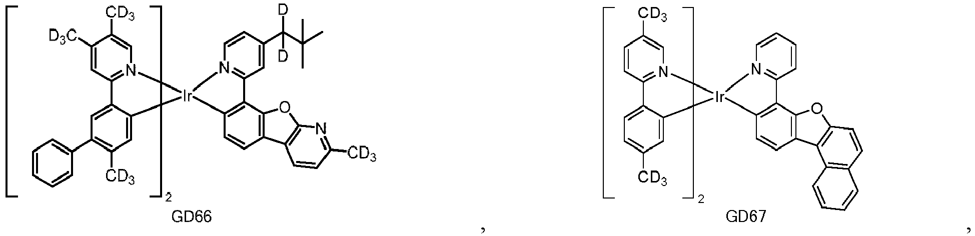 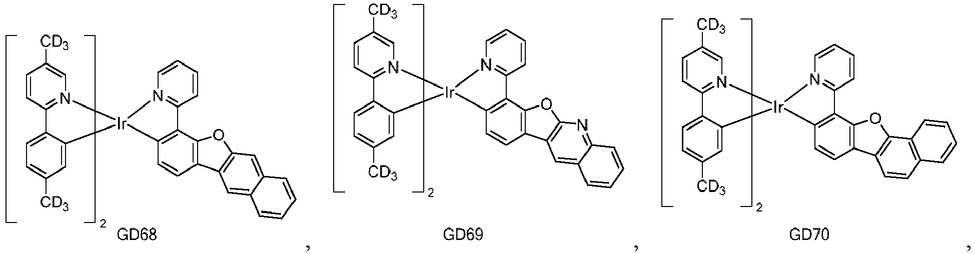 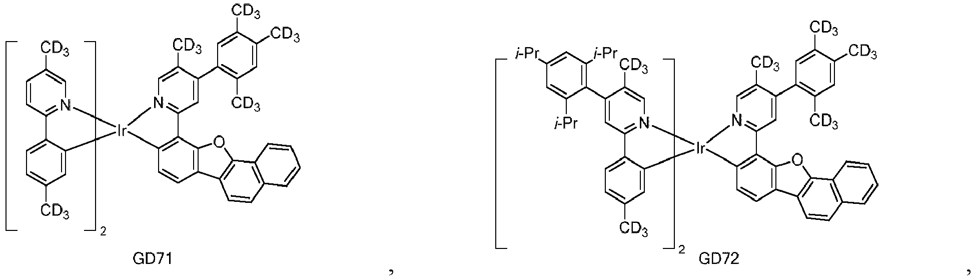 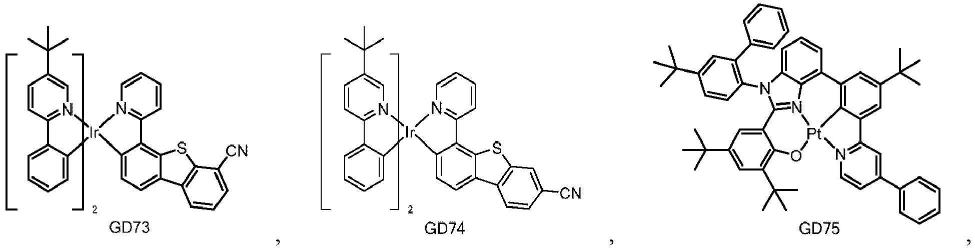 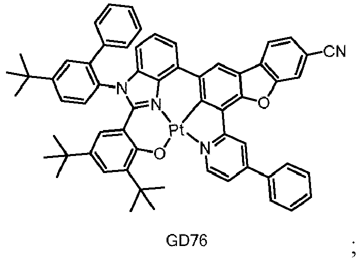
wherein optionally, hydrogens in Compound GD1 to Compound GD76 can be partially or fully substituted with deuterium.

11. The device according to claim 9, wherein the light-emitting layer further comprises a second host compound, wherein the second host compound comprises at least one chemical group selected from the group consisting of: benzene, pyridine, pyrimidine, triazine, carbazole, azacarbazole, indolocarbazole, dibenzothiophene, aza-dibenzothiophene, dibenzofuran, azadibenzofuran, dibenzoselenophene, triphenylene, azatriphenylene, fluorene, silafluorene, naphthalene, quinoline, isoquinoline, quinazoline, quinoxaline, phenanthrene, azaphenanthrene, and combinations thereof;
preferably, the second host compound comprises at least one chemical group selected from the group consisting of: benzene, carbazole, indolocarbazole, dibenzothiophene, dibenzofuran, fluorene, silafluorene, and combinations thereof.

12. The device according to claim 11, wherein the second host compound has a structure represented by Formula 12 or Formula 13: 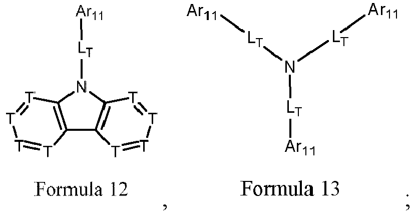 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from C, CR_{w}, or N;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof;
R_{w} is, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof; and
adjacent substituents R_{w} can be optionally joined to form a ring;
preferably, the second host compound has a structure represented by Formula 12-1, Formula 12-2, or Formula 12-3: 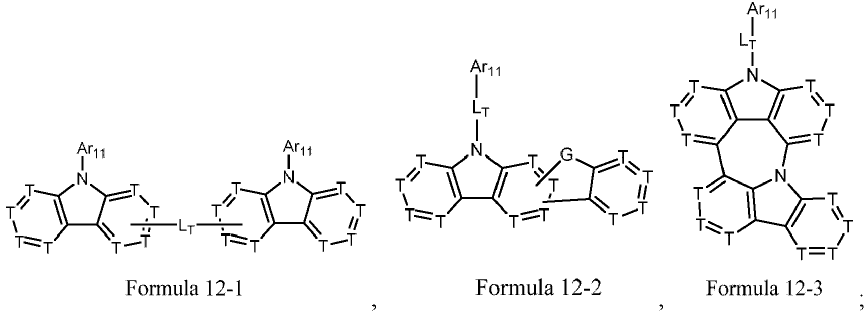 wherein
L_{T} is, at each occurrence identically or differently, selected from a single bond, substituted or unsubstituted alkylene having 1 to 20 carbon atoms, substituted or unsubstituted cycloalkylene having 3 to 20 carbon atoms, substituted or unsubstituted arylene having 6 to 20 carbon atoms, substituted or unsubstituted heteroarylene having 3 to 20 carbon atoms, or a combination thereof;
T is, at each occurrence identically or differently, selected from C, CR_{w}, or N;
G is, at each occurrence identically or differently, selected from C(R_{g})₂, NR_{g}, O, or S;
R_{w} and R_{g} are, at each occurrence identically or differently, selected from the group consisting of: hydrogen, deuterium, halogen, substituted or unsubstituted alkyl having 1 to 20 carbon atoms, substituted or unsubstituted cycloalkyl having 3 to 20 ring carbon atoms, substituted or unsubstituted heteroalkyl having 1 to 20 carbon atoms, a substituted or unsubstituted heterocyclic group having 3 to 20 ring atoms, substituted or unsubstituted arylalkyl having 7 to 30 carbon atoms, substituted or unsubstituted alkoxy having 1 to 20 carbon atoms, substituted or unsubstituted aryloxy having 6 to 30 carbon atoms, substituted or unsubstituted alkenyl having 2 to 20 carbon atoms, substituted or unsubstituted alkynyl having 2 to 20 carbon atoms, substituted or unsubstituted aryl having 6 to 30 carbon atoms, substituted or unsubstituted heteroaryl having 3 to 30 carbon atoms, substituted or unsubstituted alkylsilyl having 3 to 20 carbon atoms, substituted or unsubstituted arylsilyl having 6 to 20 carbon atoms, substituted or unsubstituted alkylgermanyl having 3 to 20 carbon atoms, substituted or unsubstituted arylgermanyl having 6 to 20 carbon atoms, substituted or unsubstituted amino having 0 to 20 carbon atoms, an acyl group, a carbonyl group, a carboxylic acid group, an ester group, a cyano group, an isocyano group, a hydroxyl group, a sulfanyl group, a sulfinyl group, a sulfonyl group, a phosphino group, and combinations thereof;
Ar₁₁ is, at each occurrence identically or differently, selected from substituted or unsubstituted aryl having 6 to 30 carbon atoms, substituted or unsubstituted heteroaryl having 3 to 30 carbon atoms, or a combination thereof; and
adjacent substituents R_{w}, R_{g} can be optionally joined to form a ring;
more preferably, the second host compound is selected from the group consisting of the following structures:

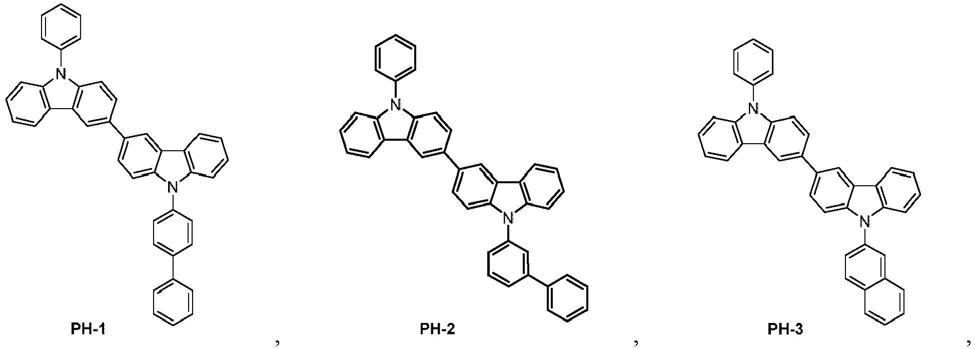

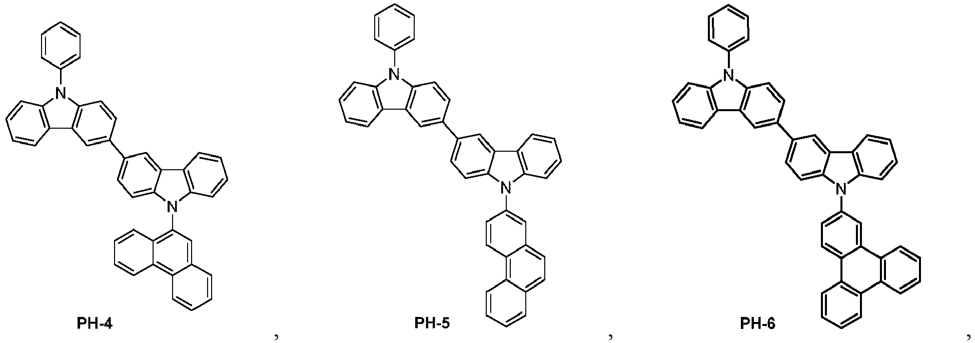

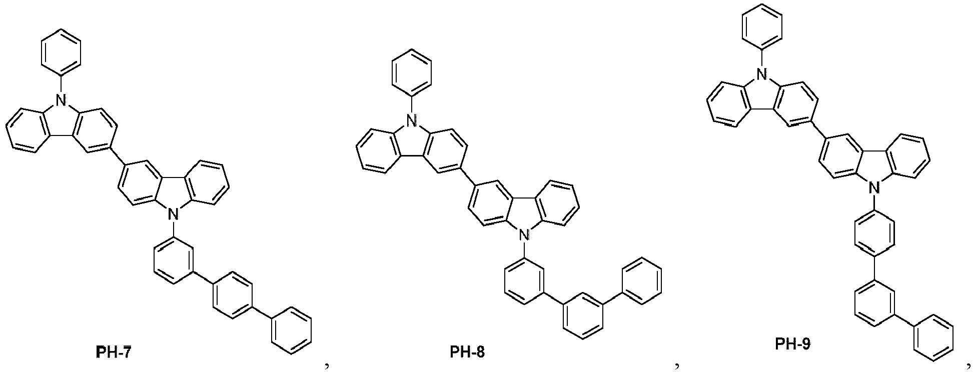

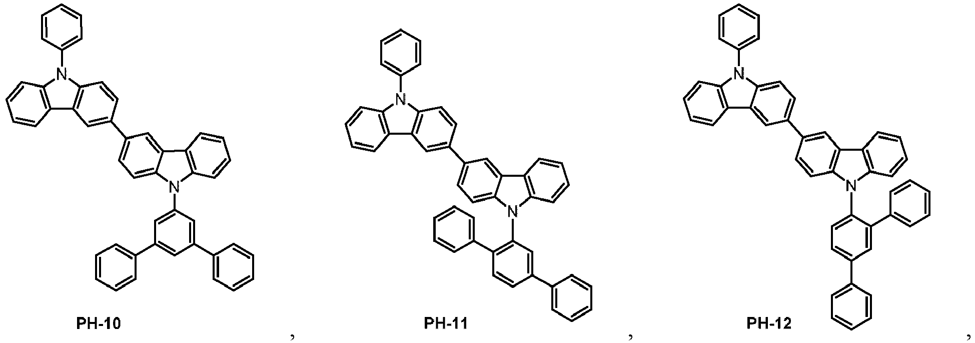

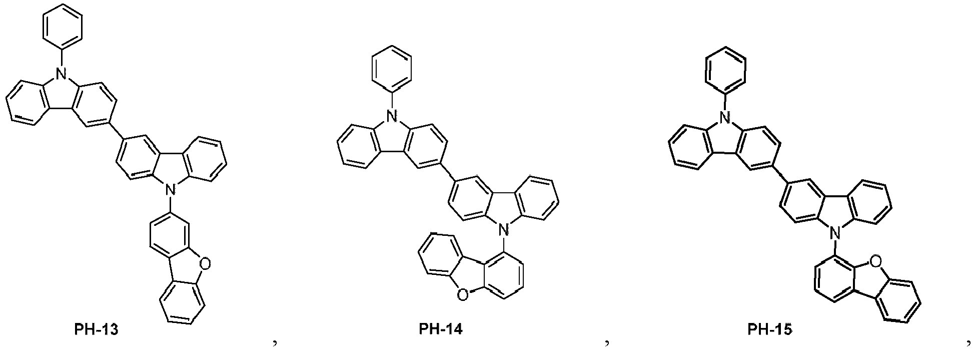

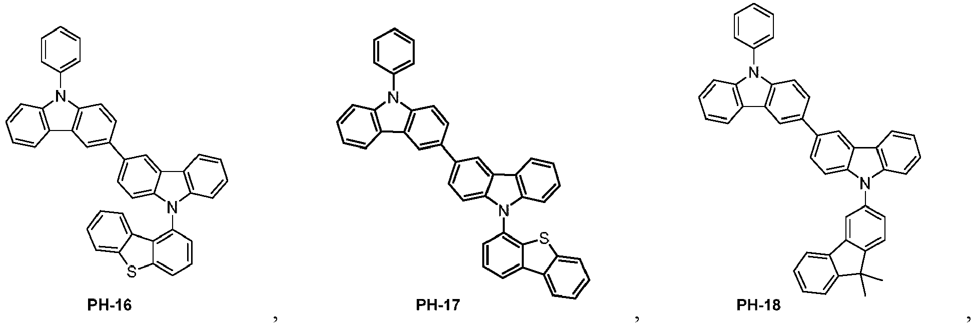

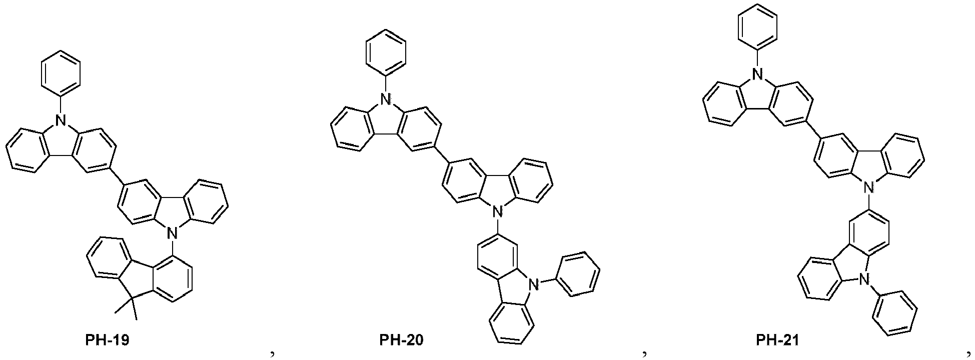

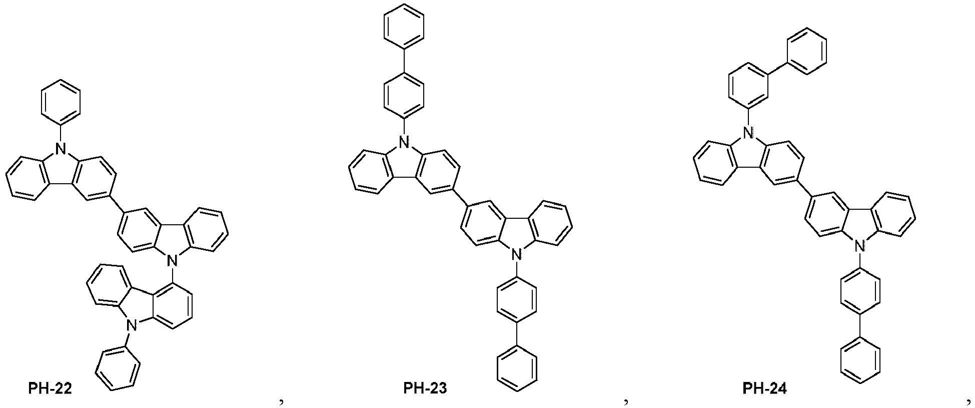

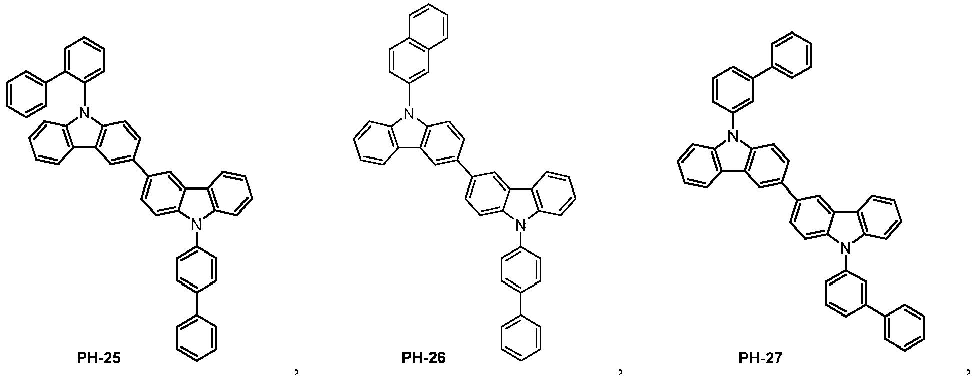

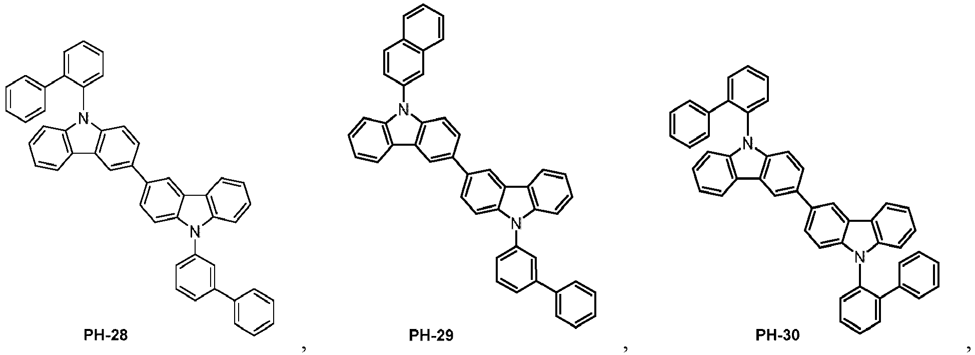

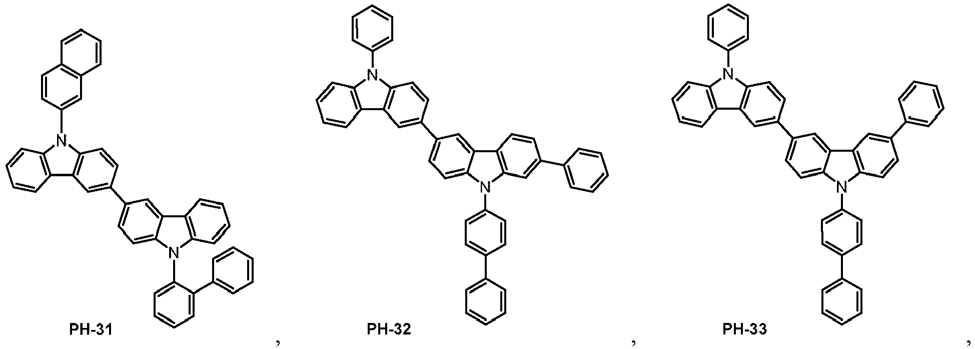

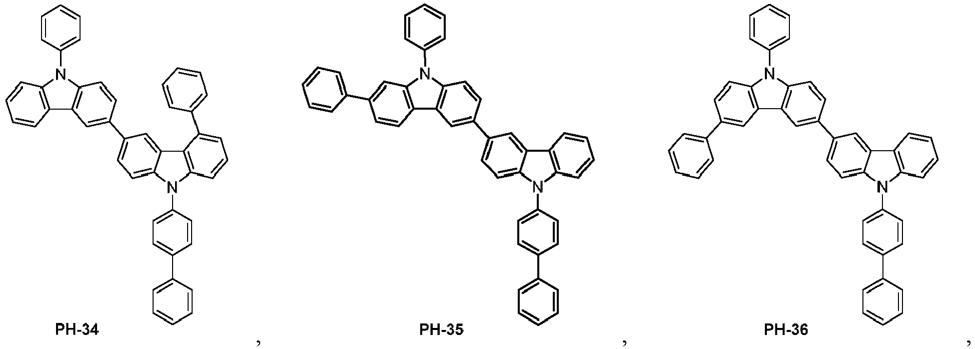

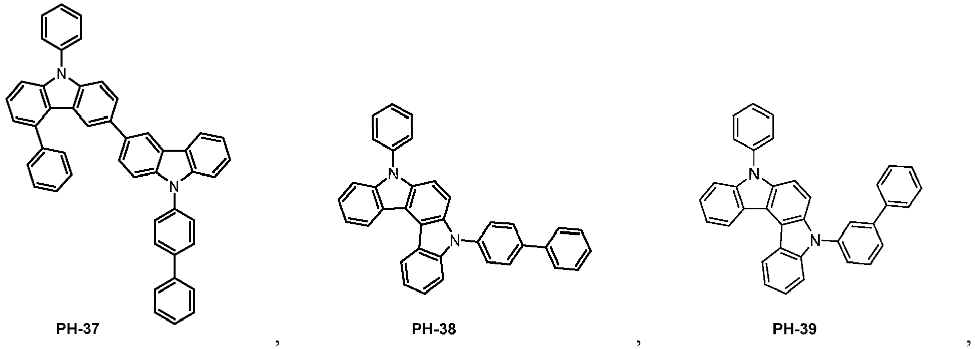

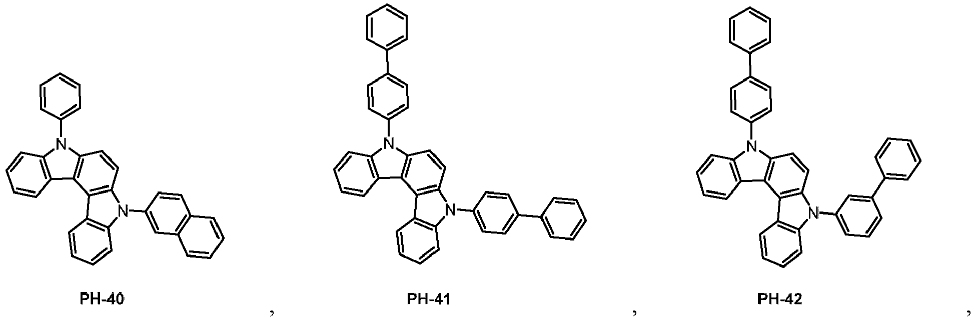

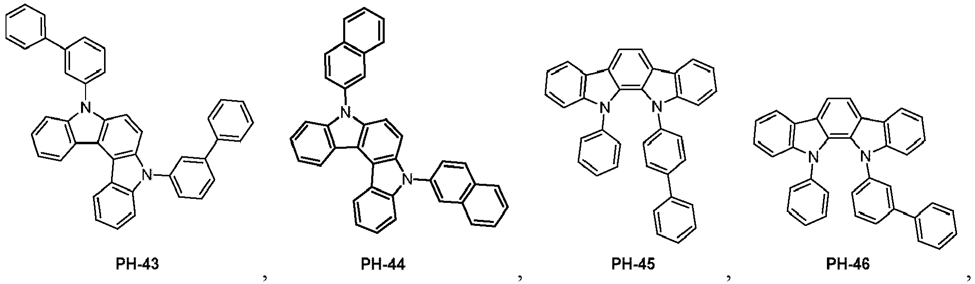

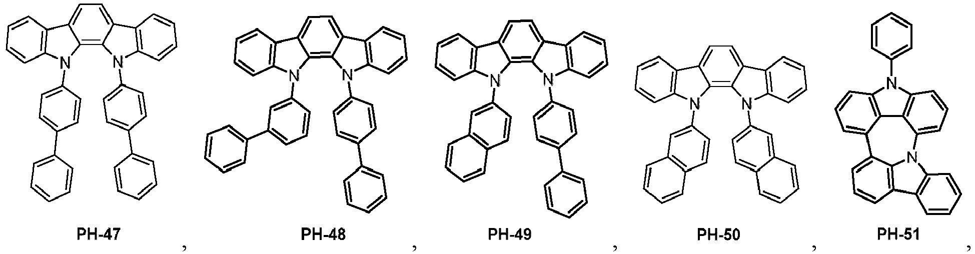

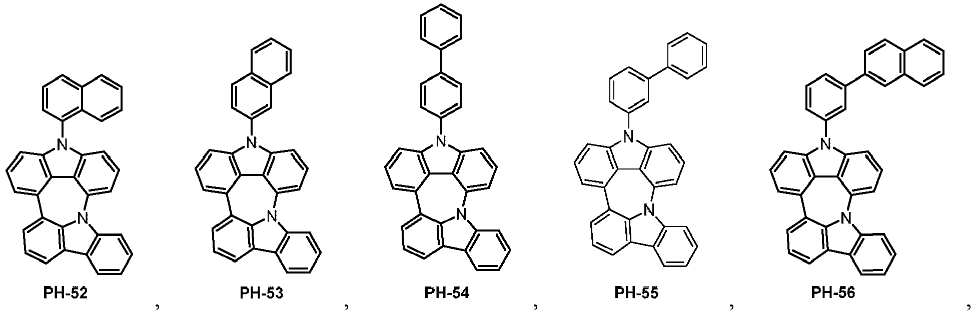

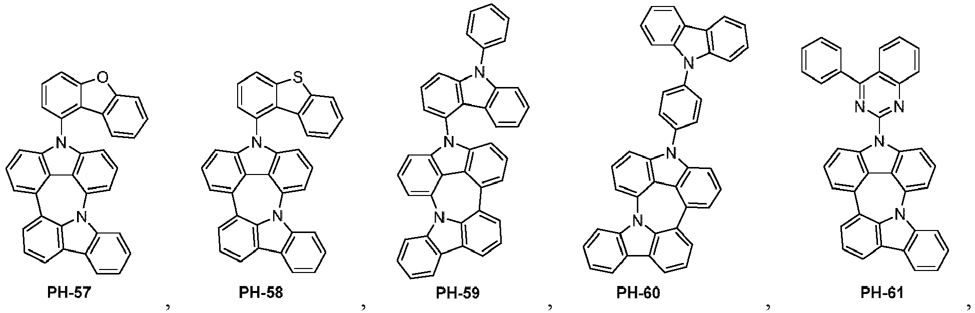

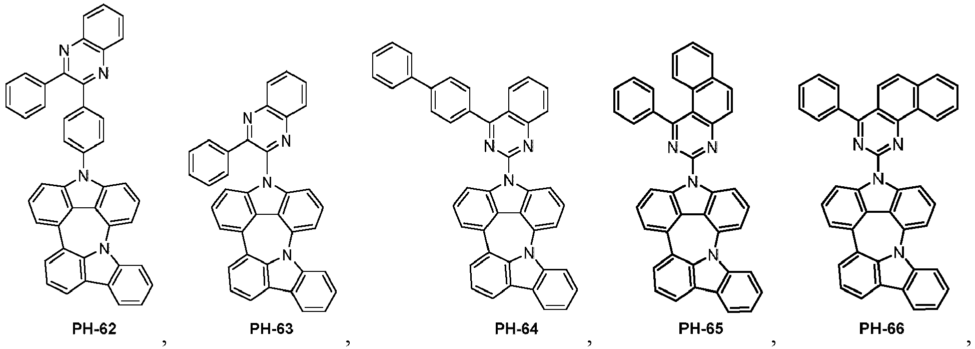

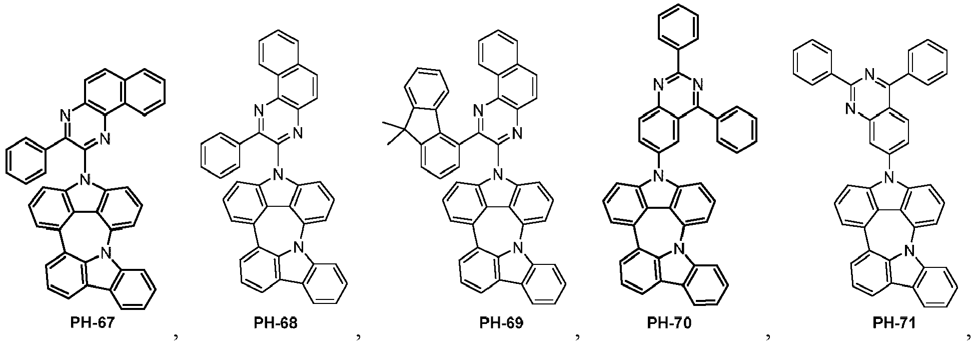

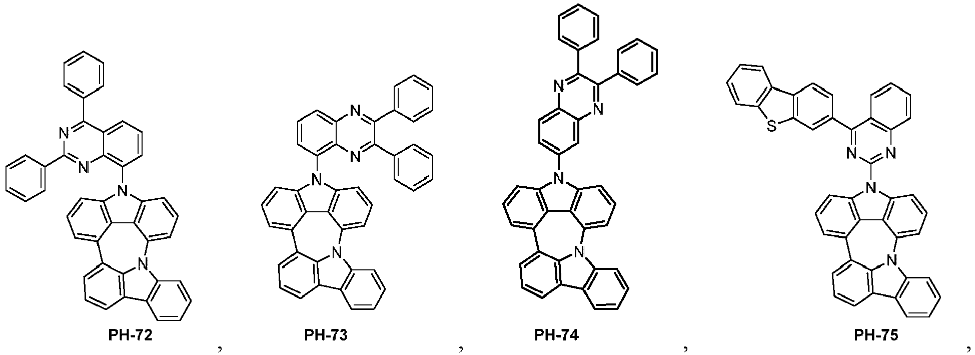

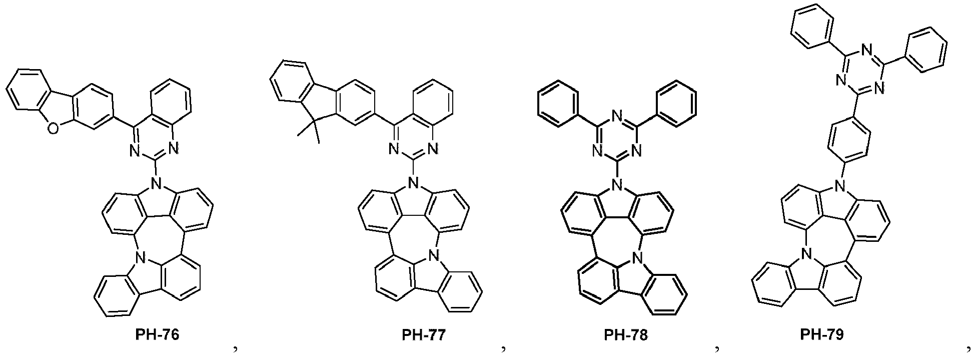

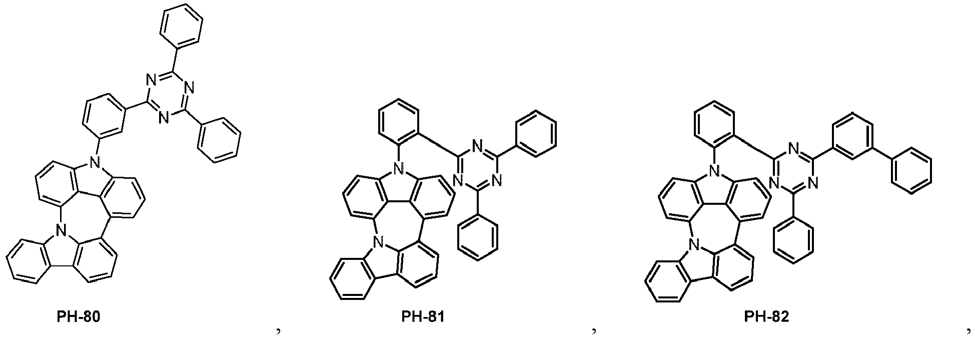

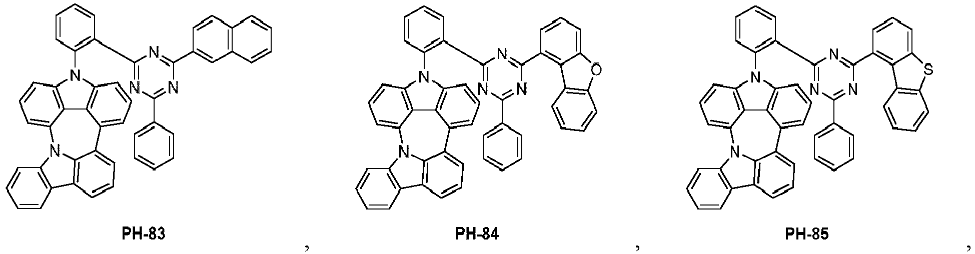

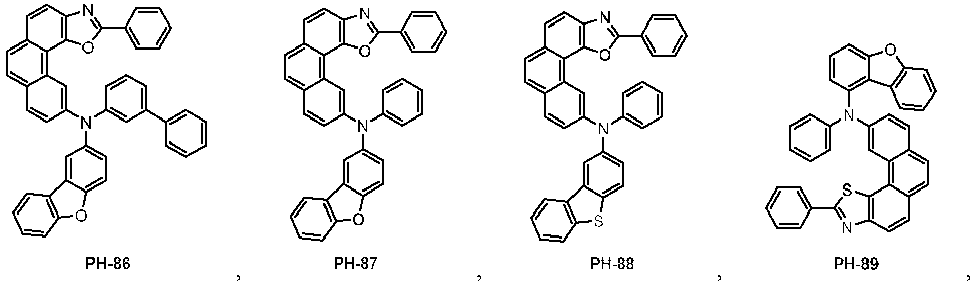

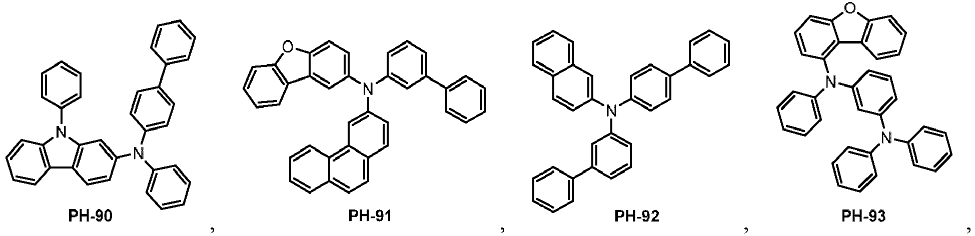

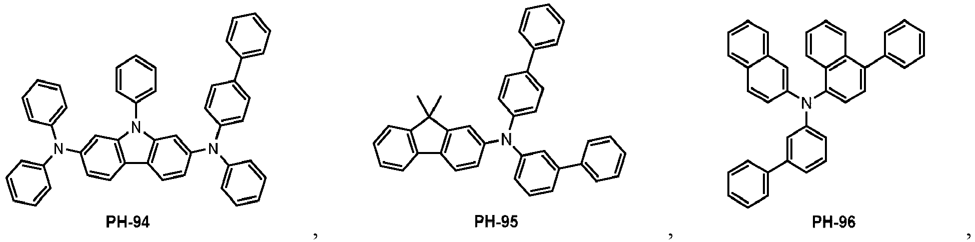

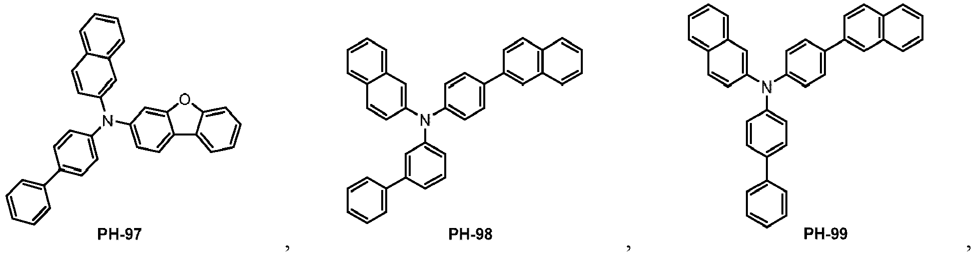

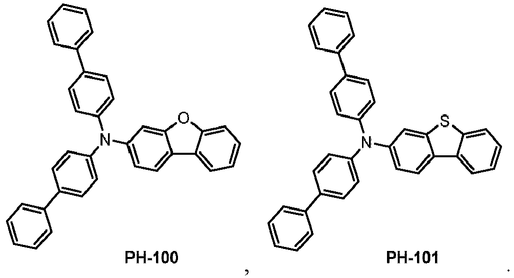

13. The device according to claim 8, wherein the organic electroluminescent device emits green light, yellow light, red light, or white light.

14. The device according to claim 11, wherein the first metal complex is doped with the compound and the second host compound, and the first metal complex accounts for 1% to 30% of the total weight of the light-emitting layer;
preferably, the first metal complex accounts for 3% to 13% of the total weight of the light-emitting layer.

15. A compound composition, comprising the compound according to any one of claims 1 to 7.
